# EUROPÄISCHE PATENTSCHRIFT

(11) **EP 0 534 216 B1**
(45) Veröffentlichungstag und Bekanntmachung des Hinweises auf die Patenterteilung: **19.08.1998**
(21) Anmeldenummer: 92115247.6
(22) Anmeldetag: 05.09.1992
(51) Int. Cl.: C07D 309/22, C07D 413/06, C07D 405/12, A01N 43/16

(54) **Dihydropyranderivate und diese enthaltende Pflanzenschutzmittel**
Dihydropyran derivatives and plant protecting agents containing them
Dérivés de dihydropyranne et produits protectifs pour plantes les contenant

(30) Priorität: 20.09.1991 DE 4131311
(43) Veröffentlichungstag der Anmeldung: 31.03.1993
(73) Patentinhaber: BASF Aktiengesellschaft, 67063 Ludwigshafen (DE)
(72) Erfinder: Mueller, Bernd, Dr., W-6710 Frankenthal (DE); Brand, Siegbert, Dr., W-6701 Birkenheide (DE); Sauter, Hubert, Dr., W-6800 Mannheim 1 (DE); Roehl, Franz, Dr., W-6700 Ludwigshafen (DE); Ammermann, Eberhard, Dr., W-6148 Heppenheim (DE); Lorenz, Gisela, Dr., W-6730 Neustadt (DE)

(56) Entgegenhaltungen:
- EP-A- 0 421 102
- EP-A- 0 438 726
- DE-B- 1 668 899
- JOURNAL OF ORGANIC CHEMISTRY, Bd. 39, Nr. 23, 15. November 1974, Washington, DC, US, Seiten 3432 - 3433 S.S. HALL ET AL.: 'The chemistry of 2-alkoxy-3,4-dihydro-2H-pyrans. II. Addition of dimethyl acetylenedicarboxylate to 2-alkoxy-6-methyl-3,4-dihydro-2H-pyrans'
- TETRAHEDRON LETTERS, Bd. 32, Nr. 24, 10. Juni 1991, Oxford, GB, H.-Y. KANG ET AL. 'Intramolecular Ä3+2Ü nitrone-alkyne cycloaddition'
- TETRAHEDRON LETTERS, Bd. 31, Nr. 42, 8. Oktober 1990, Oxford, GB, Seiten 6077 - 6080 D. MACLEOD ET AL.: 'The Pd(0)-catalysed coupling reactions of 3-(tri-n-butylstannyl)-3,4-dihydrofuran and -5,6-dihydropyran'
- SYNTHESIS, Nr. 8, August 1979, Stuttgart, DE, Seiten 610 - 613 A. LEBOUC ET AL.: 'New synthesis of dihydropyranylcarbinols, dihydropyranyl ketones, and 1-dihydropyranylalkyl carboxylates from lithiated 3,4-dihydro-2H-pyrans'
- TETRAHEDRON, Bd. 46, Nr. 5, 1990, Oxford, GB, Seiten 1625 - 1652 H. BOOTH ET AL.: 'Experimental studies of the anomeric effect. Part III. Rotameric preferences about the exocyclic C2-X bond in equatorial and axial 2-methoxy- and 2-methylaminotetrahydropyrans'
- TETRAHEDRON LETTERS, Bd. 29, Nr. 19, 1988, Oxford, GB, Seiten 2353 - 1988 P. KOCIENSKI ET AL.: 'A stereoselective synthesis of trisubstituted alkenes. The nickel-catalysed coupling of Grignard reagents with 6-alkyl-3,4-dihydro-2H-pyrans'
- CHEMICAL ABSTRACTS, vol. 111, no. 3, 17. Juli 1989, Columbus, Ohio, US; abstract no. 23340k, M.G. VORONKOV et al.: 'Regioselective addition of dichlorocarbene to 2-vinyloxy-3,4-dihydropyrans' Seite 599 ;
- CHEMICAL ABSTRACTS, vol. 107, no. 8, 24. August 1987, Columbus, Ohio, US; abstract no. 59586b, J.Y. LEE et al.: 'Synthesis of alternating head-to-head copolymers of vinyl ketones and vinyl ethers, and their properties. Ring-opening polymerisation of 2,3,6-trisu bstituted-3,4-dihydro-2H-pyrans' Seite 7 ;
- TETRAHEDRON LETTERS, Bd. 42, Nr. 15, 1986, Oxford, GB, Seiten 4333 - 4342 S.V. LEY ET AL.: 'Alkylation reactions of anions derived from 2-benzenesulphonyl tetrahydropyran and their application to spiroketal synthesis'
- ANGEWANDTE CHEMIE, SUPPLEMENT, 1982, Weinheim, DE, Seiten 1556 - 1565 W. FRANCKE ET AL.: 'Alkylsubstituierte 3,4-Dihydro-2H-pyrane: Massenspektrometrie, Synthese und Identifizierung als Insektinhaltsstoffe'
- TETRAHEDRON, Bd. 37, Nr. 23, 1981, Oxford, GB, Seiten 3997 - 4006 R.K. BOECKMAN, JR, ET AL.: 'Cyclic vinyl ether carbanions - II. Preparation and applications to the synthesis of carbonyl compounds'

## Beschreibung

Die vorliegende Erfindung betrifft Dihydropyranderivate und ihre Verwendung als Pflanzenschutzmittel, insbesondere zur Bekämpfung von Pilzen, Insekten, Nematoden und Spinnmilben.

Es ist bekannt Dihydropyranderivate, z.B. Pyracarbolid (3,4-Dihydro-6-methyl-2H-pyran-5-carboxanilid), als Fungizid zu verwenden (The Pesticide Manual, achte Auflage, Seite 902, British Crop Protection Council). Ihre biologische Wirkung ist jedoch unbefriedigend.

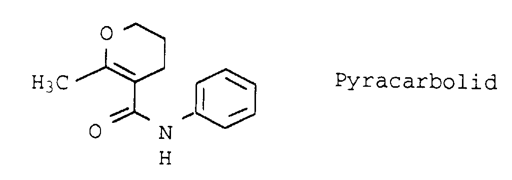

Desweiteren sind aus EP-A 421 102 Acrylsäureester bekannt, die in α-Stellung zur Carbonsäurefunktion einen Cycloalkenring oder einen partiell ungesättigten Heterocyclyl-Rest tragen. In letzterem Fall sitzt das Heteroatom im Bezug auf die Doppelbindung im Heterocyclus in der Allylposition.

Es wurde nun überraschend gefunden, daß Dihydropyranderivate der Formel I 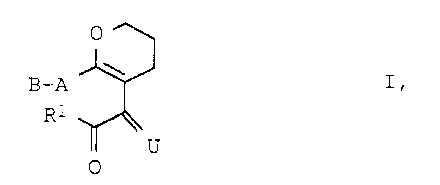 in der
- U =: CH-OR², =CH-SR², =CH-R², =CH-Halogen oder =N-OR² und
- A: eine Einfachbindung -CH₂-, -(CH₂-CH₂)ₙ-, - (CR⁹=CR⁸)ₘ-CH=CH, -C≡C-,-OCH₂, -SCH₂-, -NR⁶CH₂-, -C(=O)-OCH₂- oder R⁷C=NO-CH₂- bedeutet,
- n: 1, 2 oder 3 und
- m: 0 oder 1 bedeutet,
- B: Wasserstoff,
Cycloalkyl mit 3 bis 10 C-Atomen, Aryl mit 6, 10 oder 14 Kohlenstoffatomen, Heteroaryl mit 5 bis 14 Ringatomen, davon 1 bis 4 Heteroatome aus der Gruppe N, O, S, Heterocyclyl mit 5 bis 15 Ringatomen, davon 1 bis 4 Heteroatome aus der Gruppe N, O, S, wobei die Ringe gesättigt oder partiell ungesättigt sind oder Cycloalkenyl mit 5 bis 14 C-Atomen bedeutet, wobei diese Gruppen jeweils einen bis vier der unter R¹⁰ genannten Reste tragen können, soweit diese von den vorstehend genannten Resten verschieden sind,
- R¹: OR³ oder NR⁴R⁵ bedeutet,
- R²,: R³ und R⁶ Methyl oder Ethyl bedeuten,
- R⁴,: R⁵, R⁷, R⁸ und R⁹
Wasserstoff, Methyl oder Ethyl bedeuten,
- R¹⁰: Halogen, Cyano, Nitro oder CHO
R¹¹, OR¹¹, =NOR¹¹, CO₂R¹¹, SR¹¹, COR¹¹, SOR¹¹ oder SO₂R¹¹ bedeutet,
- R¹¹: Alkyl, Alkenyl, Alkinyl, Cycloalkyl mit 3 bis 10 C-Atomen, Aryl mit 5, 10 oder 14 Kohlenstoffatomen, Hetaryl mit 5 bis 14 Ringatomen, davon 1 bis 4 Heteroatome aus der Gruppe N, O, S, Heterocyclyl mit 5 bis 14 Ringatomen, davon 1 bis 4 Heteroatome aus der Gruppe N, O, S, wobei die Ringe gesättigt oder partiell ungesättigt sind oder Cycloalkenyl mit 5 bis 14 C-Atomen bedeutet,
wobei diese Gruppen einen bis vier der unter R¹² genannten Reste tragen können, soweit diese von den Resten R¹¹ verschieden sind,
- R¹²: Halogen, Cyano, Nitro, Alkyl, Haloalkyl, Alkoxy, Haloalkoxy, Alkoxyimino, Alkoxycarbonyl, Alkylcarbonyl, Alkenyloxyimino und Aryl bedeutet.

sowie deren pflanzenverträgliche Säureadditionsprodukte oder Basenadditionsprodukte neben einer hohen fungitoxischen, insektiziden, nematiziden und akariziden Wirkung auch eine sehr gute Pflanzenverträglichkeit besitzen.

Säuren für Säureadditionsprodukte sind z.B. Mineralsäuren, wie beispielsweise Chlorwasserstoffsäure, Bromwasserstoffsäure, Phosphorsäure, Schwefelsäure, Salpetersäure, oder Carbonsäuren, wie Ameisensäure, Essigsäure, Oxalsäure, Malonsäure, Milchsäure, Äpfelsäure, Bernsteinsäure, Weinsäure, Zitronensäure, Salicylsäure oder Sulfonsäuren, p-Toluolsulfonsäure, Dodecylbenzolsulfonsäure, oder aber auch allgemein Protonen-acide Verbindungen, z.B. Saccharin.

Basen für Basenadditionsprodukte sind z.B. Kalium-, Natrium-, -hydroxid, -carbonat, Ammoniumhydroxid.

Von der Erfindung werden sowohl die Dihydropyranderivate als solche als auch ihre Säureadditionsprodukte und Basenadditionsprodukte umfaßt. Die Säureadditionsprodukte und Basenadditionsprodukte werden hergestellt, indem man ein Dihydropyranderivat in einem organischen Lösungsmittel löst und die Lösung mit der Säure oder der Base vermischt und das Lösungsmittel abdampft.

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide, Nematizide, Akarizide oder Insektizide verwendet werden und werden von der Erfindung umfaßt.

Insbesondere falls die Gruppe U in Form von syn/anti-Isomeren vorkommen kann, werden von der Erfindung auch die synund die anti-isomeren Verbindungen umfaßt.

Die oben genannten Alkyle besitzen 1 - 10 Kohlenstoffatome und sind gegebenenfalls beliebig substituiert, beispielsweise mit 1 - 4 gleichen oder verschiedenen Substituenten R¹⁰.

Die oben genannten Alkenyle besitzen 2 - 10 Kohlenstoffatome und sind gegebenenfalls beliebig substituiert, beispielsweise mit 1 - 4 gleichen oder verschiedenen Substituenten R¹⁰.

Die oben genannten Alkinyle besitzen 2 - 10 Kohlenstoffatome und sind gegebenenfalls beliebig substituiert, beispielsweise mit 1 - 4 gleichen oder verschiedenen Substituenten R¹⁰.

Die oben genannten Cycloalkyle besitzen 3 - 10 Kohlenstoffatome und sind gegebenenfalls beliebig substituiert, beispielsweise mit 1 - 4 gleichen oder verschiedenen Substituenten R¹⁰.

Die oben genannten Aryle besitzen 6, 10 oder 14 Kohlenstoffatome und sind gegebenenfalls beliebig substituiert, beispielsweise mit 1 - 4 gleichen oder verschiedenen Substituenten R¹⁰.

Die oben genannten Hetaryle besitzen 5 - 14 Ringatome, davon 1 - 4 Heteroatome aus der Gruppe N, O, S und sind gegebenenfalls beliebig substituiert, beispielsweise mit 1 - 4 gleichen oder verschiedenen Substituenten R¹⁰.

Die oben genannten Heterocyclyle besitzen 5 - 14 Ringatome, davon 1 - 4 Heteroatome aus der Gruppe N, O, S sind gesättigt oder partiell ungesättigt und sind gegebenfalls beliebig substituiert, beispielsweise mit 1 - 4 gleichen oder verschiedenen Substituenten R¹⁰.

Die oben genannten Cycloalkenyle besitzen 5 - 14 Kohlenstoffatome und sind gegebenenfalls beliebig substituiert, beispielsweise mit 1 - 4 gleichen oder verschiedenen Substituenten R¹⁰.

Die vorstehend genannten Alkylreste bedeuten bevorzugt Methyl, Ethyl, Propyl, n-Propyl, i-Propyl, Butyl, n-Butyl, i-Butyl, t-Butyl, s-Butyl, Pentyl, Pentyl-1, Pentyl-2, Pentyl-3, 2-Methylbutyl-1, 2-Methylbutyl-2, 2-Methylbutyl-3, 3-Methylbutyl-1, 2,2-Dimethylpropyl-1, Hexyl, Hexyl-1, Hexyl-2, Hexyl-3, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Di-methylbutyl, 1-Ethylbutyl, 2-Ethylbutyl, 1,1,2-Trimethyl-propyl, 1,2,2-Trimethylpropyl, 1-Ethyl-1-methylpropyl, 1-Ethyl-2-methylpropyl, Heptyl, Heptyl-1, Heptyl-2, Heptyl-3, Heptyl-4, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, 1-Propylbutyl, 1-Isopropylbutyl, Octyl, Octyl-1, Octyl-2, Octyl-3, Octyl-4, 1-Methylheptyl, 2-Methylheptyl, 3-Methylheptyl, 4-Methylheptyl, 5-Methylheptyl, 6-Methylheptyl, 1-Ethylhexyl, 2-Ethylhexyl, 3-Ethylhexyl, 4-Ethylhexyl, 1-Propylpentyl, 2-Propylpentyl, Nonyl, 1-Nonyl, 2-Nonyl, 3-Nonyl, 4-Nonyl, 5-Nonyl, 1-Methyloctyl, 2-Methyloctyl, 3-Methyloctyl, 4-Methyloctyl, 5-Methyloctyl, 6-Methyloctyl, 7-Methyloctyl, 4-Methyl-2-propylpentyl, Decyl, 1-Decyl, 2-Decyl, 3-Decyl, 4-Decyl, 5-Decyl, 1-Ethyloctyl, 2-Ethyloctyl, 3-Ethyloctyl, 4-Ethyloctyl, 5-Ethyloctyl, 6-Ethyoctyl oder 2-Propylheptyl.

Die vorstehend genannten Alkenylreste bedeuten bevorzugt Ethenyl, 1-Propenyl, 2-Propenyl, Butenyl, 1-Butenyl, 2-Butenyl, 3-Butenyl, 1-Methyl-2-propenyl, 2-Methyl-2-propenyl, Pentenyl, 1-Pentenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, Hexenyl, 1-Hexenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, Heptenyl, Octenyl, Nonenyl oder Decenyl.

Die vorstehend genannten Alkinylreste bedeuten bevorzugt Ethinyl, 1-Propinyl, 2-Propinyl, Butinyl, 1- Butinyl, 2-Butinyl, 3-Butinyl, 1-Methyl-2-propinyl, Pentinyl, 1-Pentinyl, 2-Pentinyl, 3-Pentinyl, 4-Pentinyl, 1-Methyl-2- butinyl, 1-Methyl-3-butinyl, 2-Methyl-3-butinyl, 1,1-Dimethyl-2-methyl-2-pentinyl, Hexinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1- Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, Heptinyl, Octinyl, Noninyl oder Decinyl.

Die vorstehend genannten Halogene bedeuten Fluor, Chlor, Brom oder Iod.

Die vorstehend genannten Cycloalkylreste bedeuten bevorzugt Cyclopropyl, Cyclobutyl, Cyclopentyl, Cyclohexyl, Cycloheptyl, Cyclooctyl, Cyclononyl, Cyclodecyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonyl.

Die vorstehend genannten Cycloalkenylreste bedeuten bevorzugt Cyclopropenyl, Cyclobutenyl, Cyclopentenyl, Cyclohexenyl, Cycloheptenyl, Cyclooctenyl, Cyclononenyl, Cyclodecenyl, Bornenyl, Norbornenyl, Bicyclo[3,3,0]octenyl, Bicyclo[3,2,1]octenyl, Bicyclo[2,2,2]octenyl oder Bicyclo[3,3,1]nonenyl.

Die vorstehend genannten Haloalkyle bedeuten bevorzugt C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl oder Pentafluorethyl.

Die vorstehend genannten Haloalkoxyreste bedeuten bevorzugt C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Tri-chlorethyloxy oder Pentafluorethyloxy.

Die vorstehend genannten Aryle bedeuten bevorzugt Phenyl, 1-Naphthyl, 2-Naphthyl, 1-Anthracenyl, 2-Anthracenyl oder 9-Anthracenyl.

Die vorstehend genannten Hetaryle bedeuten bevorzugt Furyl, 2-Furyl, 3-Furyl, Thienyl, 2-Thienyl, 3-Thienyl, Pyrryl, 1-Pyrryl, 2-Pyrryl, 3-Pyrryl, Isoxazolyl, 3-Isoxazolyl, 4-Isoxazolyl, 5-Isoxazolyl, Isothiazolyl, 3-Isothiazolyl, 4-Isothiazolyl, 5-Isothiazolyl, Pyrazolyl, 1-Pyrazolyl, 3-Pyrazolyl, 4-Pyrazolyl, 5-Pyrazolyl, Oxazolyl, 2-Oxazolyl, 4-Oxazolyl, 5-Oxazolyl, Thiazolyl, 2-Thiazolyl, 4-Thiazolyl, 5-Thiazolyl, Imidazolyl, 1-Imidazolyl, 2-Imidazolyl, 4-Imidazolyl, 5-Imidazolyl, 1,2,3-Thiadiazolyl, 1,2,4-Thiadiazolyl, 1,2,5-Thiazolyl, 1,3,4-Thiadiazolyl, Tetrazolyl, 1,2,3,4-Thiatriazolyl, 1,2,3,4-Oxatriazolyl, Pyridyl, 2-Pyridyl, 3-Pyridyl, 4-Pyridyl, Pyridazinyl, 3-Pyridazinyl, 4-Pyridazinyl, Pyrimidinyl, 2-Pyrimidinyl, 4-Pyrimidinyl, 5-Pyrimidinyl, Pyrazinyl, 2-Pyrazinyl, 3-Pyra-zinyl, 1,2,4-Triazinyl, 1,3,5-Triazinyl oder 1,2,4,5-Tetrazinyl.

Dabei können benachbarte Substituenten des Heteroaromaten kondensiert sein zu einem aromatischen oder heteroaromatischen Ring, so daß Hetaryl auch kondensierte Ringsysteme umfaßt wie z.B. Benzofuranyl, Isobenzofuranyl, 1-Benzothienyl, 2-Benzothienyl, Indolyl, Isoindolyl, Benzisoxazolyl, Benzoxazol, Benzisothiazolyl, Benzthiazolyl, 2-Benzthiazolyl, 4-Benzthiazolyl, 5-Benzthiazolyl, 6-Benzthiazolyl, 7-Benzthiazolyl, Indazolyl, Benzimidazolyl, Benzthiazolyl, Benzofurazanyl, Dibenzofuranyl, Dibenzothienyl, Acridinyl, Phenanthridinyl, Carbazolyl, Chinolinyl, Isochinolinyl, Phthalazinyl, Chinazolinyl, Chinoxalinyl, Cinnolinyl, 1,5-Naphthyridinyl, 1,6-Naphthyridinyl, 1,7-Naphthyridinyl, 1,8-Naphthyridinyl, Pteridinyl, Pyrrolopyridinyl, Pyrrolopyridazinyl, Pyrrolopyrimidinyl, Pyrrolopyrazinyl, Pyrrolotriazinyl, Furopyridinyl, Furopyridazinyl, Furopyrimidinyl, Furopyrazinyl, Furotriazinyl, Thienopyridinyl, Thienopyridazinyl, Thienopyrimidinyl, Thienopyrazinyl, Thienotriazinyl, Imidazopyridinyl, Imidazopyridazinyl, Imidazopyrimidinyl, Imidazopyrazinyl, Pyrazolopyridinyl, Pyrazolopyridazinyl, Pyrazolopyrimidinyl, Pyrazolopyrazinyl, Isoxazolopyridinyl, Isoxazolopyridazinyl, Isoxazolopyrimidinyl, Isoxazolopyrazinyl, Oxazolopyridinyl, Oxazolopyridazinyl, Oxazolopyrimidinyl, Oxazolopyrazinyl, Thiazolo- pyridinyl, Thiazolopyridazinyl, Thiazolopyrimidinyl, Thiazolopyrazinyl, Isothiazolopyridinyl, Isothiazolopyridazinyl, Isothiazolopyrimidinyl, Isothiazolopyrazinyl, Triazolopyridinyl, Triazolopyridazinyl, Triazolopyrimidinyl oder Triazolopyrazinyl.

Die vorstehend genannte Heterocyclylreste bedeuten bevorzugt 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imdiazolidinyl, 4-Imdiazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Diyhdrofur-3-yl, 2,3-Dihydrothien-2-yl, 2,3-Dihydrothien-3-yl, 2,5-Dihydrothien-2-yl, 2,5-Di-hydrothien-2-yl, 2,4-Pyrrolin-2-yl, 2,3-Pyrrolin-3-yl, 2,5-Pyrrolin-2-yl, 2,5-Pyrrolin-3-yl, 2,3-Isoxazolin-3-yl, 3,4-Isoxazolin-3-yl, 4,5-Isoxazolin-2-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothia-zolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothizoalin-5-yl, 2,3-Dihydropyrazol-1-yl, 2,3-Dihydropyrazol-2yl, 2,3-Dihydropyrazol-3-yl, 2,3-Dihydropyrazol-4-yl, 2,3-Dihydropyrazol-5-yl, 3,4-Dihydroyprazol-1-yl, 3,4-Dihydropyrazol-2-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Di-hydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl, N-Morpholinyl oder Dihydrochinazolinyl.

Die neuen Verbindungen können beispielsweise nach folgenden Verfahren hergestellt werden:
Das Nitril 2 erhält man analog H. Hoffmann et al.(Synthesis 1986, 548) aus dem Dihydropyran 1. Nach Deprotonierung des Dihydropyrans 1 durch starke organische Basen und behandeln des entstandenen Anions mit einem Formylierungsmittel wie z.B. Dimethylformamid (analog A. Lozanova et al., Izv. Akad. Nauk. SSR, Ser. Khim 734 (1989; R. Boeckman et al., Tetrahedron Letters 48, 4187 (1977)) oder Reduktion des Nitrils 2 mit Diisobutylaluminiumhydrid oder Raney-Nickel erhält man den Aldehyd 3 (Schema 1). 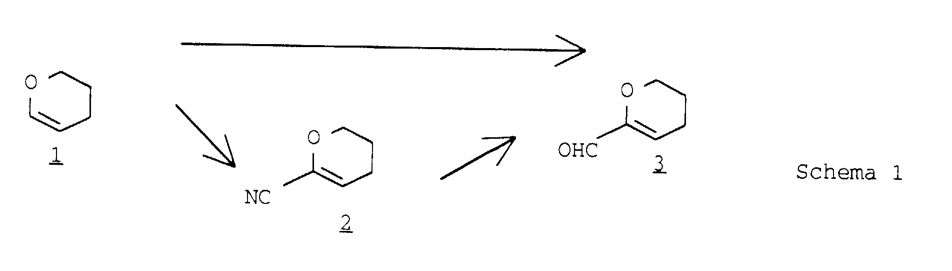 Den Aldehyd 3 kann man mit einem Metallhydrid wie z.B. Natriumborhydrid oder Lithiumaluminiumhydrid zum Alkohol 4 reduzieren (Schema 2).

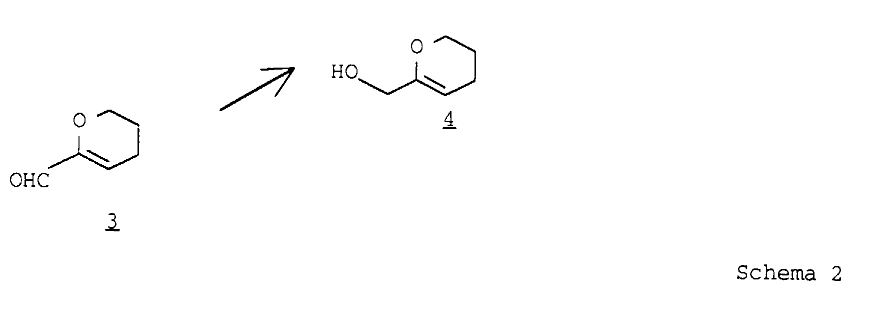

Durch Mesylierung (R. Cronland et al., J. Org. Chem. 35, 3195 (1970)) des Alkohols 4 zum Methansulfonsäureester 5 und nucleophile Substitution des Mesylrestes erhält man das Dihydropyran IV, das man analog M. Hajo et al., Synthesis 1986, 137 mit Cl-CO-CO-OR³/Pyridin zum Glyoxylat III umsetzen kann. Durch Oximierung des Ketoesters III mit H₂N-OR² oder Olefinierung von III z.B. mit (C₆H₅)₃P⁺-CH₂-O-R² (X⁻), (C₆H₅)₃P⁺-CH₂-X (X⁻) bzw. (C₆H₅)₃P⁺-CH₂-R² (X⁻) (X = Halogen) oder den entsprechenden Phosphonaten oder Phosphinoxiden erhält man die erfindungsgemäßen Verbindungen I' (Schema 3). 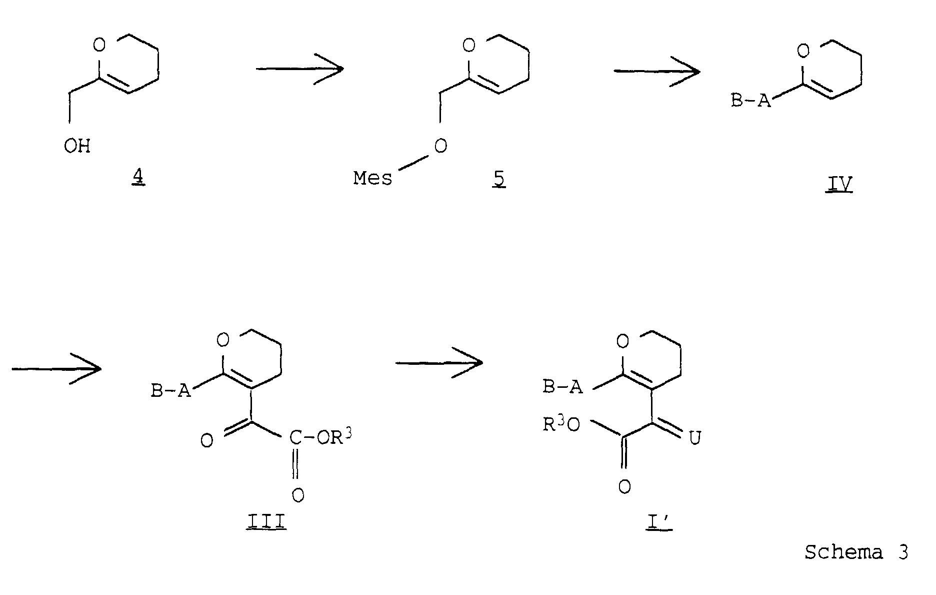
- A:: -O-CH₂-, -S-CH₂-, -NR⁶-CH₂-, -C(=O)-O-CH₂, -(R⁷)C=N-O-CH₂-
- U:: =CH-OR², =CHR², =CH-Halogen, =NOR²

Außerdem kann die Carbonylverbindung 3 in einer Wittig-Reaktion umgesetzt werden zu dem zwei- oder dreifach ungesättigten Derivat 6. Dieses reagiert mit Cl-CO-CO-OR³/Pyridin (vgl. M. Hojo et al., Synthesis 1986, 137) zum Ketoester XIV'. Durch Oximierung des Glyoxylats XIV' mit H₂N-OR² oder durch Olefinierung von XIV z.B. mit (C₆H₅)₃P⁺-CH₂-OR² (X⁻), (C₆H₅)₃P⁺-CH₂-X (X⁻) bzw. (C₆H₅)₃P⁺-CH₂-R² (X⁻) (X = Halogen) oder den entsprechenden Phosphonaten oder Phosphinoxiden erhält man die erfindungsgemäßen Verbindungen Ia' (Schema 4). 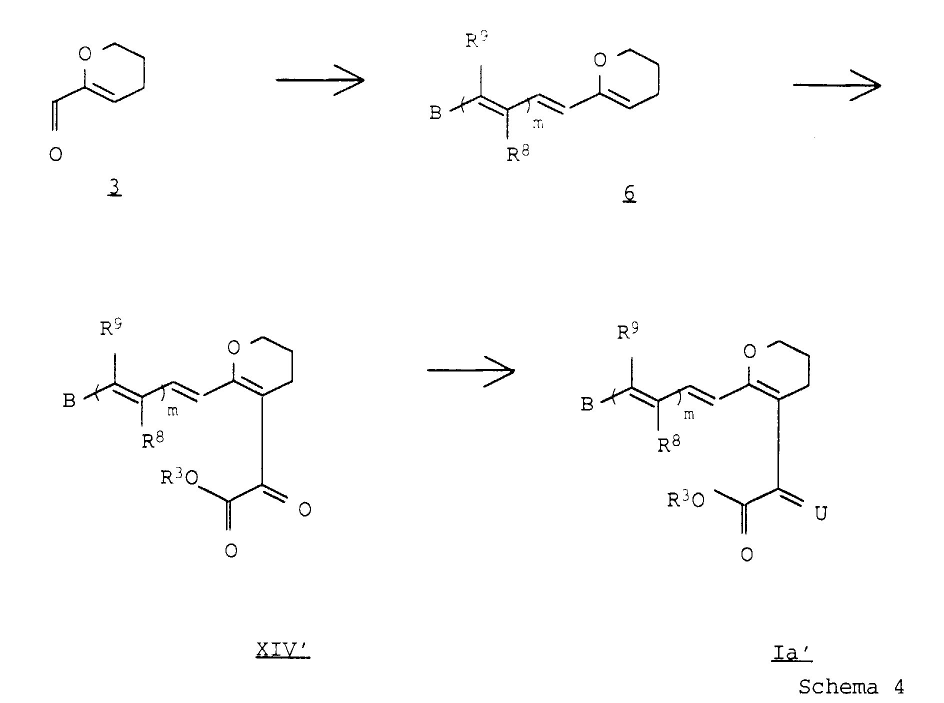
- U:: =CH-OR², =CHR², =CH-Halogen, =NOR²

Außerdem können die mehrfach ungesättigten Verbindungen XIV' selektiv mit Wasserstoff in Gegenwart geeigneter Katalysatoren wie Pd, Pt oder Ni in der Seitenkette hydriert werden, so daß man Dihydropyranderivate XIII' erhält. Diese lassen sich durch Oximierung mit H₂N-OR² bzw. durch Olefinierung z.B. mit (C₆H₅)₃P⁺-CH₂-OR² (X⁻), (C₆H₅)₃P⁺-CH₂-X (X⁻) oder (C₆H₅)₃P⁺-CH₂-R² (X⁻) (X = Halogen) oder den entsprechenden Phosphonaten oder Phosphinoxiden in die erfindungsgemäßen Verbindungen Ib' überführen (Schema 5).

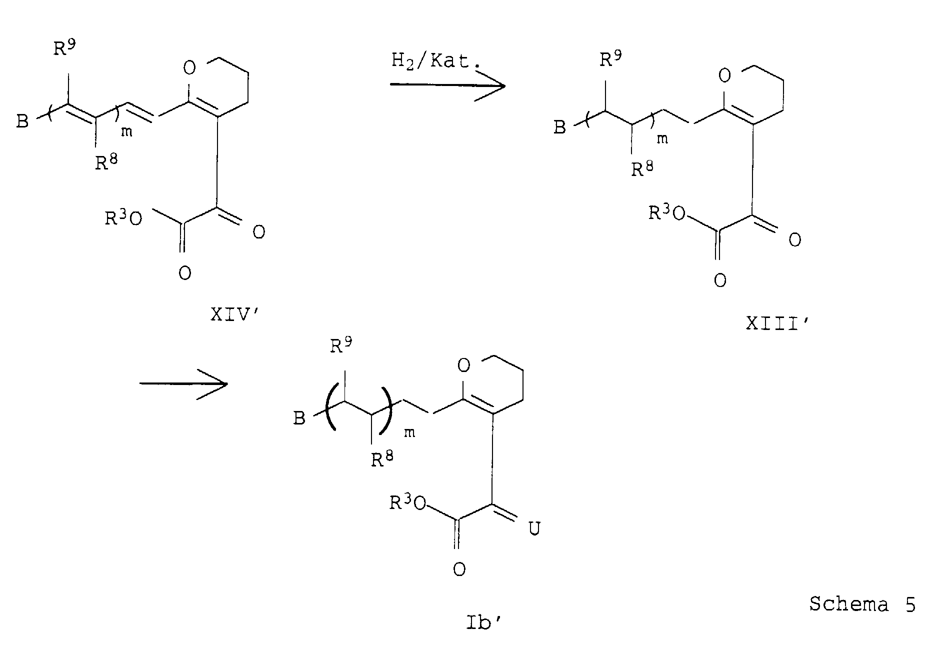

Die Thioenolether VIII sind zugänglich durch Umsetzung der Derivate IX' mit Mercaptanen unter basischen Bedingungen (Schema 6). 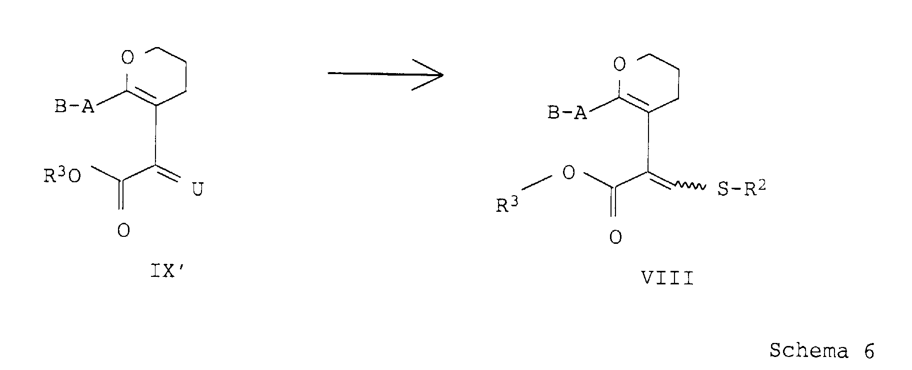
- U:: = CHX (X = Halogen), =CH-OR²

Die Ester VII reagieren mit Aminen HNR⁴R⁵ zu den entsprechenden Amiden VI (Schema 7)

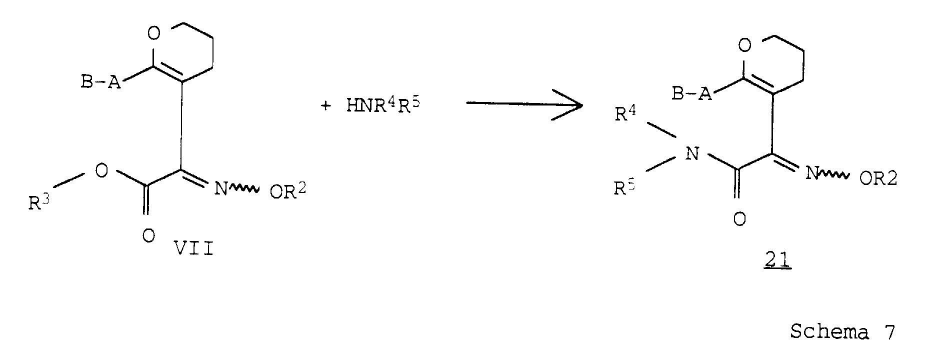

Acetylene der Formel Ic können nach literaturbekannten Methoden erhalten werden, z.B. durch Addition von Halogen an die Doppelbindung der Seitenketten von 7, wobei man das Dihalogenid 8 erhält, und anschließender thermischer oder basenkatalysierter Eliminierung von HX (X = Halogen; Schema 89).

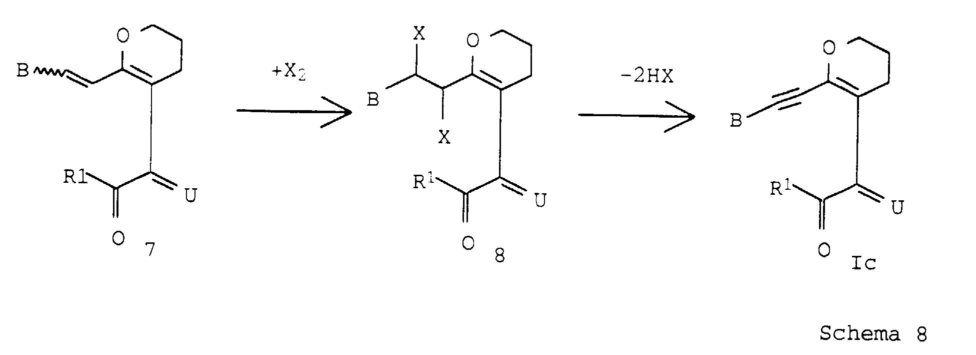

Alternativ können die erfindungsgemäßen Verbindungen auch unter Verwendung von Schutzgruppen synthetisiert werden. So kann man z.B. die Carbonylverbindung 3 in ihr cyclisches oder acyclisches Acetal, Thioacetal oder Hemithioacetal 9 überführen (SG1 = Schutzgruppe der Carbonylgruppe; siehe z.B.: T. Greene: Protective Groups in Organic Synthesis, J. Wiley & Sons 1981, S. 152ff). Die geschützte Carbonylverbindung 9 kann dann analog Schemata 3, 4, 6, 7, in die Verbindung 10 übergeführt werden, aus der durch Abspaltung der Schutzgruppe (siehe z. B.: T. Greene, Protective Groups in Organic Synthesis, J. Wiley & Sons 1981, S. 152ff) die freie Carbonylverbindung 11 zugänglich ist. Alternativ kann 11 durch Oxidation von 14 erhalten werden. Anschließend kann 11 analog Schemata 4, 5, 6, 7, 8, zu den Wirkstoffen I umgesetzt werden (Schema 9).

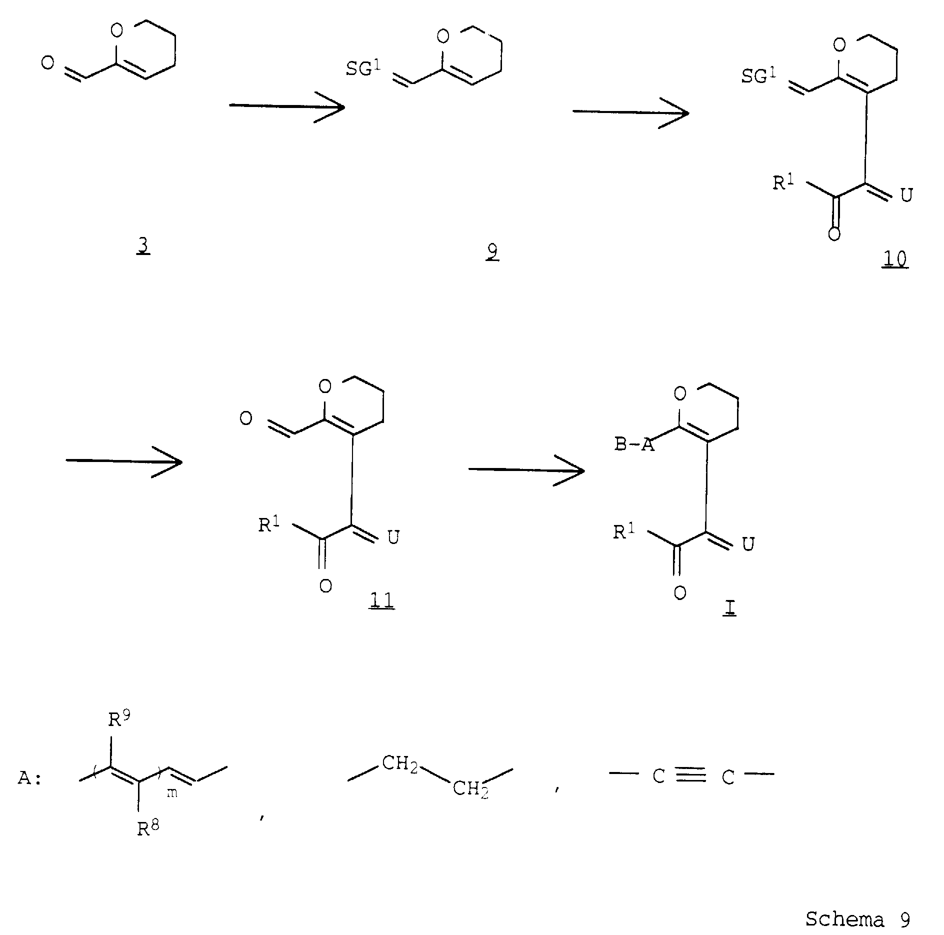

Außerdem kann auch der Alkohol 4 geschützt werden (SG2 = Schutzgruppe der Hydroxylfunktion), z.B. als Ether, Ester oder Acetal 12 (siehe z. B.: T. Greene, Protective Groups in Organic Synthesis, J. Wiley & Sons 1981, S. 10ff). Der geschützte Alkohol 12 kann dann analog Schemata 3, 6, 7, in die Verbindung 13 überführt werden, aus der durch Abspaltung der Schutzgruppe (siehe z. B.: T. Greene, Protective Groups in Organic Synthesis, J. Wiley & Sons 1981, S. 10ff) der freie Alkohol 14 zugänglich ist. Alternativ kann 14 auch durch Reduktion von 11 erhalten werden. 14 kann analog Schemata 3, 6, 7 zu den Wirkstoffen I umgesetzt werden (Schemata 10). 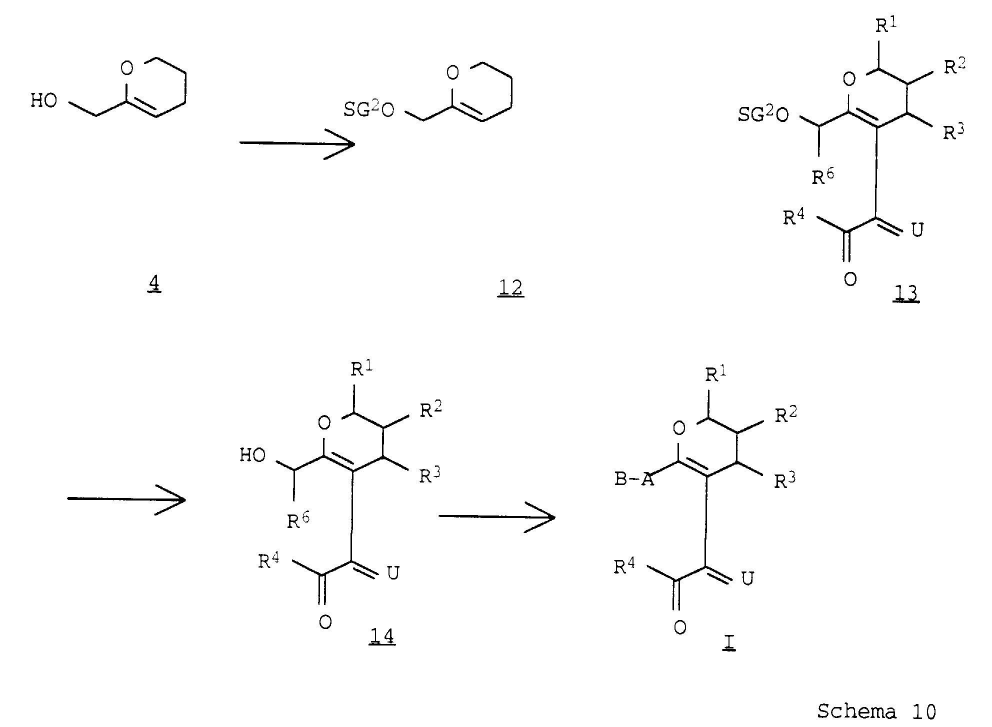
- A:: -O-CH₂-, -S-CH₂-, -NR⁶-CH₂-, -C(=O)-OCH₂-, -CR⁷=N-O-CH₂-

Die folgenden Beispiele erläutern die Herstellung der neuen Verbindungen.

### Beispiel 1

### 2-(6-Phenethenyl-2,3-dihydropyranyl-5)-crotonsäuremethylester (Tabelle 1, Nr. III/1)

a) 6-Formyl-2,3-dihydropyran
   100 g (0,91 mol) 6-Cyano-2,3-dihydropyran (H. Hoffmann et al., Synthesis 1986, 548) in 200 ml Toluol wird bei -70°C bis -60°C tropfenweise mit 750 ml 1,5 m Diisobutylaluminium-hydridlösung in Toluol (1,1 mol) versetzt. Man rührt ca. 30 min bei -60°C und gießt die Reaktionsmischung auf Eiswasser. Dann säuert man mit konz. Salzsäure vorsichtig an, so daß sich der gesamte Niederschlag auflöst. Man trennt die organische Phase ab und extrahiert die wässrige noch dreimal mit Methylenchlorid. Die vereinigten organischen Phasen werden mit 10 %iger Salzsäure und Wasser gewaschen, über MgSO4 getrocknet und i. Vak. eingeengt. Der Rückstand wird destilliert. Man ehält 80 g (78 %) der Titelverbindung (vgl. R. Boeckman et al., Tetrahedron Letters 48, 4187 (1977)) als farblose Flüssigkeit.
   Kp^{0,3}= 45 - 47°C
   ¹H-NMR (CDCl₃) :
      δ (ppm). 9,1 (s, 1H, CHO); 5,9 (t, 1H, J = 4 Hz, HC=C); 4,15 (t, 2H, J = 5 Hz, O-CH₂), 2,15 (m, 2H, CH2); 1,95 (m, 2H, CH₂)
b) 6-Phenethenyl-2,3-dihydropyran
   150 g (0,39 mol) Benzyltriphenylphosphoniumchlorid in 500 ml Tetrahydrofuran wird bei 0 - 10°C portionsweise mit 40 g (0,36 mol) Kalium-t-butanolat versetzt. Man rührt die orange Reaktionsmischung 30 min bei ca. 0°C. Danach gibt man 37,5 g (0,34 mol) 6-Formyl-2,3-dihydropyran (Beispiel la) hinzu, entfernt die Kühlung und rührt weitere 30 min. Anschließend gießt man die Reaktionsmischung auf NH₄Cl-Lösung und extrahiert die wässrige Phase mit Methylenchlorid. Die vereinigten organischen Phasen werden über MgSO4 getrocknet und eingeengt. Der halbfeste Rückstand wird mit 300 ml Ether aufgerührt und über eine kurze Kieselgelsäule abgesaugt. Das Filtrat wird eingeengt und der Rückstand wird mit Cyclohexan/Essigsester 3:1 chromatographiert. Man erhält 53,5 g (0,29 mol = 85 %) der Titelverbindung (Isomerengemisch ca. 5:1) als helles Öl.
   ¹H-NMR (CDCl₃) :
      δ (ppm): 7,1 - 7,9 (m, 5H, Aromat), 6,8 (d, 1H (Nebenkomponente), J = 16 Hz, -C=C-H); 6,45 (d, 1H (Nebenkomponente), J = 16 Hz, -C=C-H); 6,3 (d, 1H (Hauptkomponente); J = 13 Hz, -C=C-H); 5,85 (d, 1H (Hauptkomponente), J = 13 Hz, -C=C-H); 4,9 (m, 1H, O-C=C-H); 4,1 (t, 2H (Nebenkomponente), J = 5 Hz, O-CH₂), 3,9 (t, 2H (Hauptkomponente), J = 5 Hz, O-CH₂), 2,1 (m, 2H, CH₂); 1,8 (m, 2H, CH₂)
c) 6-trans-Phenethenyl-2,3-dihydropyranyl-5-glyoxylsäuremethylester
   53,5 g (0,29 mol) 6-Phenethenyl-2,3-dihydropyran (Beispiel 1b) und 31 g (0,4 mol) Pyridin in 250 ml Methylendchlorid werden tropfenweise mit 44 g (0,36 mol) Oxalsäuremethylester versetzt. Dabei erwärmt sich die Reaktionsmischung zum Rückfluß. Man rührt über Nacht bei Raumtemperatur (20°C). Anschließend extrahiert man die Reaktionsmischung mit verdünnter Salzsäure und Wasser. Die organische Phase wird über MgSO₄ getrocknet und eingeengt. Der Rückstand wird säulenchromatographisch mit Cyclohexan/Essigester-Gemischen gereinigt. Man erhält 82,5 g (ca. 90 %ige Reinheit; 0,27 mol - 94 %) der Titelverbindung(trans-Konfiguration) als farbloses Öl.
   ¹H-NMR (CDCl₃) :
      δ (ppm): 7,2 - 7,4 (m, 7H, Aromat, H-C=C-H); 4,25 (t, 2H, (O-CH₂); 3,8 (s, 3H, OCH₃), 2,5 (t, 2H, CH₂); 1,95 (t, 2H, CH₂)
d) 2- (6-trans-Phenethenyl-2,3-dihydropyranyl-5) -transcrotonsäuremethylester (Tabelle 1, Nr III/1)
   21 g (50,2 mmol) Ethyl-triphenylphosphoniumchlorid in 100 ml Tetrahydrofuran wird bei 0 - 5°C portionsweise mit 4,1 g (36,5 mmol) Kalium-t-butanolat versetzt. Man rührt 15 min bei 0 - 5°C, kühlt auf -20°C und gibt 7 g (26 mmol) 6-trans-Phenethenyl-2,3-dihydropyranyl-5-glyoxylsäuremethylester (Beispiel 1c) hinzu. Danach läßt man die Reaktionsmischung auf Raumtemperatur erwärmen, gießt auf NH₄Cl-Lösung und extrahiert die wässrige Phase dreimal mit Methylenchlorid. Die vereinigten organischen Phasen werden über MgSO₄ getrocknet und eingeengt. Der Rückstand wird säulenchromatographisch mit Cyclohexan/Essigester-Gemischen gereinigt. Man erhält 2,7 g (9,5 mmol = 37 %) der Titelverbindung als farbloses Öl.
   ¹H-NMR (CDCl₃):
      δ (ppm): 7,15 - 7,4 (m, 5H, Aromat); 7,1 (q, 1H, Me-C=C-H); 6,8 (d, 1H, J = 16 Hz, -H-C=C-); 6,45 (d, 1H, J = 16 Hz, -C=C-H-); 4,15 (m, 2H, O-CH₂); 3,65 (s, 3H, OCH₃); 2,2 (m, 2H, CH₂); 1,95 (t, breit, 2H, CH₂); 1,75 (d, 3H, J = 8 Hz, CH₃)

### Beispiel 2

### 2-(6'-Phenethyl-2',3'-dihydropyranyl-5')-3-methoxy-acrylsäuremethylester (Tabelle 4, Nr. I/422)

a) 6-Phenethyl-2,3-dihydropyranyl-5-glyoxylsäuremethylester
   30 g (0,11 mol) 6-Phenethenyl-2,3-dihydropyranyl-5-gly-oxylsäuremethylester und 3 g PtO₂ (5 % auf SiO₂) in 250 ml Methanol werden unter einer H₂-Atmosphäre bei Raumtemperatur 24 Stunden kräftig gerührt. Dann gibt man zusätzlich 1 g PtO₂ (5 % auf SiO₂) hinzu und rührt 8 Stunden bei 50°C und anschließend über Nacht bei Raumtemperatur. Dann wird der Katalysator abgesaugt und das Filtrat eingeengt. Der Rückstand wird säulenchromatographisch mit Cyclohexan/Essigester-Gemischen gereinigt. Man erhält 15,6 g (57 mmol = 52 %) der Titelverbindung als farbloses Öl.
   ¹H-NMR (CDCl₃):
      δ (ppm): 7,25 (m, 5 H, Aromat); 4,15 (t, 2H, J = 6 Hz, O-CH₂), 3,85 (s, 3H, OCH₃); 2,8 (m, 4H, Ph-CH₂-CH₂), 2,35 (t,2H, J = 6 Hz, CH₂); 1,85 (m, 2H, CH₂)
b) 2- (6'-Phenethyl-2',3' -dihydropyranyl-5') -3-methoxyacrylsäuremethylester (Tabelle 4, Nr. I/422)
   10 g (29 mmol) Methoxy-triphenylphosphoniumchlorid in 40 ml Tetrahydrofuran wird bei 0 - 5°C portionsweise mit 2,8 g (25 mmol) Kalium-t-butanolat versetzt. Man rührt 15 Minuten bei 0°C, kühlt auf -60°C und gibt 4 g (14,6 mmol) 6-Phenethyl-2,3-dihydropyranyl-5-glyoxylsäuremethylester (Beispiel 2a) hinzu. Man läßt auf Raumtemperatur erwärmen, gießt auf NH₄Cl-Lösung und extrahiert die wässrige Phase dreimal mit Diethylether. Die vereinigten organischen Phasen werden über MgSO₄ getrocknet und eingeengt. Der Rückstand wird säulenchromatographisch mit Hexan/Essigester-Gemischen gereinigt. Man erhält 2,1 g (6,9 mmol - 48 %) des trans-Isomeren (farblose Kristalle, Fp = 83 - 85°C) und 1,6 g (5,3 mmol - 36 %) des cis-Isomeren (farbloses Öl) der Titelverbindung.
   trans-Isomeres, ¹H-NMR (CDCl₃):
      δ (ppm): 7,3 (s, 1H, -C=C-H); 7,1 - 7,3 (m, 5H, Aromat); 4,05 (t, 3H, J - 6 Hz, OCH₂); 3,8 (s, 3H, OCH3); 3,65 (s, 3H, OCH₃); 2,75 (m, 2H, CH₂); 2,25 (m, 2H, CH₂); 2,05 (t, breit, 2H, CH₂), 1,9 (m, 2H, CH₂)
   cis-Isomeres, ¹H-NMR (CDCl₃):
      δ (ppm): 7 - 7,4 (m, 5H, Aromat), 5,45 (s, 1H, = -H); 4,05 (t, 2H, J - 6 Hz, O-CH₂); 3,65 (s, 3H, OCH₃); 3,6 (s, 3H, OCH₃); 2,75 (t, 2H, J - 8 Hz, CH₂); 2,35 (t, 2H, J - 8 Hz, CH₂); 2,0 (t, breit, 2H, CH₂); 1,85 (m, 2H, -CH₂)

### Beispiel 3

### 2-(6'-trans-ortho-Methylphenethenyl-2',3'-dihydropyranyl-5')-3-chlor-acrylsäuremethylester (Tabelle 7, Nr. 42)

12 g (34 mmol) Chlormethyl-triphenylphosphoniumchlorid, 50 g (17 mmol) trans-6-ortho-Methylphenethenyl-2,3-dihydropyranyl-5-glyoxylsäuremethylester (hergestellt analog Beispiel 1c) und 1 ml Methanol in 100 ml Tetrahydrofuran werden bei Raumtemperatur portionsweise mit 2,8 g (25 mmol) Kalium-t-butanolat versetzt. Man rührt 3 Stunden bei Raumtemperatur und gießt die Reaktionsmischung auf Wasser. Die wässrige Phase wird dreimal mit Diethylether extrahiert. Die vereinigten organischen Phasen werden über MgSO₄ getrocknet und eingeengt. Der halbfest Rückstand wird mit Diethylether verrührt und abgesaugt. Anschließend engt man das Filtrat ein und reinigt den zurückbleibenden Rückstand säulenchromatographisch mit Hexan/Essigester-Gemischen. Man erhält das cis-Chlor- und das trans-Chlor-acrylsäureester-Isomere der Titelverbindung als Gemisch, 0,9 g (2,8 mmol = 16 %; hellgelbes Öl) im Verhältnis cis/trans 1:10 und 3,7 g (11,5 mmol = 67 %; hellgelbes Öl) im Verhältnis cis/trans 1:3.
¹H-NMR (CDCl₃) :
   δ (ppm): 7,5 (s, 1H (trans), C=CCl-H);7,04 - 7,45 (m, 5H, Aromat, -C=C-H); 6,65 (d, 1H (cis), J = 16 Hz, -C=C-H), 6,4 (s, 1H (cis), C=CCl-H); 6,3 (d, 1H (trans), J = 16 Hz, -C=C-H); 4,2 (m, 2H, O-CH₂); 3,8 (s, 3H (cis), OCH₃); 3,75 (s, 3H (trans), OCH₃); 2,35 (s, 3H, CH₃); 2,25 (t, breit, 2H, CH₂); 2,0 (m, 2H, CH₂)

### Beispiel 4

### 2-(6'-trans-ortho-Methylphenethenyl-2',3'-dihydropyranyl-5')-3-trans-methylthio-acrylsäuremthylester (Tabelle 7, Nr. 44)

0,9 g (2,8 mmol) 2-(6'-trans-ortho-Methylphenethenyl-2',3'-dihydropyranyl-5')-3-trans-chlor-acrylsäuremethylester (Beispiel 3) in 8 ml Dimethylformamid wird mit 0,22 g (3,1 mmol) Natriumthiomethanolat versetzt, wobei sich die Reaktionsmischung auf ca. 35°C erwärmt. Man rührt ca. 30 min, gibt die Reaktionsmischung auf Wasser und extrahiert die wässrige Phase dreimal mit Diethylether. Die vereinigten organischen Phasen werden über MgSO₄ getrocknet und eingeengt. Der Rückstand wird säulenchromatographisch mit Hexan/Essigester-Gemischen gereinigt. Man erhält 0,68 g (2 mmol - 72 %) der Titelverbindung als hellgelbes Öl.

¹H-HMR (CDCl₃) :
δ (ppm): 7,7 (s, 1H, C=C(SMe)-H);
7,4 (m, 1H, Aromat); 7 - 7,2 (m,4H, Aromat, -C=C-H); 6,35 (d, 1H, J = 16 Hz, -H-C=C-); 4,15 (t, 1H, J = 6 Hz, O-CH₂); 3,7 (s, 3H, OCH₃); 2,4 (s, 3H, CH₃); 2,35 (s, 3H, CH₃); 2,2 (m, 2H, CH₂); 1,95 (m, 2H, CH₂)

### Beispiel 5

### 2-[6'-(4'''-Chlorphenyl-3''-isoxazolyl-5''-ethenyl)-2',3'dihydropyranyl-5']-3-methoxy-acrylsäuremethylester (Tabelle 2, Nr. I/396)

a) 6' -(4'''-Chlorphenyl-3''-isoxazolyl-5''-ethenyl)-2',3'-dihydropyran
   10 g (30 mmol) 4'''-Chlorphenyl-3''-isoxazolyl-5''-methylphosphonsäurediethylester in 100 ml Tetrahydrofuran wird unter Kühlung mit einem Wasserbad portionsweise mit 0,8 g (33 mmol) Natriumhydrid versetzt. Man rührt 15 Minuten bei Raumtemperatur und gibt dann 3 g (27 mmol) 6-Formyl-2,3-dihydropyran (Beispiel la) hinzu. Man rührt 1 Stunde bei Raumtemperatur, gießt die Reaktionsmischung auf verdünnte Salzsäure (pH - 3 - 4) und extrahiert die wässrige Phase dreimal mit Methylenchlorid. Die vereingten organischen Phasen werden über MgSO₄ getrocknet und eingedampft. Der zurückbleibende Festkörper wird mit Methyl-t-butylether verrührt und abgesaugt. Als Rückstand erhält man 5,2 g (18 mmol - 67 %) der Titelverbindung als hellgelbe Kristalle.
   1H-NMR (CDCl3).
      δ (ppm): 7,7 (d, 2H, J - 8 Hz, Phenyl); 7,4 (d, 2H, J = 8 Hz, Phenyl); 6,65 (s, 2H, H-C=C-H); 6,4 (s, 1H, Isoxazolyl); 5,15 (t, 2H, J - 4 Hz, -C=C-H); 4,1 (t, 2H, J = 5 Hz, O-CH₂); 2,2 (m, 2H, CH₂); 1,9 (m, 2H, CH₂)
b) 6'-(4'''-Chlorphenyl-3''-isoxazolyl-5''-transethenyl)-2',3'-dihydropyranyl-5'-glyoxylsäuremthylester
   5,5 g (19 mmol) 6'-(4'''-Chlorphenyl-3''-isoxazolyl-5''ethenyl)-2', 3'-dihydropyran (Beispiel 5 a), 2,5 g (32 mmol) Pyridin und 3 g (24,6 mmol) Oxalsäuremethylesterchlorid werden 4 Tage bei Raumtemperatur gerührt. Man gießt die Reaktionsmischung auf Wasser und extrahiert die wässrige Phase dreimal mit Methylenchlorid.
   Die vereinigten organischen Phasen werden über MgSO₄ getrocknet und eingedampft. Der Rückstand wird aus Methylt-butylether umkristallisiert. Man erhält 3,4 g (9,1 mmol = 48 %) der Titelverbindung als hellgelbe Kristalle.
   ¹H-NMR (CDCl₃) :
      δ (ppm): 7,75 (d, 2H, J = 8 Hz, Phenyl); 7,65 (d, 1H, J = 16 Hz, Vinyl); 7,4 (d, 2H, J = 8 Hz, Phenyl); 7,15 (d, 1H, J = 16 Hz, Vinyl); 6,65 (s, 1H, Isoxazolyl); 4,25 (t, 2H, J = 5 Hz, O-CH₂); 3,9 (s, 3H, OCH₃); 2,5 (t, 2H, J = 6 Hz, CH₂); 2,0 (m, 2H, CH₂)
c) 2-[6'-(4'''-Chlorphenyl-3''-isoxazolyl-5''trans-ethenyl)-2', 3'-dihydropyranyl-5']-3-methoxy-acrylsäuremethylester (Tabelle 2, Nr. I/396)
   7,5 g (22 mmol) Methoxy-triphenylphosphoniumchlorid in 50 ml Tetrahydrofuran wird bei 0 - 5°C portionsweise mit 2,1 g (19 mmol) Kalium-t-butanolat versetzt. Man rührt 30 min bei 0 - 5°C, gibt 3,4 g (9,1 mmol) 6'-(4'''-Chlorphenyl-3''-isoxazolyl-5''-transethenyl)-2', 3'-dihydropyranyl-5'-glyoxylsäuremethylester (Beispiel 5 b) hinzu und läßt auf Raumtemperatur erwärmen. Nach 30 Minuten gießt man die Reaktionsmischung auf Wasser und extrahiert die wässrige Phase dreimal mit Ether. Die vereinigte etherischen Phasen werden über MgS04 getrocknet und eingedampft. Man erhält einen öligen Rückstand, der säulenchromatographisch mit Hexan/Essigester-Gemischen gereinigt wird. Man erhält 1,4 g (3,5 mmol = 38 %; farblose Kristalle, Fp = 170°C) des trans-Methoxy-Isomeren und 0,44 g (1,1 mmol = 12 %, farblose Kristalle, Fp = 181°C) des cis-Methoxy-Isomeren der Titelverbindung.
   trans-Isomeres, 1H-NMR (CDCl3):
      δ (ppm): 7,75 (d, 2H, J = 8 Hz, Phenyl); 7,45 (s, 1H, C-C(OMe)-H); 7,4 (d, 2H, J = 8 Hz, Phenyl); 6,85 (d, 1H, J = 16 Hz; -C=C-H); 6,7 (d, 1H, J - 16 Hz, -C=C-H); 6,4 (s,1H, Isoxazolyl); 4,15 (t, 2H, J - 5 Hz, O-CH₂); 3,85 (s, 3H, OCH₃); 3,75 (s, 3H, OCH₃), 2,25 (t, breit, CH₂); 1,95 (t, breit, CH₂)
   cis-Isomeres, 1H-NMR (CDCl₃):
      δ (ppm): 7,75 (d, 2H, J = 8 Hz, Phenyl); 7,45 (d, 2H, J = 8 Hz, Phenyl); 7,1 (d, lH, J 16 Hz, -C=C-H); 6,75 (d, 1H, J = 16 Hz, =-H); 5,4, 6,45 (2s, je 1 H, Isoxazolyl und C=C(OMe)-H); 4,15 (t, 2H, J = 5 Hz, O-CH₂); 3,9 (s, 3H, OCH₃); 3,8 (s, 3H, OCH₃); 2,3 (t, 2H, J = 6 Hz, CH₂); 19 (m, 2H, CH₂)

### Beispiel 6

### 6'-(2'', 5''-Dimethylphenyl-trans-ethenyl)-2'-3'-dihydropyranyl-5'-glyoxylsäuremethylamid-trans-O-methyloxim (Tabelle 7, Nr. 47)

Eine Mischung von 0,6 g (1,8 mmol) 6'-(2'', 5''-Dimethylphenyl-trans-ethenyl)-2',3 '-dihydropyranyl-5'-glyoxylsäure-methylester-trans-O-methyloxim (Tabelle 1, Nr. II/133; hergestellt analog Beispiel 1 a - c und 7 e, f) und 10 ml 40 %iger wäßriger Methylaminlösung wird bei 50°C 2 Stunden gerührt. Anschließend läßt man abkühlen und extrahiert die heterogene Reaktionsmischung dreimal mit Methylenchlorid. Die vereinigten organischen Phasen werden über MgSO₄ getrocknet und eingedampft. Nach chromatographischer Reinigung des Rückstandes mit Hexan/Essigester-Gemischen erhält man 0,45 g (1,4 mmol = 76 %) der Titelverbindung als farblosen Festkörper.
Fp = 165 - 168°C.
¹H-NMR (CDCl₃):
   δ (ppm): 6,9 - 7,2 (m, 4H, Aromat, Vinyl); 6,7 (s, breit, 1H, N-H); 6,1 (d, lH, J = 16 Hz, Vinyl); 4,2 (t, 2H, J = 5 Hz, O-CH₂), 4,0 (s, 3H, OCH₃); 2,9 (d, 3H, J = 5 Hz, N-CH₃); 2,3 (m, 8 H, 2 x CH₃, CH₂); 2,0 (m, 2H, CH₂)

### Beispiel 7

### 6-Phenoxymethyl-2,3-dihydropyranyl-5-glyoxylsäuremethylester-trans-O-methyloxim (Tabelle 7, Nr. 23)

a) 6-Hydroxymethyl-2,3-dihydropyran
   56 g (0,5 mol) 6-Formyl-2,3-dihydropyran (Beispiel 1 a) in 400 ml Isopropanol wird unter Kühlung mit einem Eis-Wasser-Bad portionsweise mit 9,5 g (0,25 mol) Natriumborhydrid versetzt. Dabei erwärmt sich die Reaktionsmischung auf 40°C. Man rührt 1 Stunde bei 0°C, gießt anschließend die Reaktionsmischung auf Wasser und extrahiert die wäßrige Phase dreimal mit Methylenchlorid. Die vereinigten organischen Phasen werden über MgSO₄ getrocknet und eingeengt. Man erhält 45 g der Titelverbindung als gelbes Öl, das als Rohprodukt in der nächsten Reaktion eingesetzt wird.
b) 6-Hydroxymethyl-2,3-dihydropyran-methansulfonsäureester
   45 g (0,39 mol) 6-Hydroxymethyl-2,3-dihydropyran (Beispiel 7 a) und 50 g (0,5 mol) Triethylamin in 400 ml Methylenchlorid werden bei 0 - 5°C tropfenweise mit 51,5 g (0,45 mol) Methansulfonylchlorid versetzt. Man rührt 1 Stunde bei 0°C und gießt die Reaktionsmischung auf Wasser. Man trennt die wässrige Phase ab und extrahiert die organische Phase noch einmal mit Wasser. Dann wird die organische Phase über MgSO₄ getrocknet und eingeengt. Man erhält 84 g der Titelverbindung als rotes Öl, das direkt in der nächsten Reaktion eingesetzt wird.
c) 6-Phenoxymethyl-2,3-dihydropyran
   20,5 g (0,22 mol) Phenol in 200 ml Dimethylformamid wird bei Raumtemperatur portionsweise mit 6,0 g (0,25 mol) Natriumhydrid versetzt. man rührt 30 Minuten bei Raumtemperatur und tropft anschließend unter leichter Kühlung 42 g (0,22 mol) 6-Hydroxymethyl-2,3-dihydropyranmethansulfonsäureester (Beispiel 7 b) zu. Man rührt 4 Stunden bei Raumtemperatur und gießt anschließend die Reaktionsmischung auf Wasser. Man extrahiert die wäßrige Phase dreimal mit Diethylether, trocknet die organische Phase über MgSO₄ und engt i. Vak. ein. Der Rückstand wird destilliert. Man erhält 27 g (0,14 mol - 57 % bezogen auf 6-Formyl-2,3-dihydropyran) der Titelverbindung als hellgelbes Öl.
   Kp_{0,3} = 70°C.
   ¹H-NMR (CDCl₃):
      δ (ppm): 7,3 (t, 2H, J - 8 Hz, Phenyl); 6,95 (m, 3H, Phenyl); 4,9 (t, 1H, J - 4 Hz, Vinyl), 4,35 (s, 2H, O-CH₂); 4,1 (t, 2H, J - 5 Hz, O-CH₂); 2,05 (m, 2H, CH₂); 1,85 (m, 2H, CH₂)
d) 6-Phenoxymethyl-2,3-dihydropyranyl-5-glyoxylsäuremethylester
   31 g (0,16 mol) 6-Phenoxymethyl-2,3-dihydropyran (Beispiel 7 c), 39,5 g (0,5 mol) Pyridin und 2,5 g (0,02 mol) p-Dimethylaminopyridin in 200 ml Methylenchlorid werden tropfenweise mit 35 g (0,29 mol) Oxalsäuremethylesterchlorid versetzt. Dabei erwärmt sich die Reaktionsmischung auf 30 - 35°C. Man rührt über Nacht und gießt den Reaktionsansatz anschließend auf Wasser. Man trennt die wäßrige Phase ab und extrahiert die organische noch einmal mit Wasser. Dann wird die organische Phase über MgSO₄ getrocknet und eingeengt. Der Rückstand wird säulenchromatographisch mit Hexan/EssigesterGemischen gereinigt. Man erhält 16 g Ausgangsprodukt und 8,5 g (0,031 mol - 19 %, hellgelbes Öl) der Titelverbindung.
   ¹H-NMR (CDCl₃):
      δ (ppm): 7,3 (t, 2H, J = 8 Hz, Phenyl); 6,8 - 7,1 (m, 3H, Phenyl); 4,8 (s, 2H, O-CH₂); 4,2 (t, 2H, J - 5Hz, O-CH₂); 3,6 (s, 3H, OCH₃); 2,4 (t, 2H, J - 6 Hz, CH₂); 1,9 (m, 2H, CH₂)
e) 6-Phenoxymethyl-2, 3-dihydropyranyl-5-glyoxylsäuremethylester-cis-O-methyloxim (Tabelle 7, Nr. 23 a)
   6 g (22 mmol) 6-Phenoxymethyl-2,3-dihydropyranyl-5-glyoxylsäuremethylester (Beispiel 7 d), 3 g (38 mmol) Pyridin und 3 g (36 mmol) o-Methylhydroxylamin-hydrochlorid in 50 ml Methanol werden über Nacht bei Raumtemperatur gerührt. Dann wird die Reaktionsmischung i. Vak. eingeengt, in Methylenchlorid aufgenommen und mit Wasser extrahiert. Anschließend wird die organische Phase über MgSO4 getrocknet und eingeengt. Der Rückstand wird säulenchromatographisch mit Hexan/Essigester-Gemischen gereinigt. Man erhält 2,9 g (9,5 mmol = 43 %) der Titelverbindung als farbloses Öl.
   ¹H-NMR (CDCl₃):
      δ (ppm): 7,3 (t, 2H, J = 8 Hz, Phenyl); 6,9 (m, 3H, Phenyl); 4,6 (s, 2H, OCH₂); 4,1 (t, 2H, J - 5 Hz, O-CH₂); 3,85 (s, 3H, OCH₃); 2,3 (t, 2H, J - 6 Hz, CH₂); 1,9 (m, 2H, CH₂)
f) 6-Phenoxymethyl-2,3-dihydropyranyl-5-gloxylsäuremethylester-trans-O-methyloxim (Tabelle 7, Nr. 23 b)
   5 g (16 mmol) 6-Phenoxymethyl-2,3-dihydropyranyl-5-glyoxylsäuremethylester-cis-O-methyloxim (Beispiel 7 e) in 20 ml Methylenchlorid wird mit 1 ml 4 m Salzsäuregas in Diethylether versetzt und über Nacht bei Raumtemperatur gerührt. Anschließend wird die Reaktionslösung i. Vak. eingeengt und der Rückstand säulenchromatographisch mit Hexan/Essigester-Gemischen gereinigt. Man erhält 0,8 g Ausgangsmaterial und 2,2 g (7,2 mmol = 44 %) der Titelverbindung als hellgelbes Öl.
   ¹H-NMR (CDCl₃):
      δ (ppm): 7,25 (t, 2H, J - 8 Hz, Phenyl); 6,9 (m, 3H, Phenyl); 4,35 (s, 2H, OCH₂); 4,15 (t, 2H, J = 5 Hz, OCH₂); 3,95 (s, 3H, OCH₃); 3,75 (s, 3H, OCH₃); 2,2 (t, 2H, J - 6 Hz, CH₂); 1,9 (m, 2H, CH₂)

### Beispiel 8

### 6-(6'-Ethylpyridyl-2'-oxymethyl)-2,3-dihydropyranyl-5glyoxylsäuremethylester-trans-O-methyloxim (Tabelle 7, Nr. 25)

a) 6- (6'-Ethylpyridyl-2'-oxymethyl) -2,3-dihydropyran
   28 g (0,227 mol) 2-Hydroxy-6-ethylpyridin in 400 ml Dimethylformamid wird unter leichter Kühlung portionsweise mit 6,6 g (0,275 mol) Natriumhydrid versetzt. Man rührt 30 Minuten bei Raumtemperatur und tropft anschließend 41 g (0,213 mol) 6-Hydroxymethyl-2,3-dihydropyran-methansulfonsäureester (Beispiel 7 b) gelöst in 50 ml Dimethylformamid zu. Man rührt über Nacht bei Raumtemperatur, gießt die Reaktionsmischung auf Wasser und extrahiert die wäßrige Phase dreimal mit Diethylether. Die vereinigten organischen Phase werden einmal mit Wasser gewaschen, über MgSO₄ getrocknet und eingeengt. Der Rückstand wird destillativ gereinigt. Man erhält 21,5 g (0,098 mol - 43 % bezogen auf 6-Formyl-2,3-dihydropyran) der Titelverbindung als hellgelbes Öl.
   ¹H-NMR (CDCl₃):
      δ (ppm): 7,45 (t, 1H, J = 8 Hz, Pyridyl); 6,7 (d, 1H, J = 8 Hz, Pyridyl); 6,6 (d, 1H, J - 8 Hz, Pyridyl); 4,95 (t, 1H, J - 4 Hz, Vinyl); 4,7 (s, 2H, OCH₂); 4,1 (t, 2H, J = 5 Hz, O-CH₂); 2,7 (q, 2H, J = 8 Hz, CH₂); 2,1 (t, breit, 2H, CH₂); 2,8 (m, 2H, CH₂); 1,3 (t, 3H, J = 8 Hz, CH₃)
b) 6- (6'-Ethylpyridyl-2'-oxymethyl) -2,3-dihydropyranyl-5-glyoxylsäuremthylester
   9 g (46,5 mmol) 6-(6'-Ethylpyridyl-2'-oxymethyl)-2,3-dihydropyran (Beispiel 8 a), 8 g (100 mmol) Pyridin und 8 g (65 mmol) Oxalsäuremethylesterchlorid in 100 ml Methylenchlorid werden über Nacht bei Raumtemperatur gerührt. Anschließend extrahiert man die Reaktionslösung mit Wasser, trocknet über MgSO₄ und engt i. Vak. ein. Der Rückstand wird säulenchromatographisch mit Hexan/Essigester-Gemischen gereinigt. Man erhält 1,9 g Ausgangsmaterial und 6,6 g (21,6 mmol = 47 %) der Titelverbindung als hellgelbes Öl.
   ¹H-NMR (CDCl₃):
      δ (ppm): 7,5 (t, 1H, J = 8 Hz, Pyridyl); 6,75 (d, 1H, J = Hz, Pyridyl); 6,55 (d, 1H, J = 8 Hz, Pyridyl); 5,15 (s, 2H, OCH₂); 4,15 (t, 2H, J - 5 Hz, O-CH₂), 3,7 (s, 3H, OCH₃); 2,65 (q, 2H, J - 8 Hz, CH₂); 2,4 (t, 2H, J = 6 Hz, CH₂); 1,9 (m, 2H, CH₂); 1,3 (t, 3H, J = 8 Hz, CH₃)
c) 6- (6'-Ethylpyridyl-2'-oxymethyl) -2,3-dihydropyranyl-5-glyoxylsäuremethylester-cis-O-methyloxim (Tabelle 7, Nr. 25 a)
   12,2 g (40 mmol) 6-(6'-Ethylpyridyl-2'-oxymethyl)-2,3-dihydropyranyl-5-glyoxylsäuremethylester (Beispiel 8 b), 7 g (88 mmol) Pyridin und 6 g (71 mmol) O-Methylhydroxylamin-hydrochlorid in 100 ml Methanol werden bei Raumtemperatur über Nacht gerührt. Anschließend engt man die Reaktionsmischung ein, nimmt den Rückstand in Methylenchlorid auf und extrahiert die organische Phase mit Wasser. Danach wird die organische Phase über MgSO₄ getrocknet und eingedampft. Der Rückstand wird säulenchromatographisch mit Hexan/EssigesterGemischen gereinigt. Man erhält 4,5 g (13,5 mmol = 34 %) der Titelverbindung als hellgelbes Öl.
   ¹H-NMR (CDCl₃):
      δ (ppm): 7,45 (t, 1H, J = 8 Hz, Pyridyl); 6,7 (d, 1H, J = 8 Hz, Pyridyl); 6,6 (d, 1H, J = 8 Hz, Pyridyl); 4,95 (s, 2H, O-CH₂); 4,1 (t, 2H, J = 5Hz, O-CH₂); 3,8 (s, 3H, OCH₃); 3,7 (s, 3H, OCH₃); 2,7 (q, 2H, J = 8 Hz, CH₂); 2,3 (t, 2H, J - 6 Hz, CH₂); 1,9 (m, 2H, CH₂); 1,3 (t, 3H, J - 8 Hz, 3H)
d) 6-(6'-Ethylpyridyl-2'-oxymethyl)-2,3-dihydropyranyl-5-glyoxylsäuremethylester-trans-O-methyloxim (Tabelle 7, Nr. 25 b)
   8 g (26 mmol) 6-(6'-Ethylpyridyl-2'-oxymethyl)-2,3-dihydropyranyl-5-glyoxylsäuremthylester-cis-O-methyloxim (Beispiel 8 c) in 50 ml Methylenchlorid wird in einer offenen Kristallisierschale mit 2 ml 2n Salzsäuregaslösung in Ether versetzt und während ca. 5 Stunden von oben mit einer 300 W UV-Lampe bestrahlt. Verdampftes Lösungsmittel wird von Zeit zu Zeit ersetzt. Anschließend wird die Reaktionslösung in einen Kolben überführt und eingedampft. Der Rückstand wird säulenchromatographisch mit Hexan/Essigester-Gemischen gereinigt. Man erhält 2,8 g Ausgangsmaterial und 1,8 g (5,9 mmol = 23 %) der Titelverbindung als hellgelbes Öl.
   ¹H-NMR (CDCl₃):
      δ (ppm): 7,45 (t, 1H, J - 8 Hz, Pyridyl); 6,7 (d, 1H, J - 8 Hz, Pyridyl); 6,55 (d, 1H, J - 8 Hz, Pyridyl); 4,7 (s, 2H, OCH₂); 4,15 (t, 2H, J = 5 Hz, OCH₂); 4,0 (s, 3H, OCH₃); 3,8 (s, 3H, OCH₃); 2,65 (q, 2H, J = 8 Hz, CH₂); 2,2 (t, breit, CH₂); 1,95 (m, 2H, CH₂); 1,25 (t, 3H, J = 8 Hz, CH₃)

### Beispiel 9

### 6-[1'-(4''-Bromphenyl)-1'-methyl-iminooxymethyl-4']-2,3dihydropyranyl-5-glyoxylsäuremethylester-trans-O-methyloxim (Tabelle 7, Nr. 26)

a) 6-[1'-(4''-Bromphenyl)-1'-methyl-iminooxymethyl-4']-2,3-dihydropyran
   46 g (0,215 mol) 4-Bromacetophenonoxim in 300 ml Dimethylformamid wird portionsweise mit 6 g (0,25 mol) Natriumhydrid versetzt. Man rührt ca. 1 Stunde bei Raumtemperatur und tropft anschließend unter leichter Kühlung 41,5 g (0,213 mol) 6-Hydroxymethyl-2,3-dihydropyran-methansulfonsäureester (Beispiel 7 b) zu. Man rührt über Nacht bei Raumtemperatur, gießt die Reaktionsmischung auf Wasser und extrahiert die wässrige Phase dreimal mit Diethylether. Die etherische Phase wird einmal mit Wasser gewaschen, über MgSO₄ getrocknet und eingeengt. Der Rückstand wird säulenchromatographisch mit Hexan/Essigester-Gemischen gereingt. Man erhält 53 g (0,171 mol = 80 %) der Titelverbindung als hellgelbes Öl.
   ¹H-NMR (CDCl₃):
      δ (ppm): 7,5 (m, 4H, Phenyl); 4,9 (t, 1H, J = 4 Hz, Vinyl); 4,55 (s, 2H, OCH₂); 4,05 (t, 2H, J = 5 Hz, O-CH₂); 2,25 (s, 3H, CH₃); 2,05 (m, 2H, CH₂); 1,85 (m, 2H, CH₂)
b) 6-[1'-(4''-Bromphenyl) -1'-methyl-iminooxymethyl-4']-2,3-dihydropyranyl-5-glyoxylsäuremethylester
   53 g (0,17 mol) 6-[1'-(4''-Bromphenyl)-1'-methyl-iminooxymethyl-4']-2,3-dihydropyran (Beispiel 9 a) und 20 g (0,38 mol) Pyridin in 30 ml Methylenchlorid werden tropfenweise mit 28 g (0,23 mol) Oxalsäuremethylesterchlorid versetzt. Man rührt über Nacht bei Raumtemperatur, gießt die Reaktionsmischung auf Wasser und extrahiert die wäßrige Phase dreimal mit Diethylether. Die vereinigten etherischen Phase werden über MgSO₄ getrocknet und eingeengt. Der Rückstand wird säulenchromatographisch mit Hexan/Essigester-Gemischen gereinigt. Man erhält 25,7 g (0,065 mol - 38 %) der Titelverbindung als gelbes Öl.
   ¹H-NMR (CDCl₃):
      δ (ppm): 7,5 (m, 4H, Phenyl); 5,0 (s, 2H, OCH₂); 4,2 (t, 2H, J - 5 Hz, OCH₂); 3,8 (s, 3H, OCH₃); 2,4 (t, 2H, J = 6 Hz, CH₂); 2,9 (m, 2H, CH₂)
c) 6-[1'-(4''-Bromphenyl) -1'-methyl-iminooxymethyl-4']-2,3-dihydropyranyl-5-glyoxylsäuremethylestercis-O-methyloxim (Tabelle 7,Nr. 26 a)
   25,7 g (65 mmol) 6-[1'-(4''-Bromphenyl)-1'-methyliminooxymethyl-4']-2,3-dihydropyranyl-5-glyoxylsäuremethylester (Beispiel 9 b), 10 g (127 mmol) Pyridin und 8,5 g (100 mmol) O-Methylhydroxylamin-hydrochlorid in 200 ml Methanol werden über Nacht bei Raumtemperatur gerührt. Anschließend engt man die Reaktionsmischung i. Vak. ein und nimmt den Rückstand in Methylenchlorid auf. Man extrahiert die organische Phase mit Wasser, trocknet über MgSO₄ und engt ein. Der Rückstand wird säulenchromatographisch mit Hexan/Essigester-Gemischen gereinigt.
   Man erhält 7,3 g (17 mmol - 26 %) der Titelverbindung als farblosen Festkörper.
   Fp - 87 - 89°C
   ¹H-NMR (CDCl₃):
      δ (ppm): 7,55 (d, 2H, J - 8 Hz, Phenyl); 7,45 (d, 2H, J - 8 Hz, Phenyl); 4,8 (s, 2H, OCH₂); 4,1 (t, 2H, J = 5 Hz, OCH₂); 3,8 (s, 3H, OCH₃); 2,3 (t, 2H, J = 6 Hz, CH₂); 2,2 (s, 3H, CH₃); 2,9 (m, 2H, CH₂)
d) 6-[1'-(4''-Bromphenyl) -1'-methyl-iminooxymethyl-4']-2,3-dihydropyranyl-5-glyoxylsäuremethylestertrans-O-methyloxim (Tabelle 7, Nr. 26 b)
   7,3 g (17 mmol) 6-[1'-(4''-Bromphenyl)-1'-methyl-iminooxymethyl-4']-2,3-dihydropyranyl-5-glyoxylsäuremethylester-cis-O-methyloxim (Beispiel 9 c) in 20 ml Methylenchlorid wird in einer offenen Kristallisierschale 8 Stunden mit einer 300 W UV-Lampe bestrahlt. Dabei wird verdampftes Lösungsmittel von Zeit zu Zeit ersetzt. Anschließend gibt man 2 ml 2n etherische Salzsäurelösung hinzu und bestrahlt weitere 4 Stunden. Dann wird die Reaktionsmischung i. Vak. eingedampft und der Rückstand säulenchromatographisch mit Hexan/Essigester-Gemischen gereinigt. Man erhält 4 g Ausgangsprodukt und 1,2 g (2,8 mmol - 17 %) der Titelverbindung als hellgelbes Öl.
   ¹H-NMR (CDCl₃):
      δ (ppm): 7,5 (m, 4H, Phenyl); 4,5 (s, 2H, OCH₂); 4,1 (t, 2H, J - 5 Hz, OCH₂); 4,0 (s, 3H, OCH₃); 3,8 (s, 3H, OCH₃); 2,2 (m, 5H, CH₂, CH₃); 1,95 (m, 2H, CH₂)

In entsprechender Weise können die in den folgenden Tabellen aufgeführten Verbindungen hergestellt werden. In den einzelnen Tabellen sind die Indizes I, II und III wie folgt zu verstehen.

In Tabelle I hat die Verbindung Nr. 1 die folgende Formel:

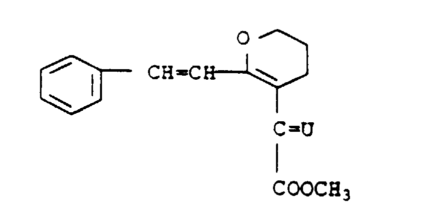

Die Verbindung aus Tabelle 1 Nr. I/1 hat die folgende Formel:

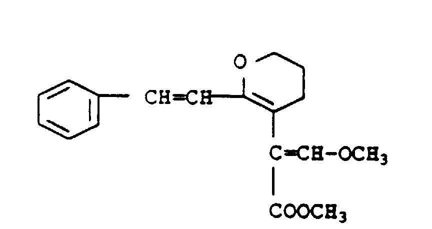

Die Verbindung Tabelle 1, Nr. II/1 hat die folgende Formel:

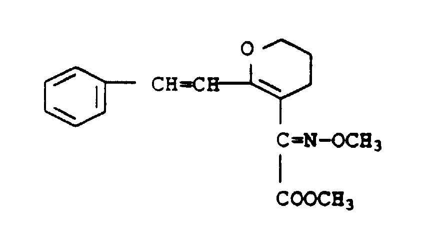

Die Verbindung aus Tabelle 1, Nr. III/1 hat die folgende Formel:

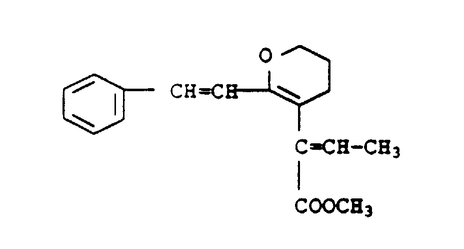

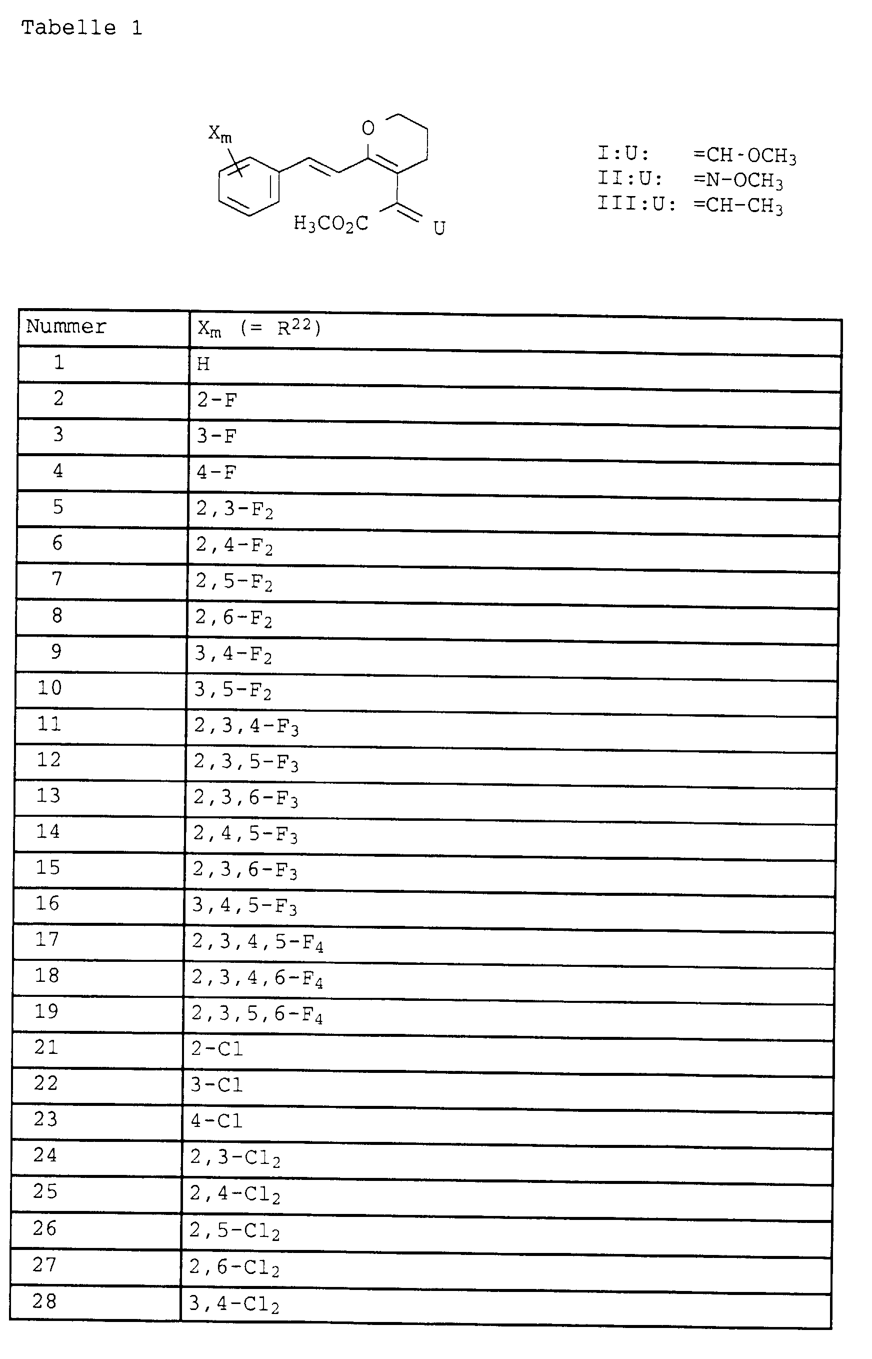

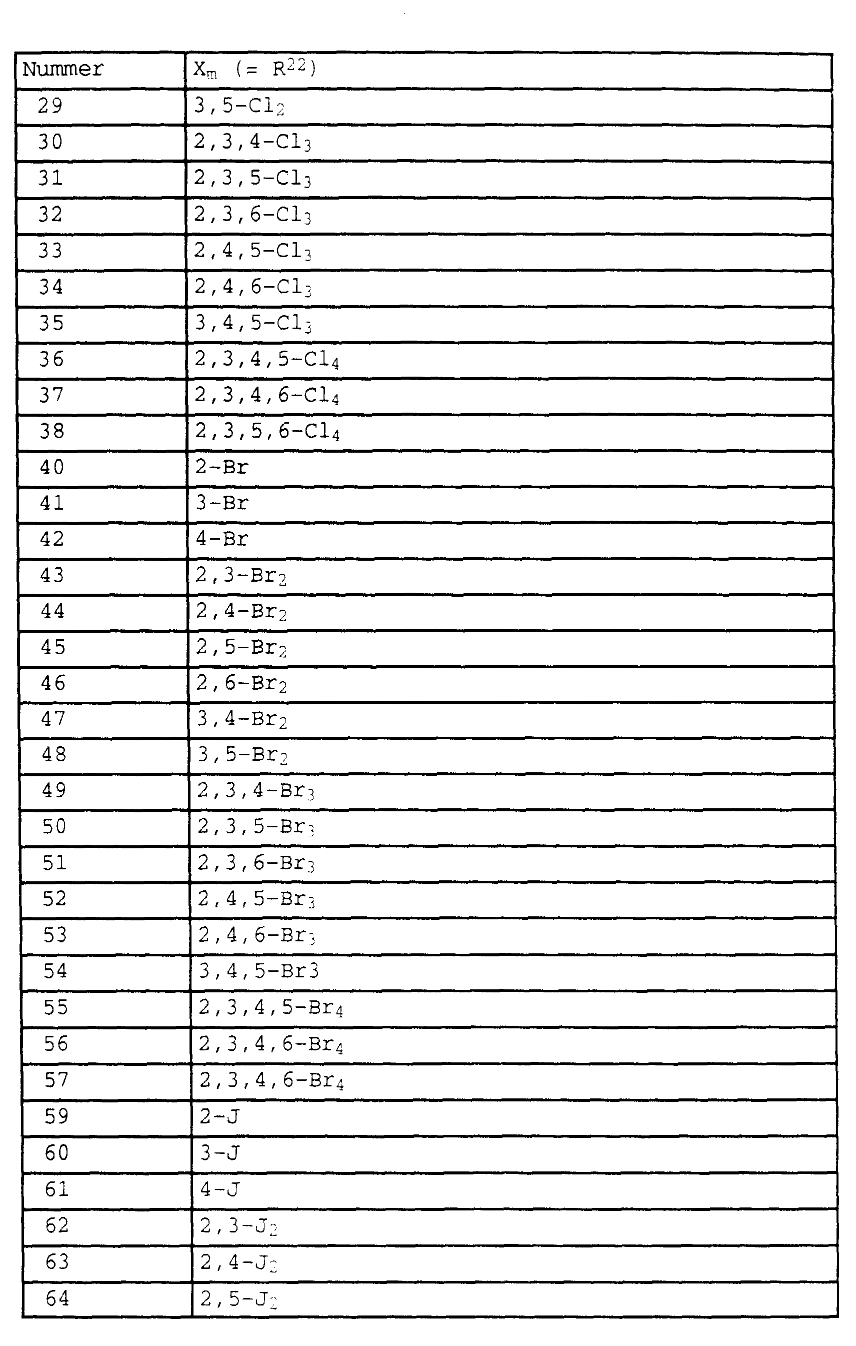

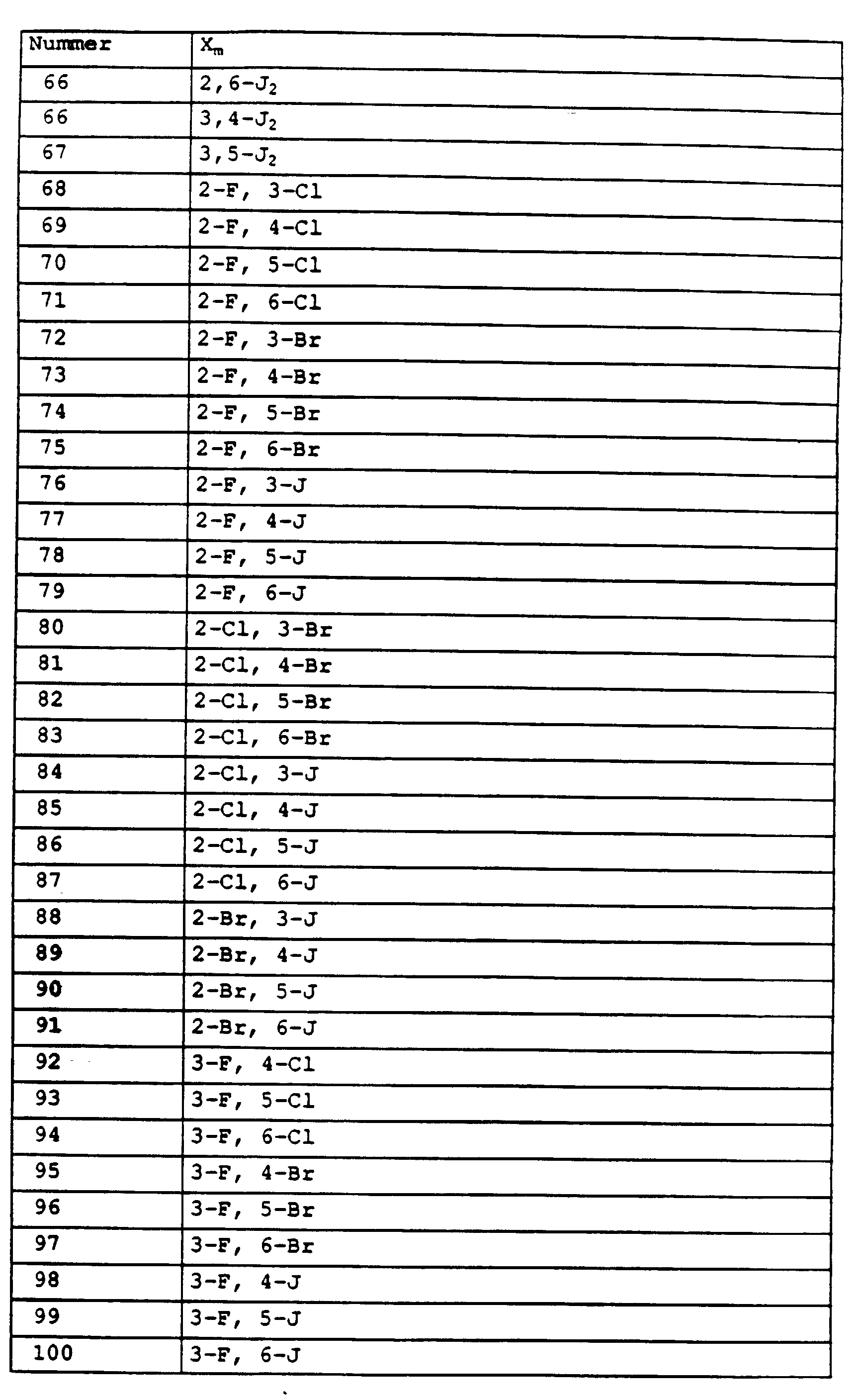

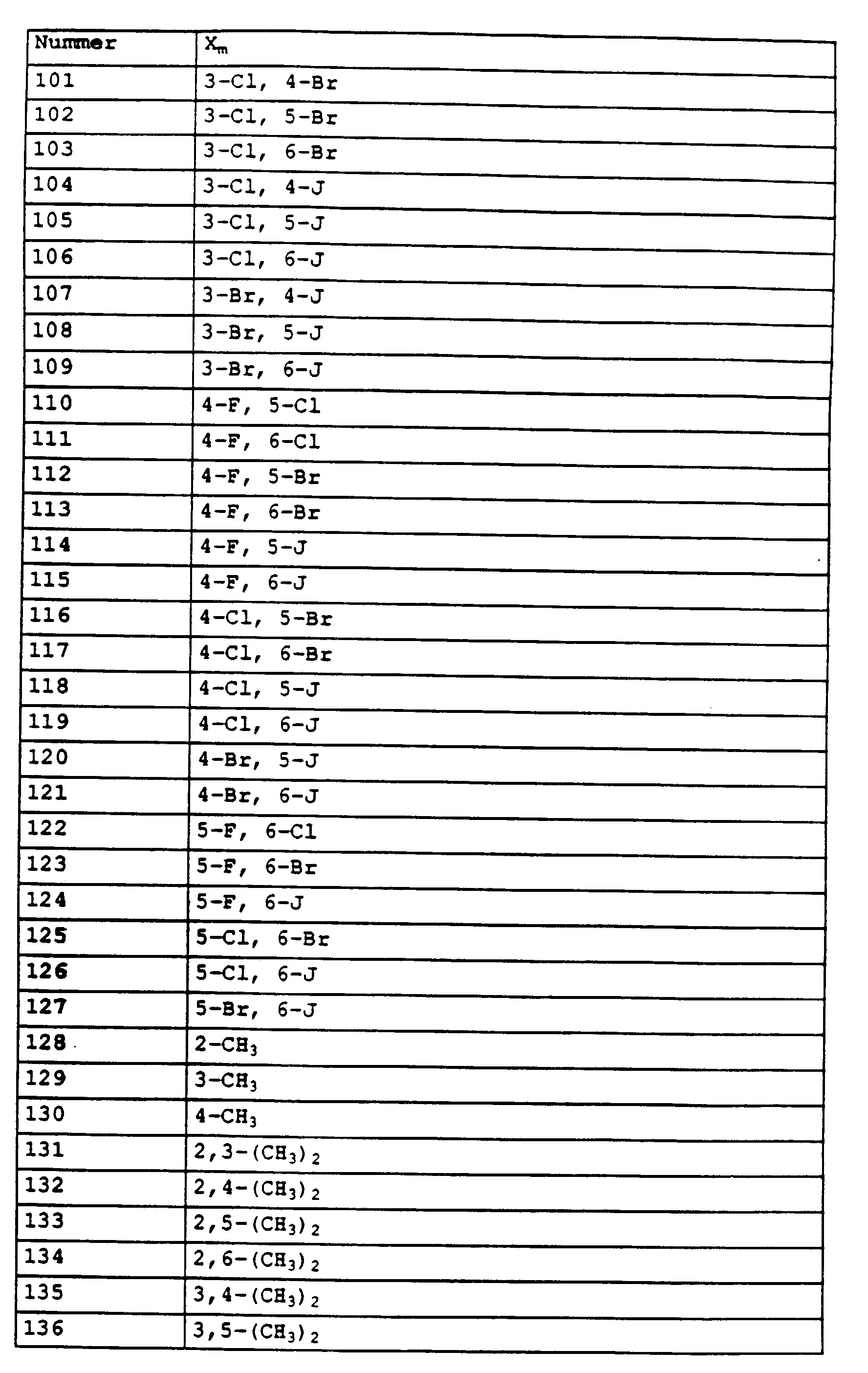

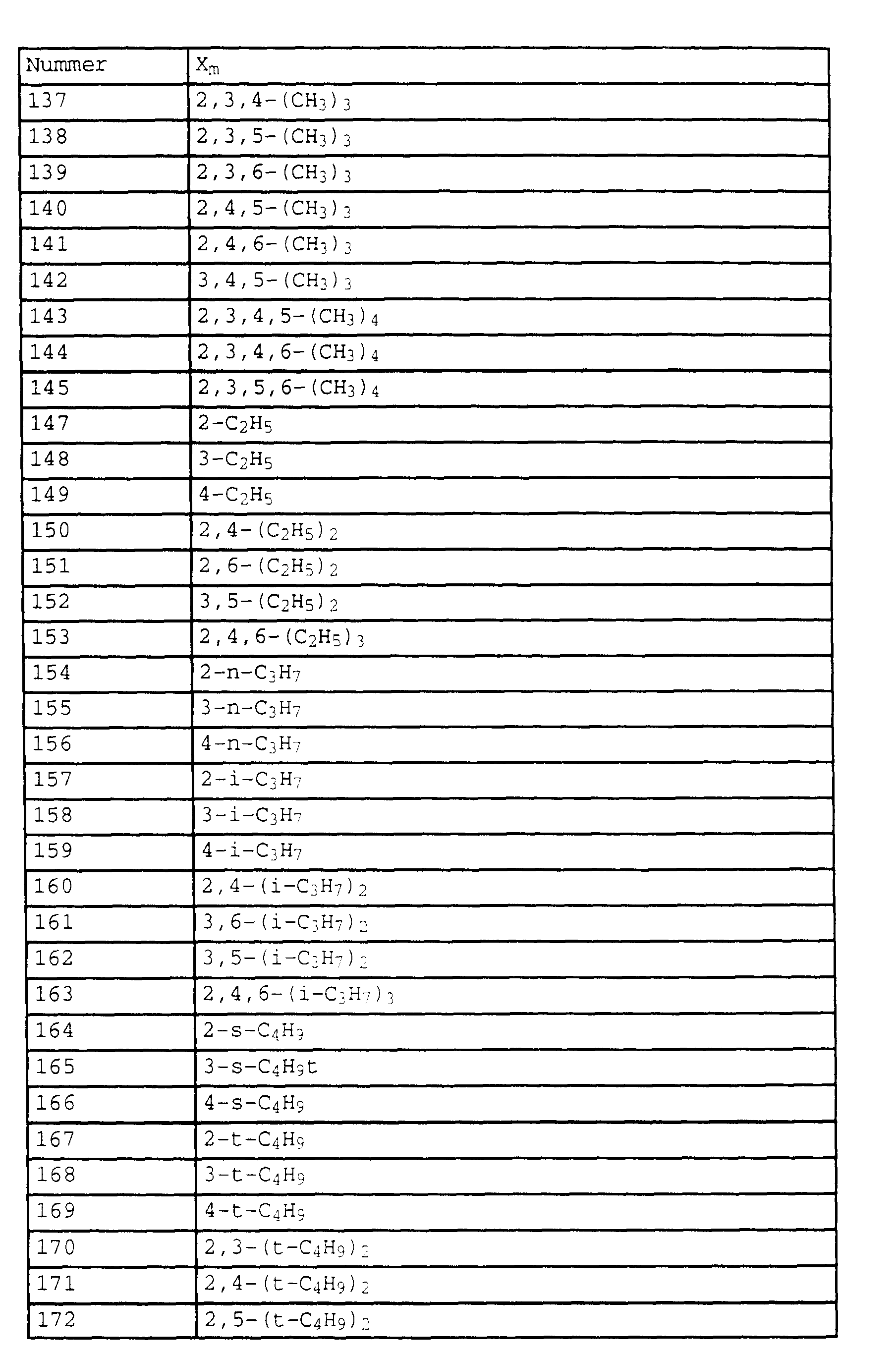

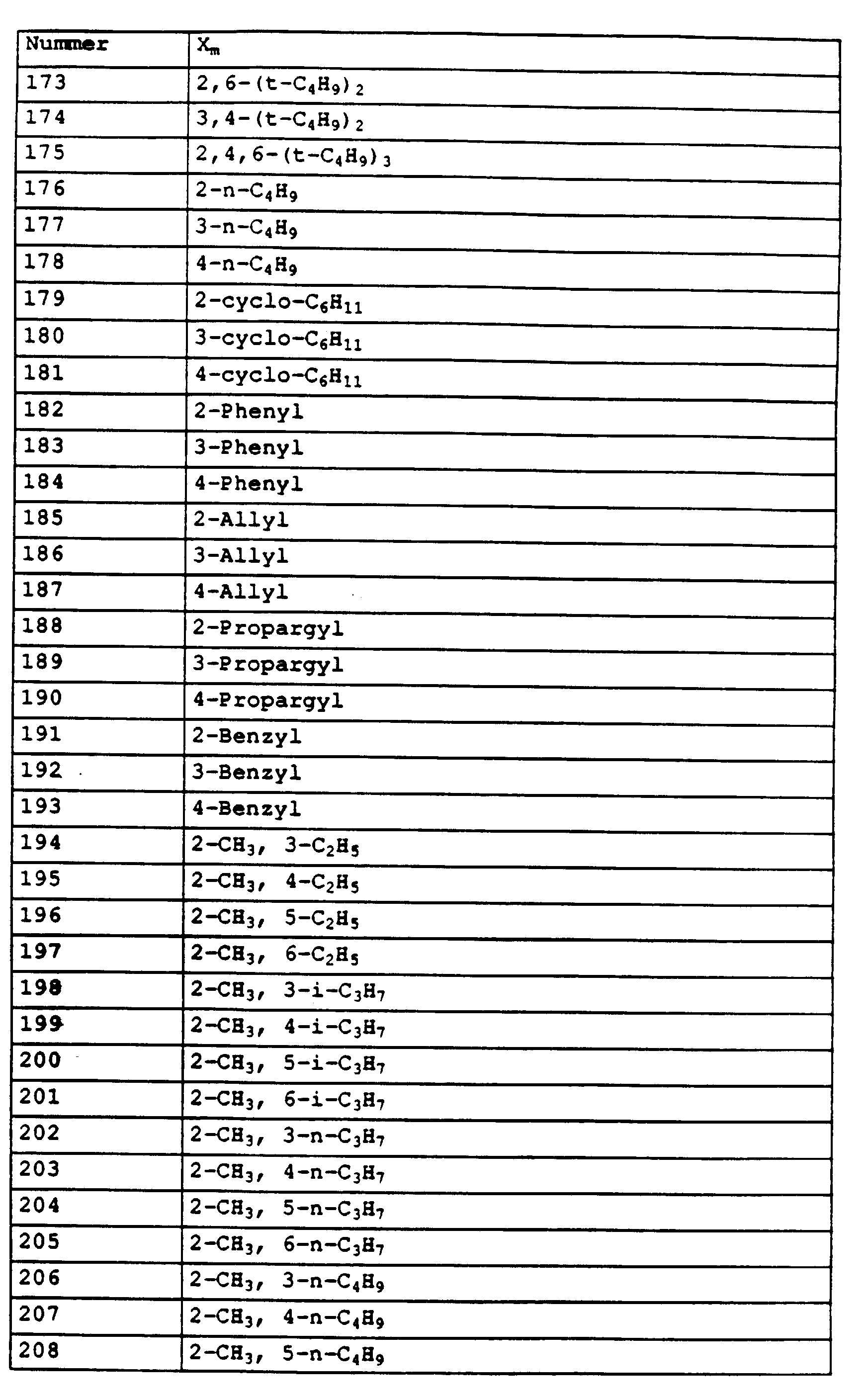

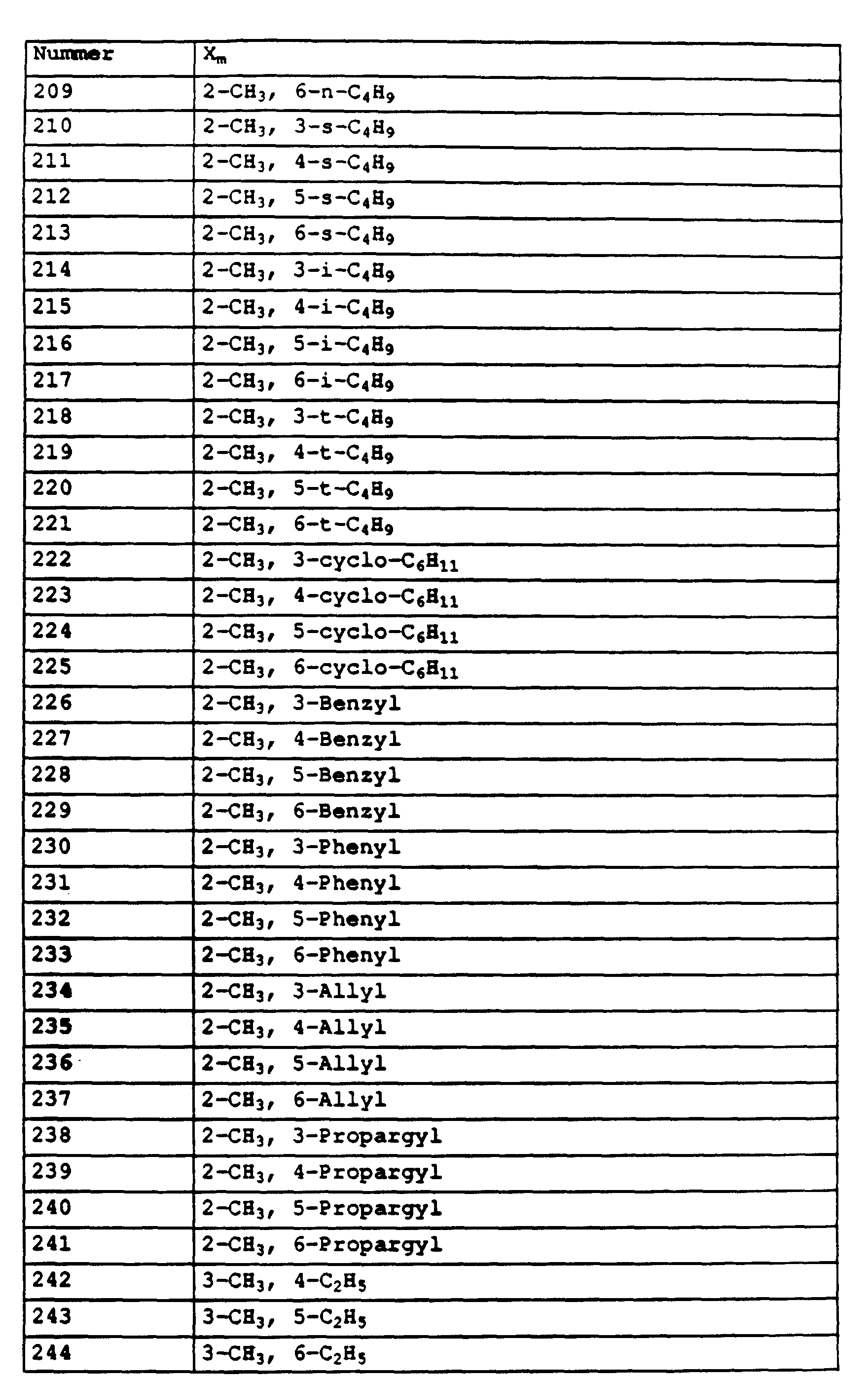

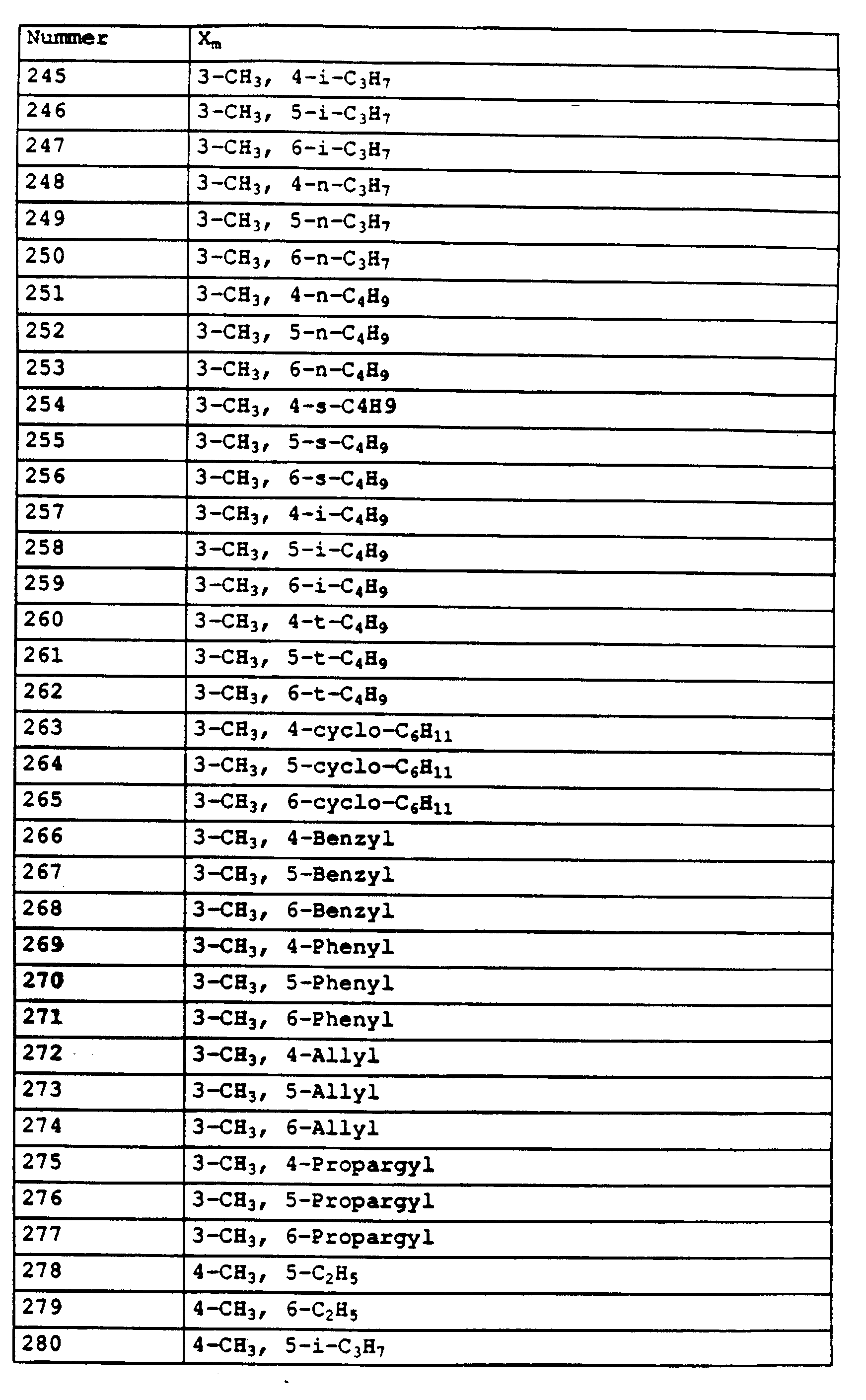

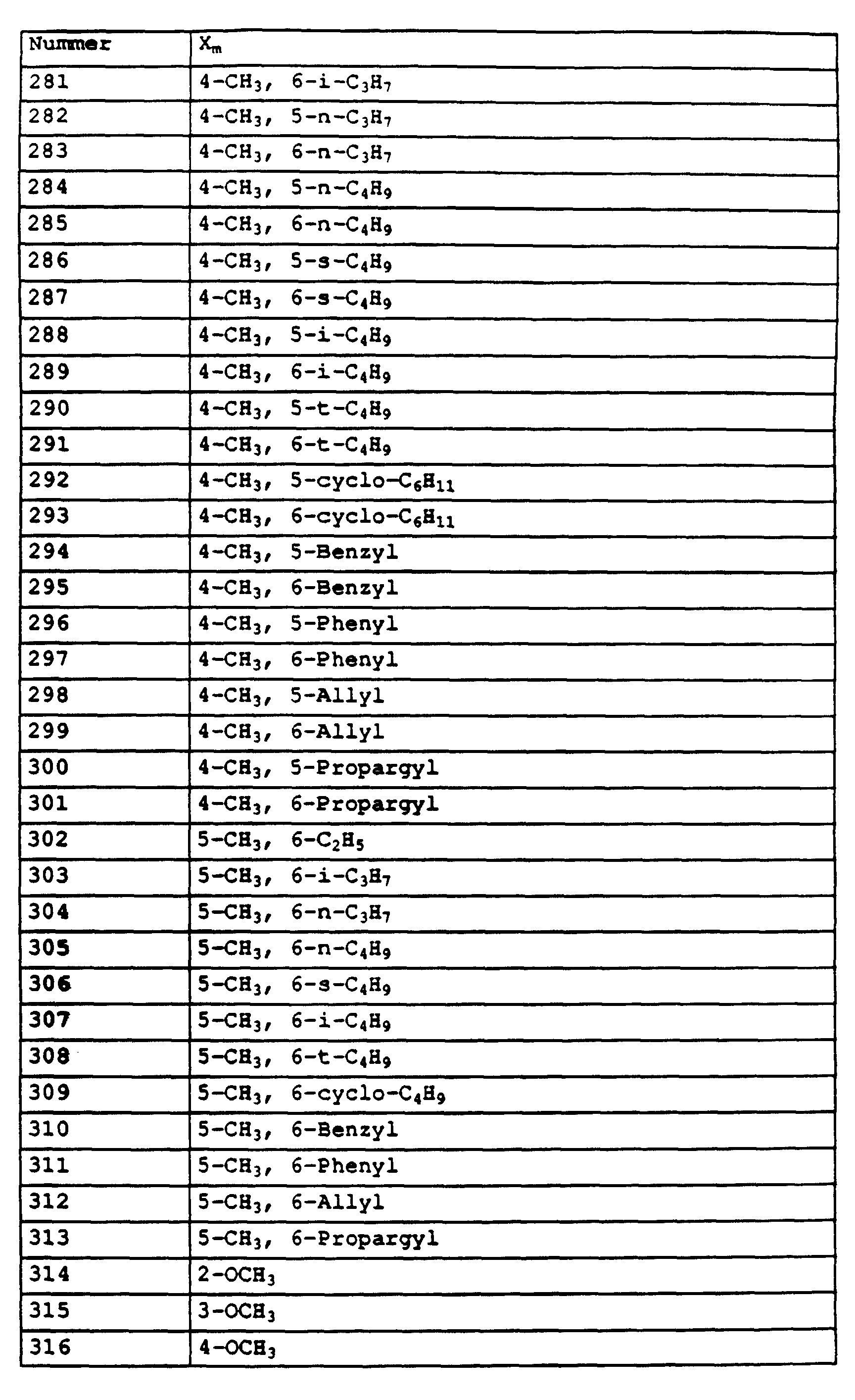

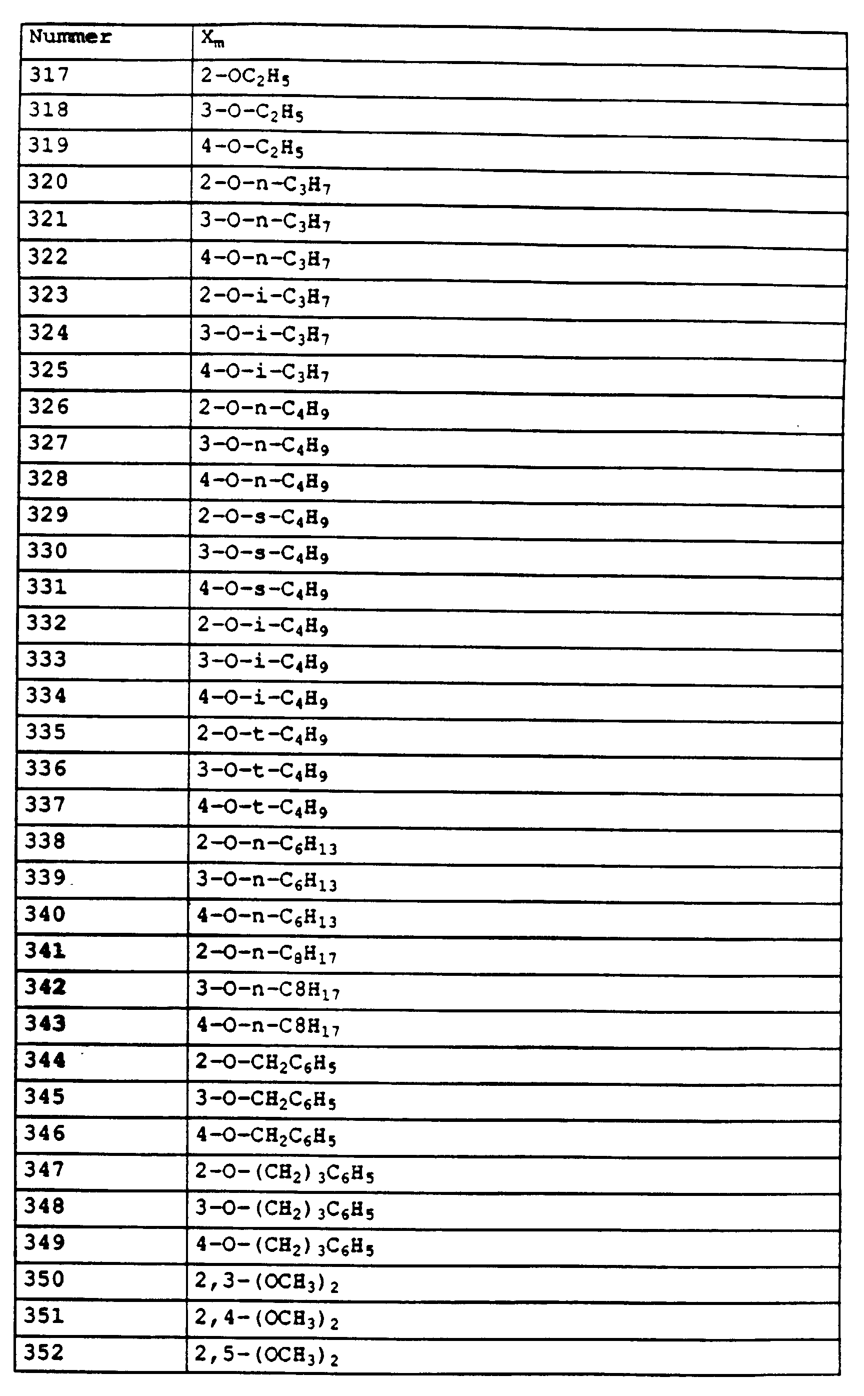

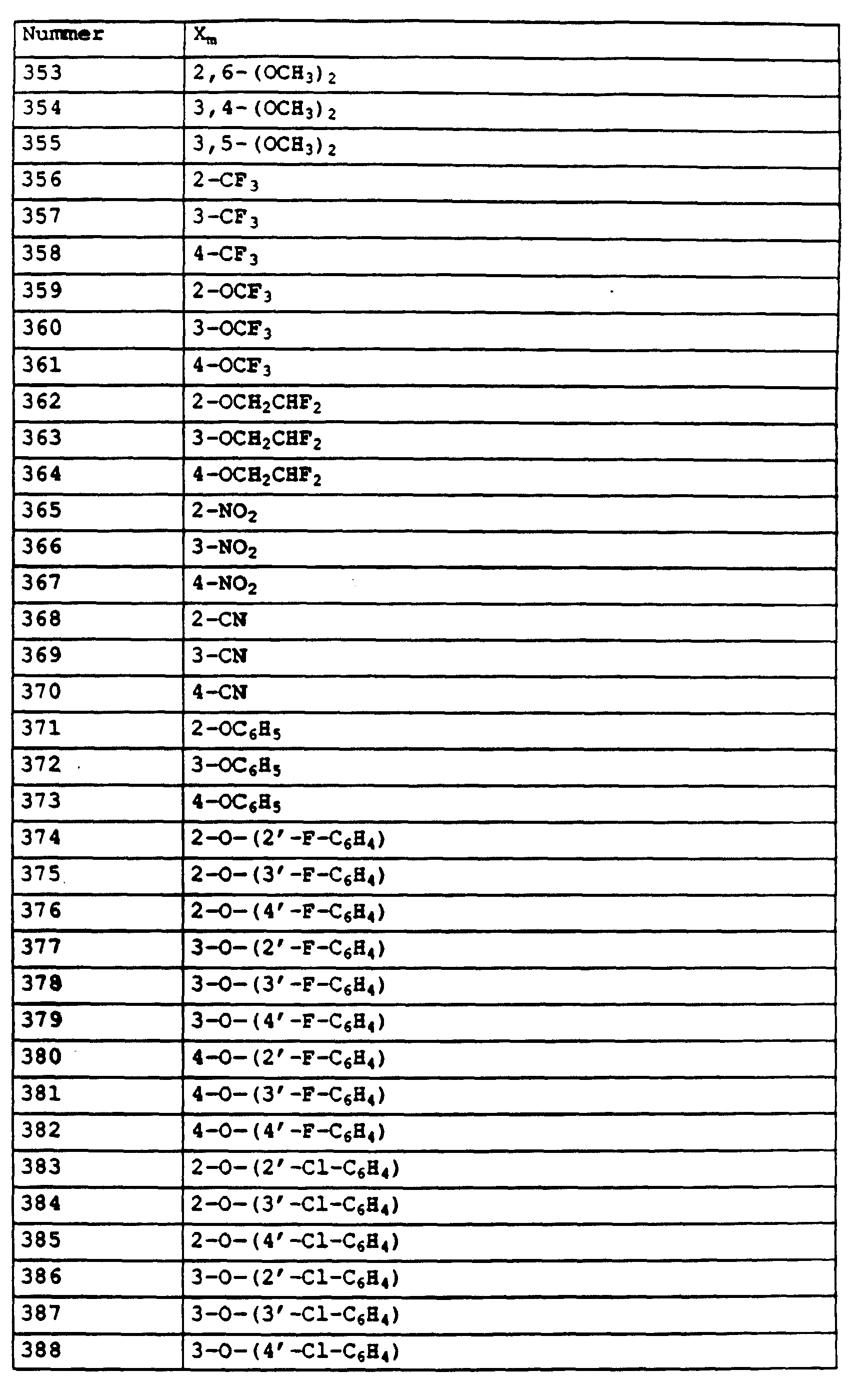

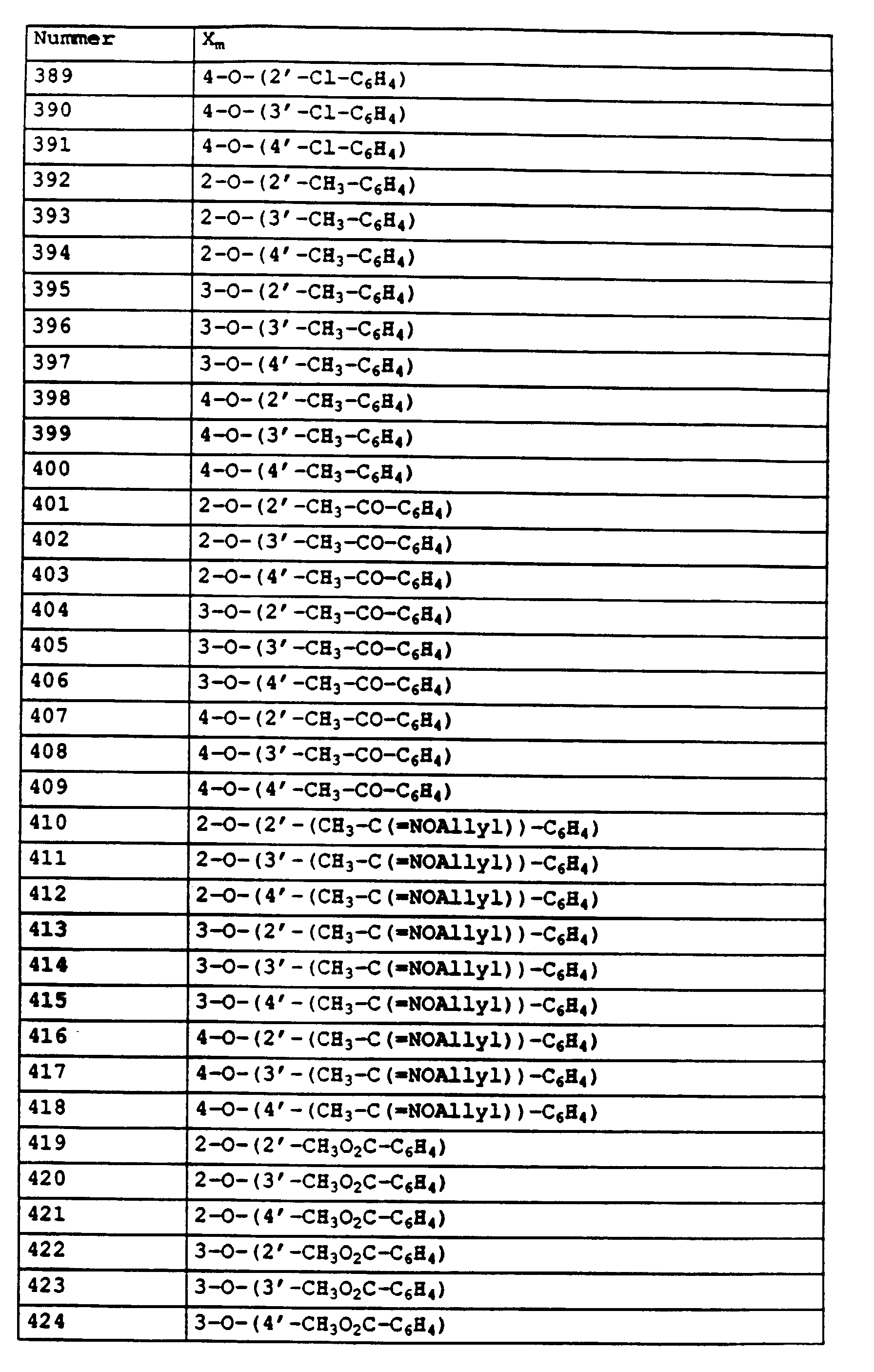

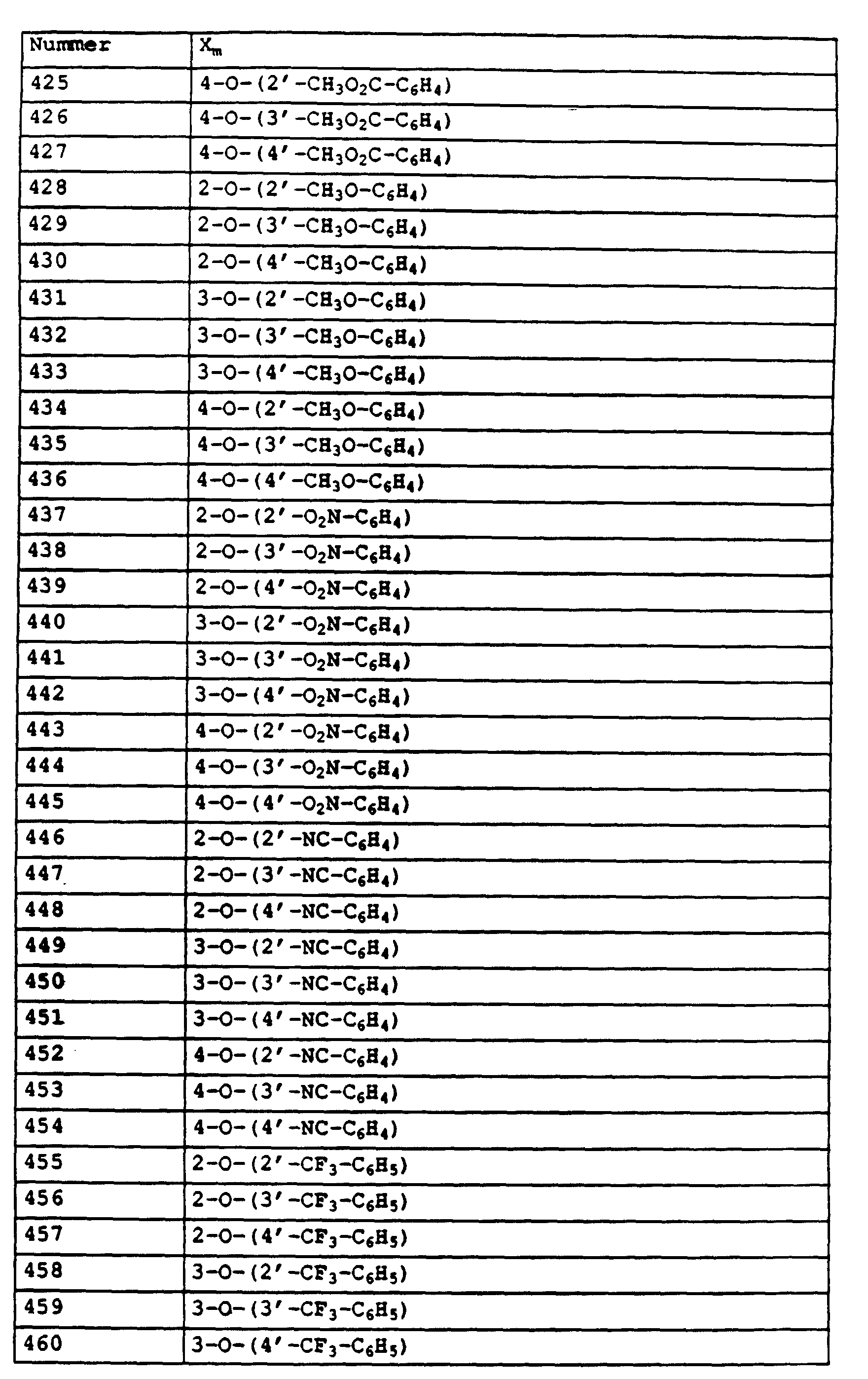

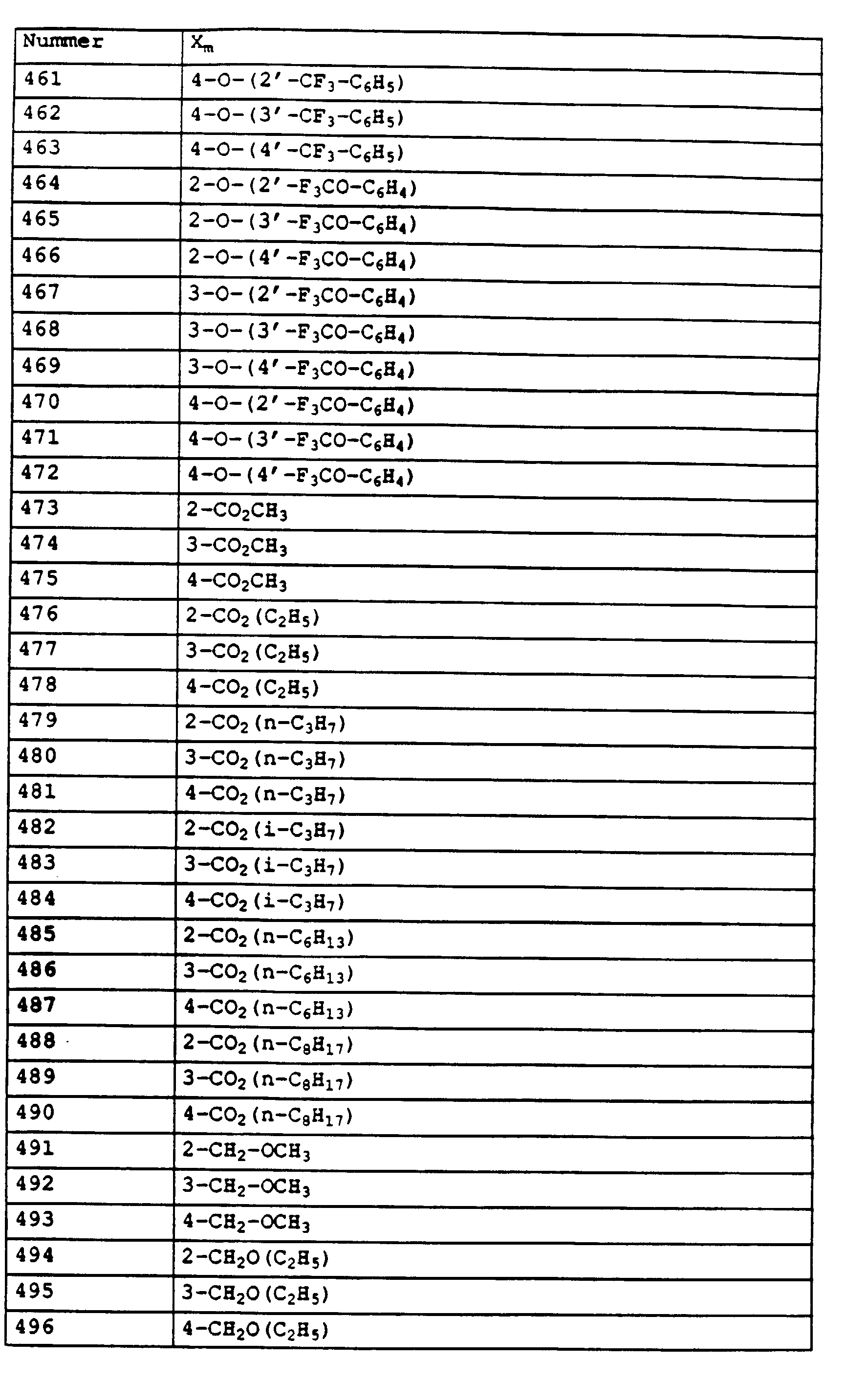

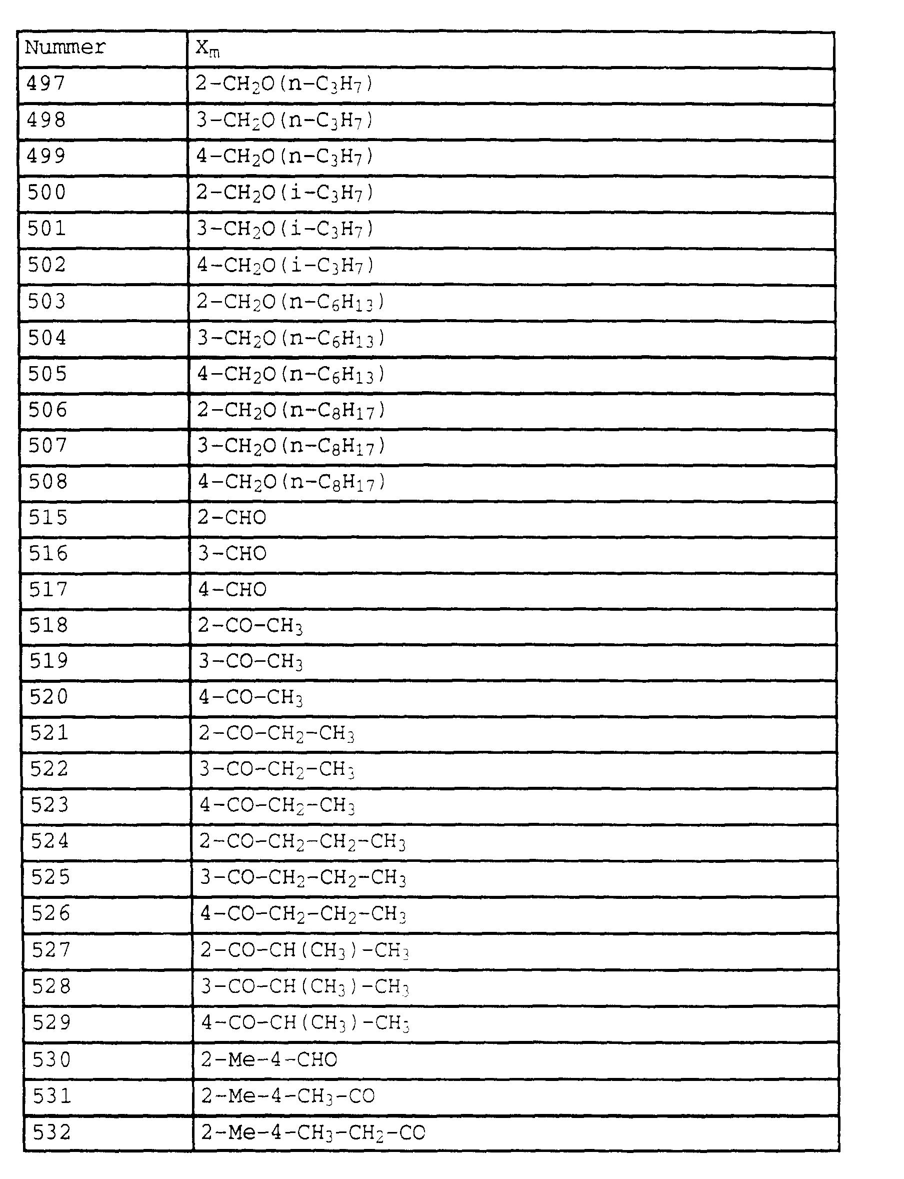

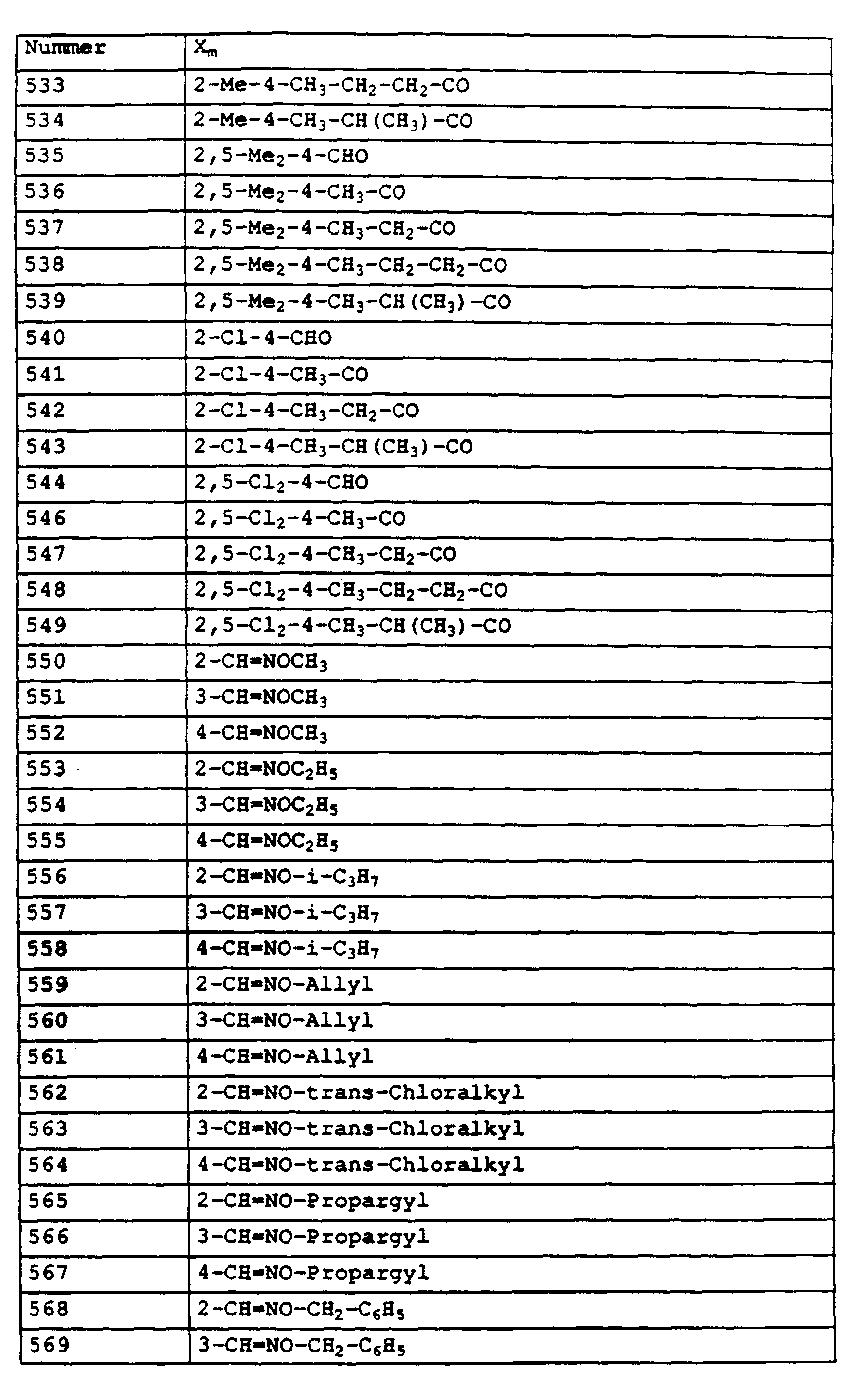

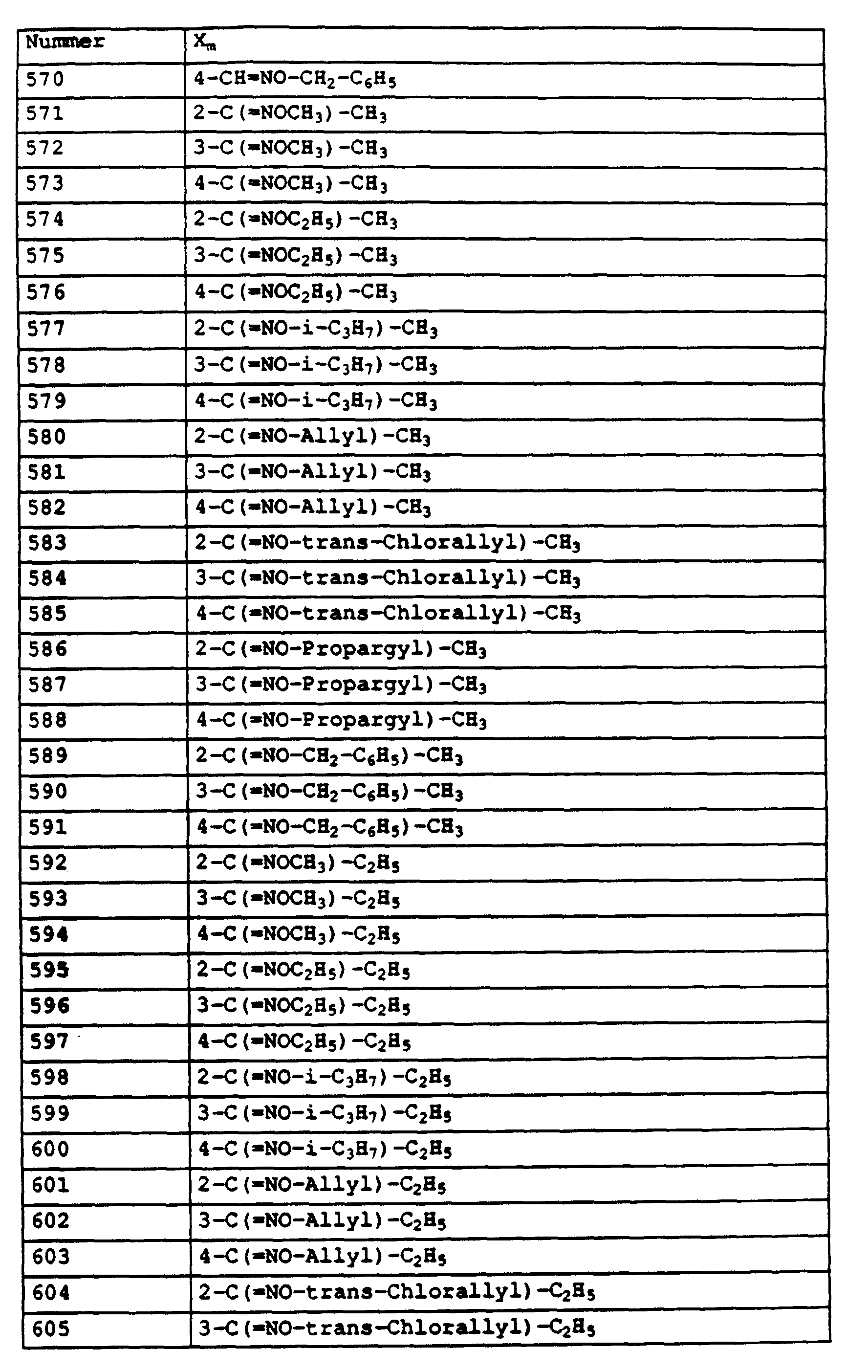

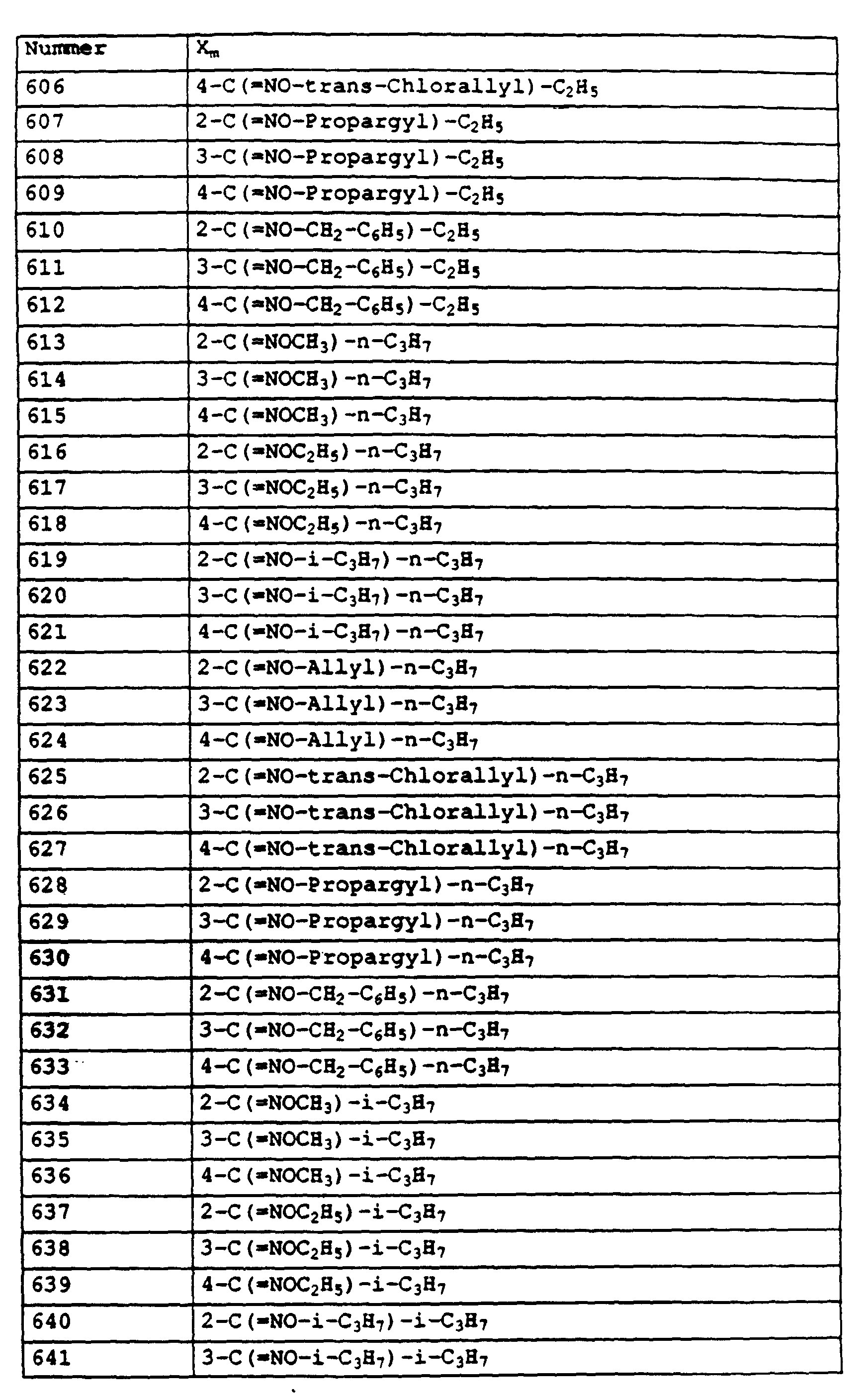

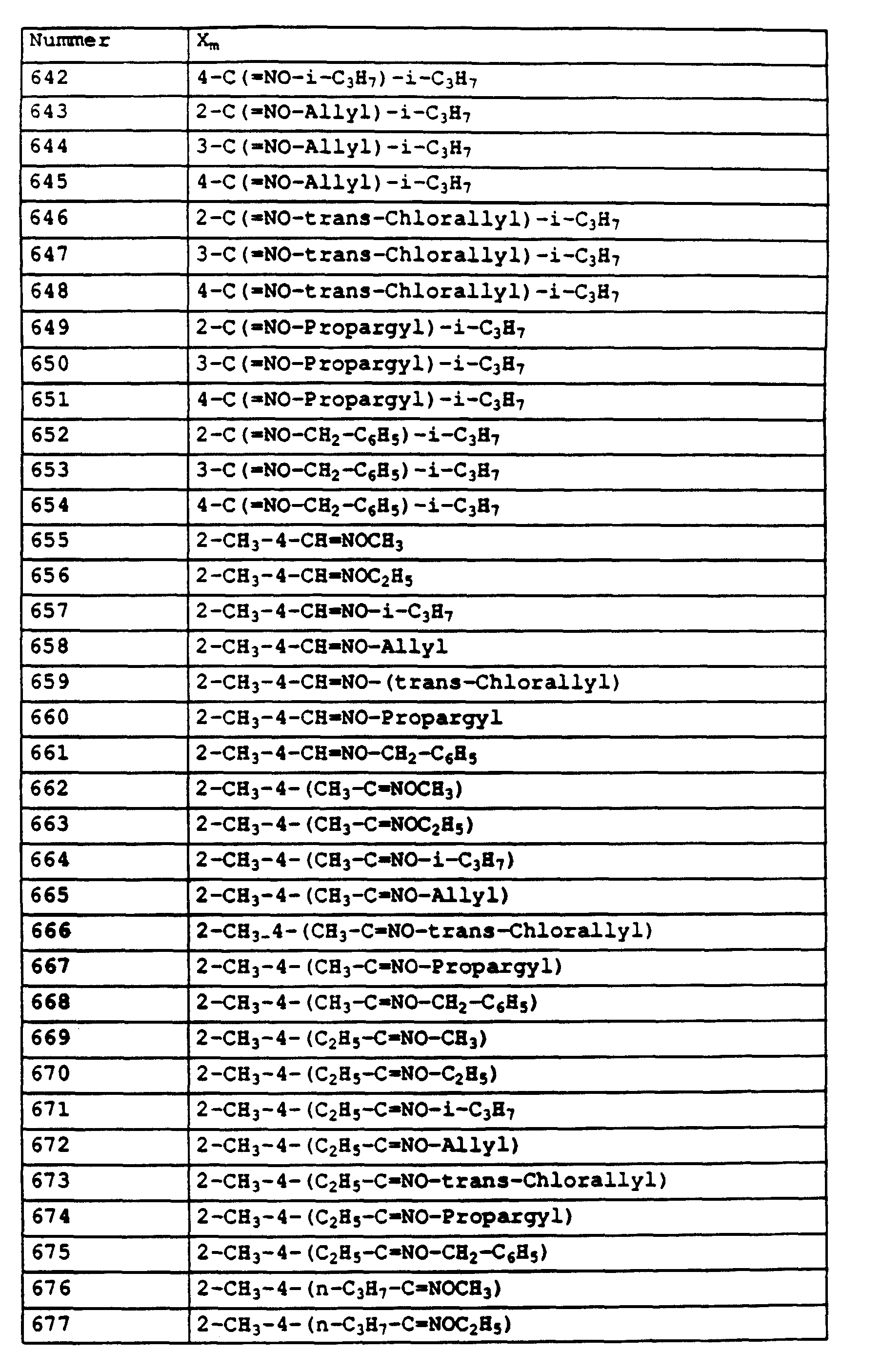

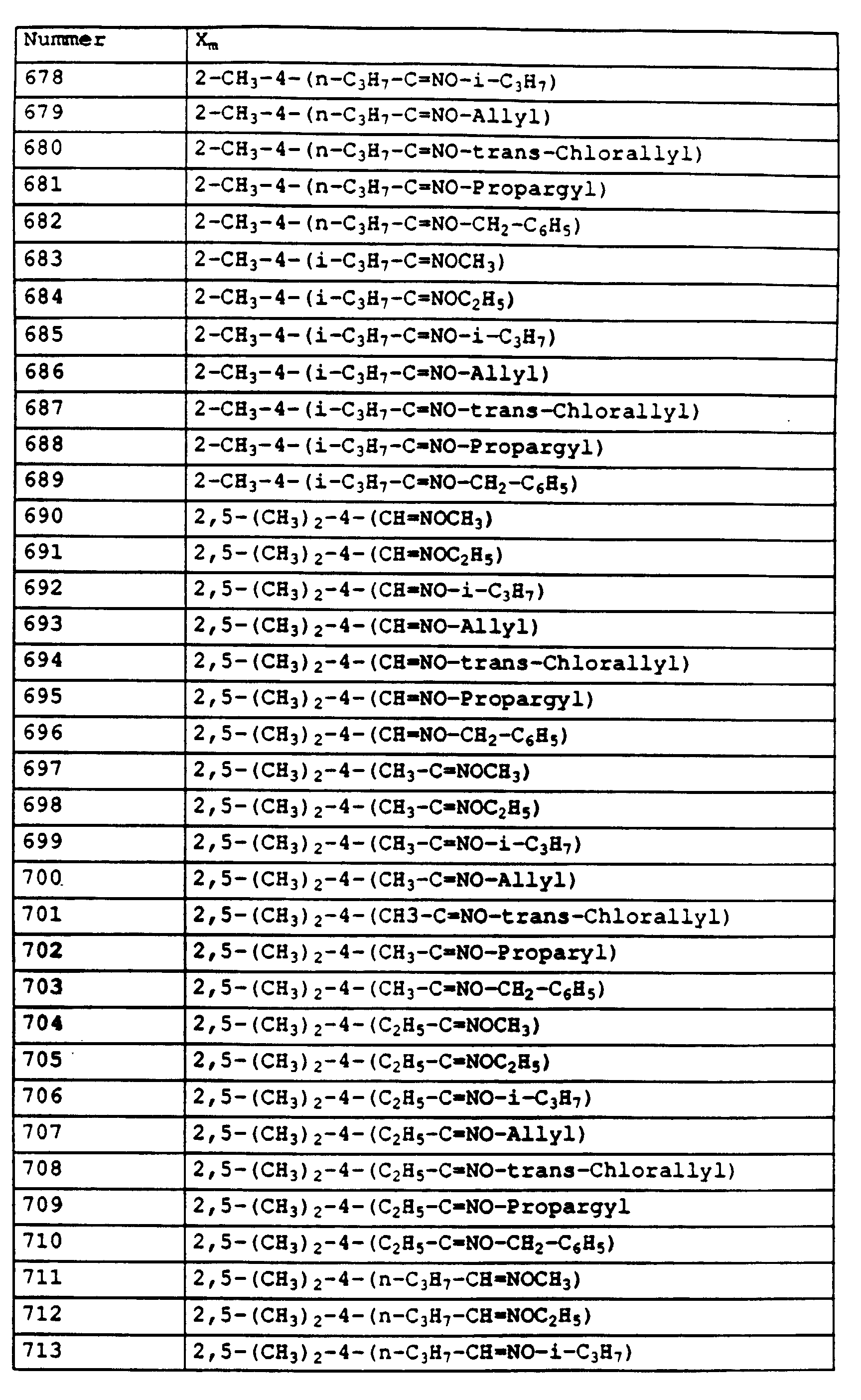

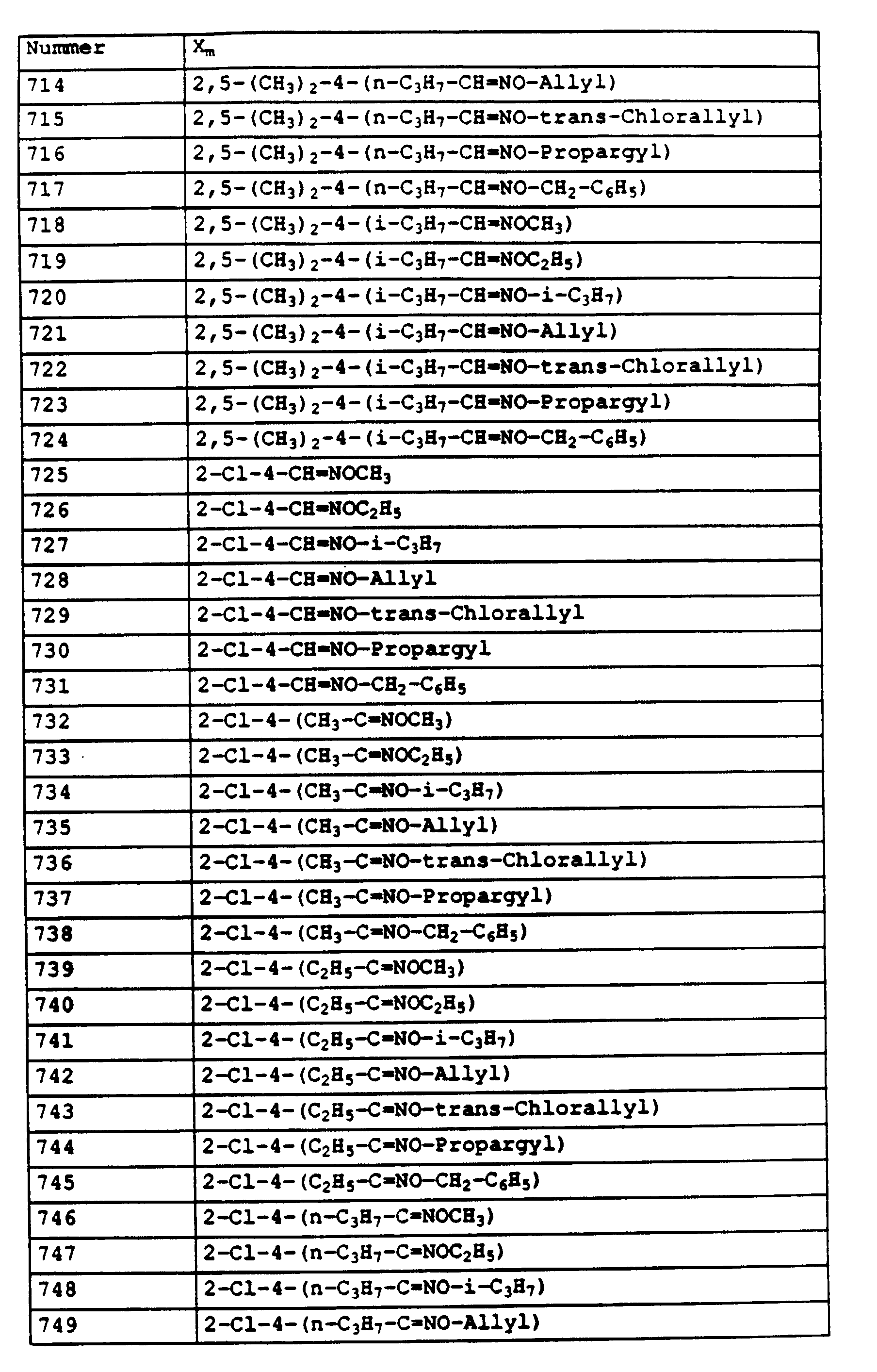

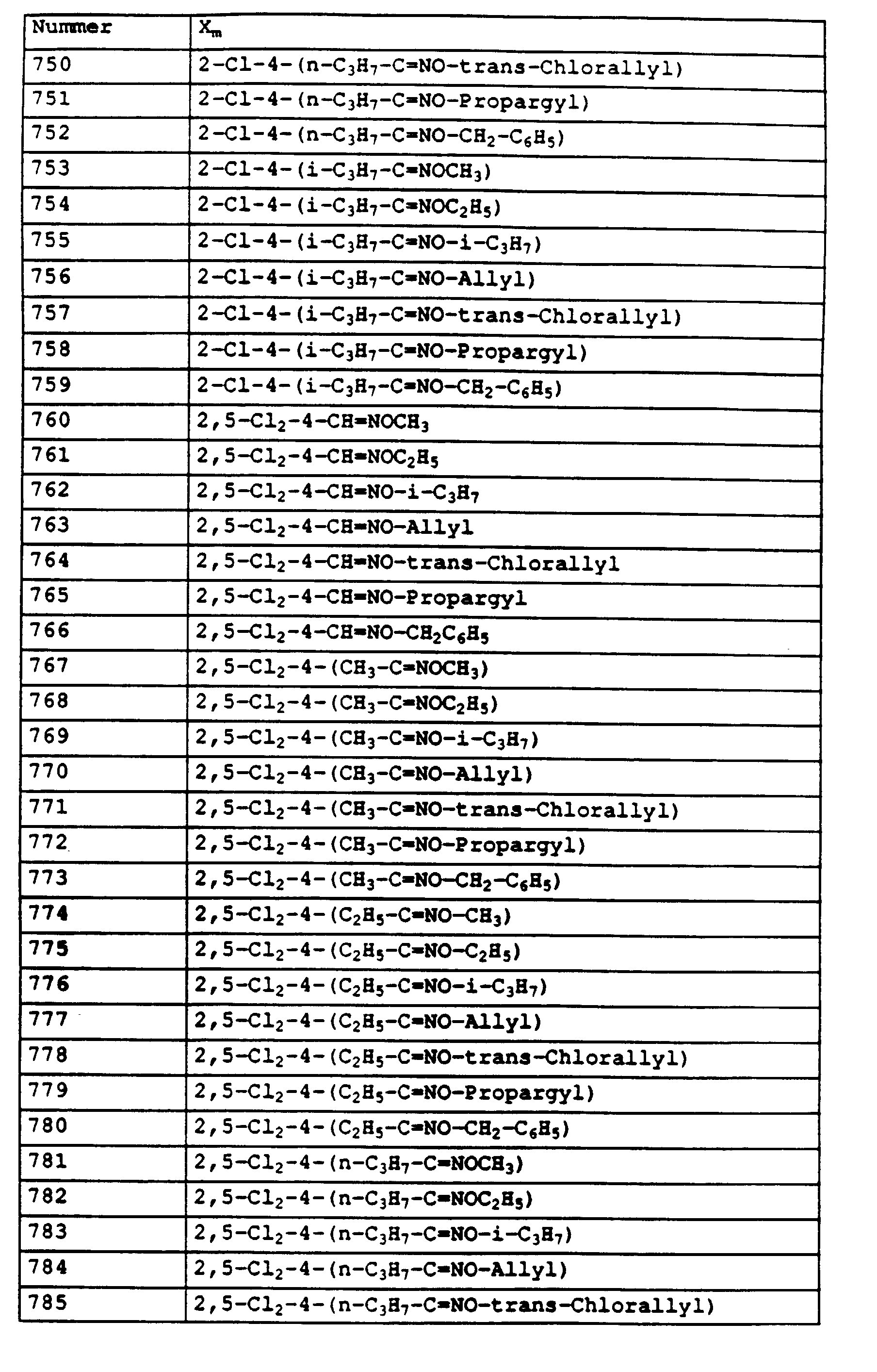

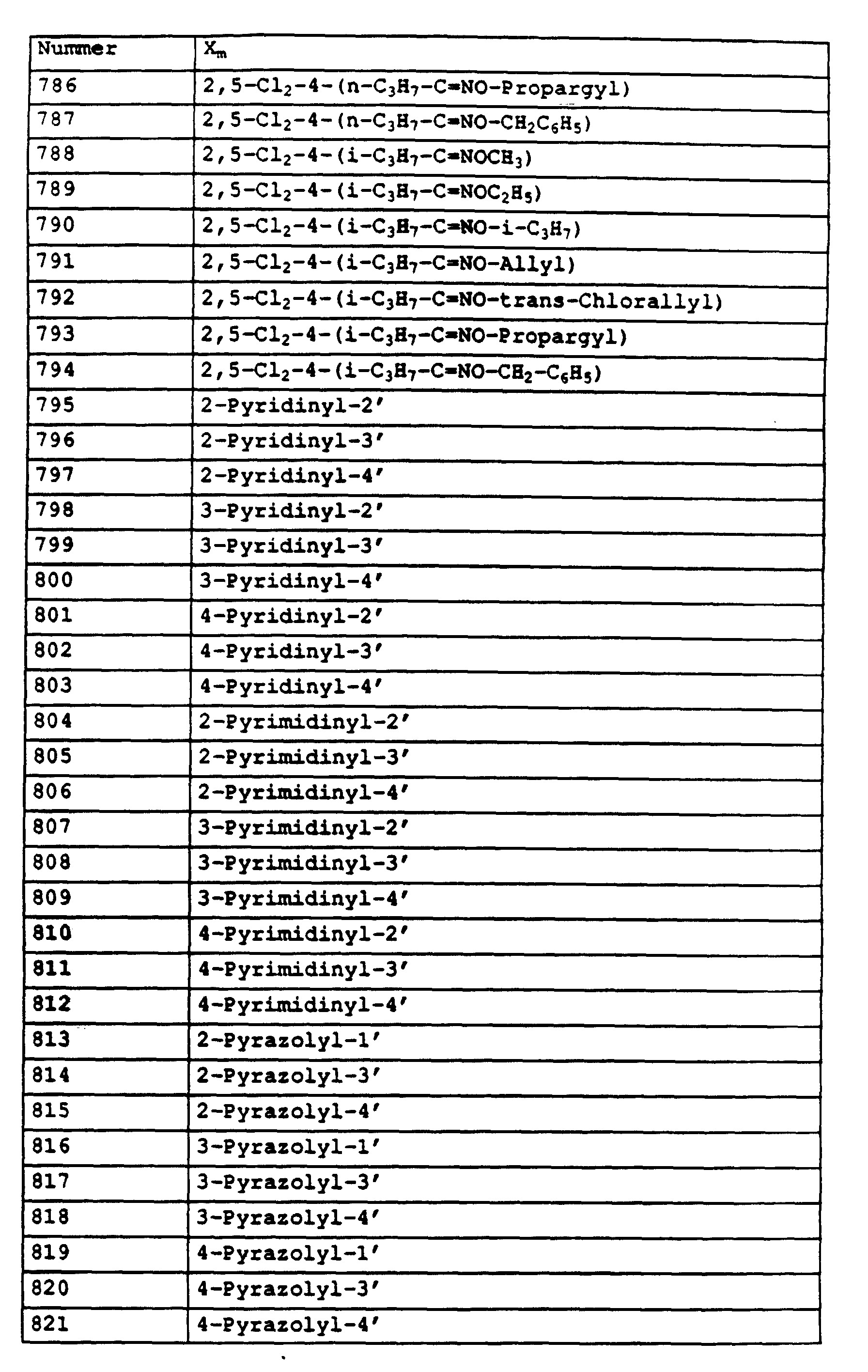

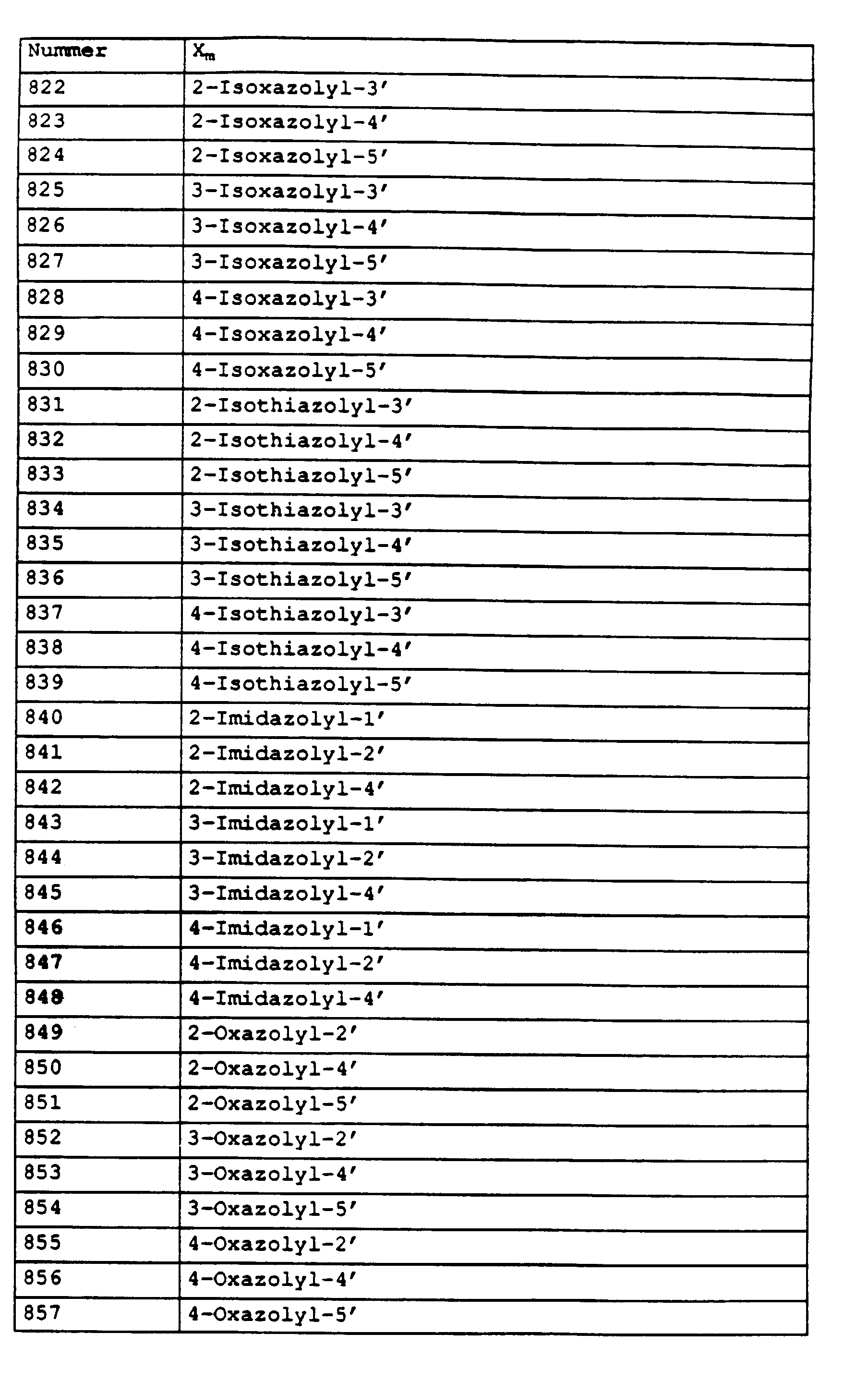

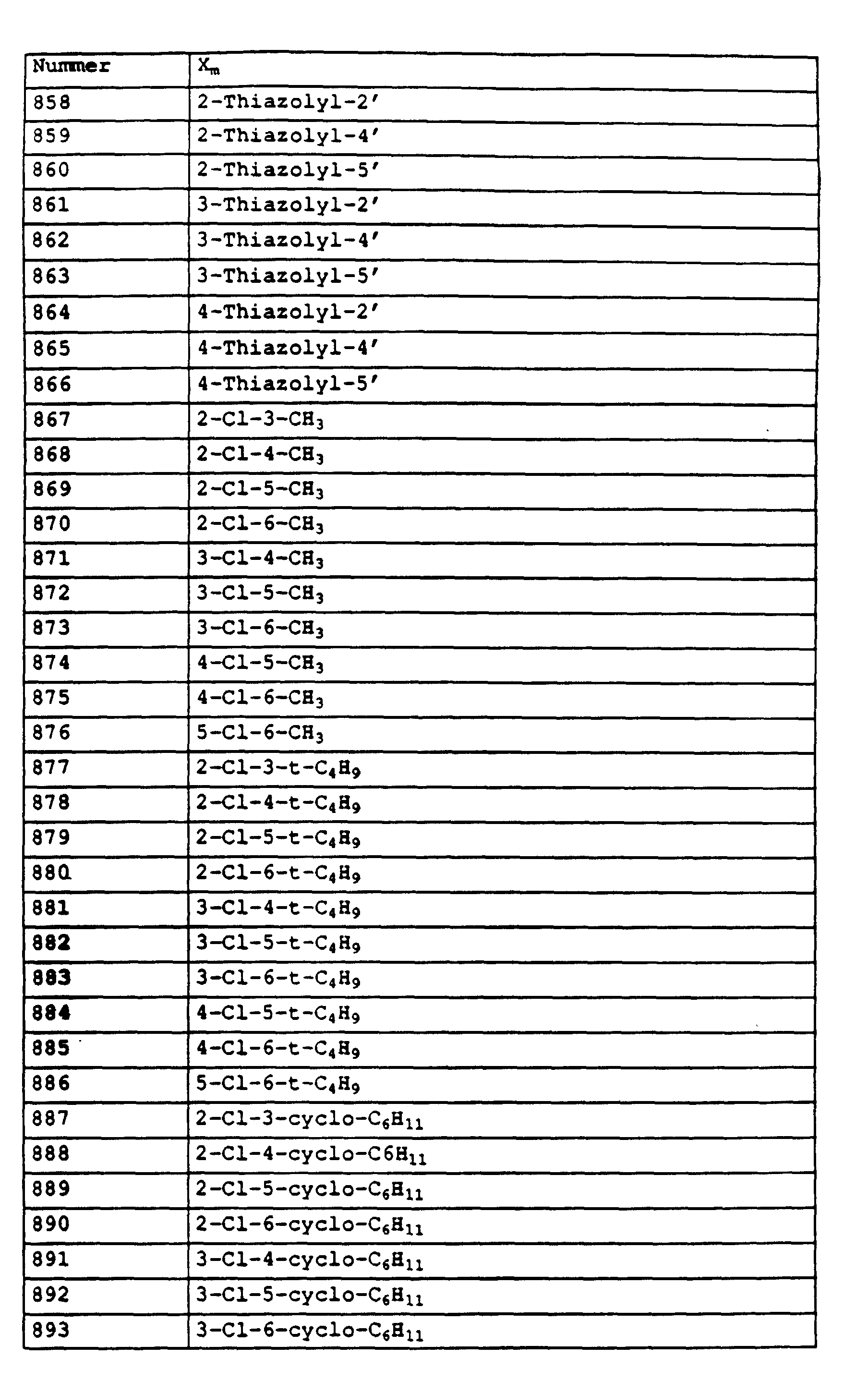

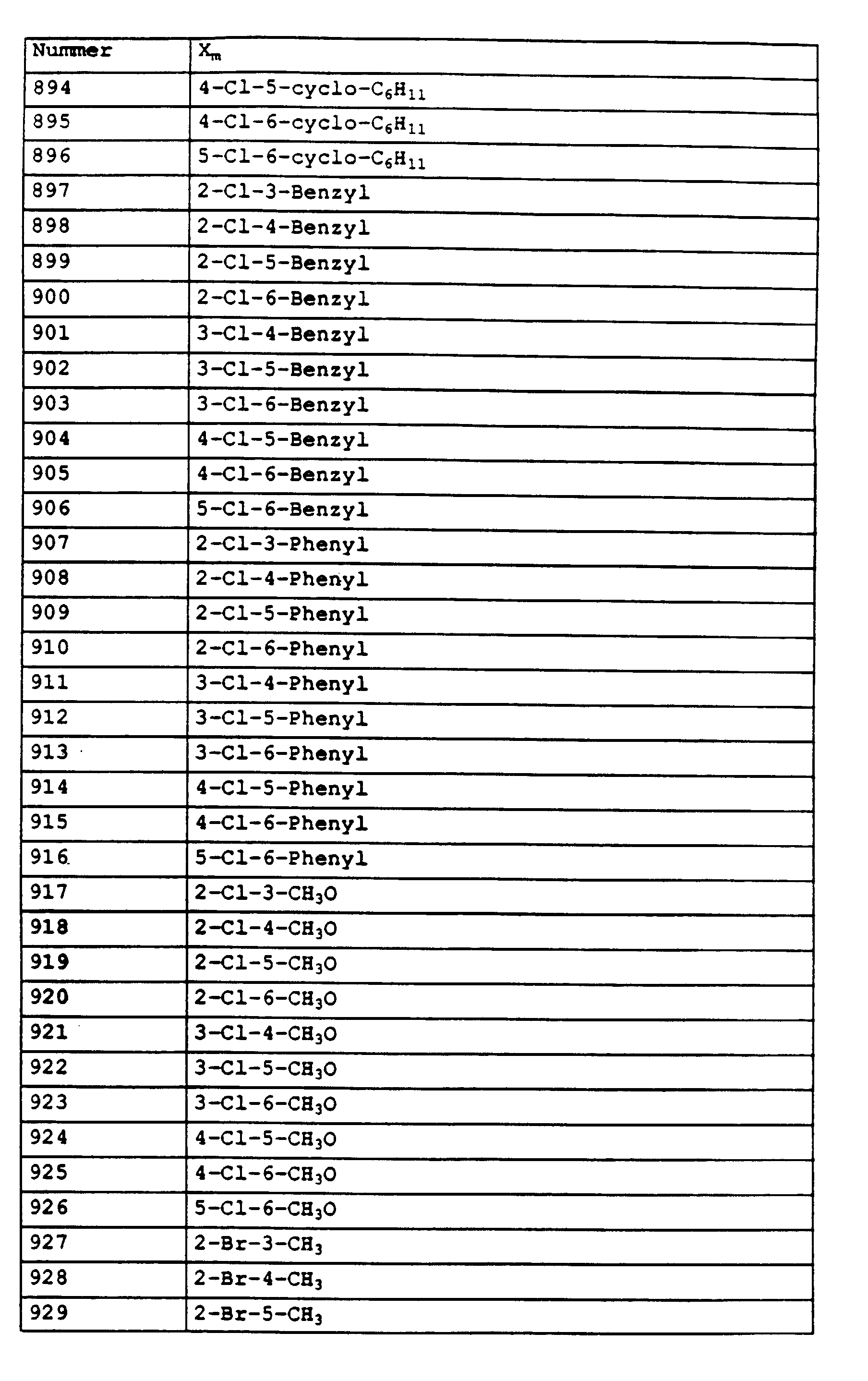

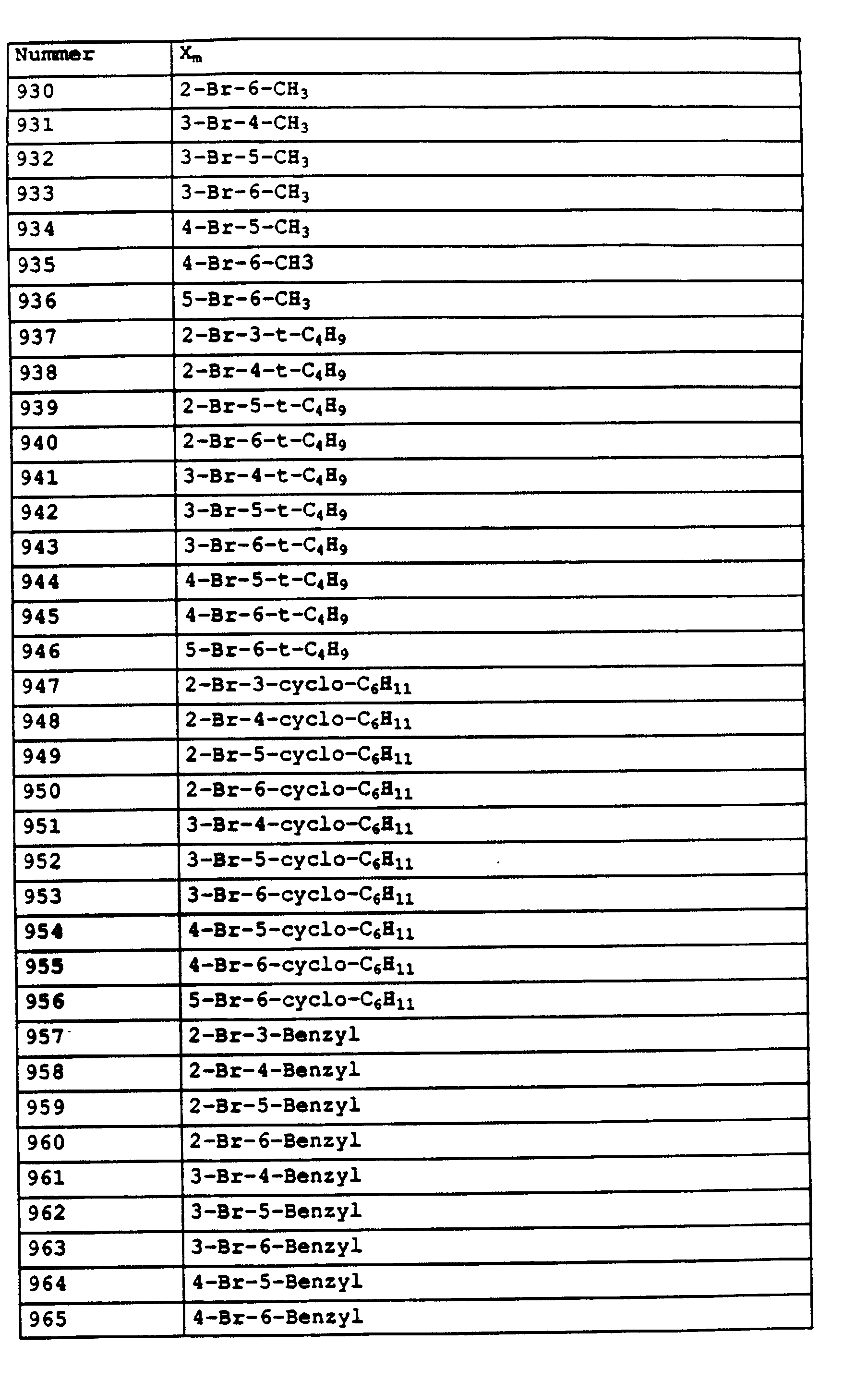

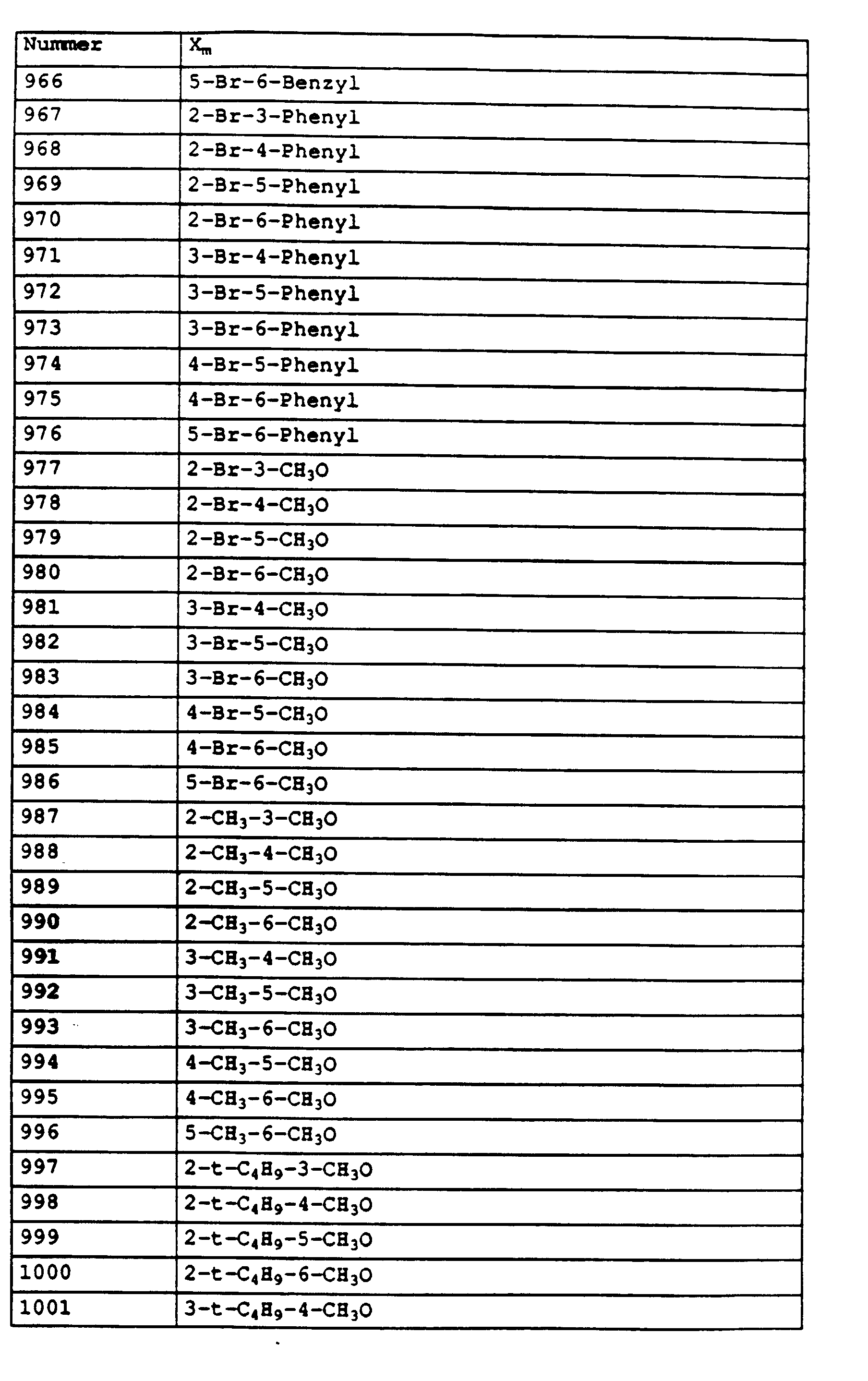

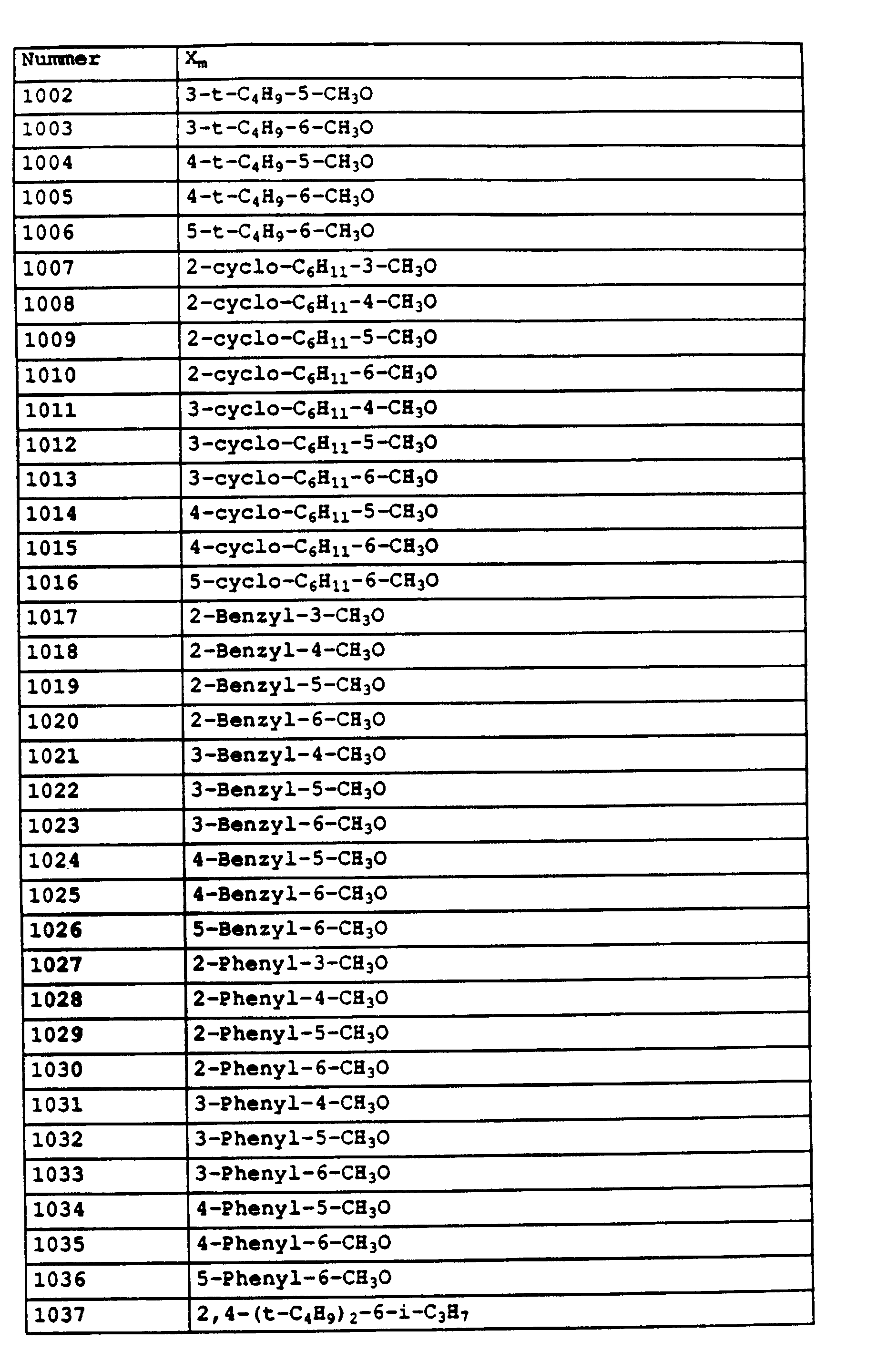

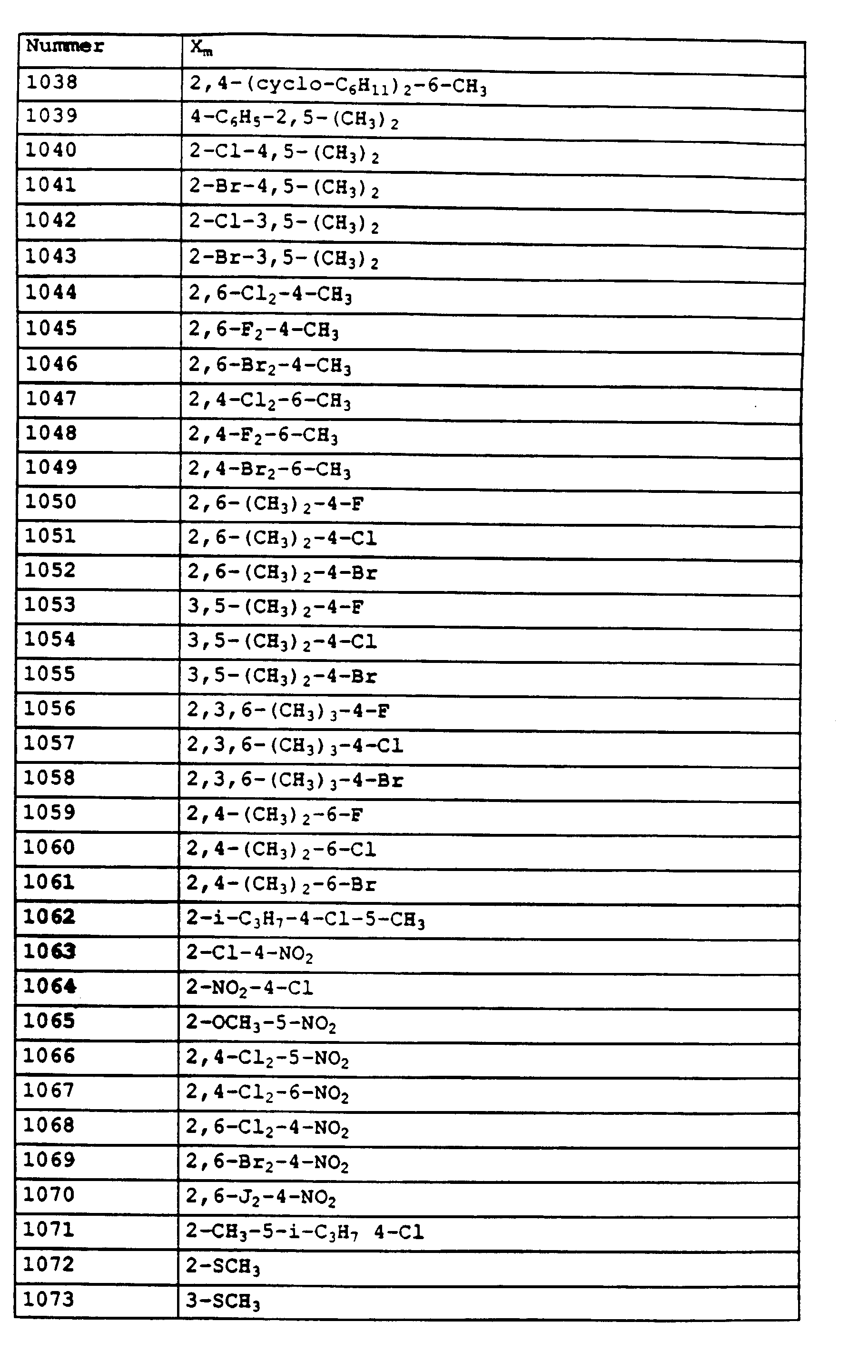

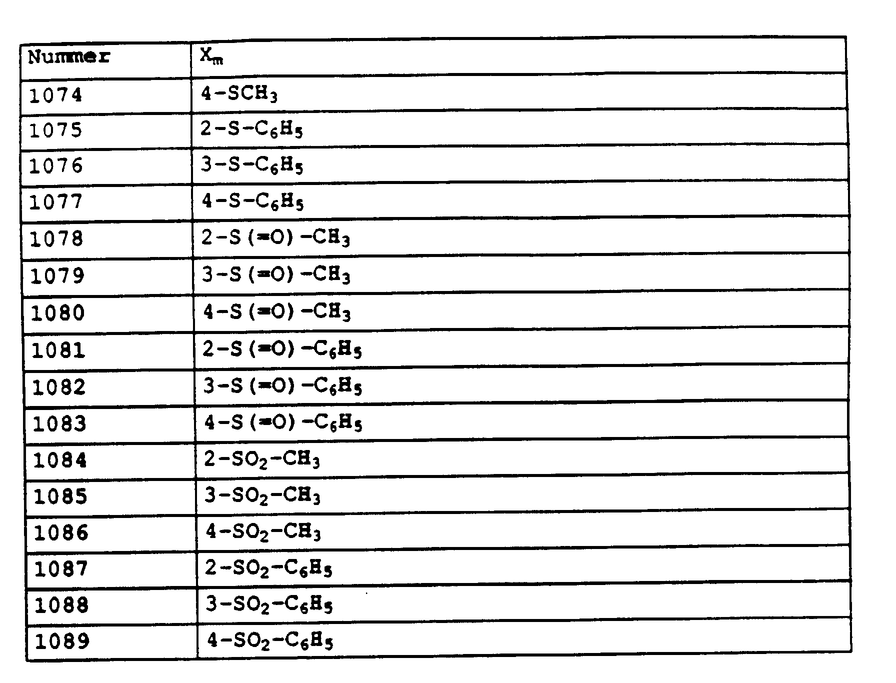

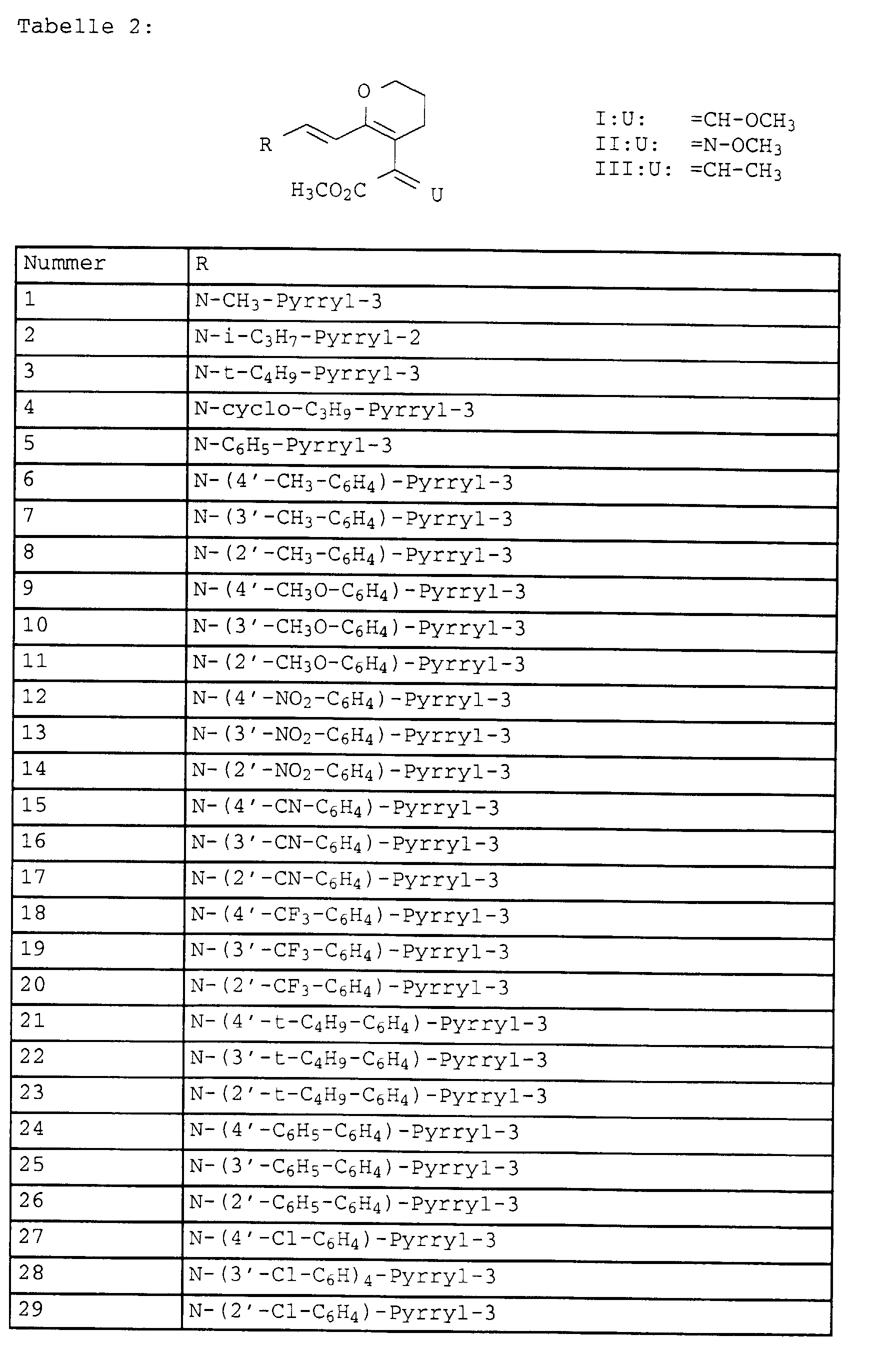

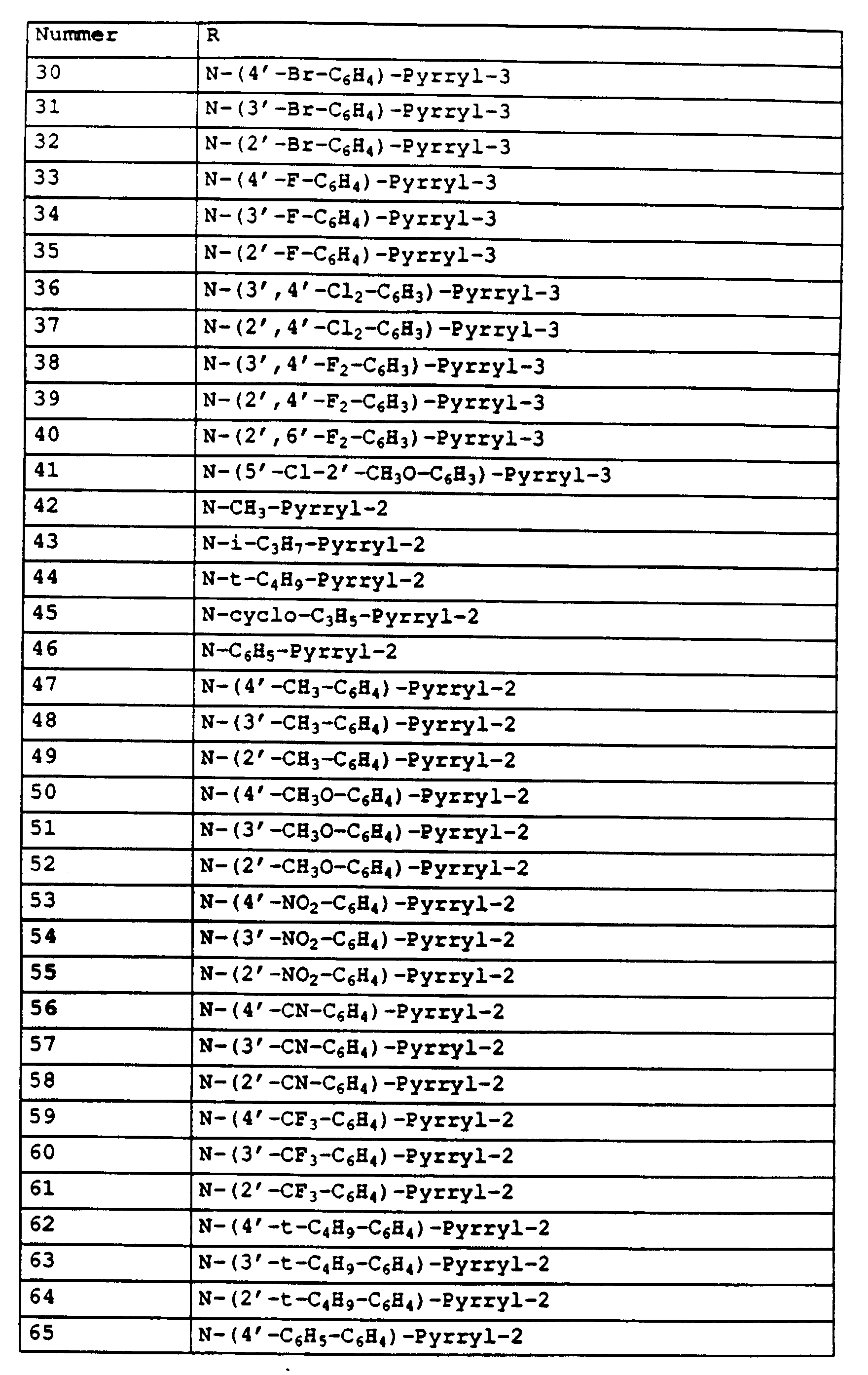

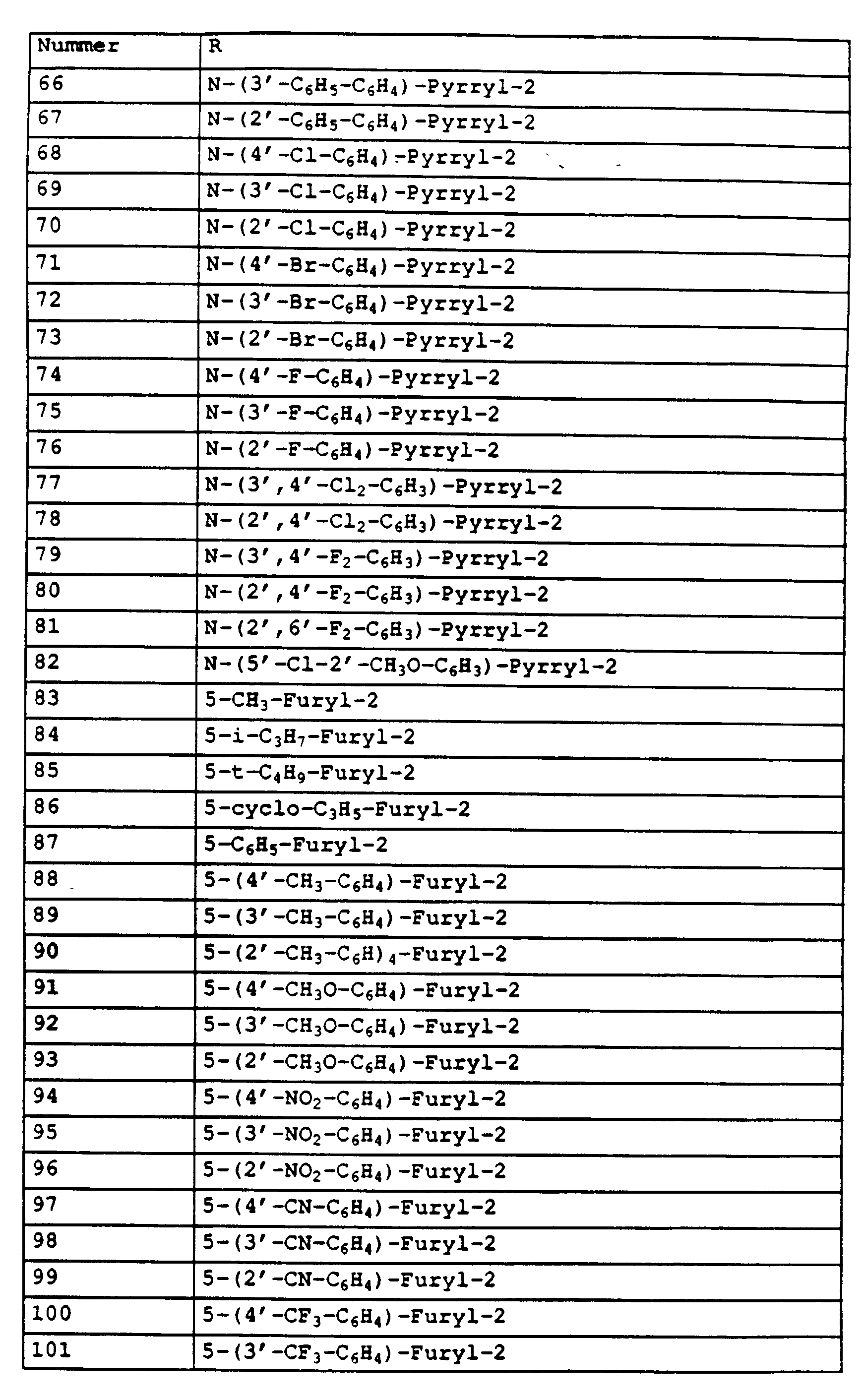

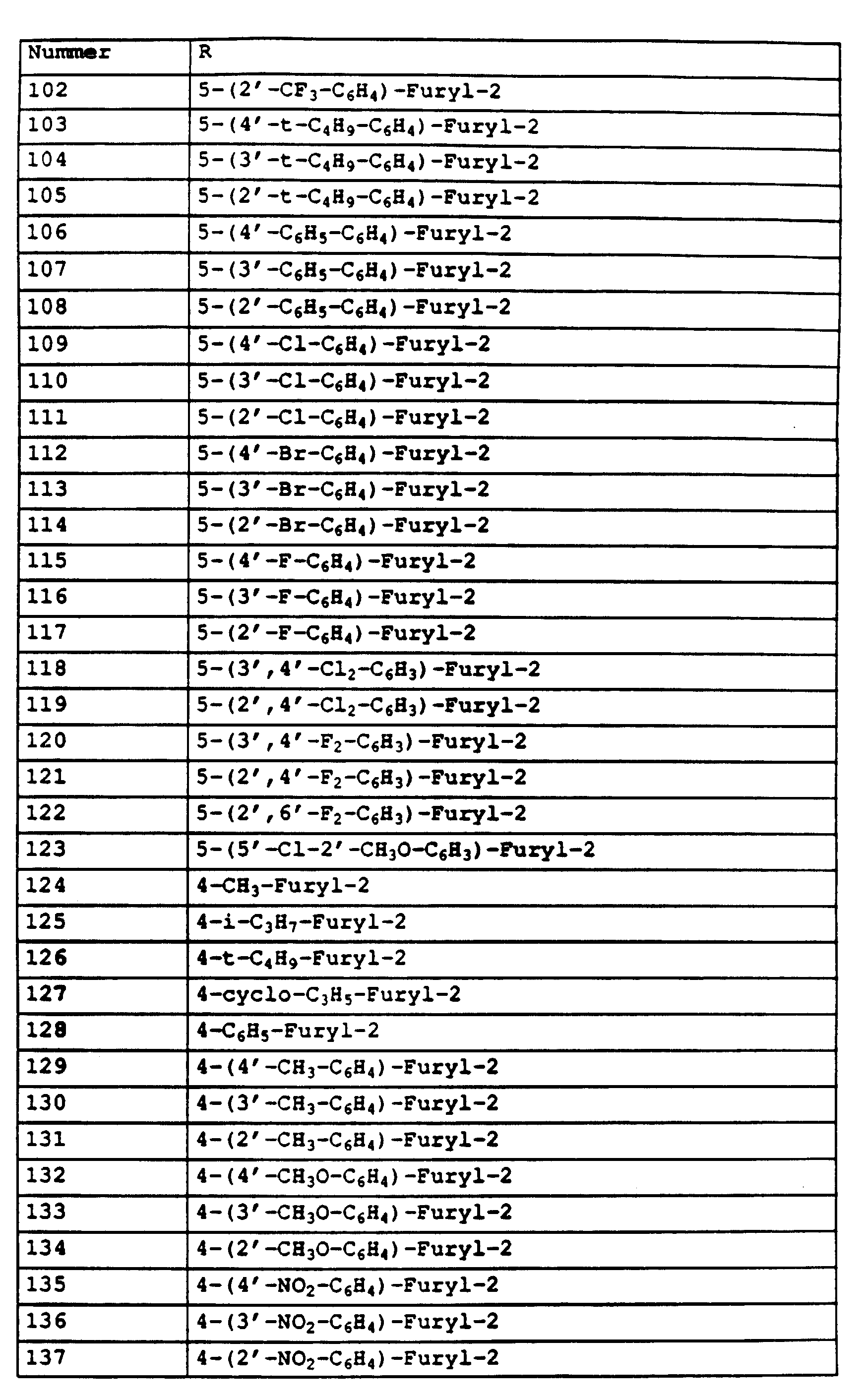

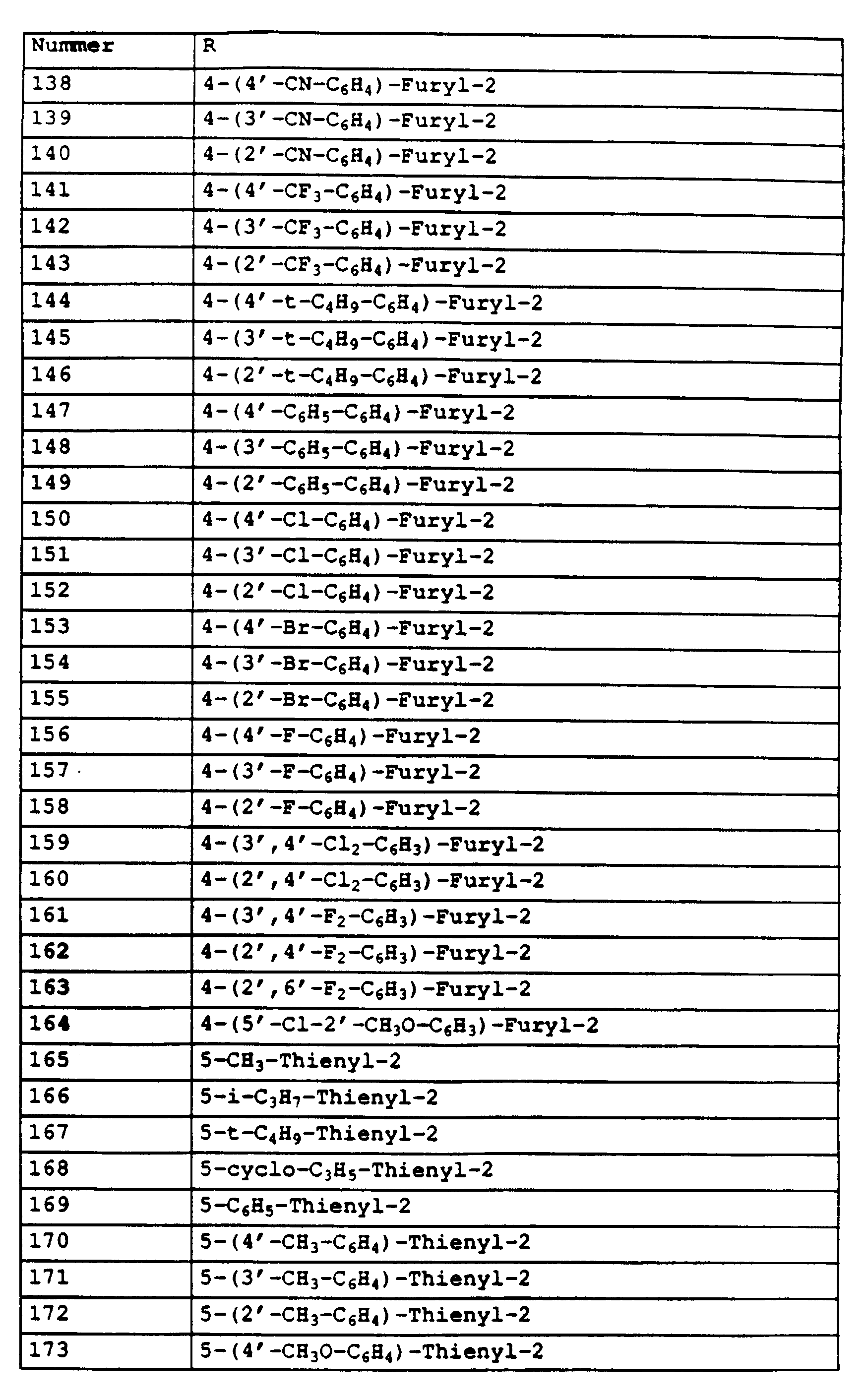

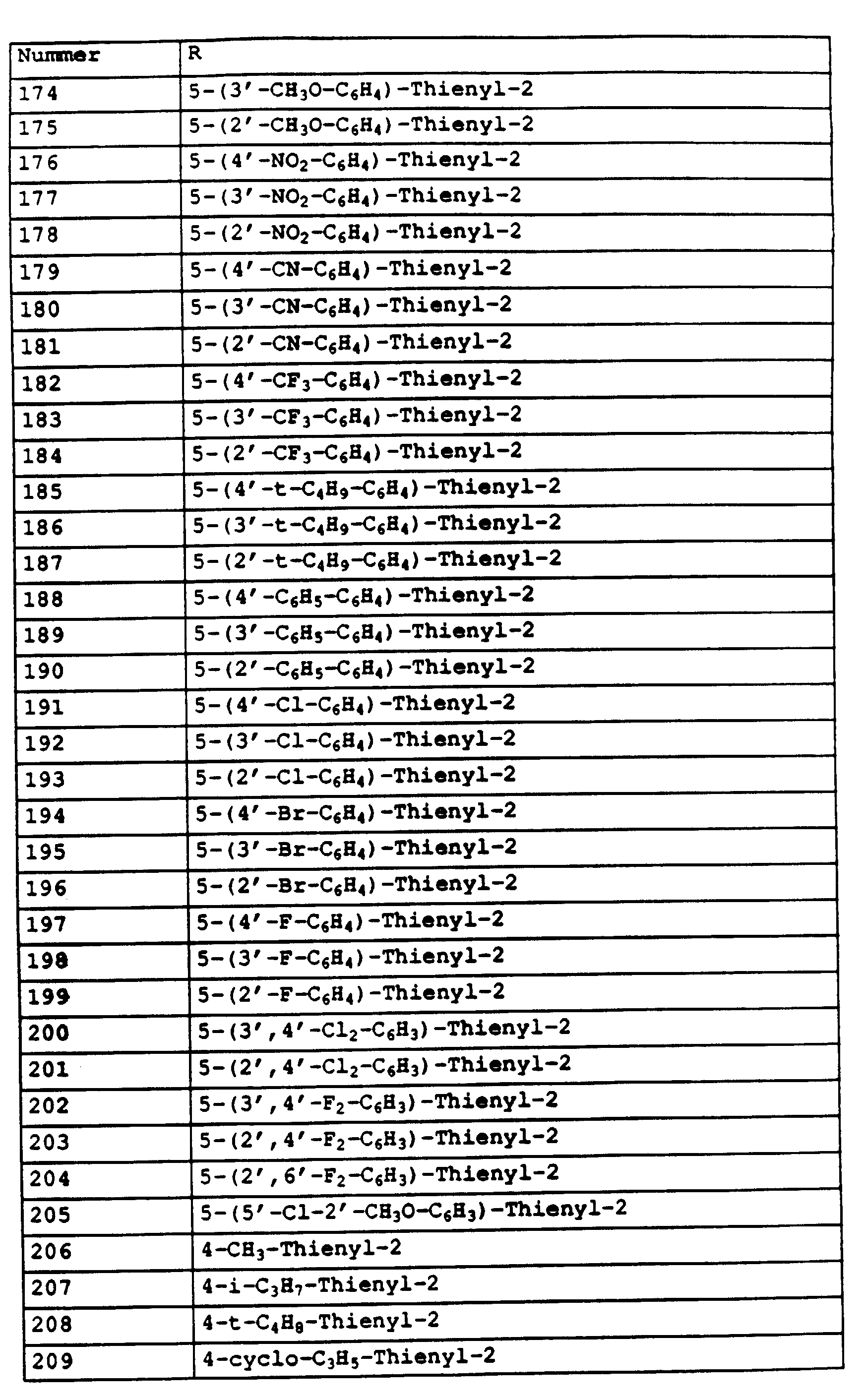

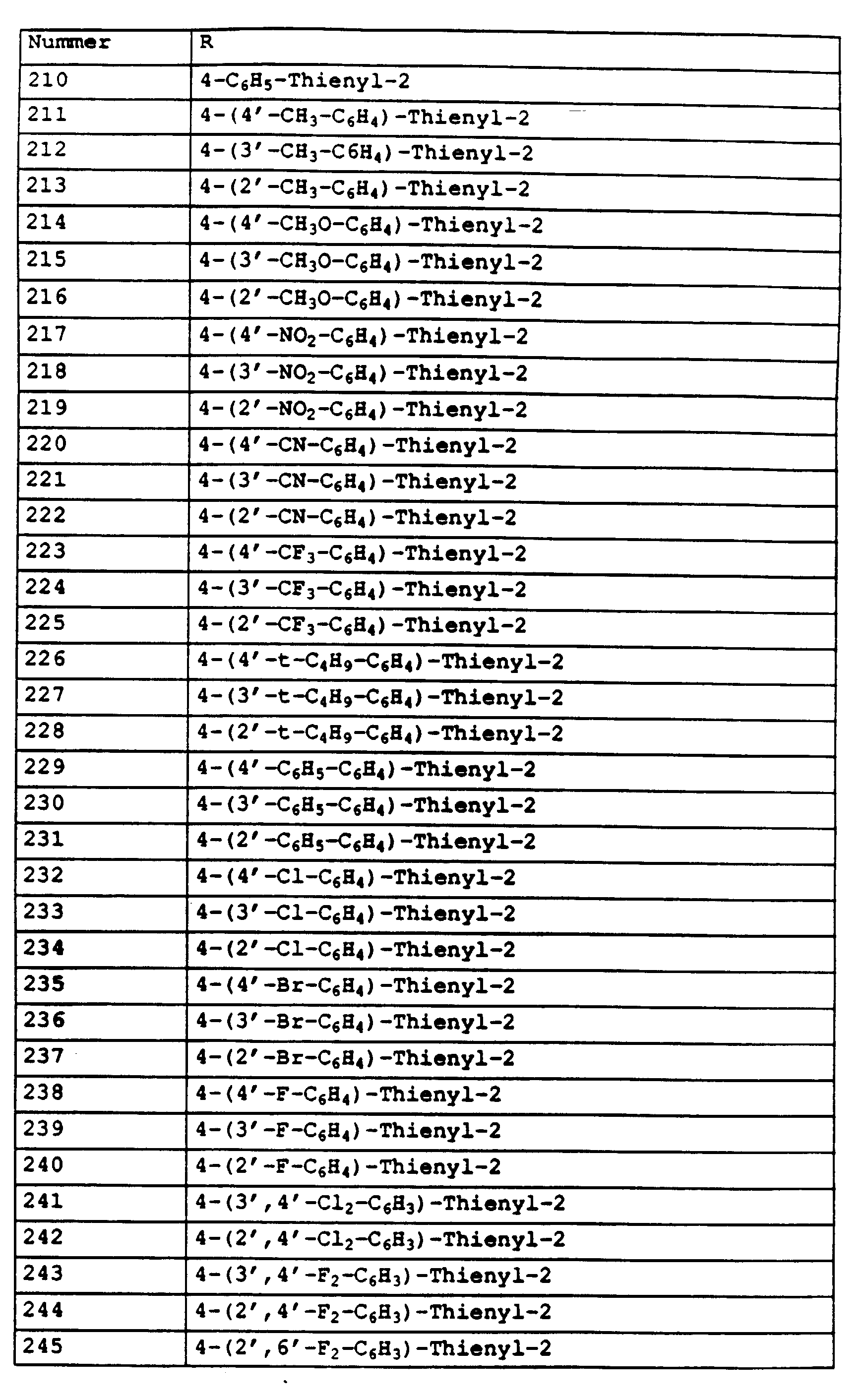

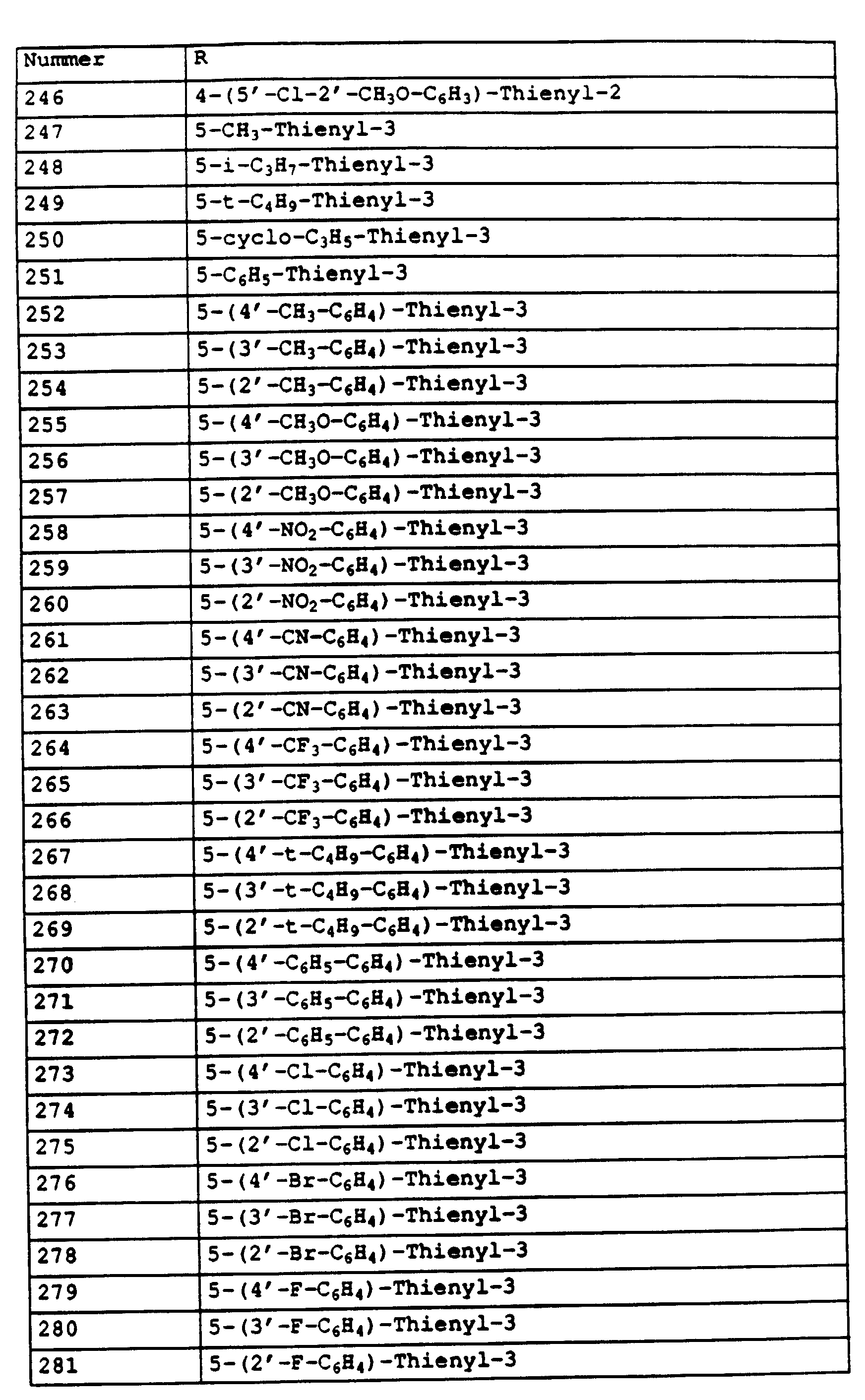

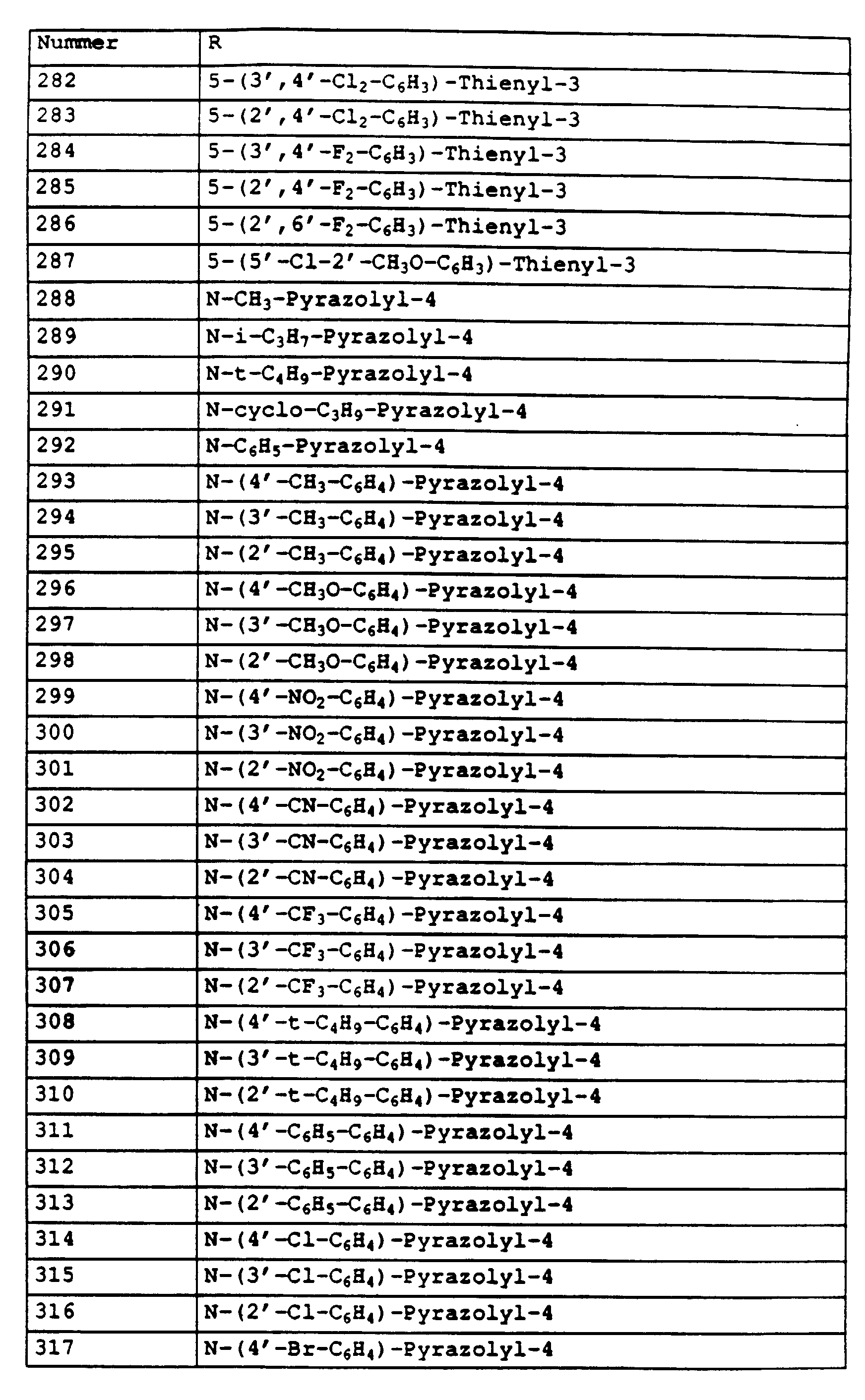

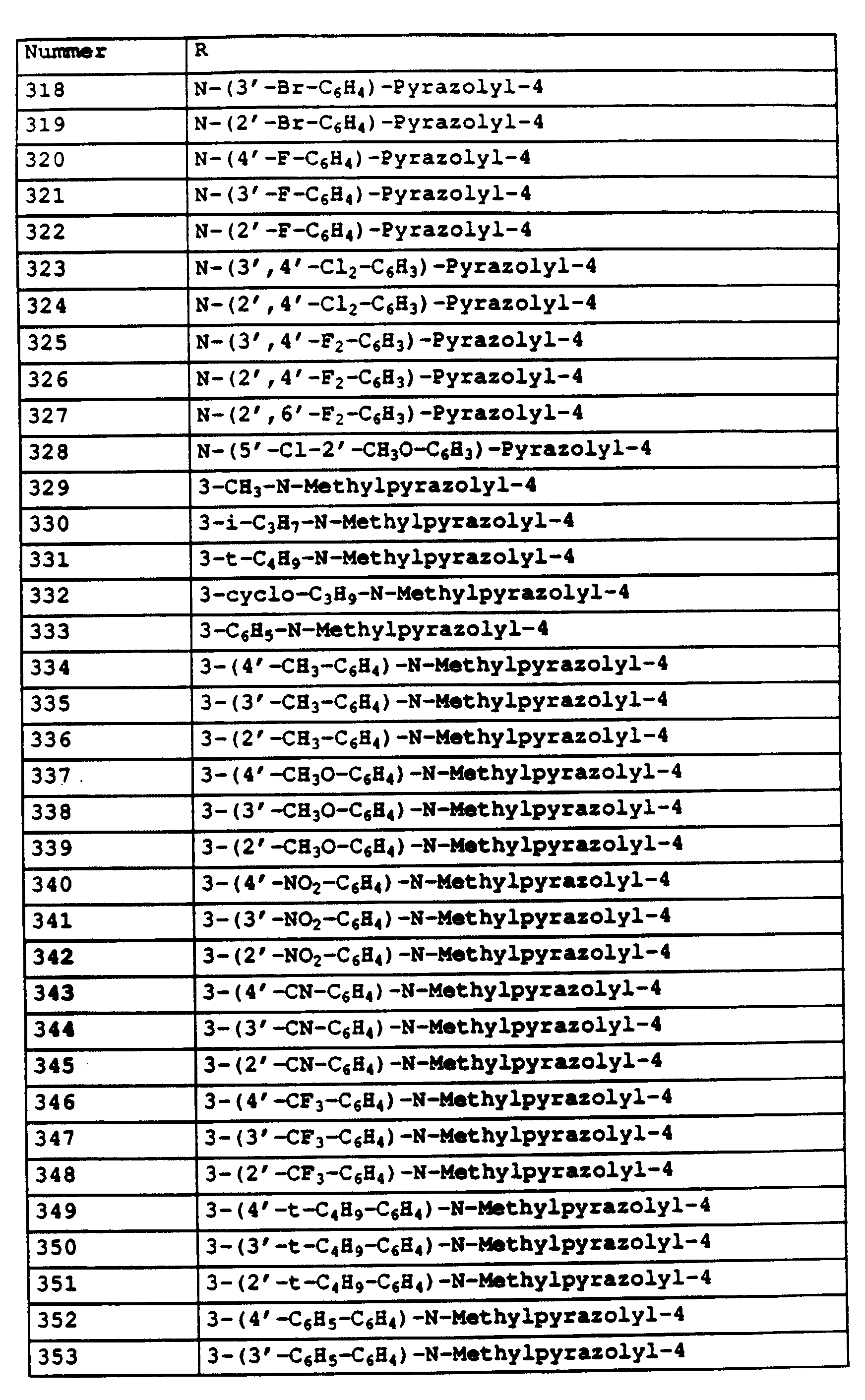

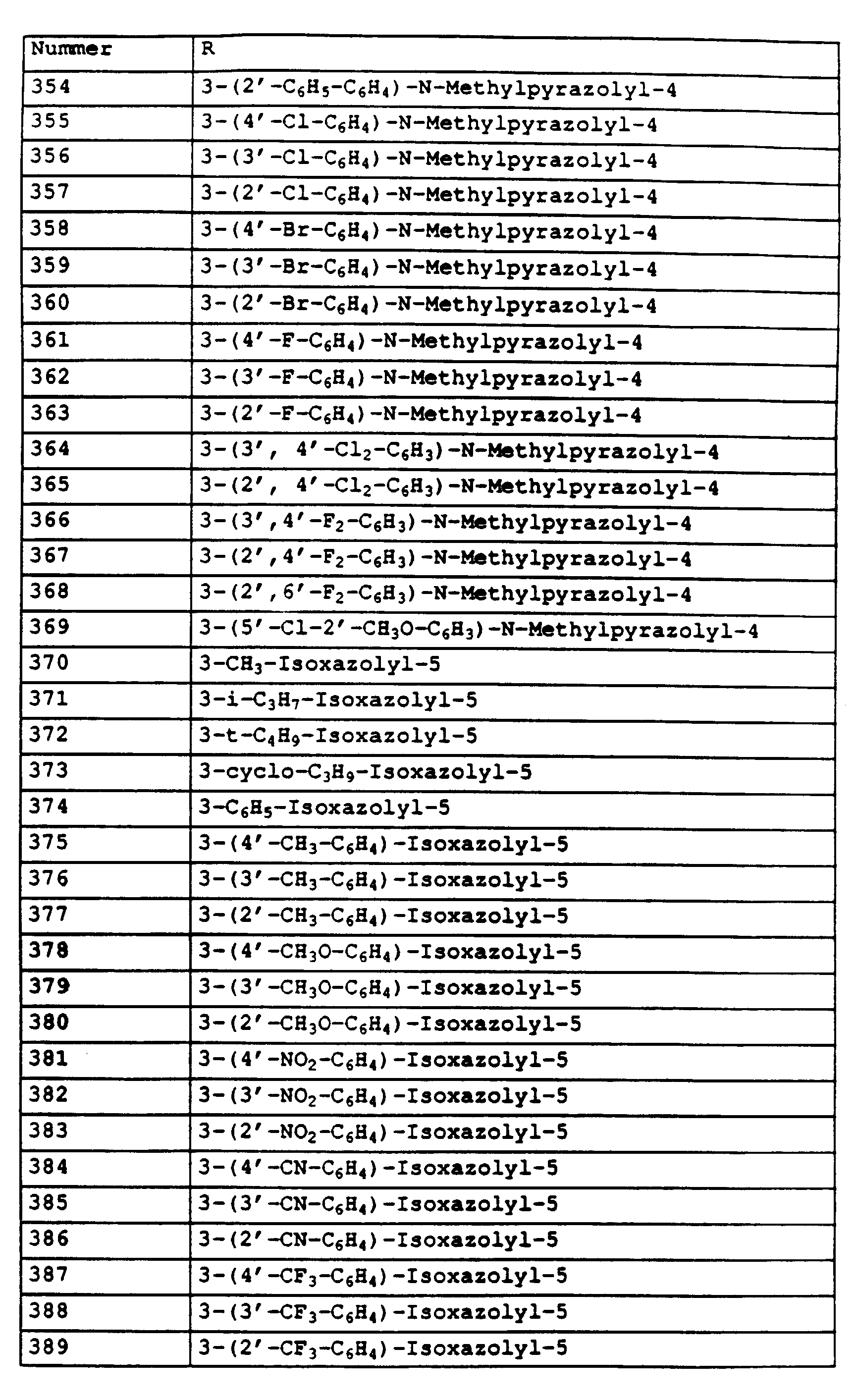

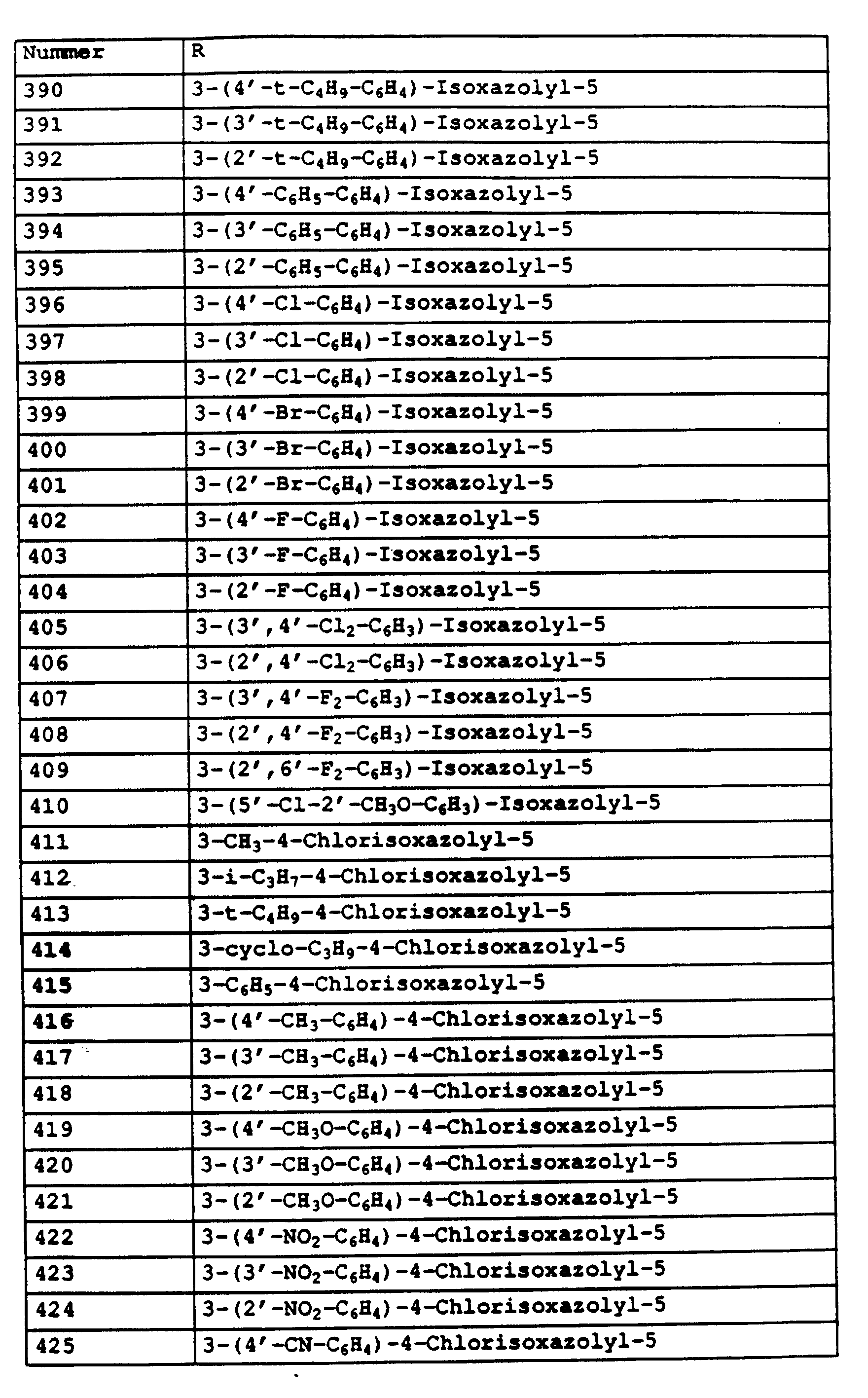

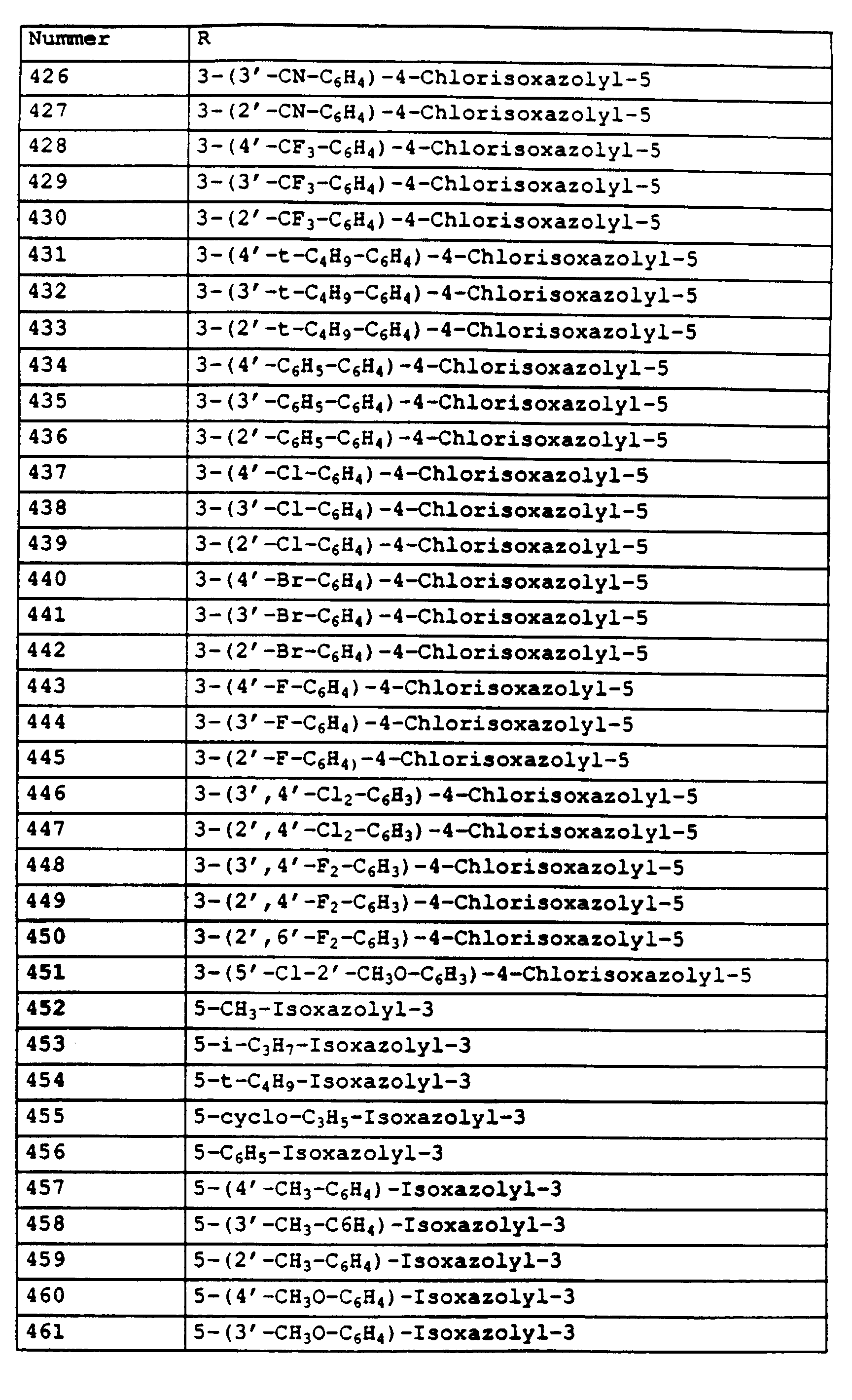

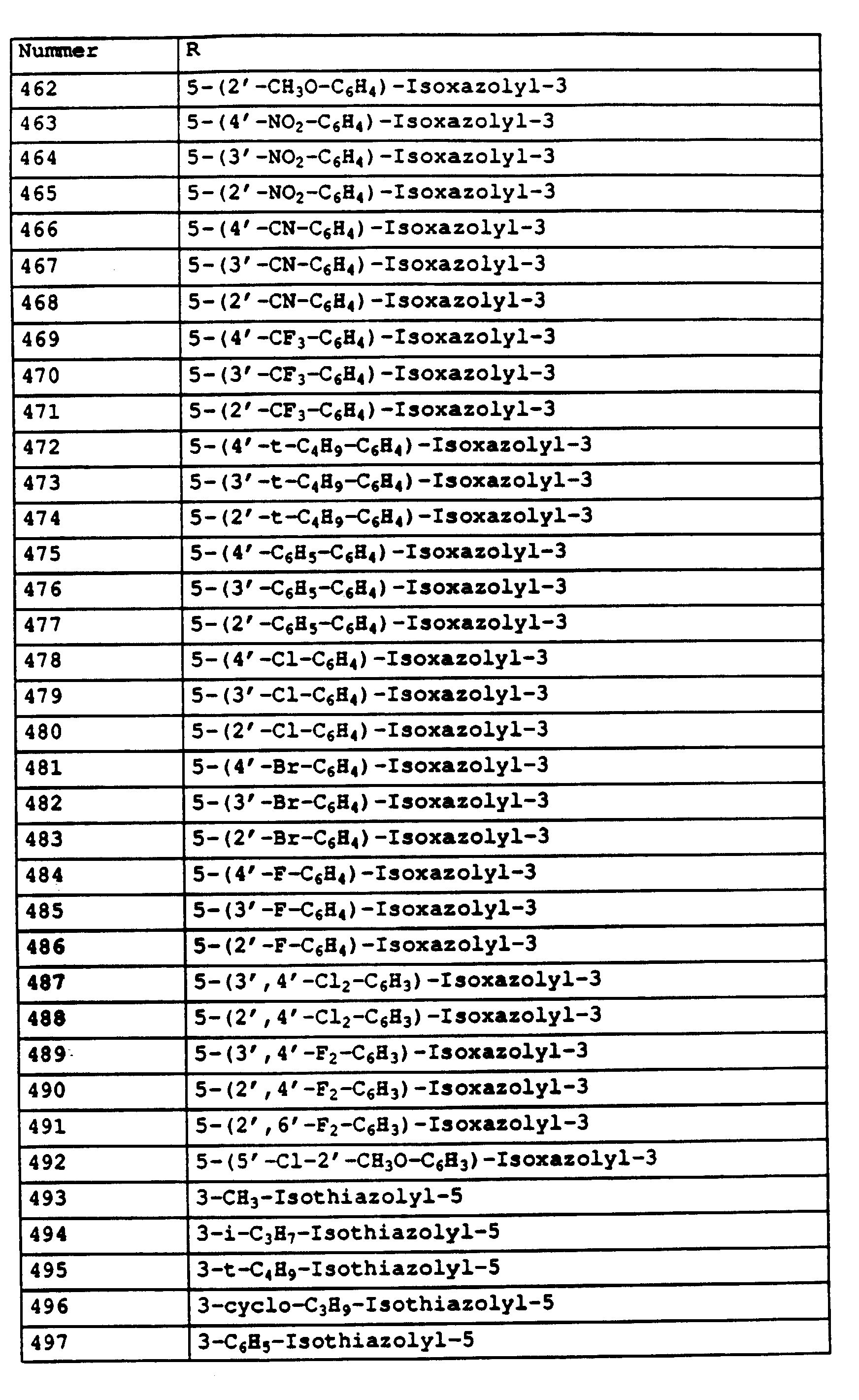

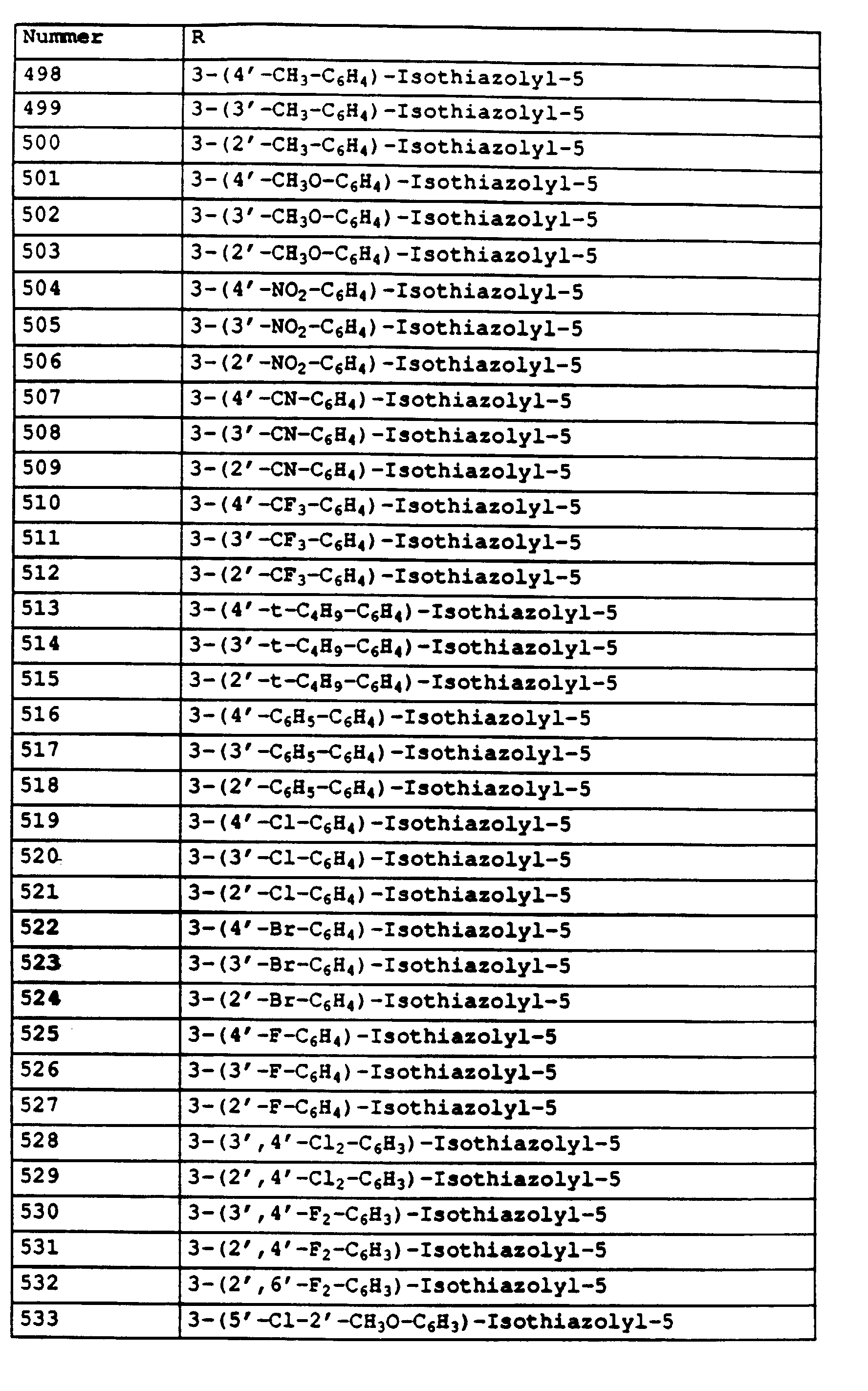

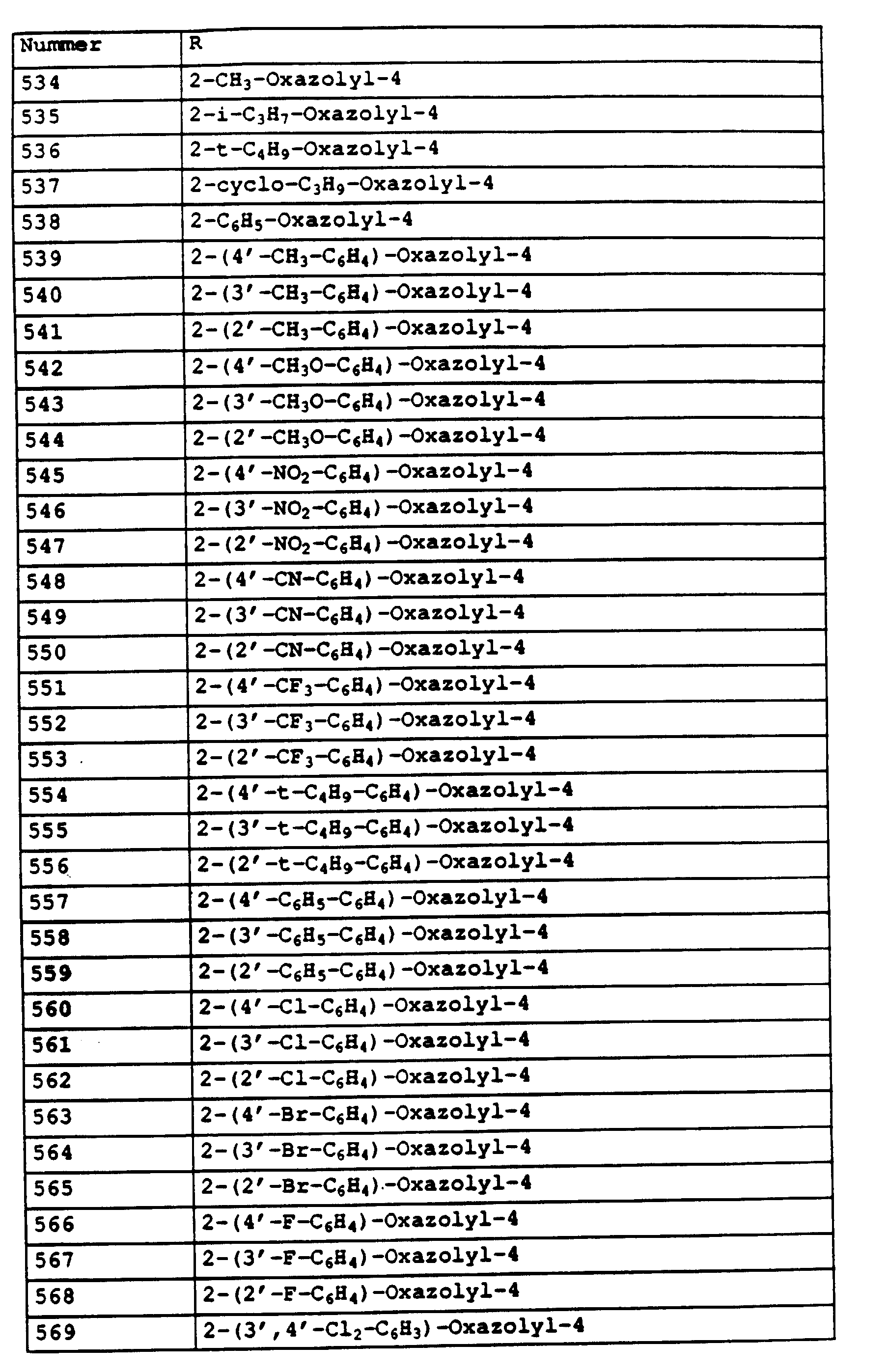

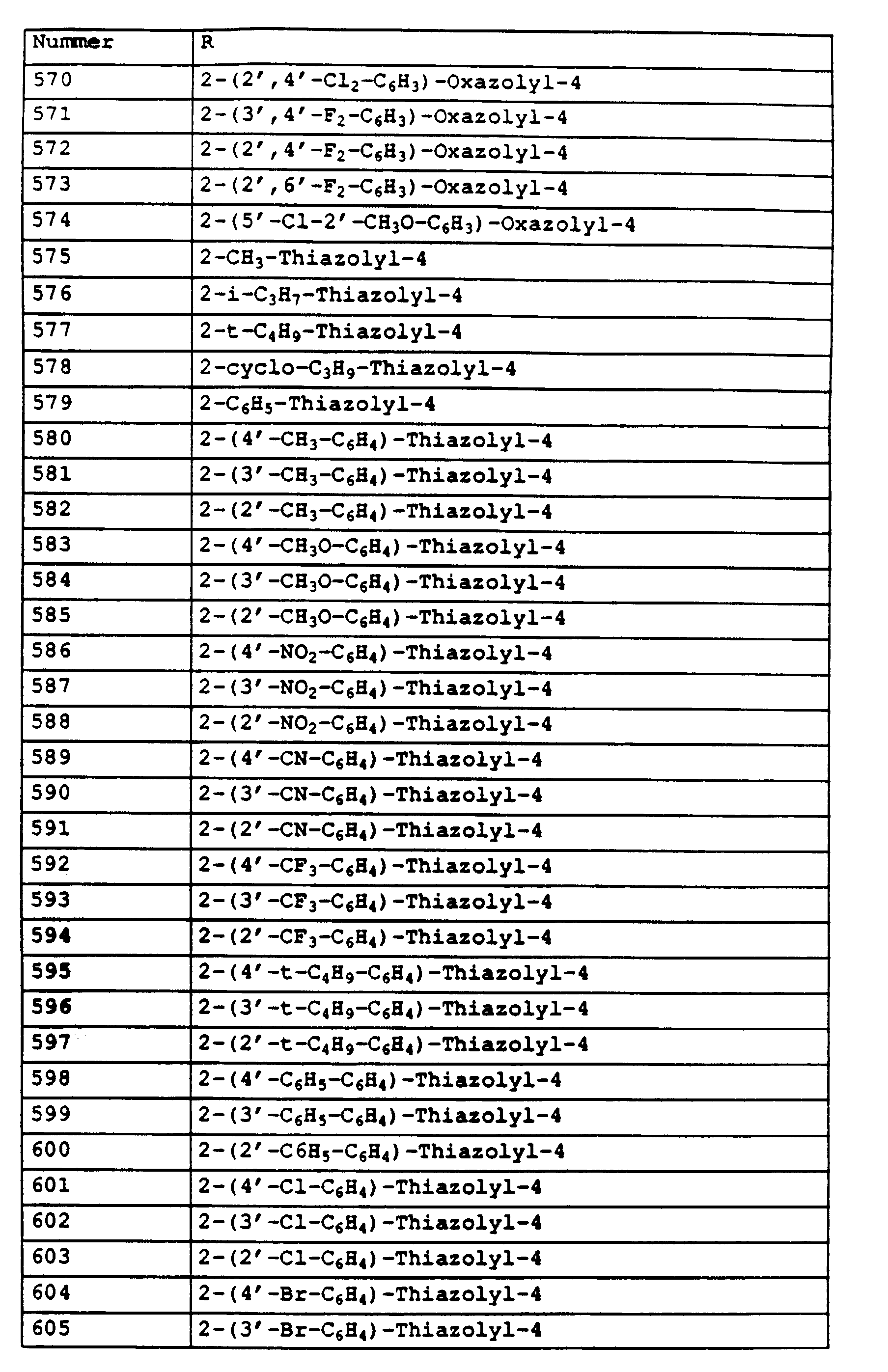

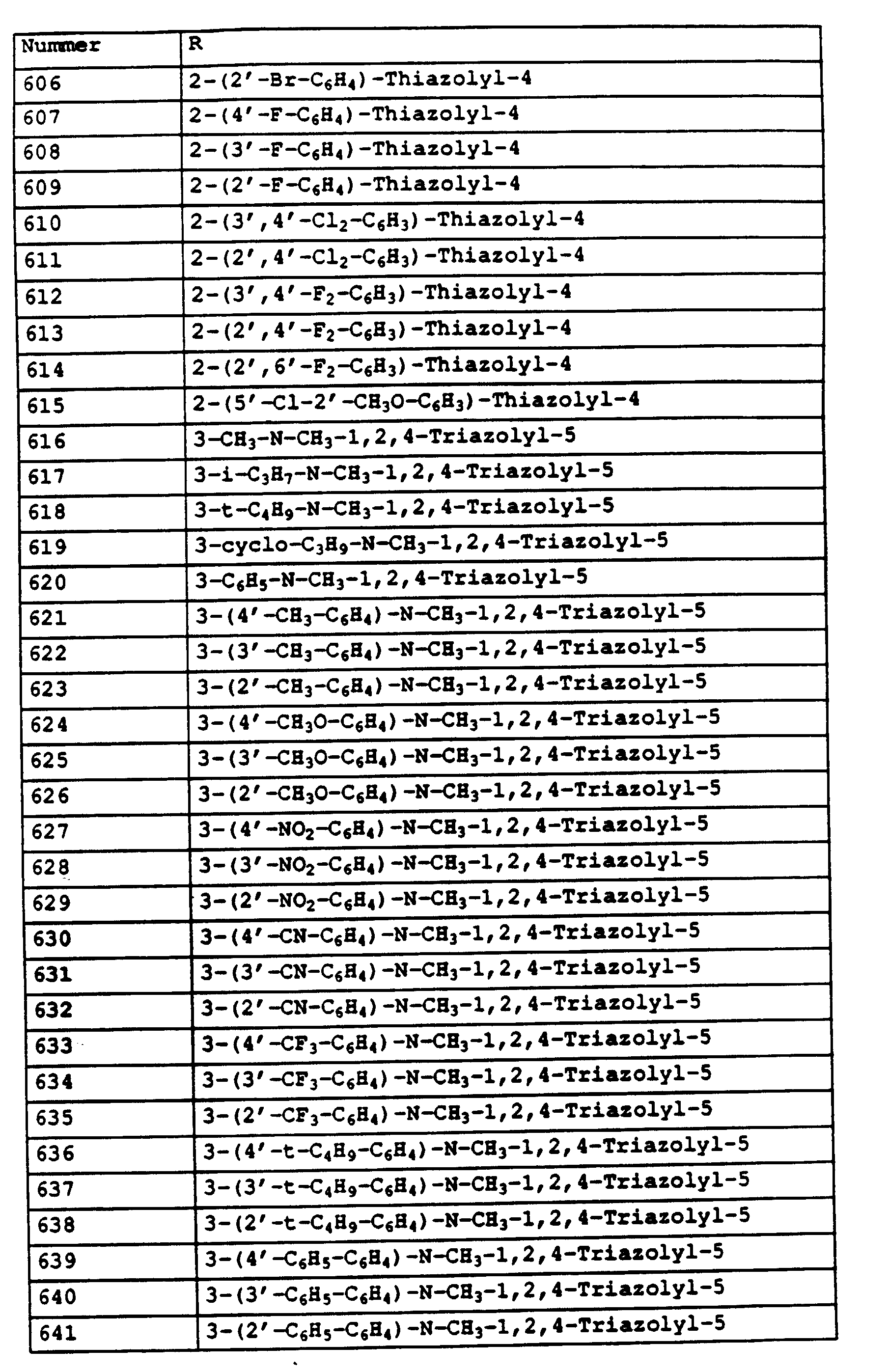

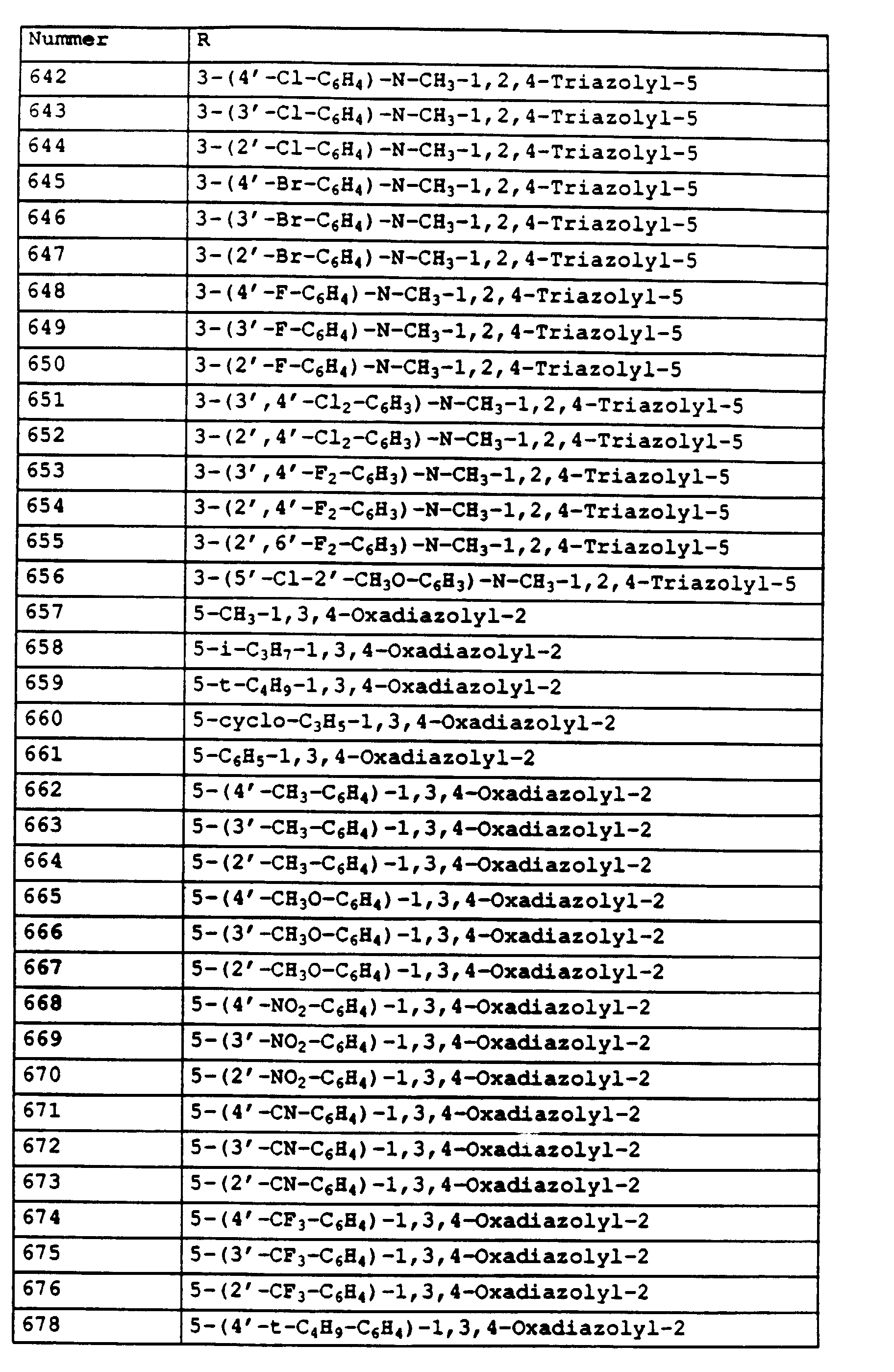

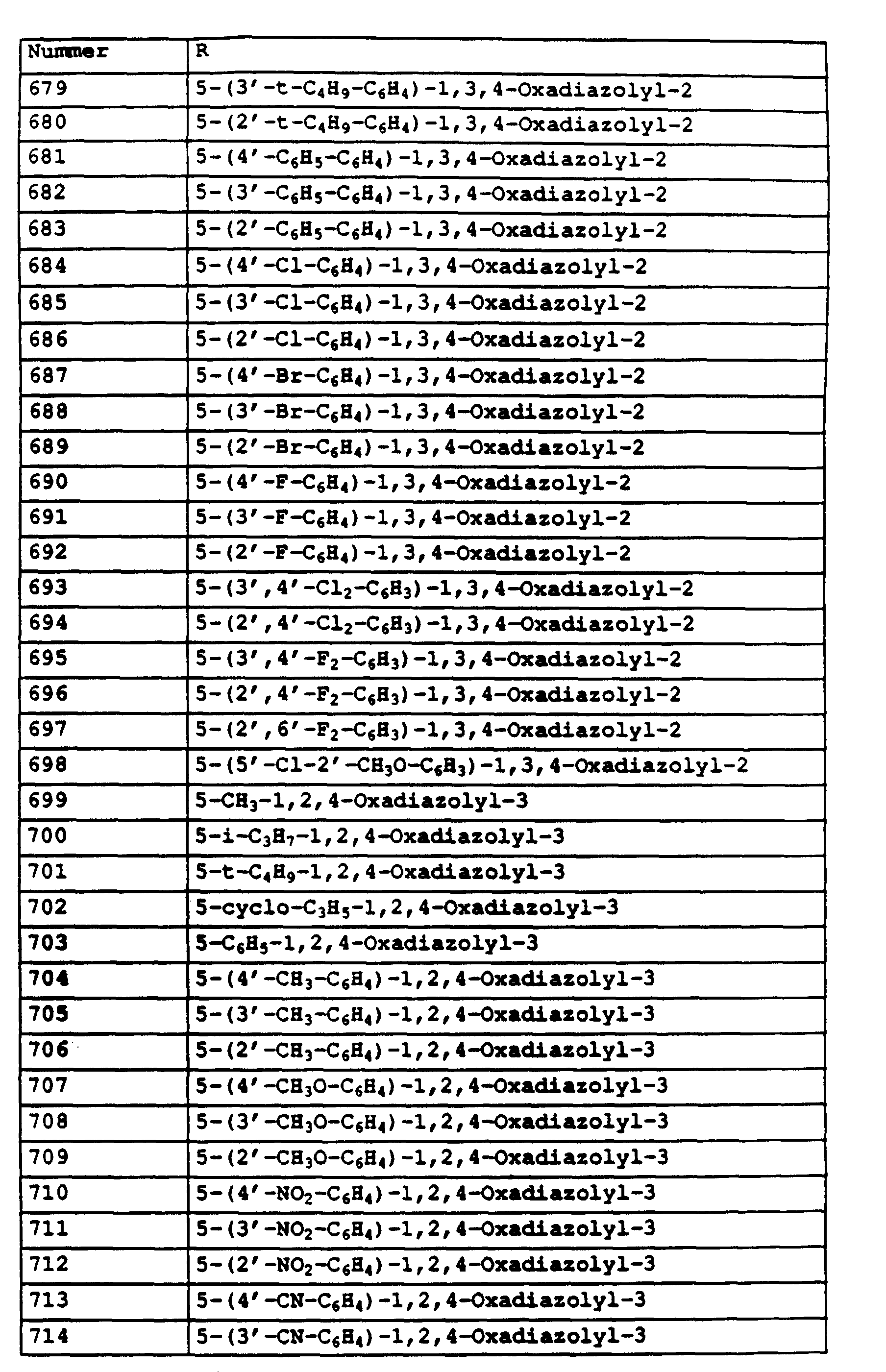

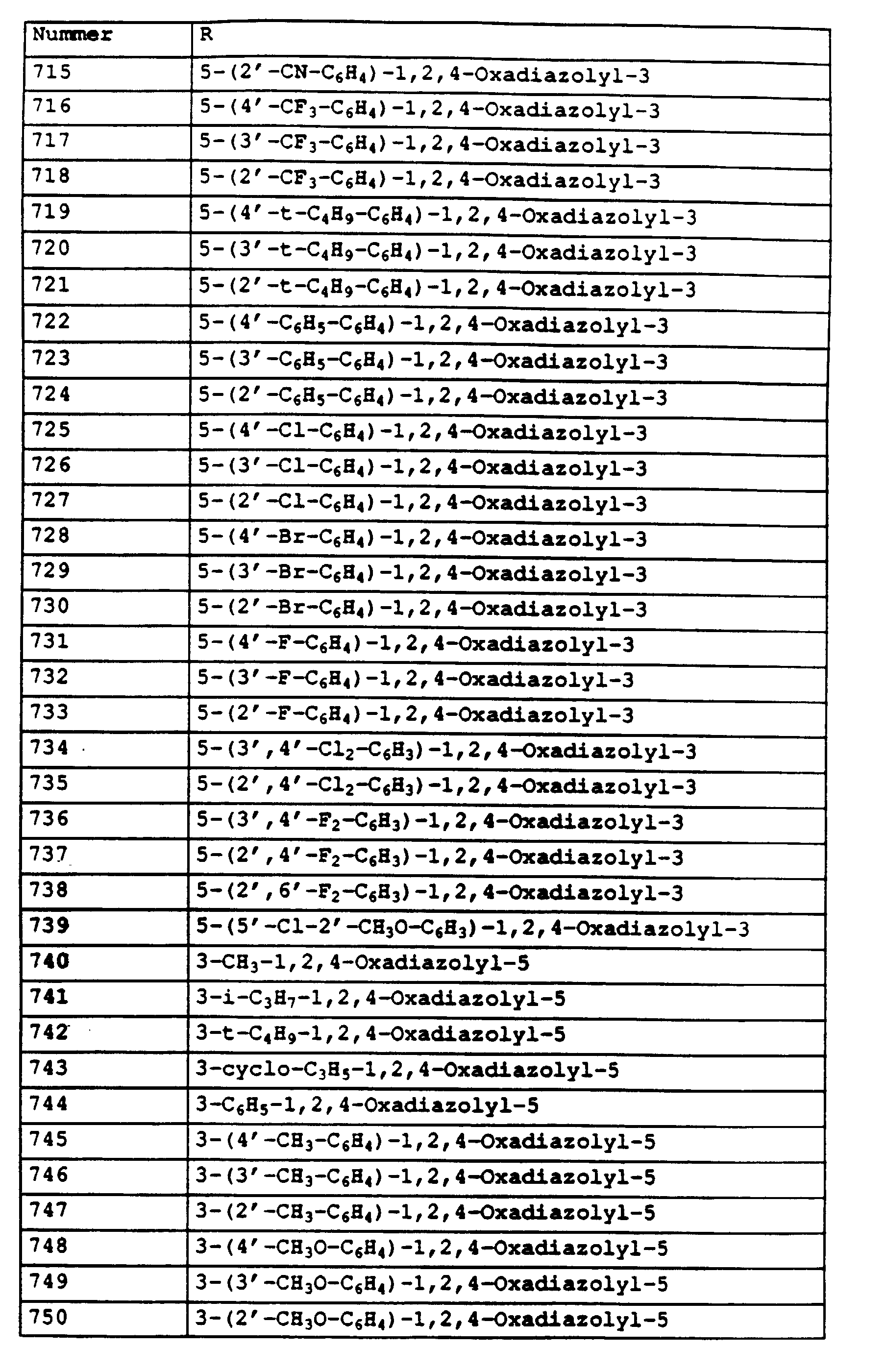

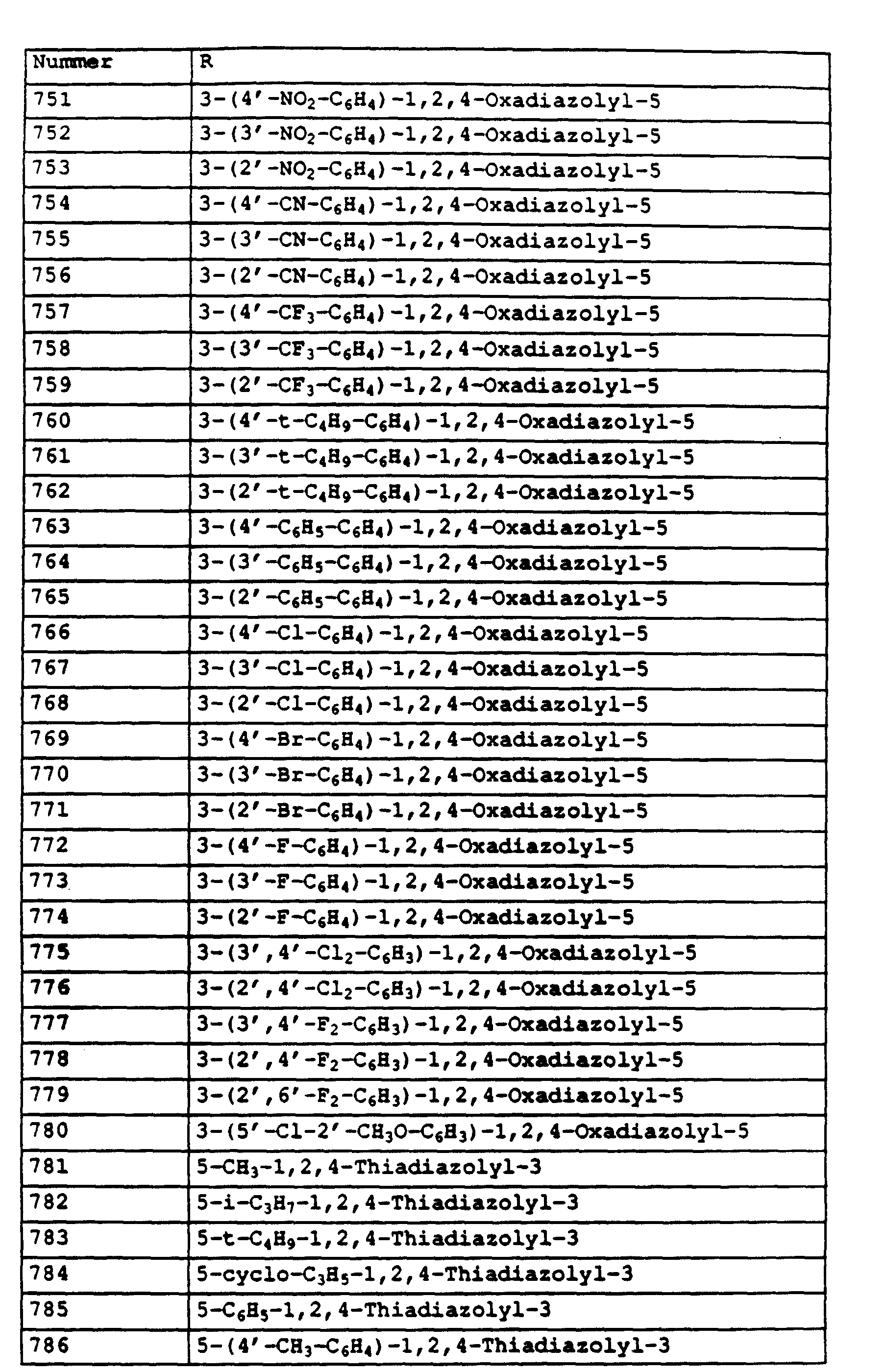

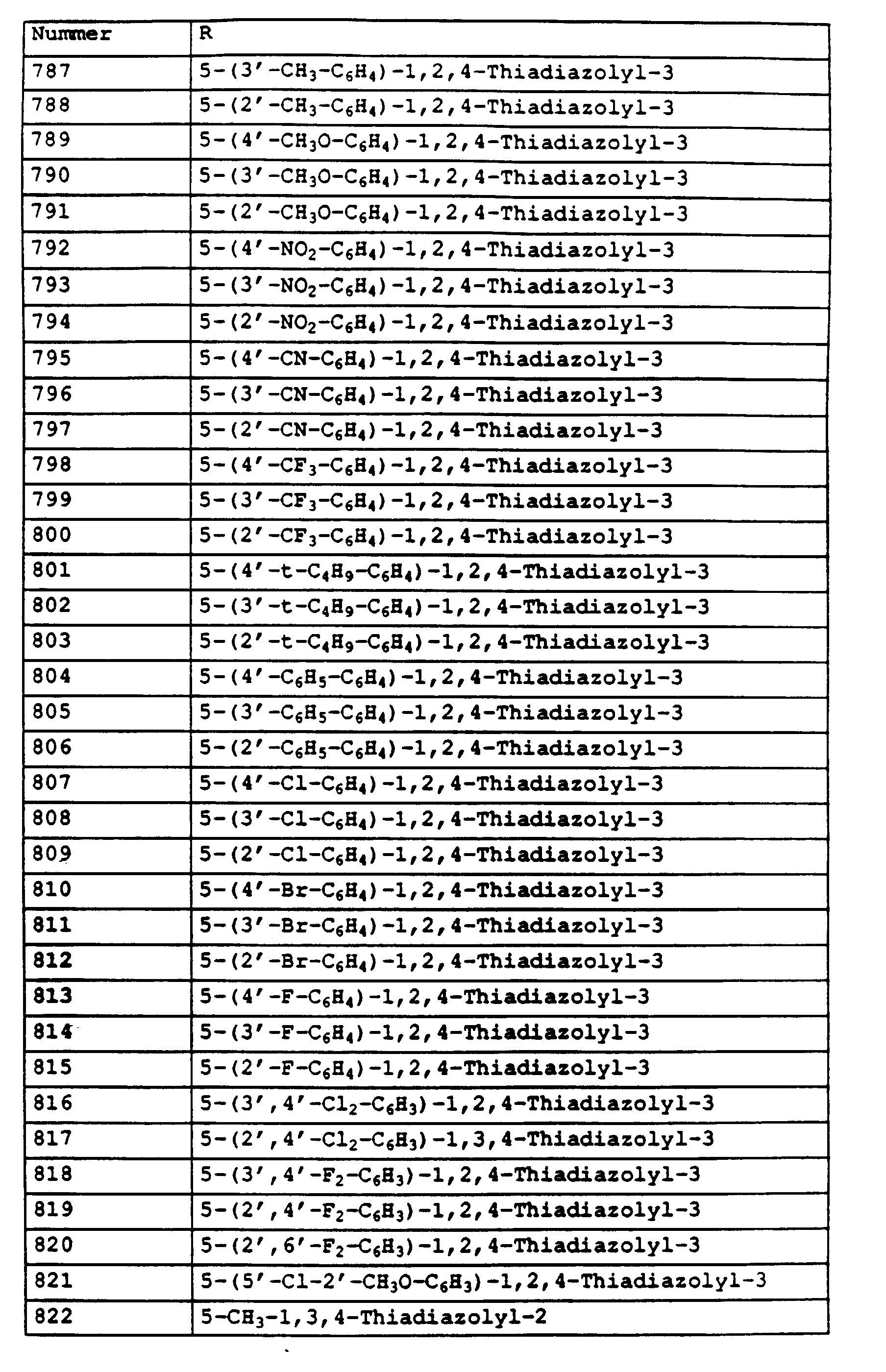

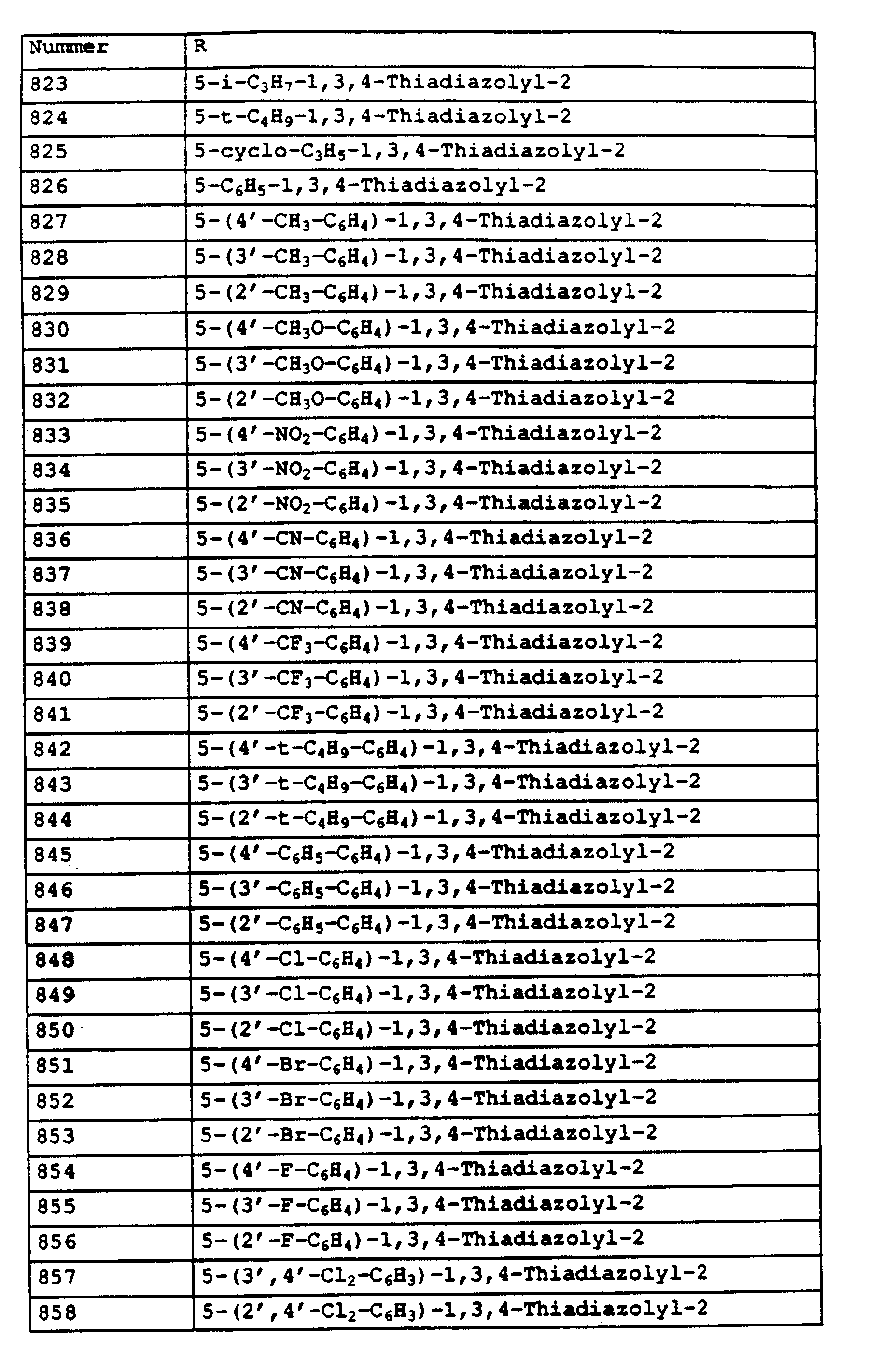

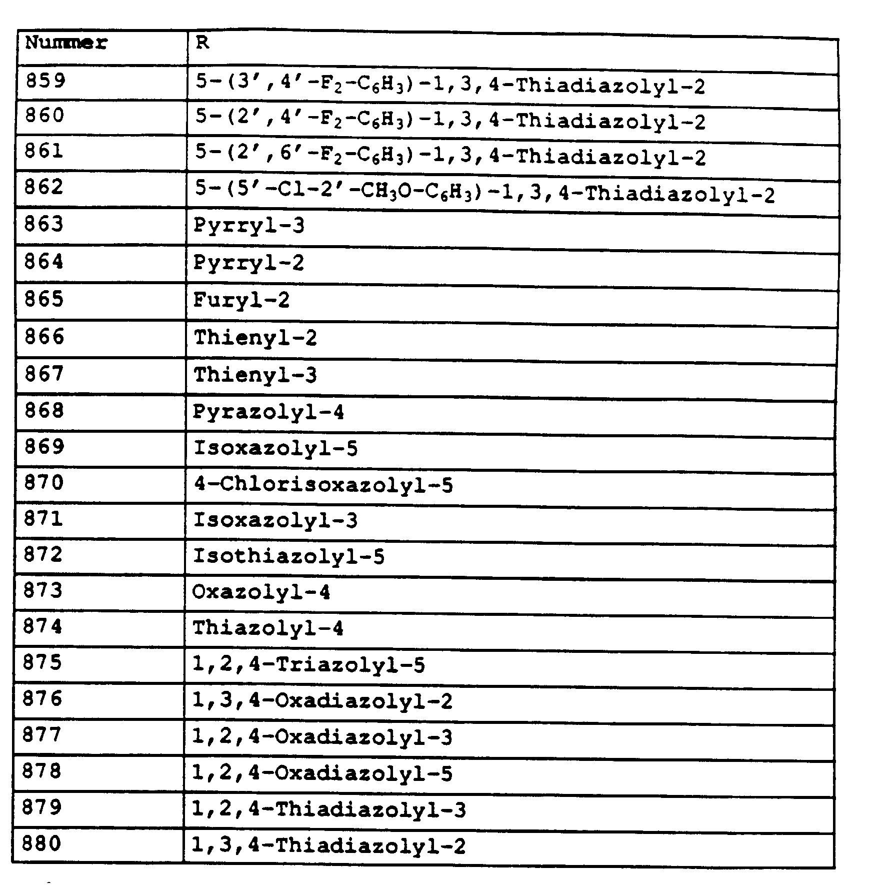

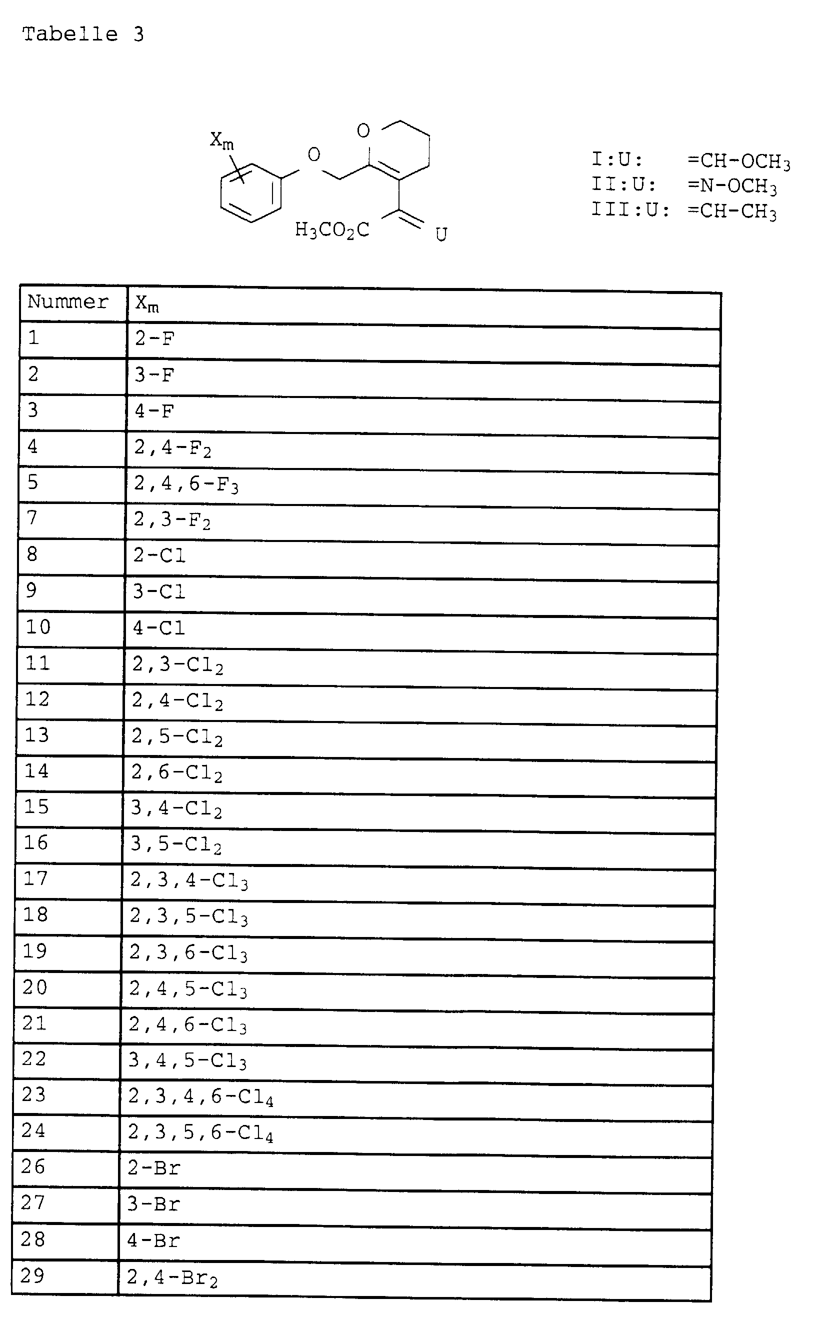

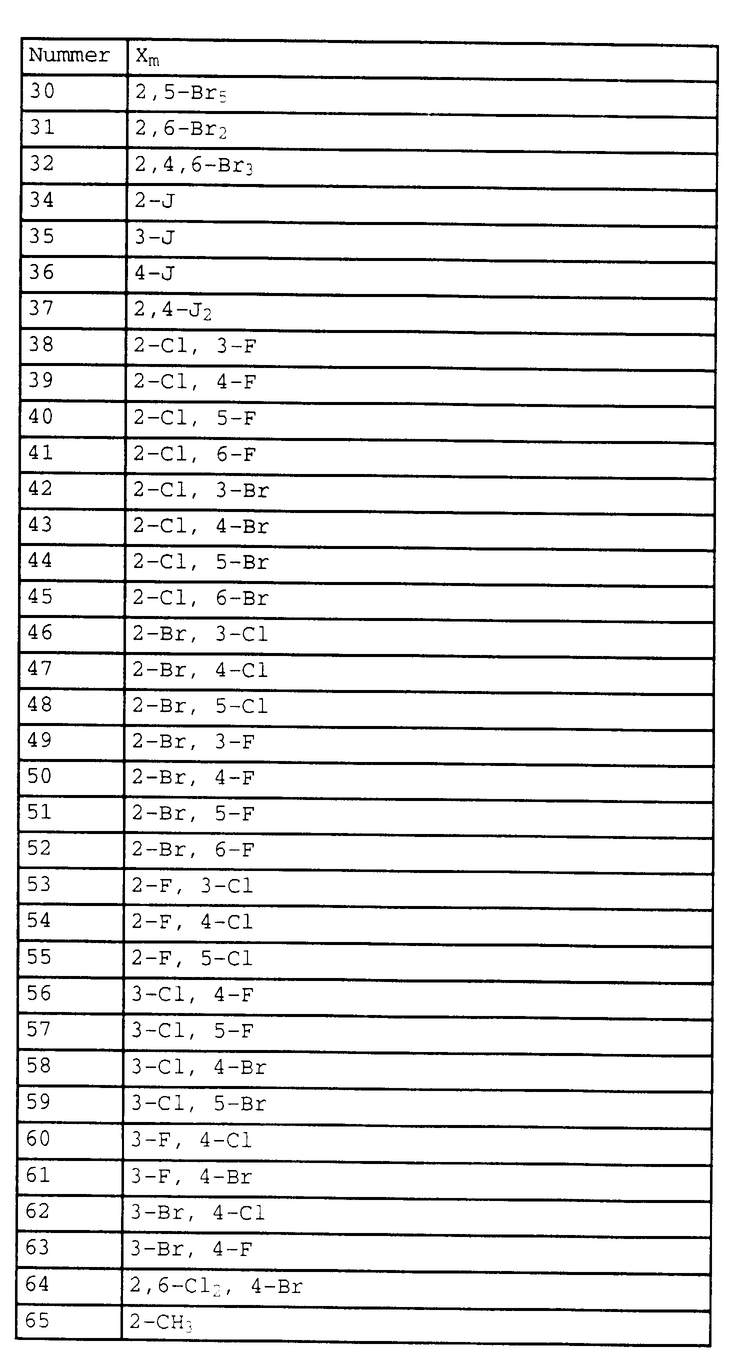

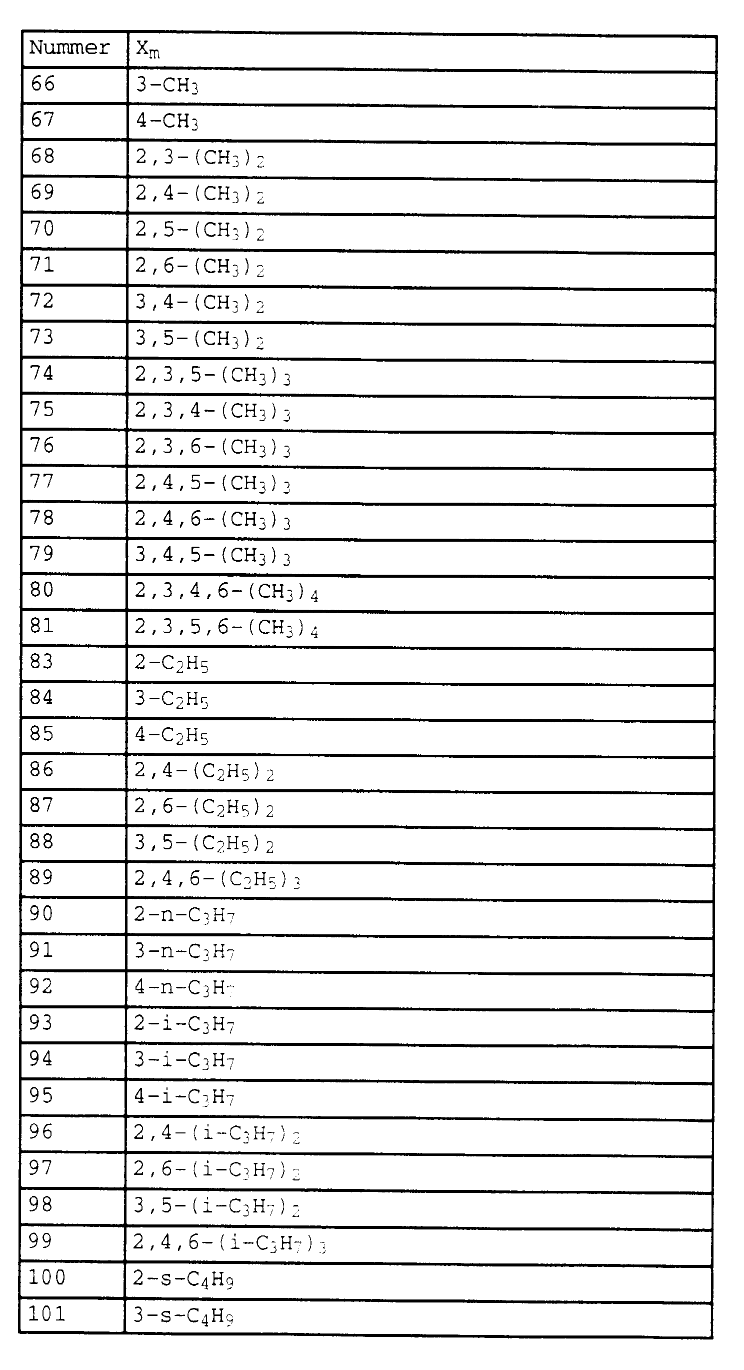

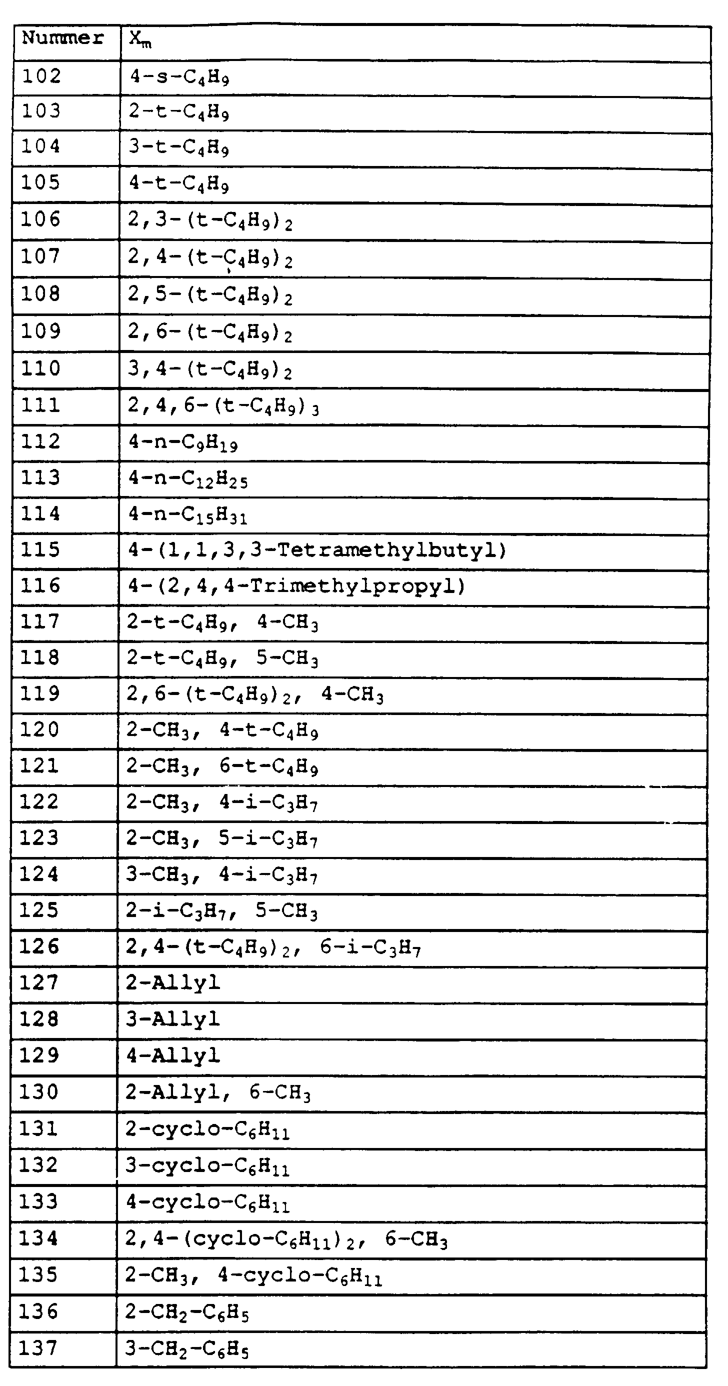

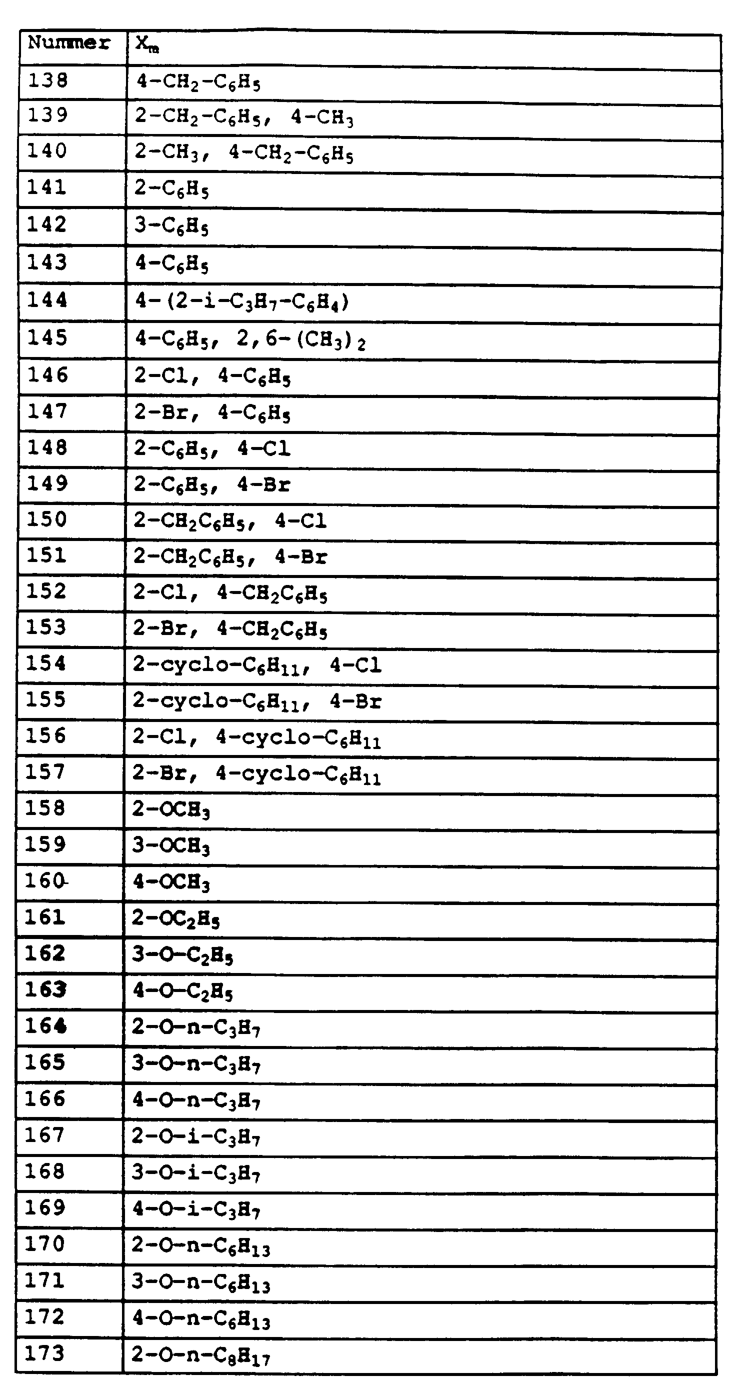

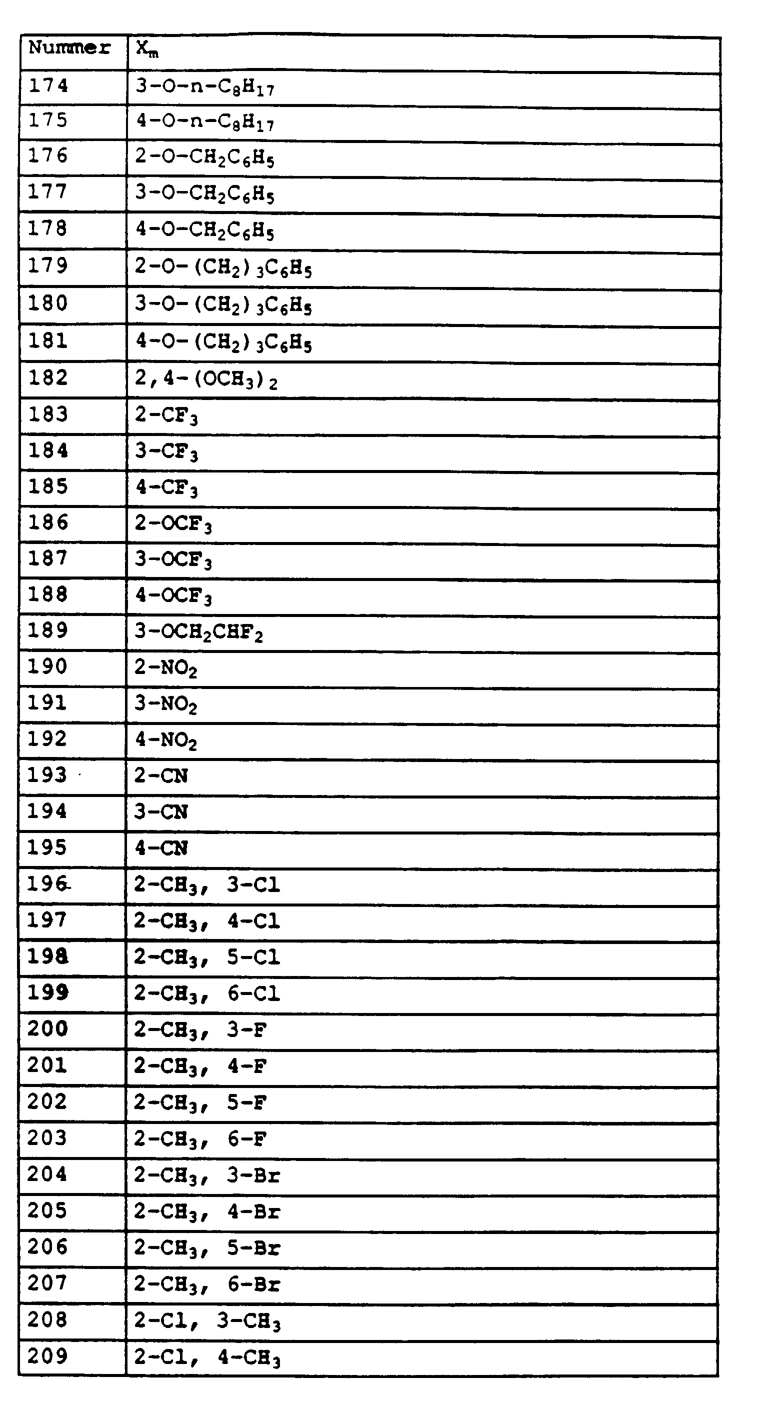

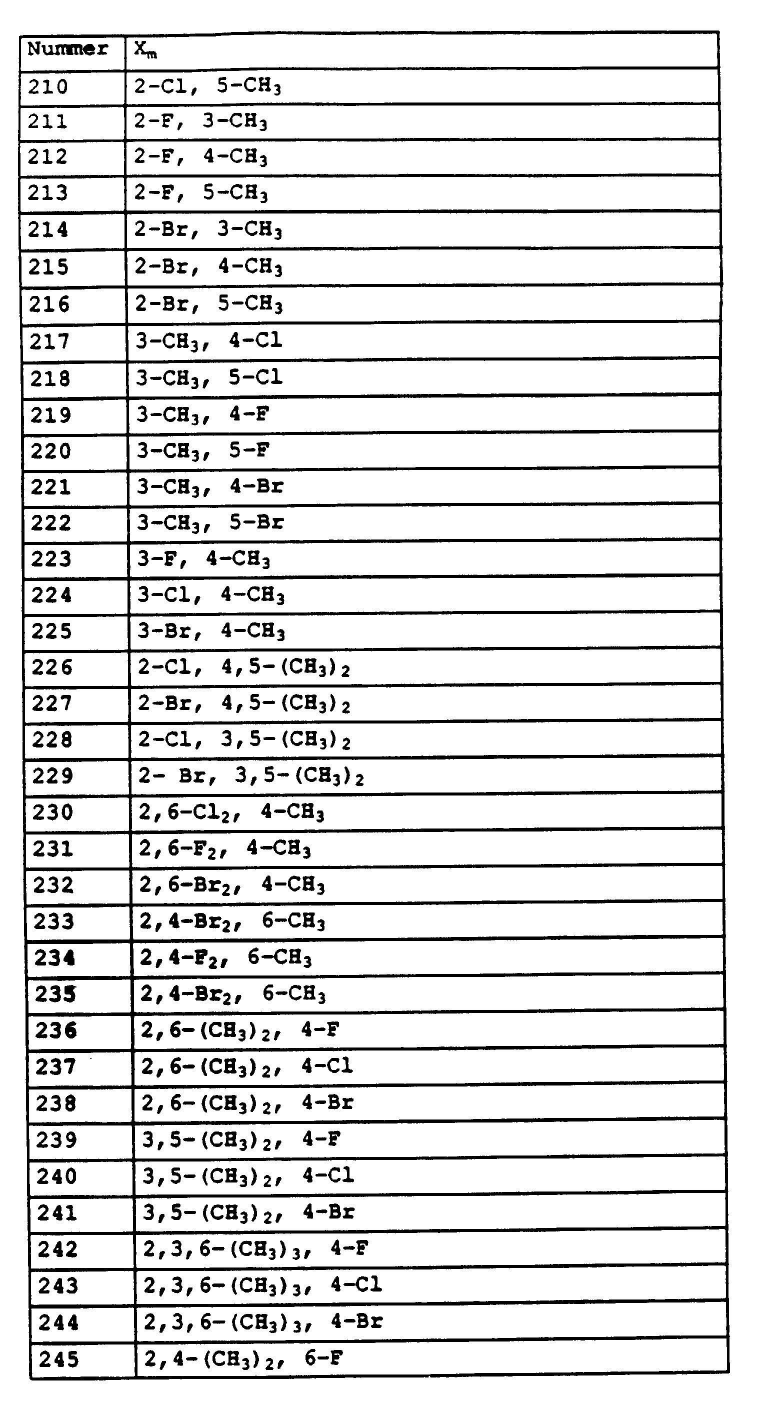

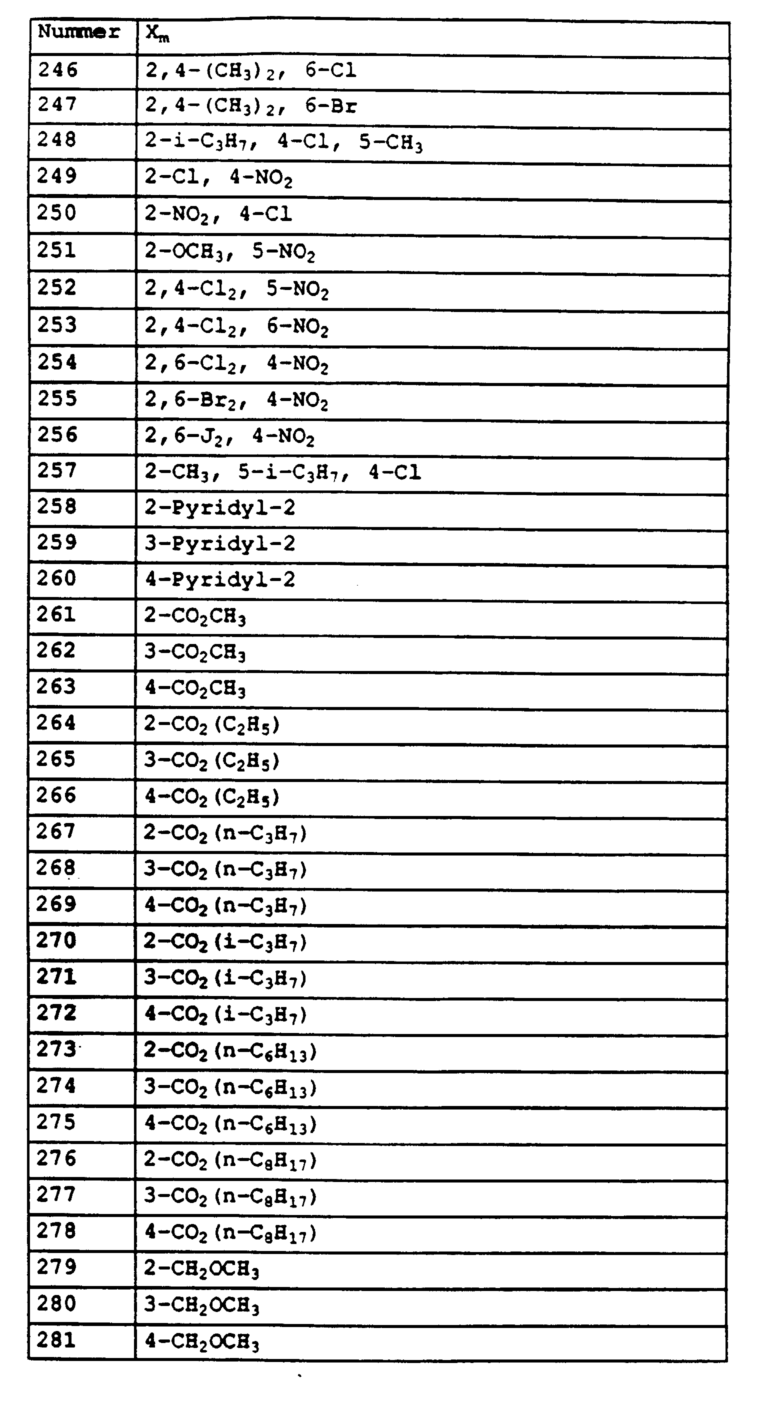

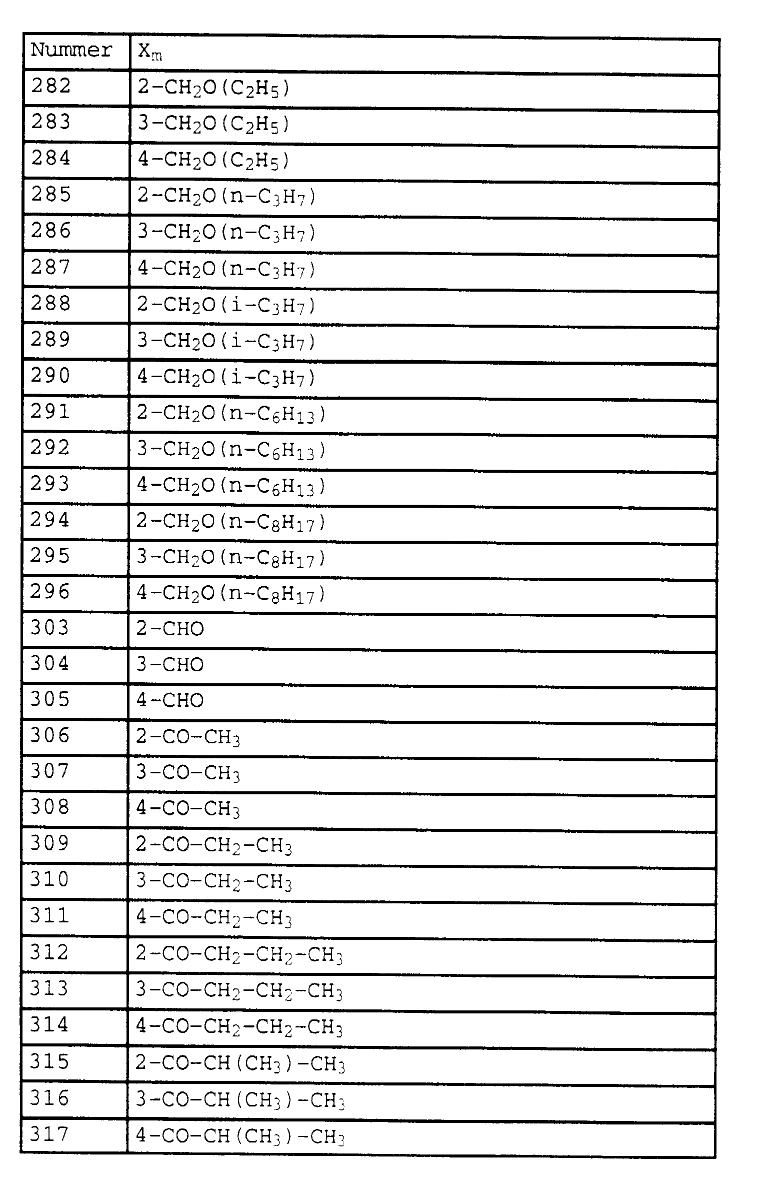

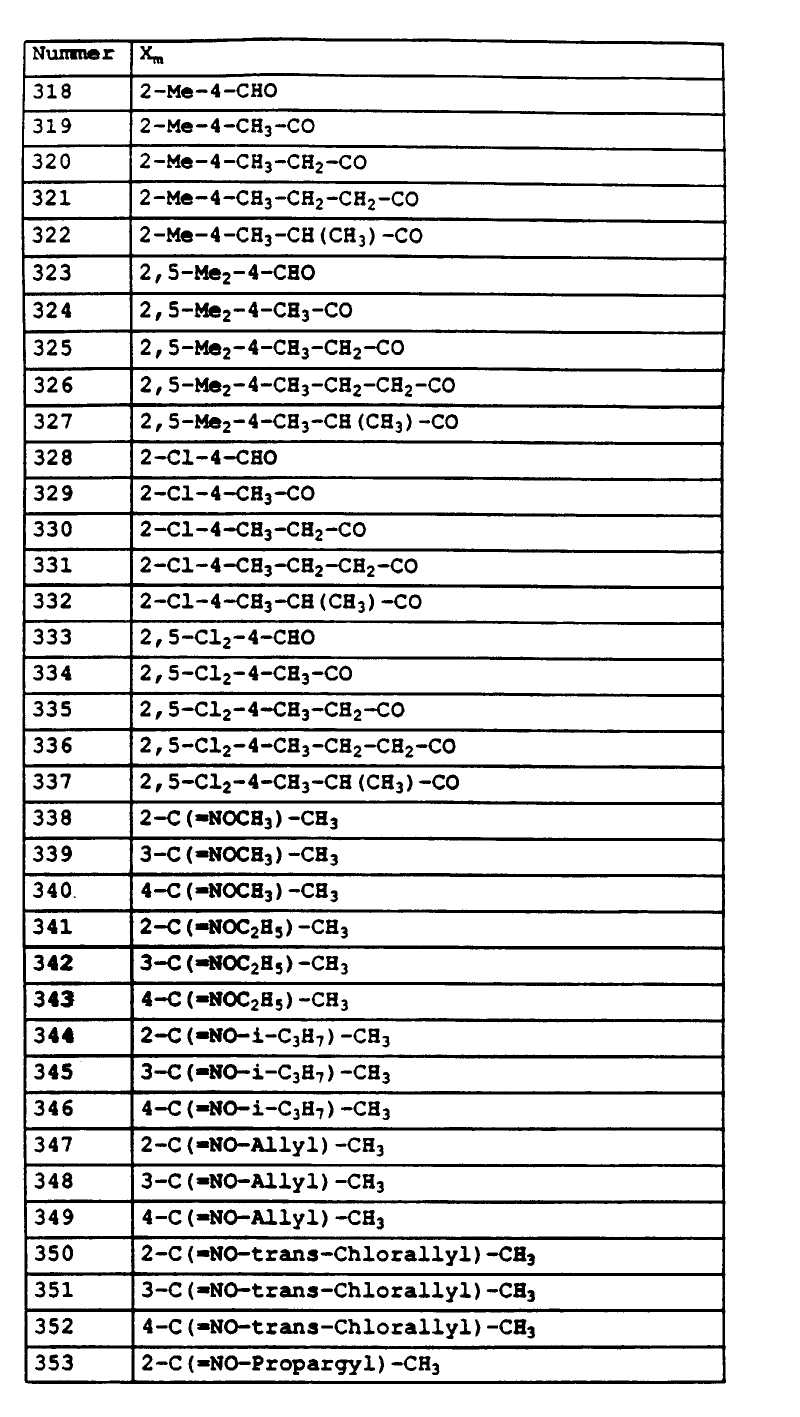

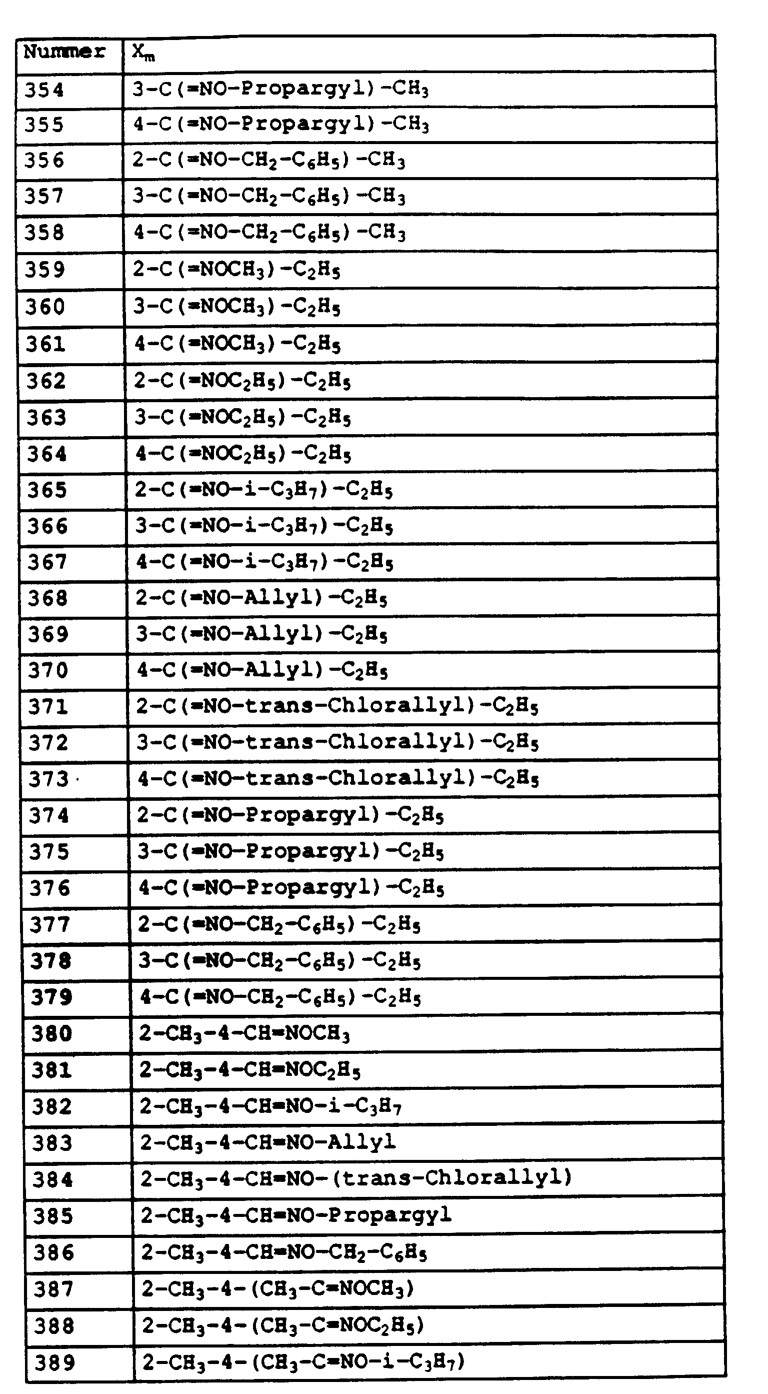

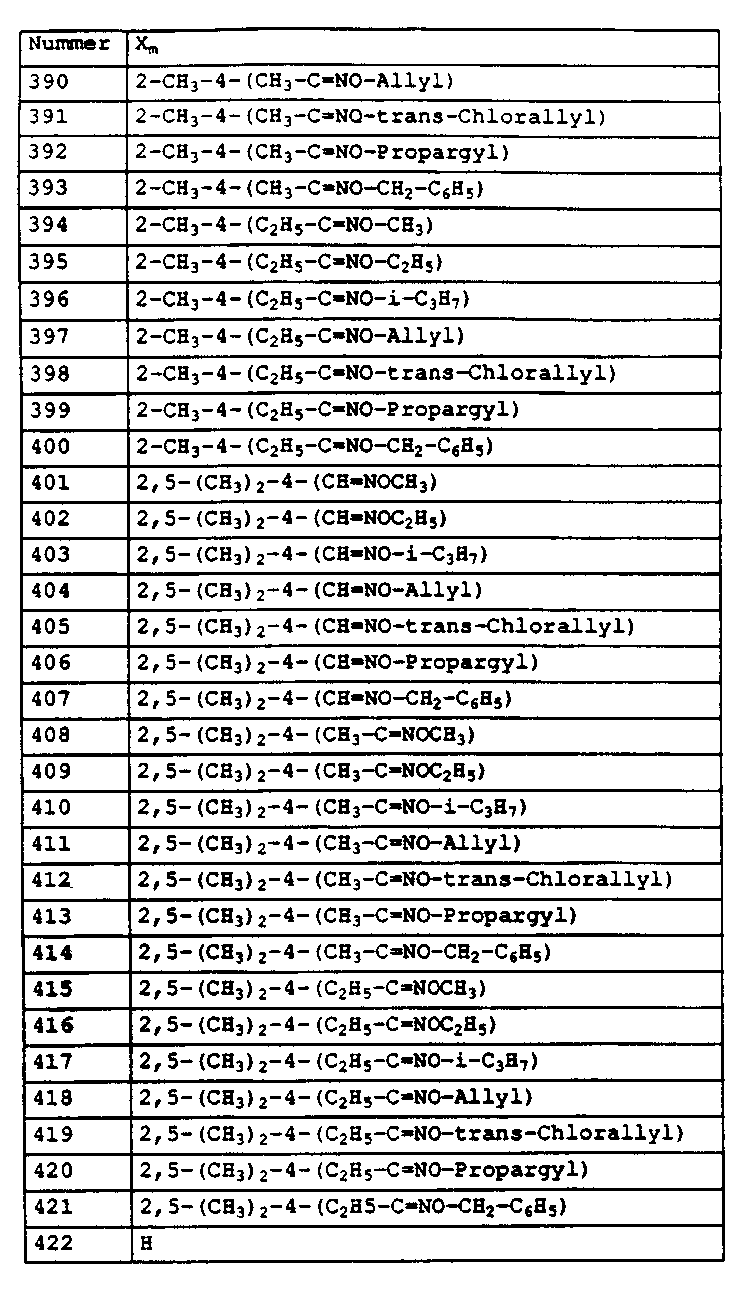

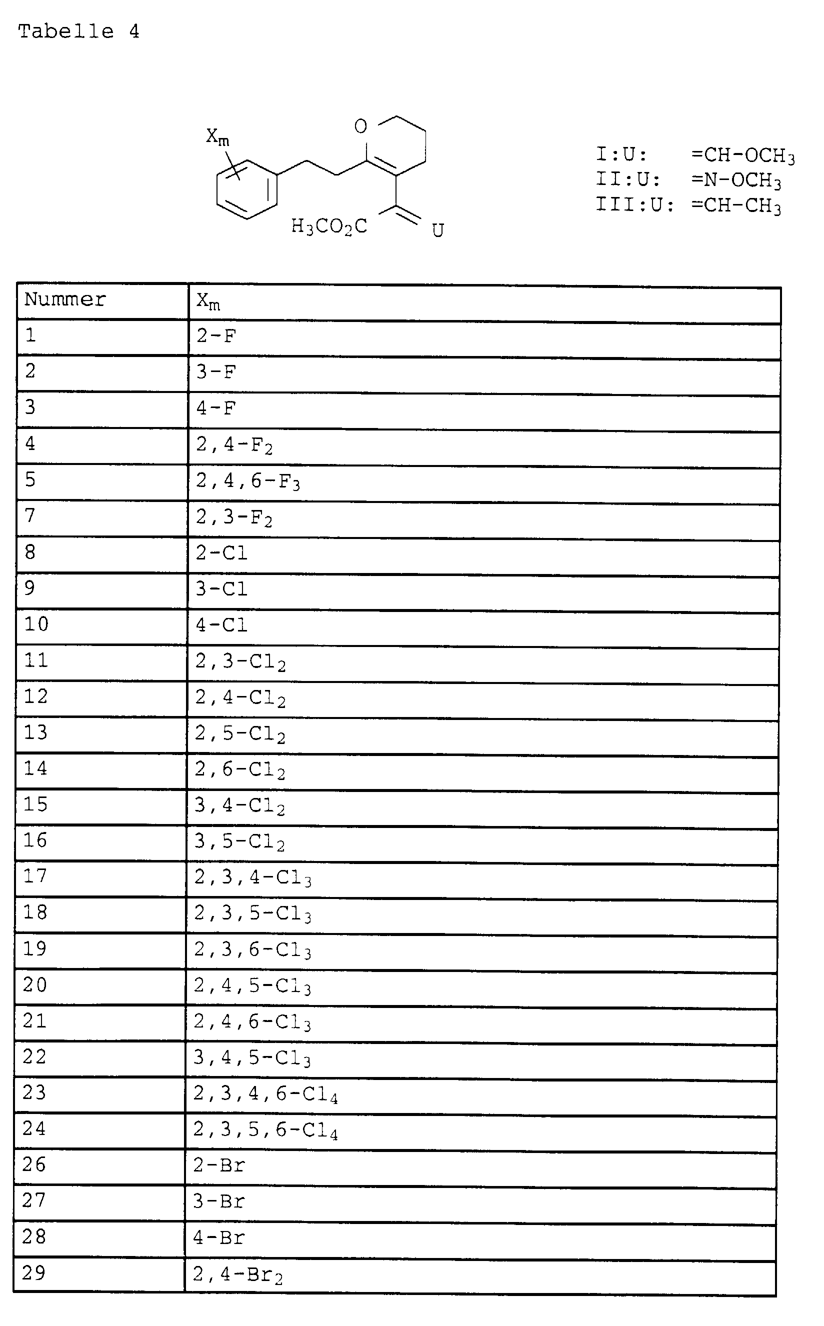

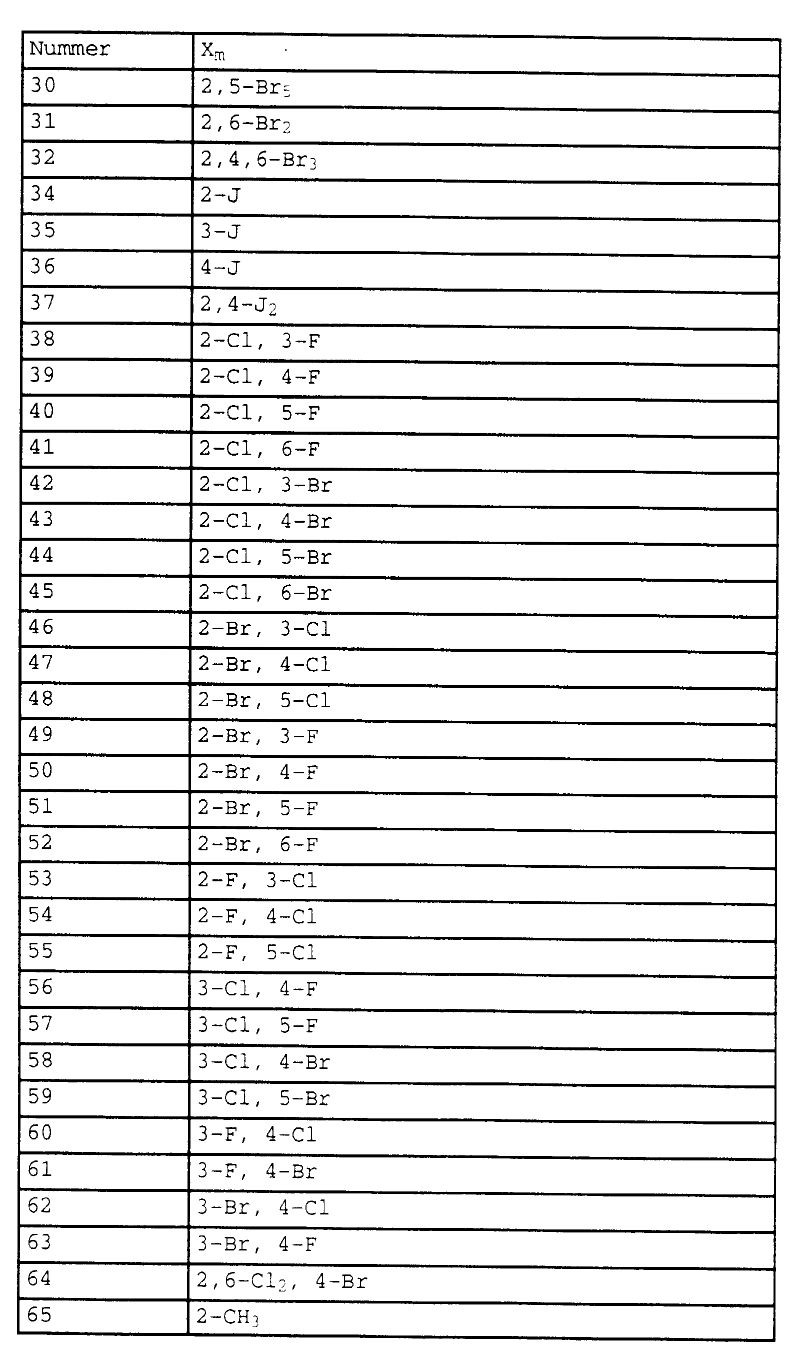

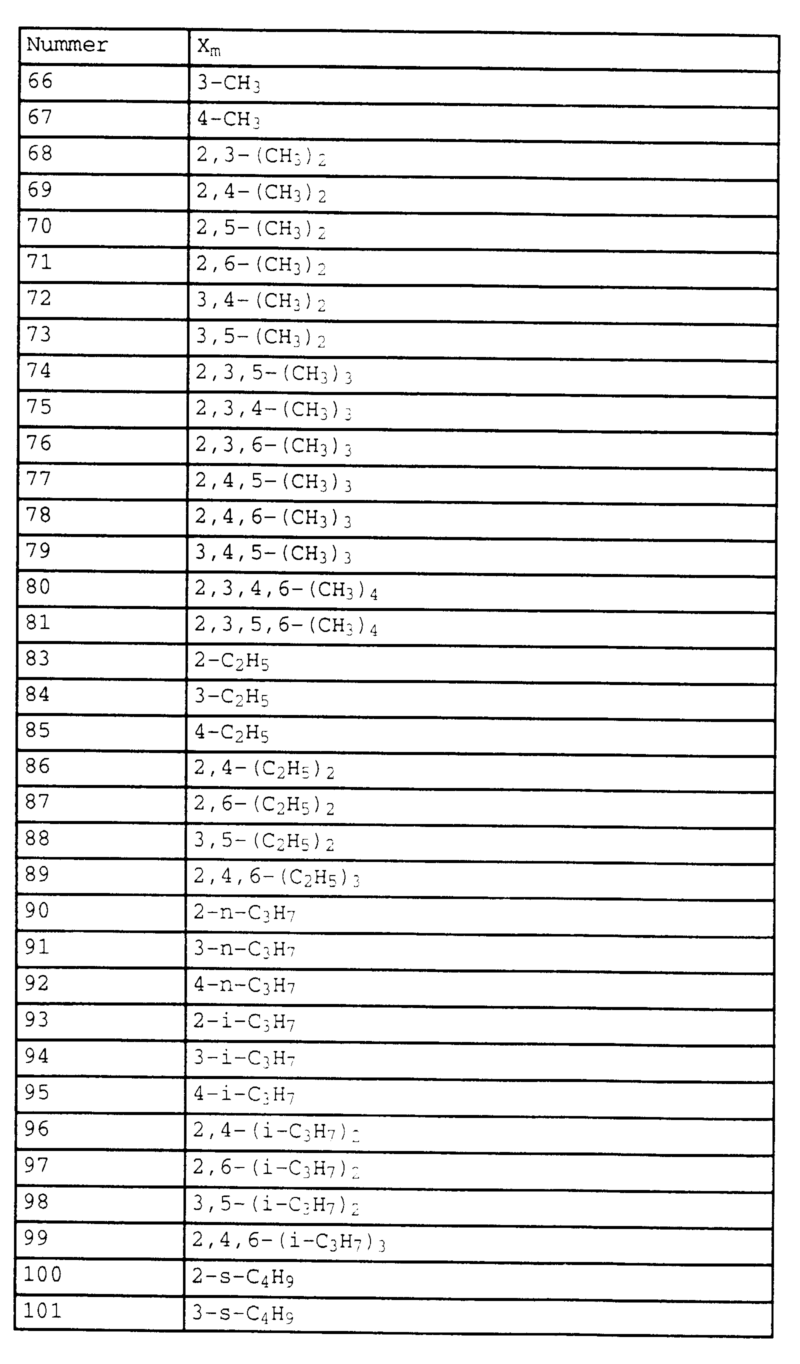

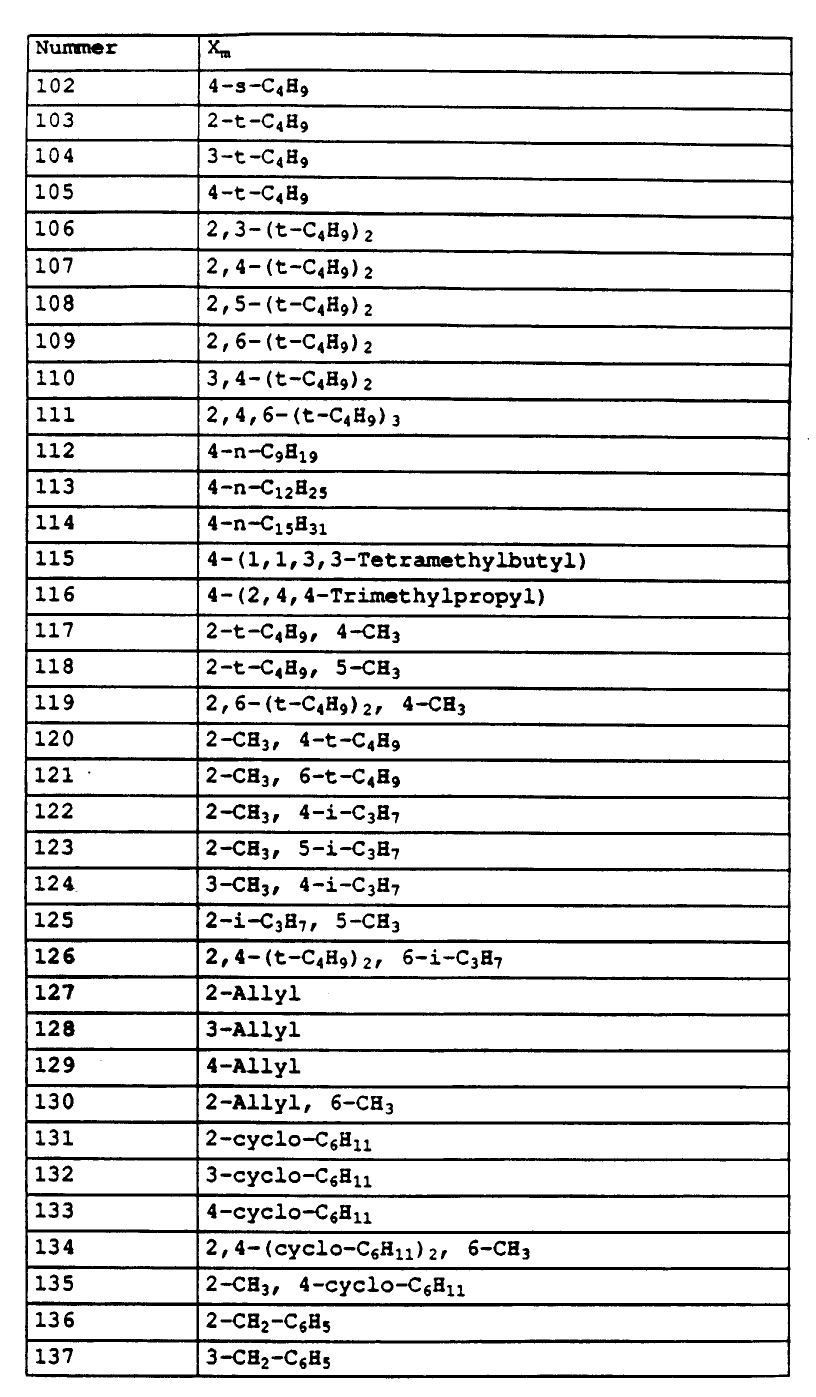

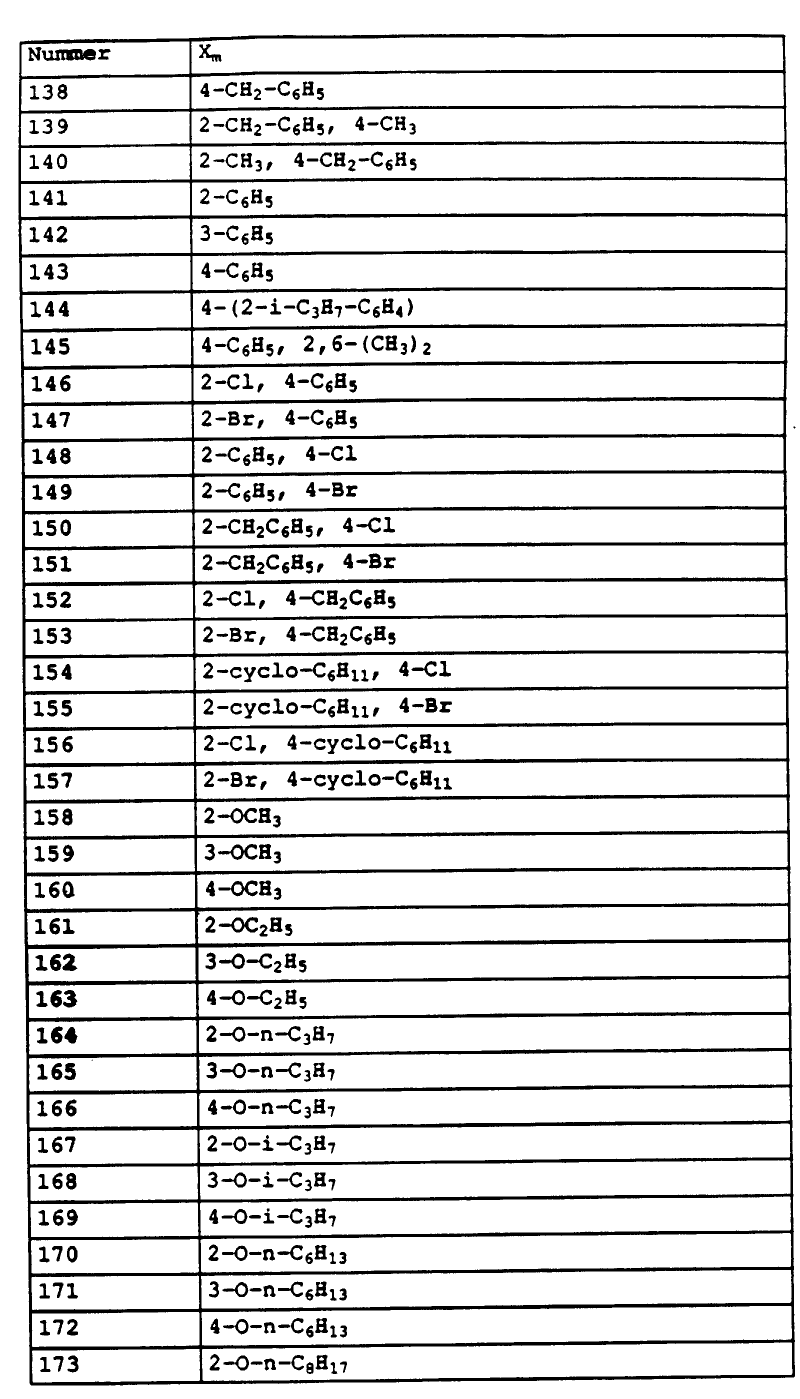

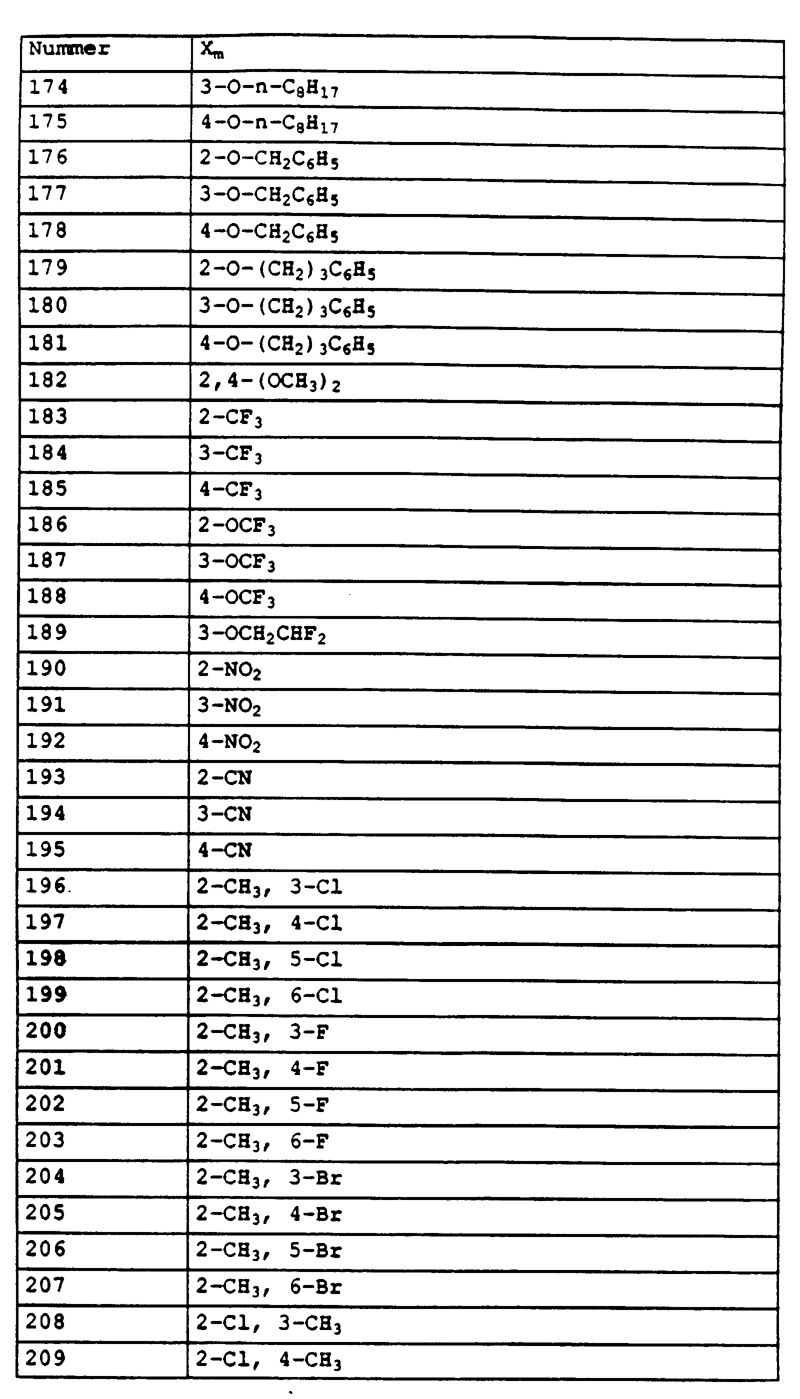

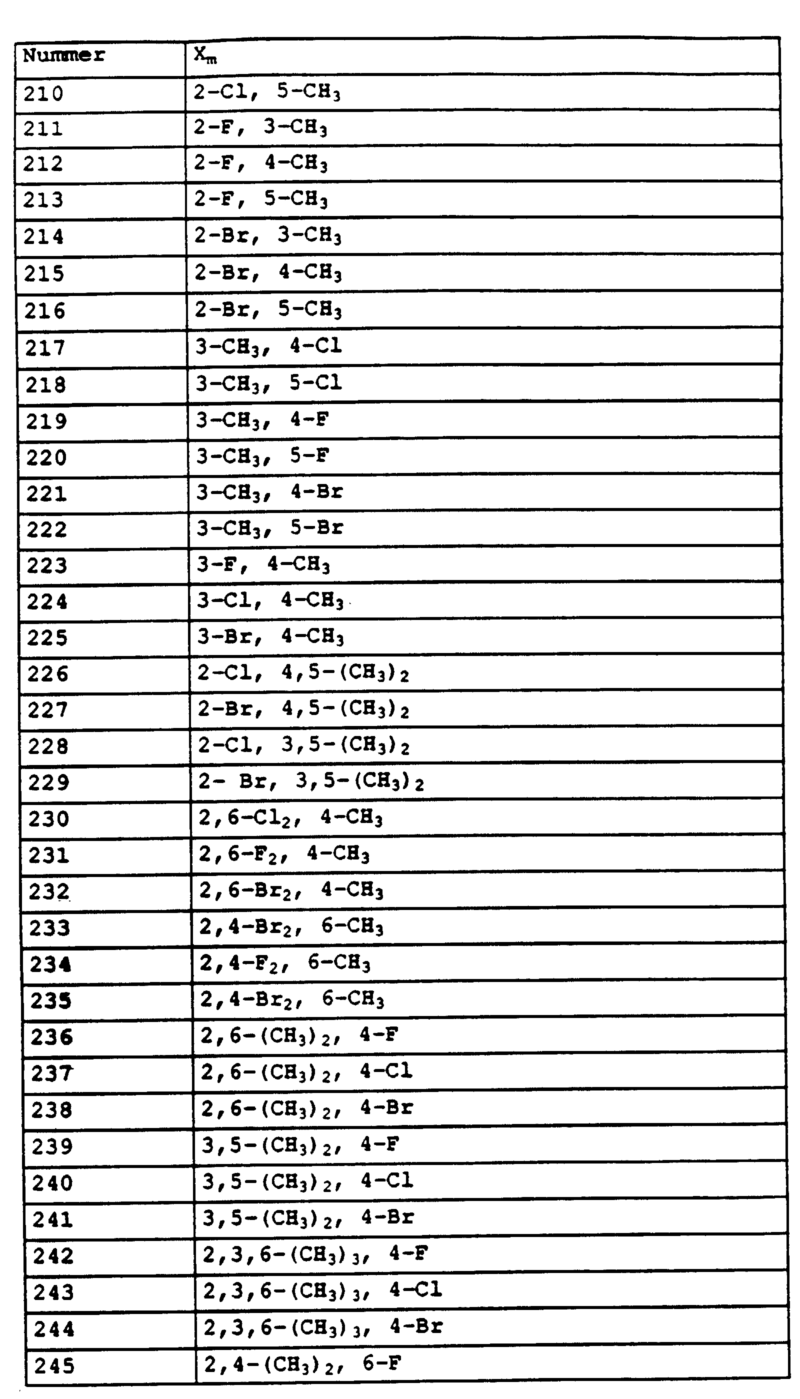

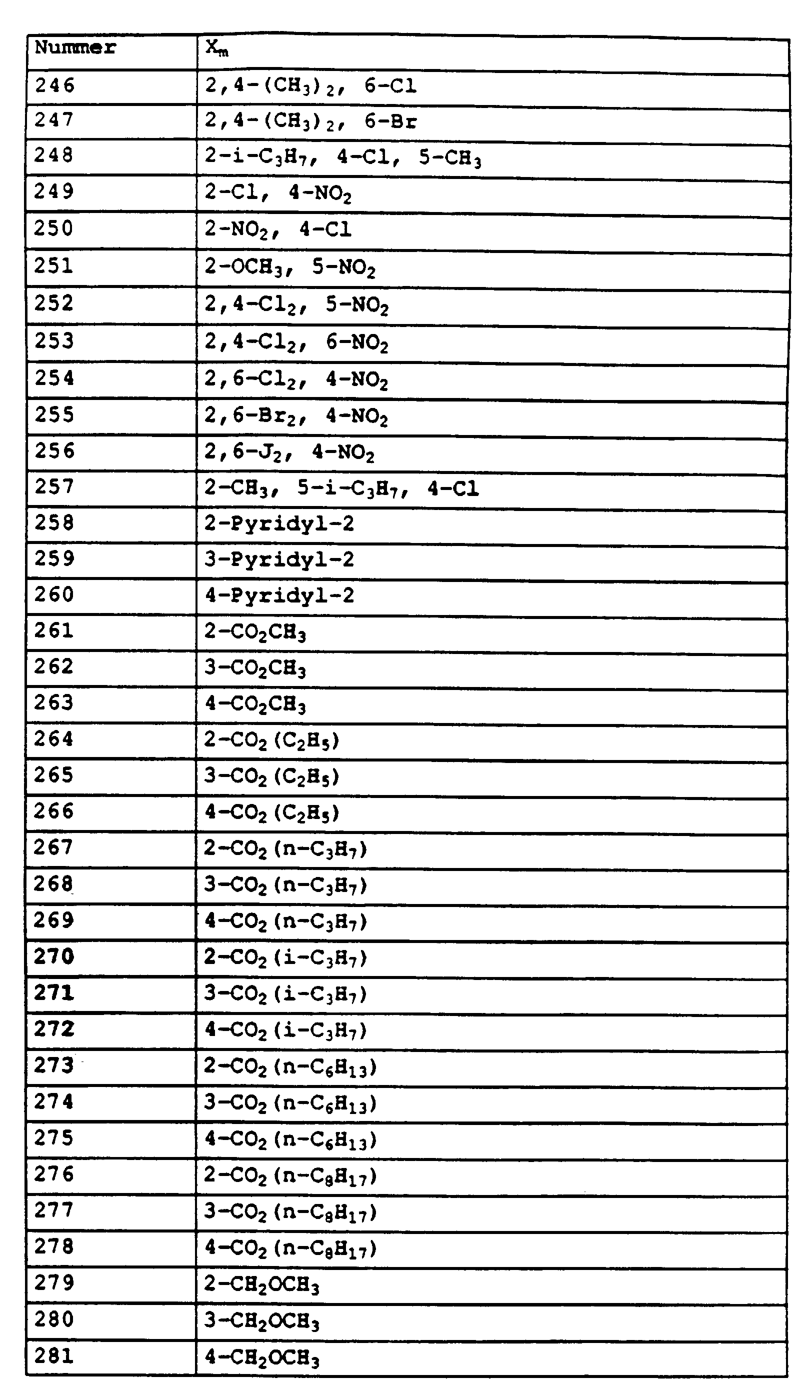

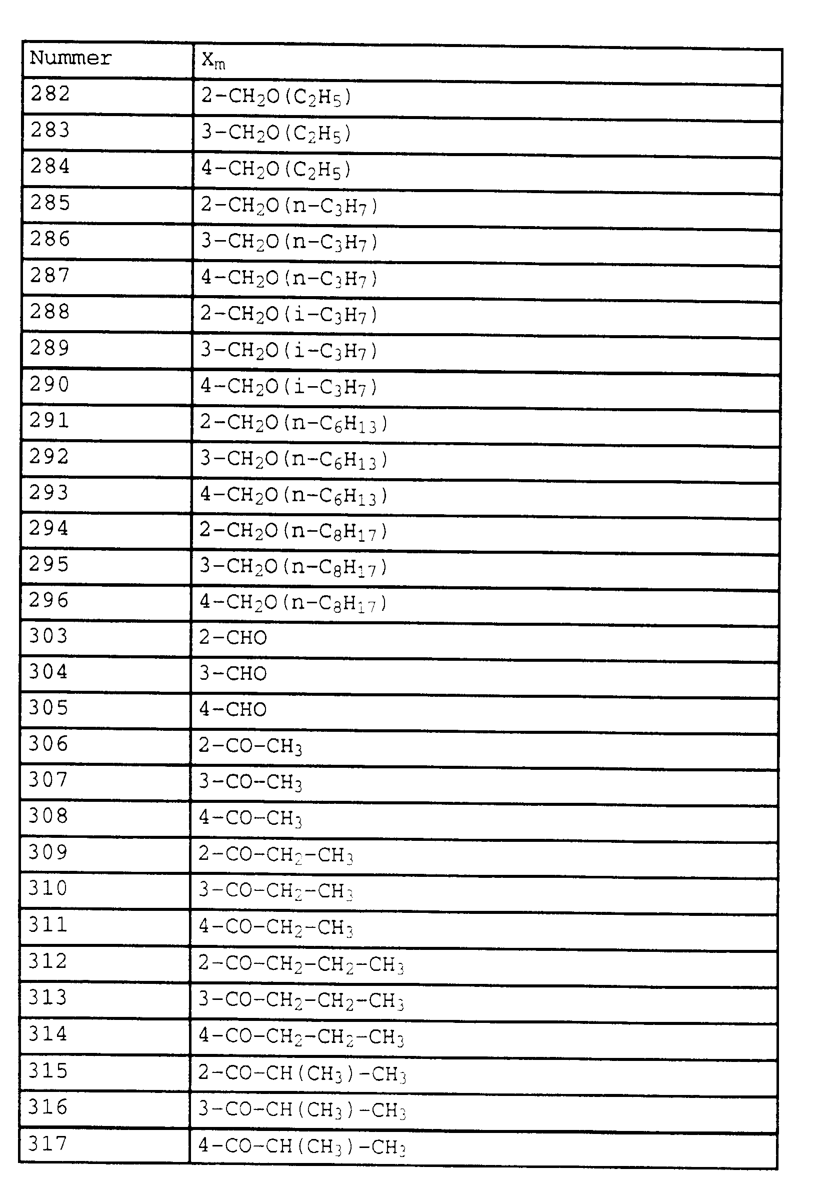

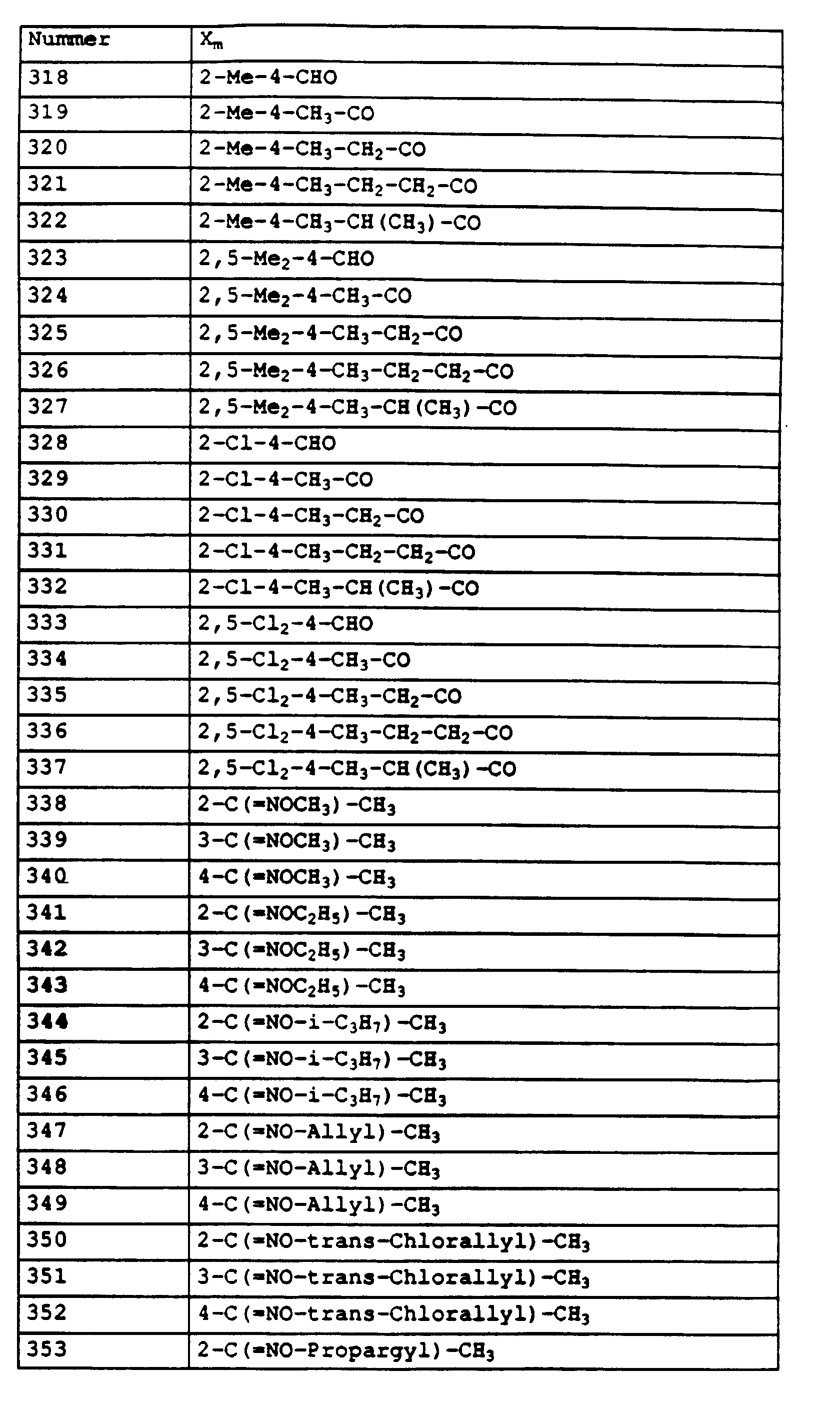

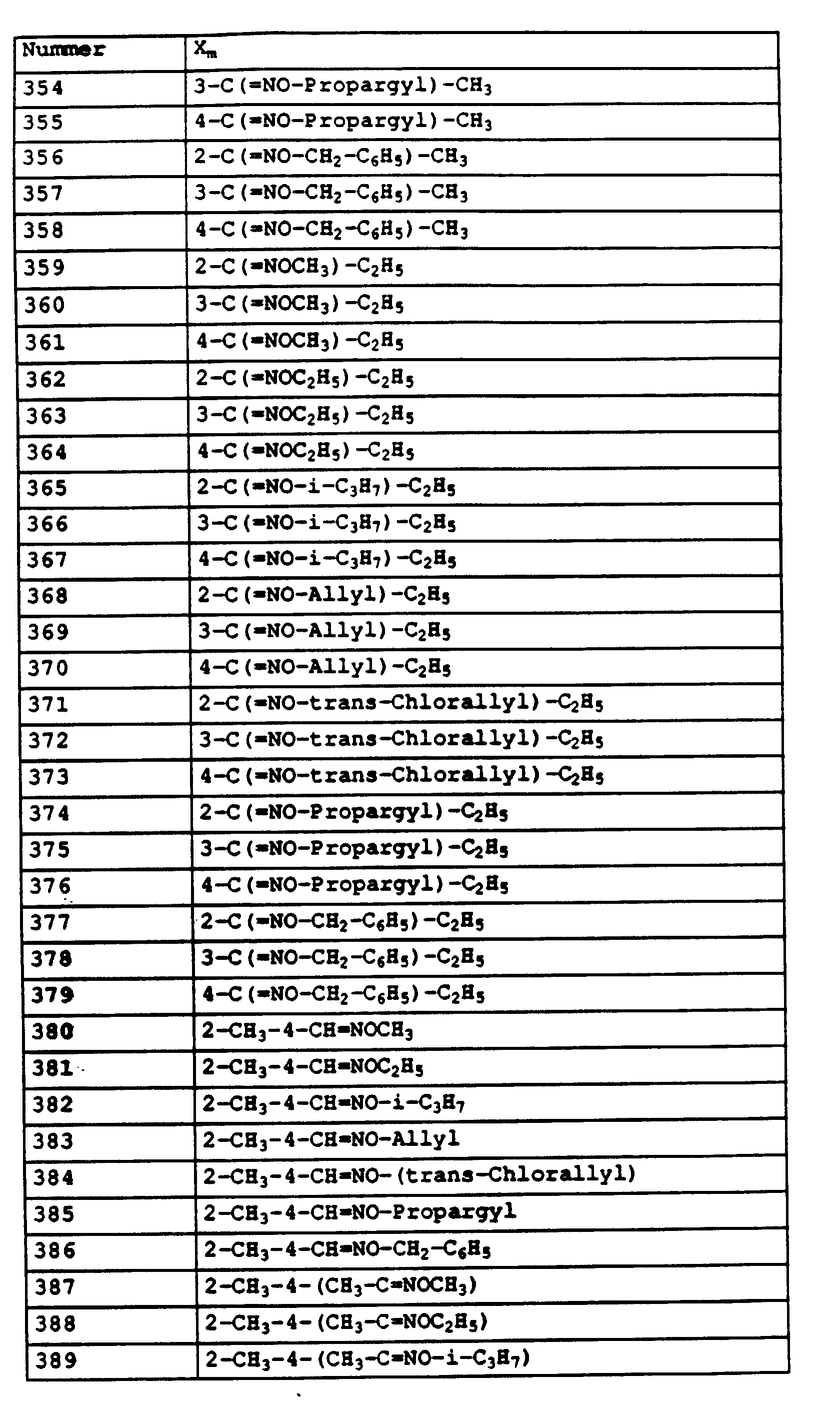

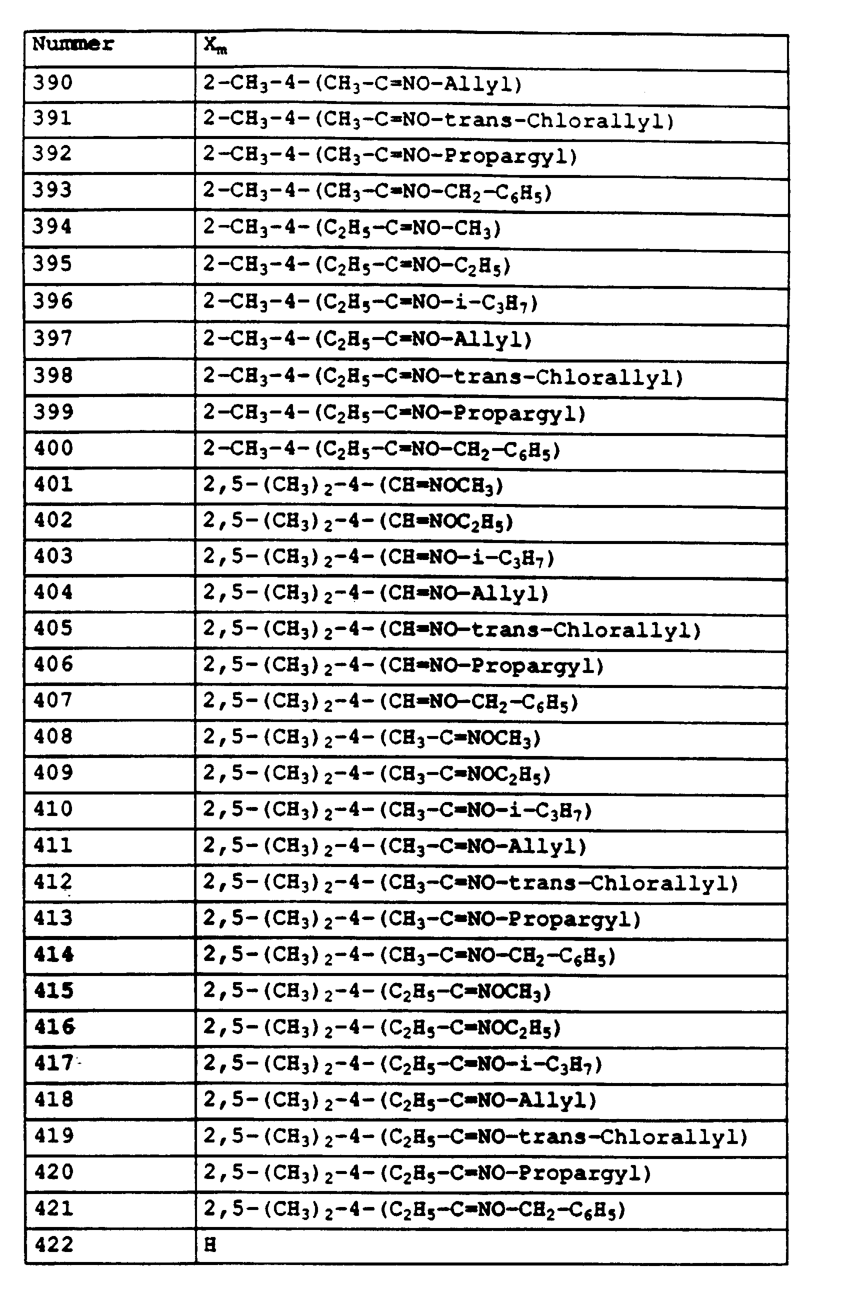

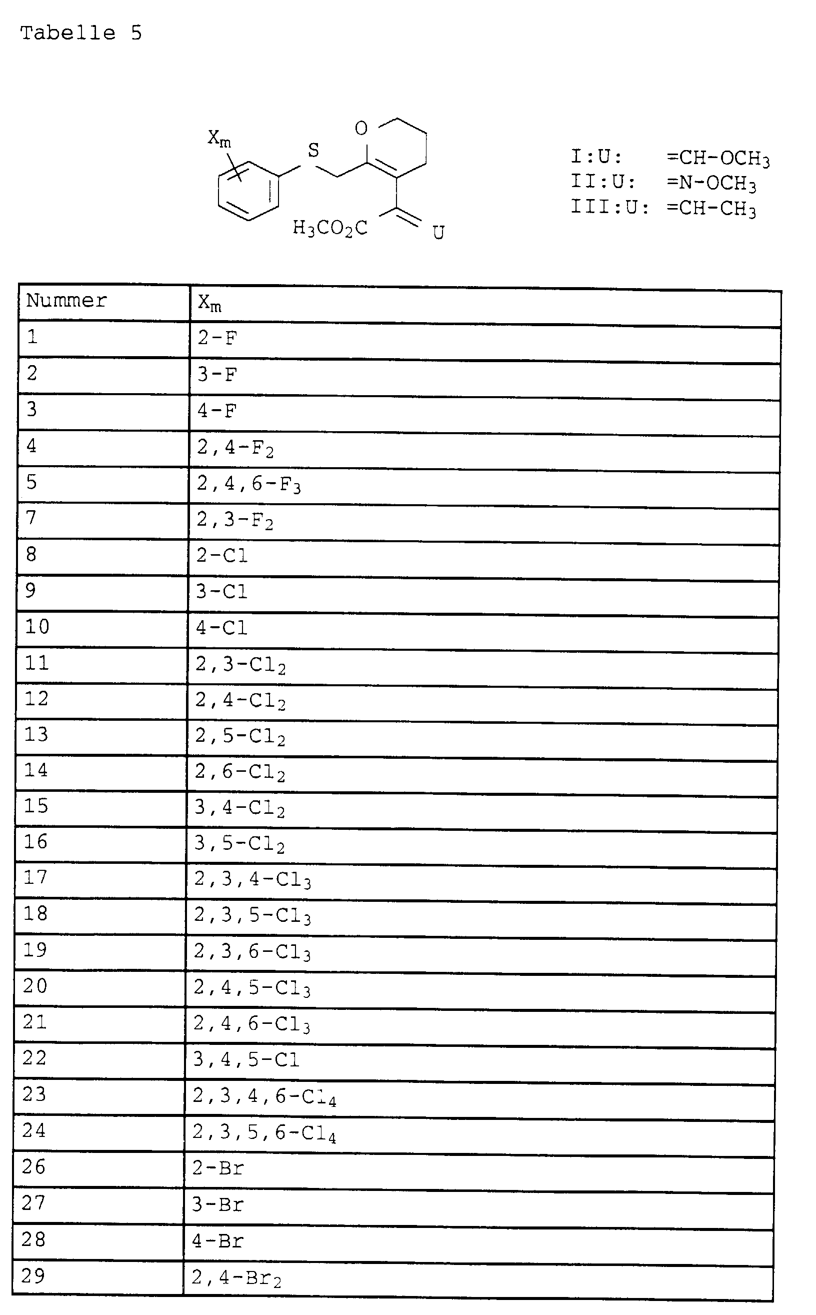

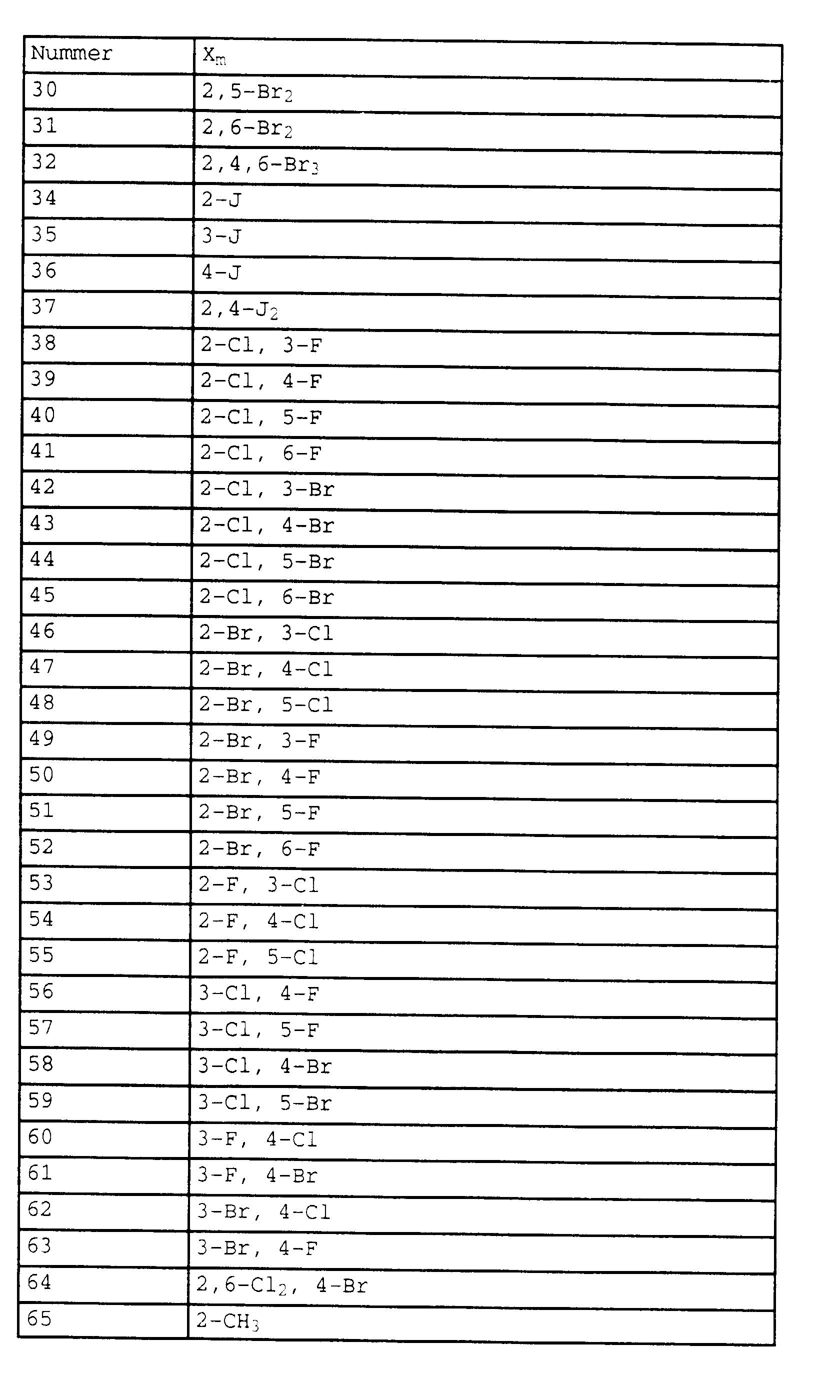

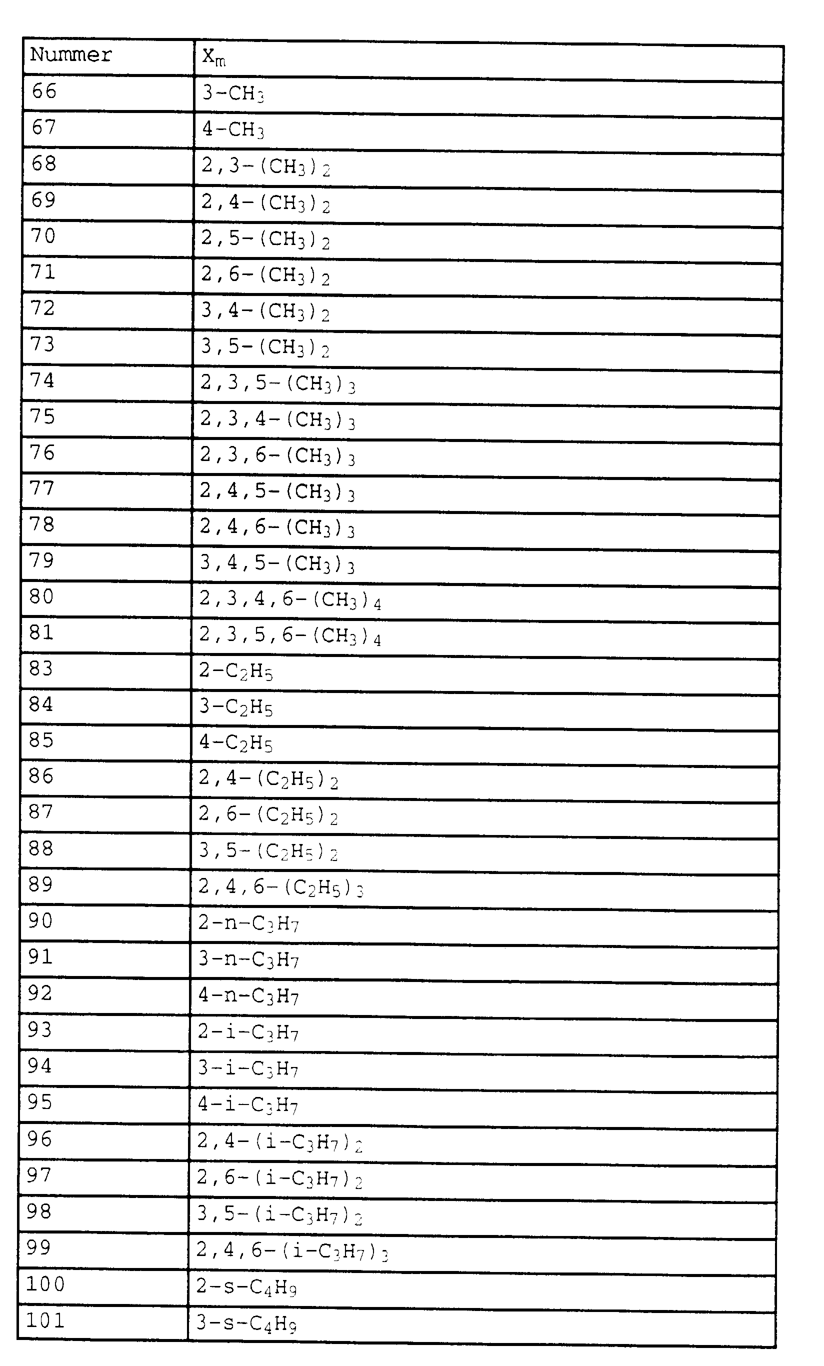

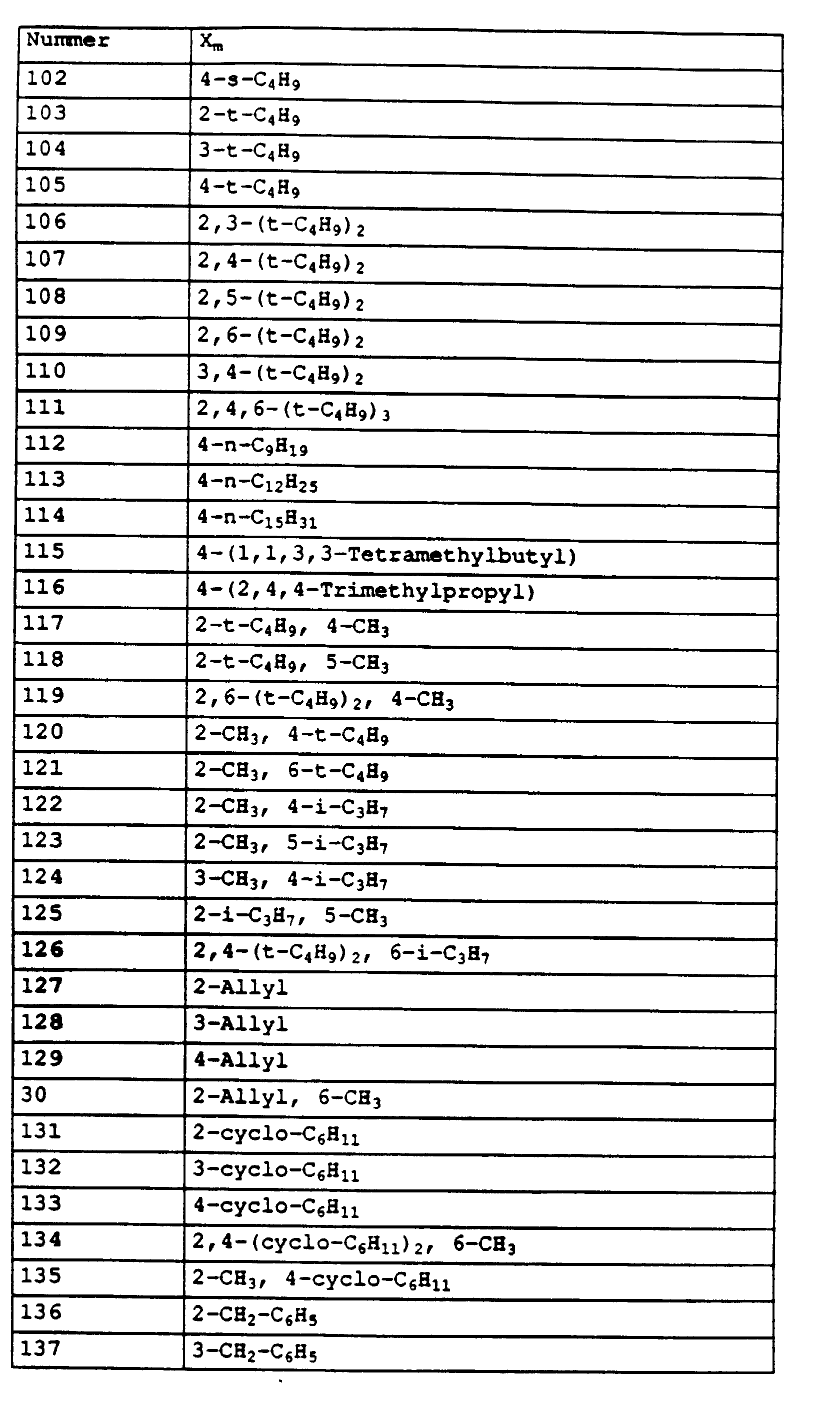

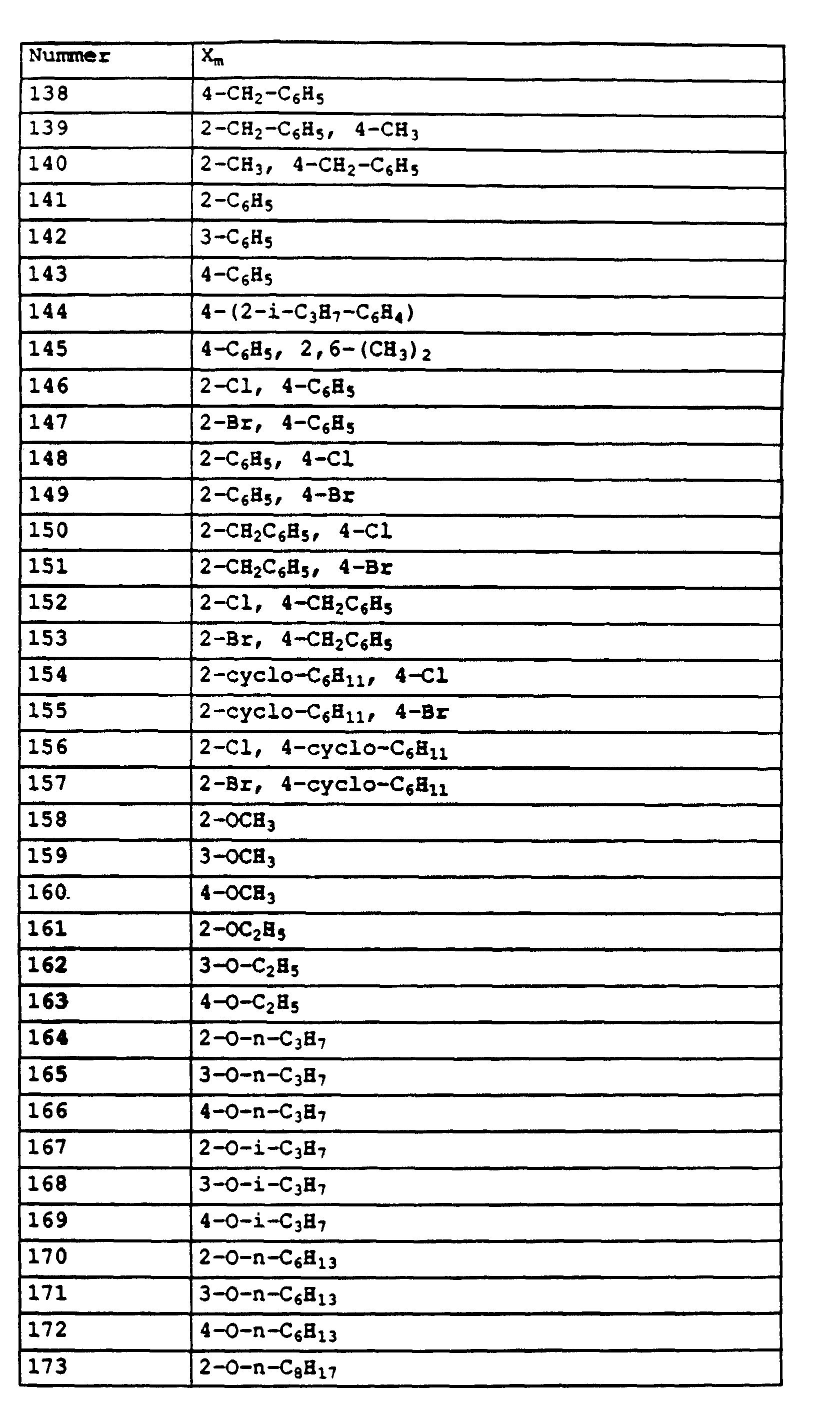

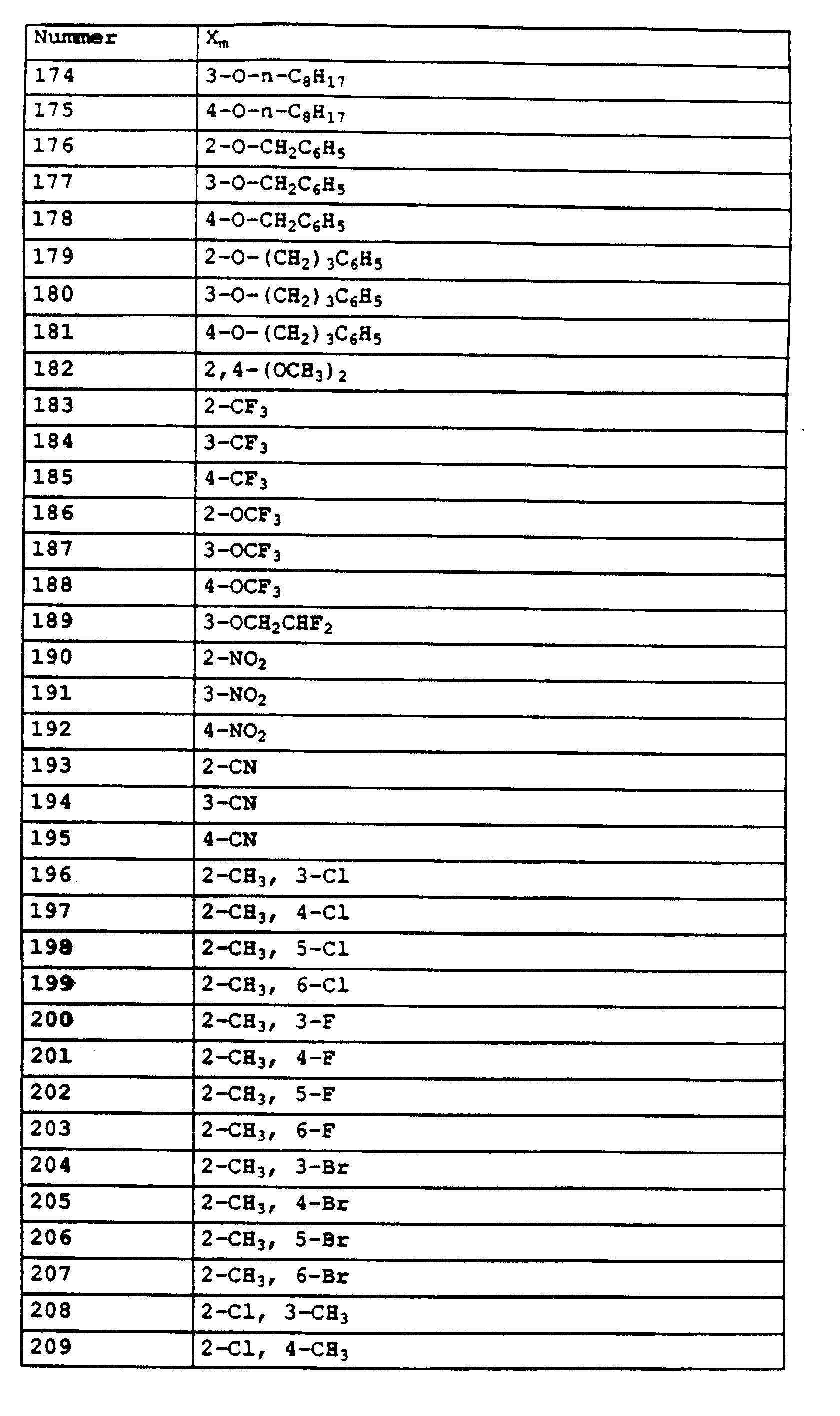

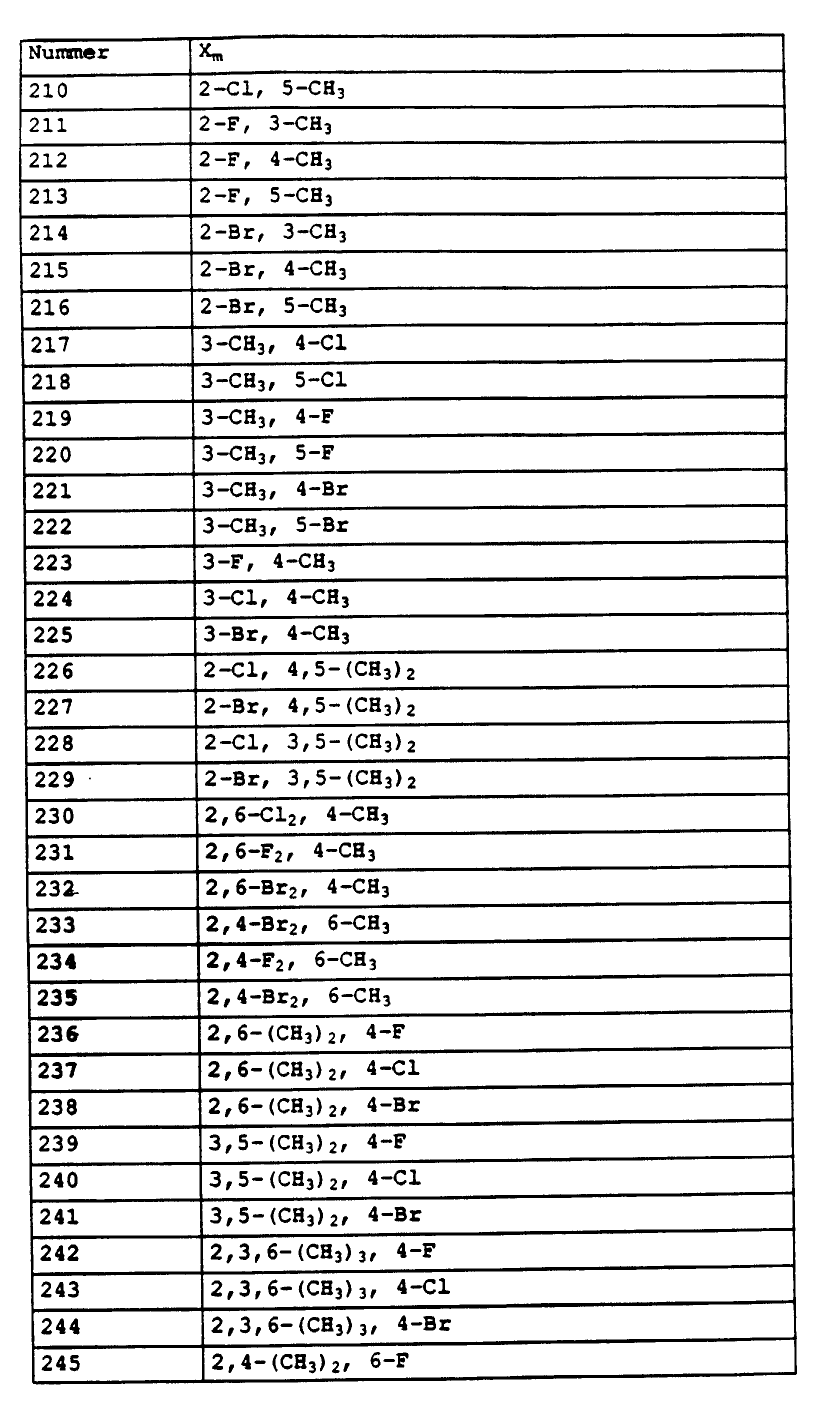

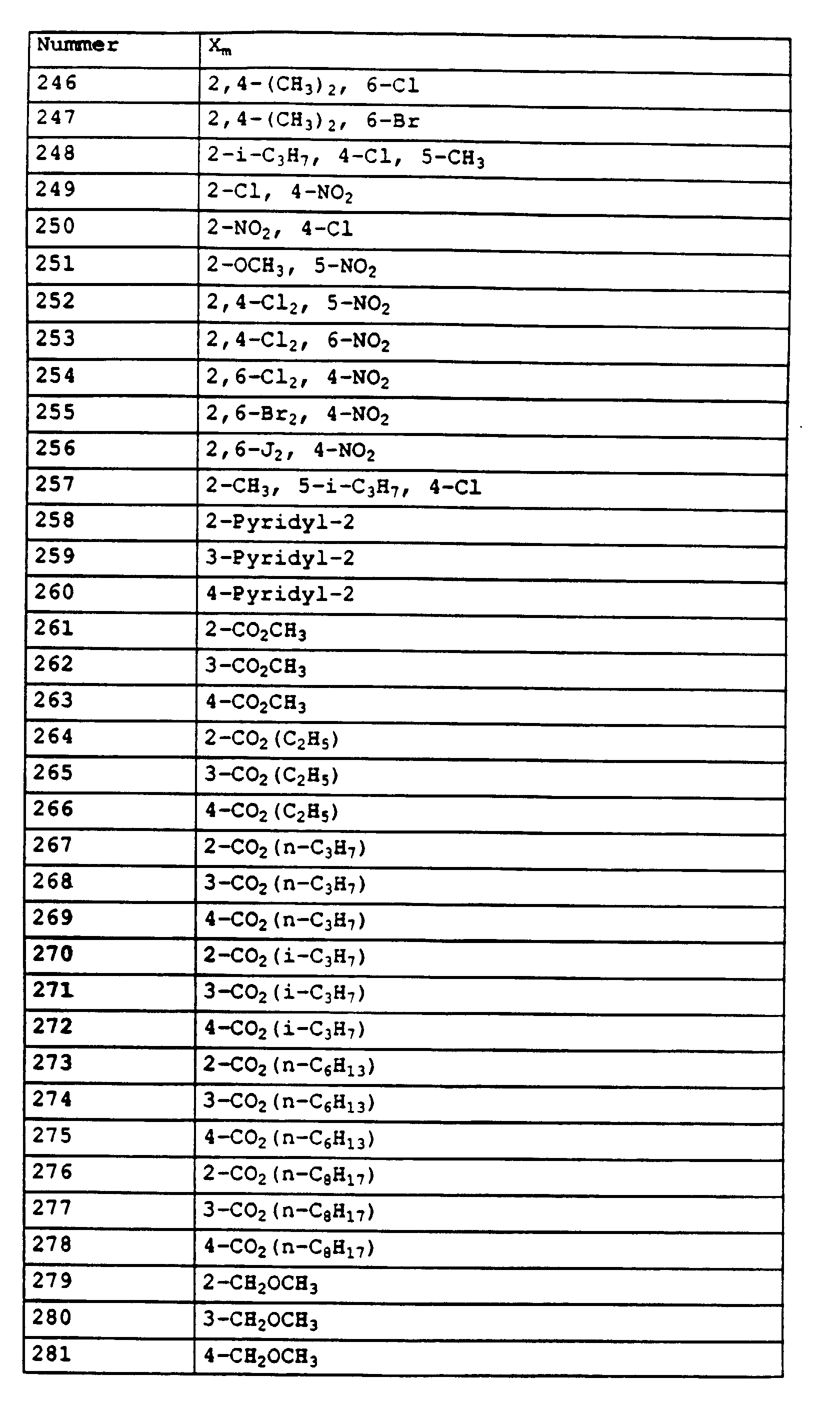

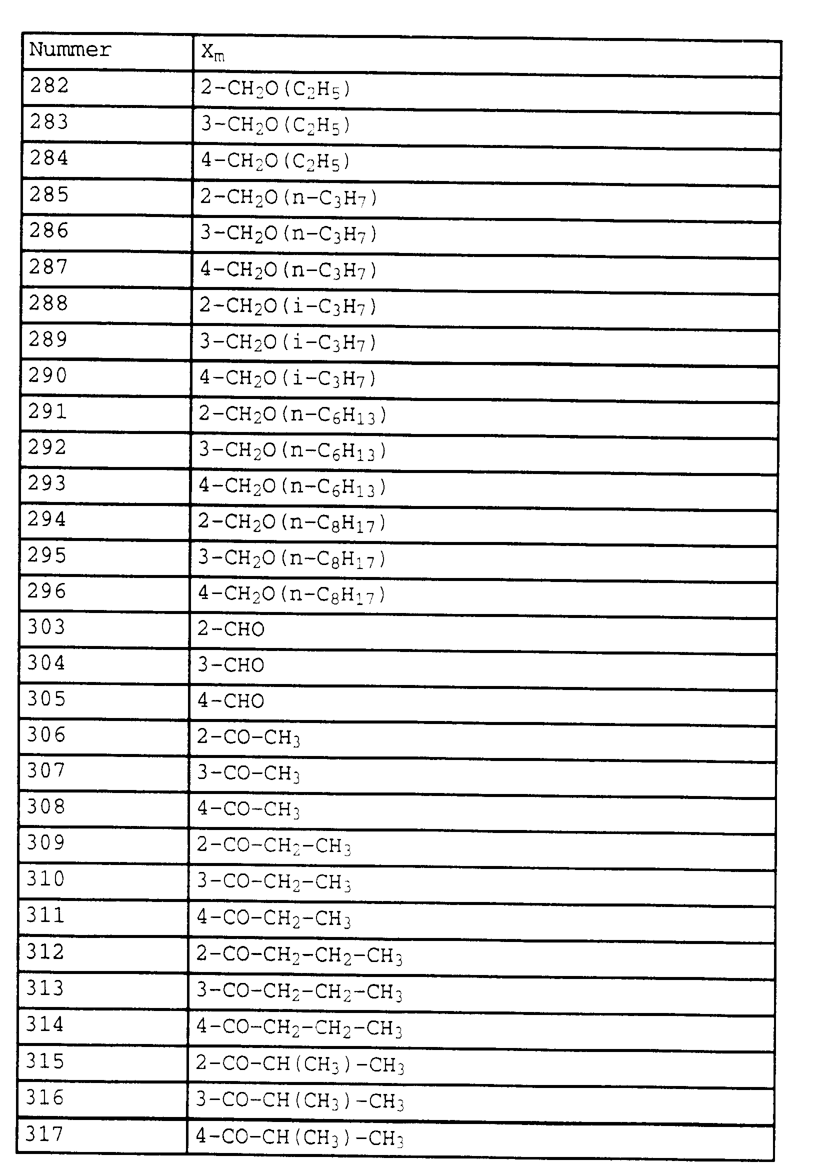

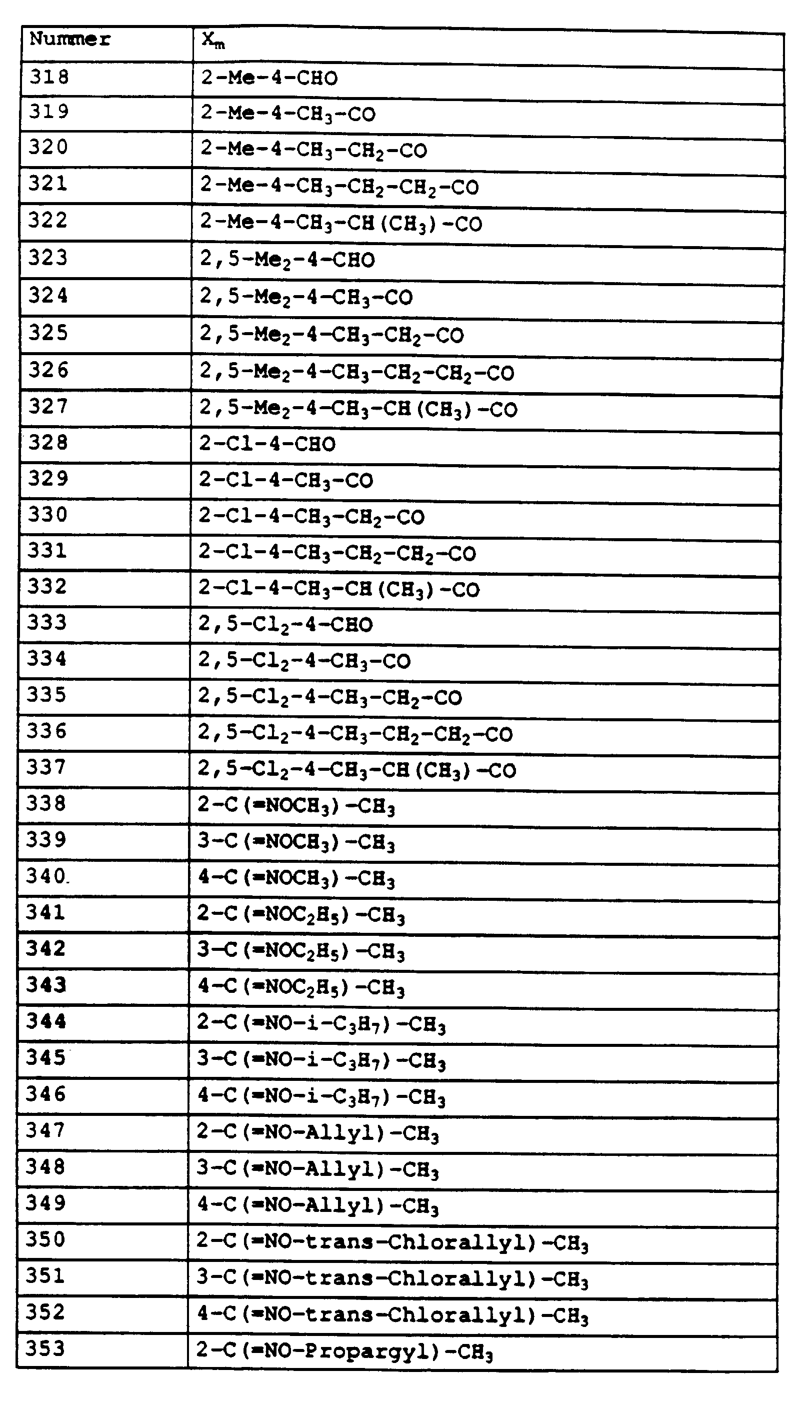

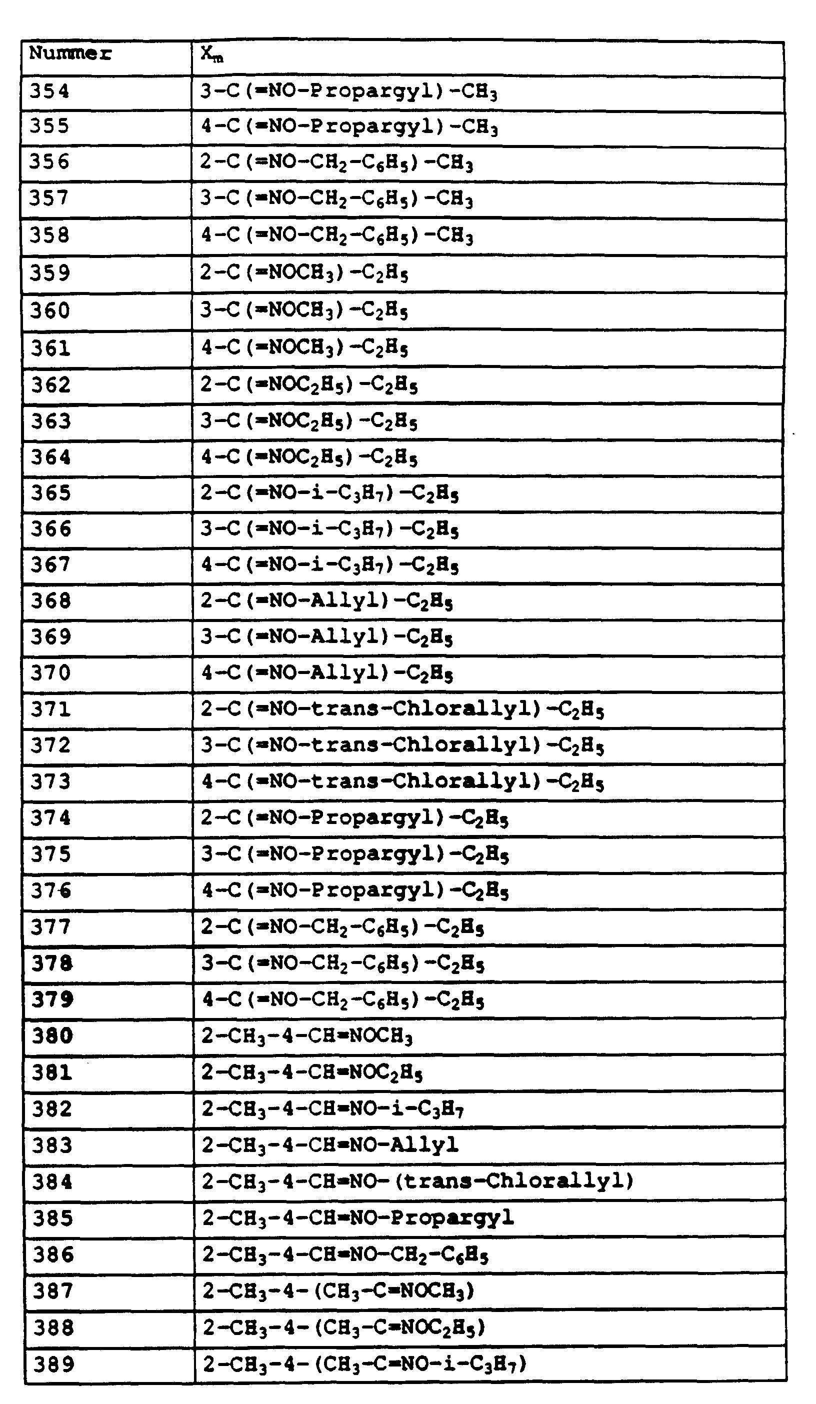

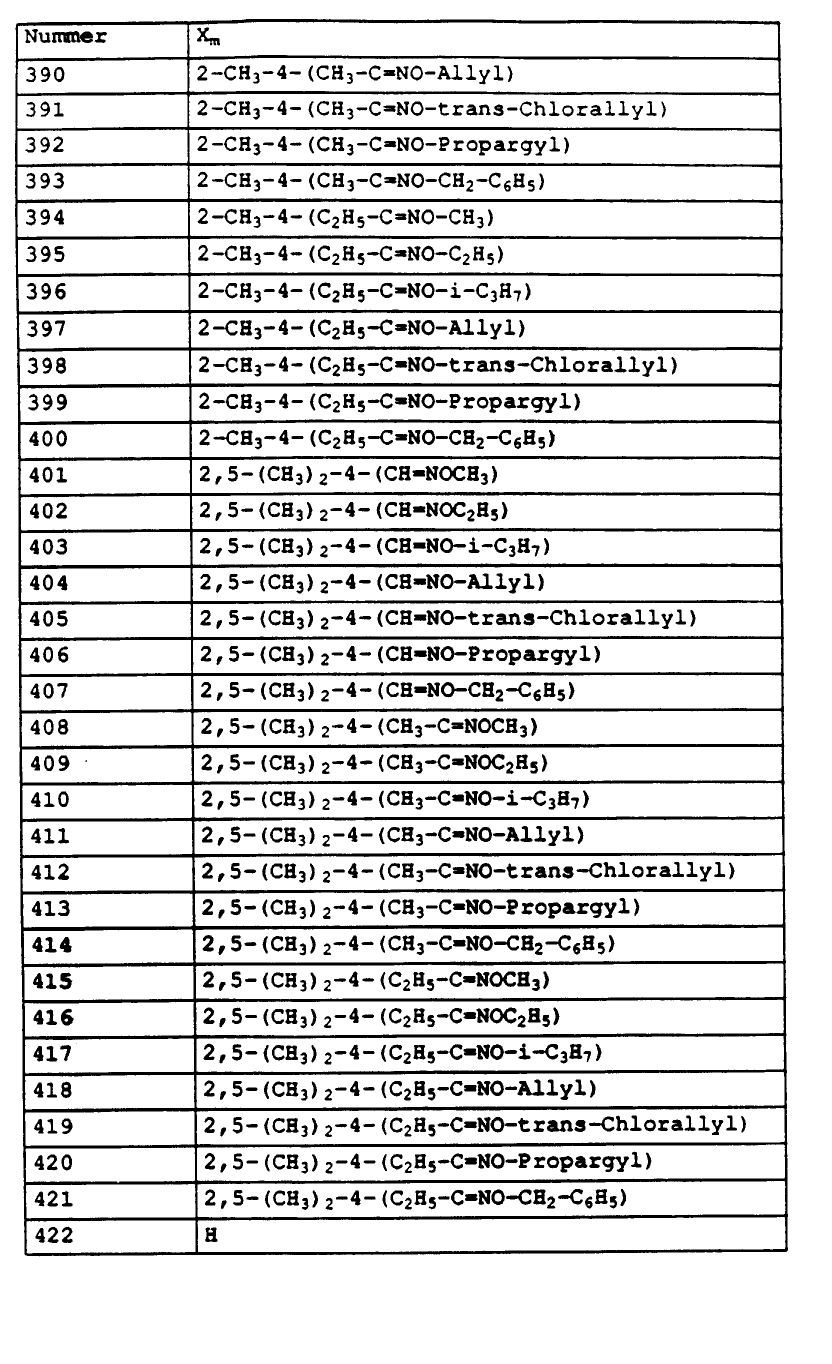

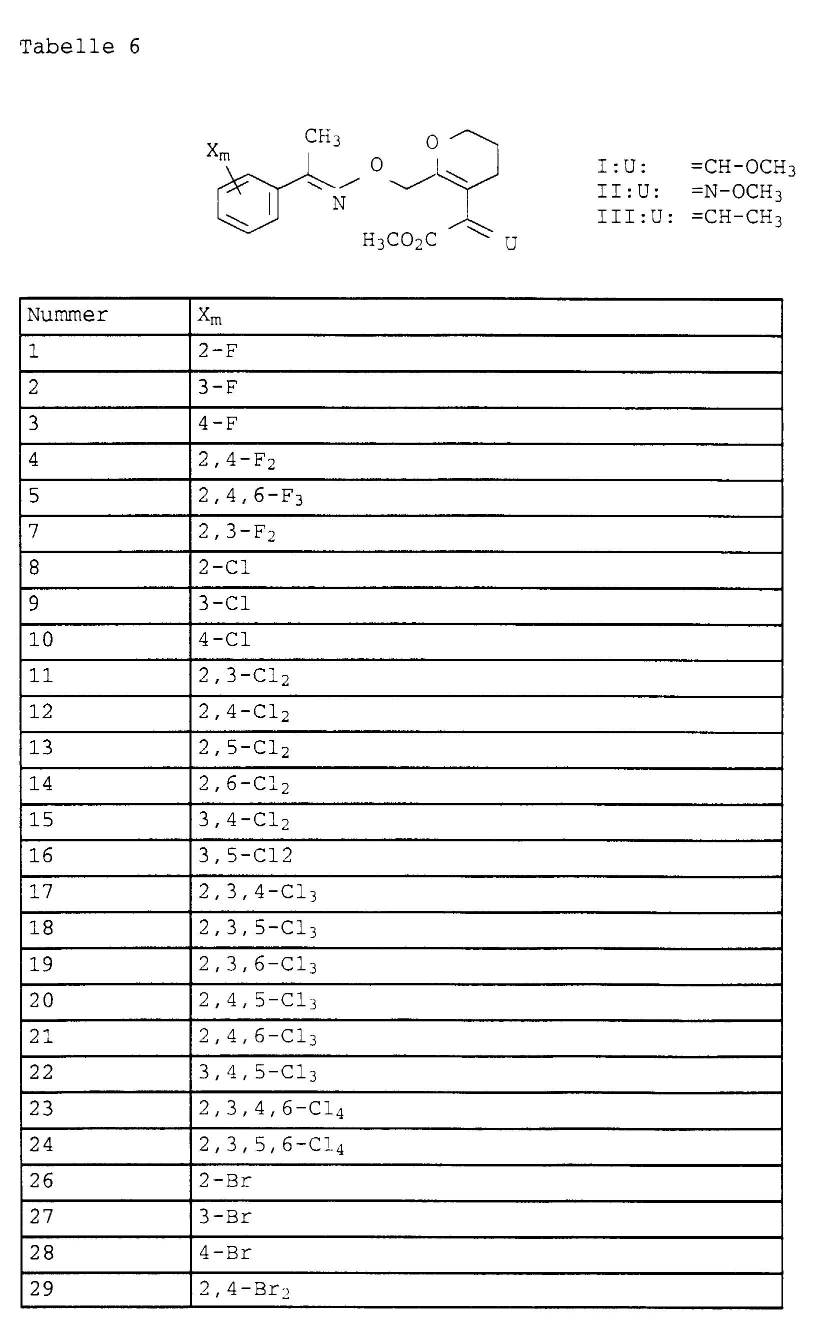

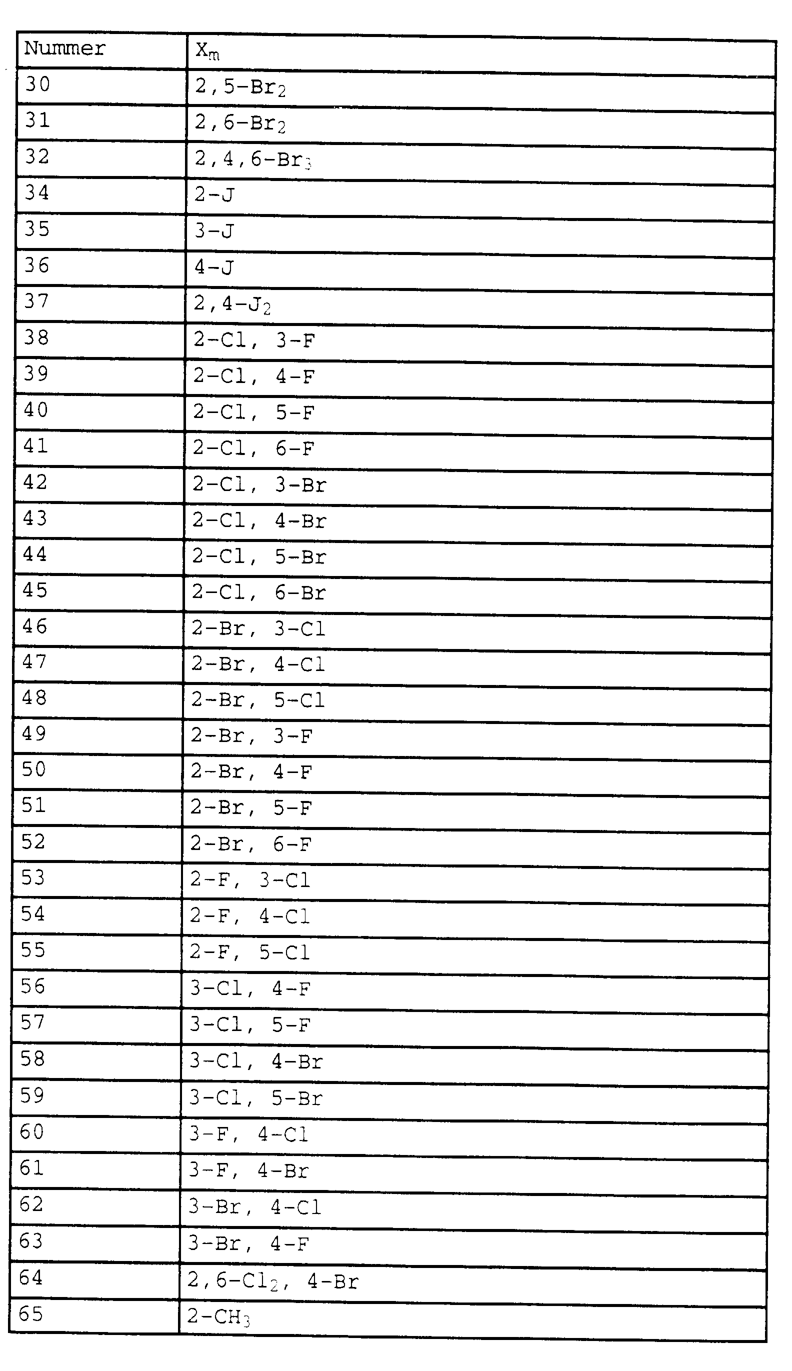

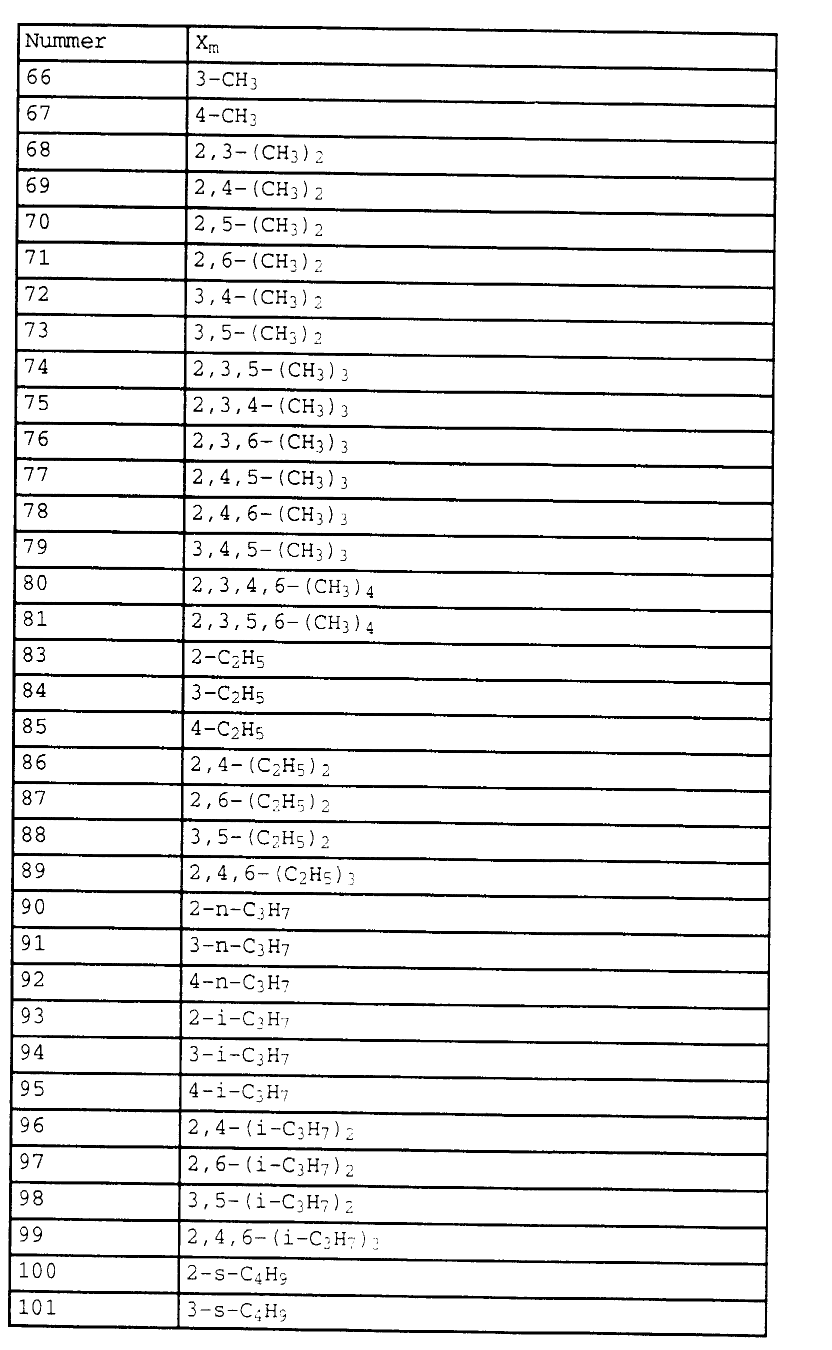

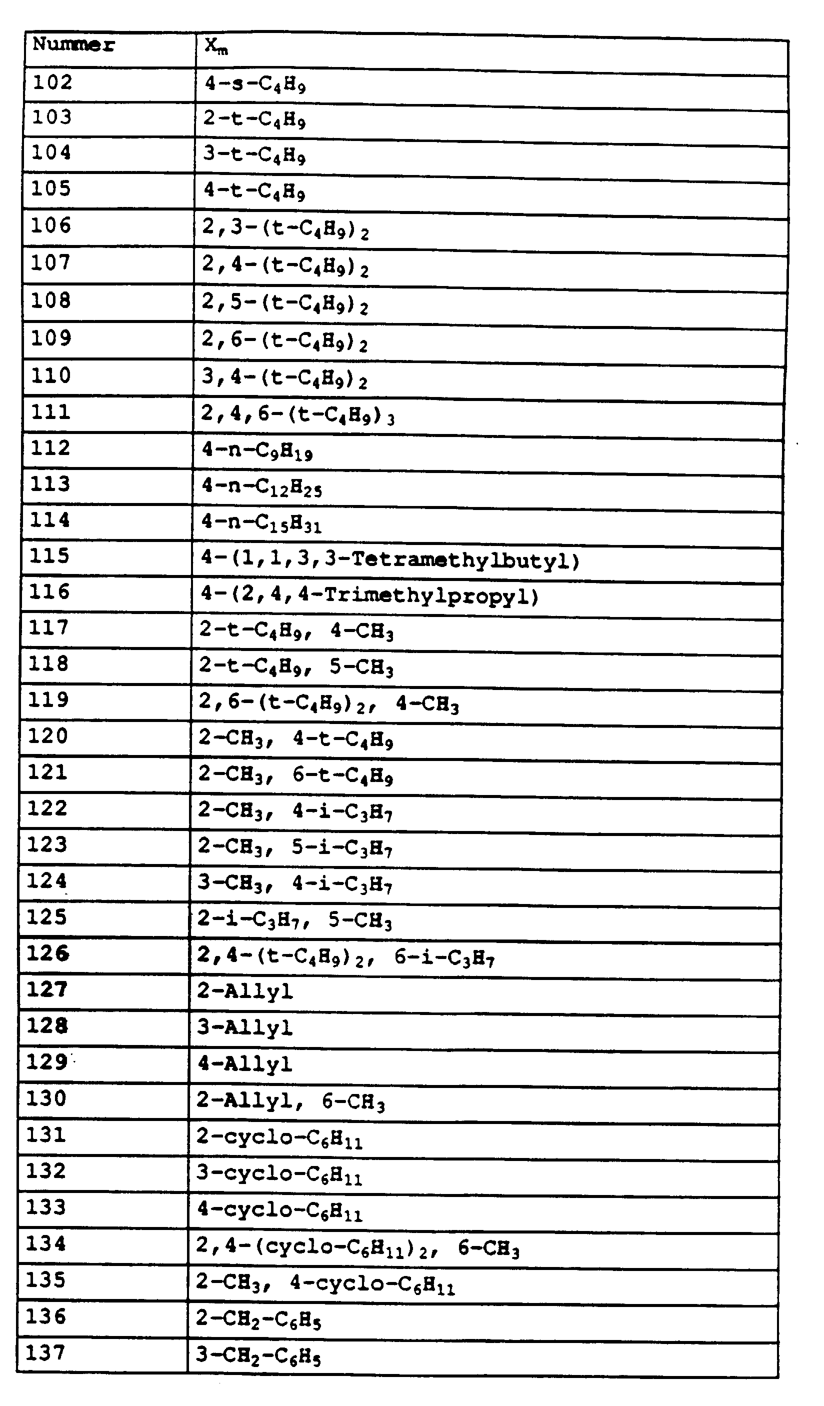

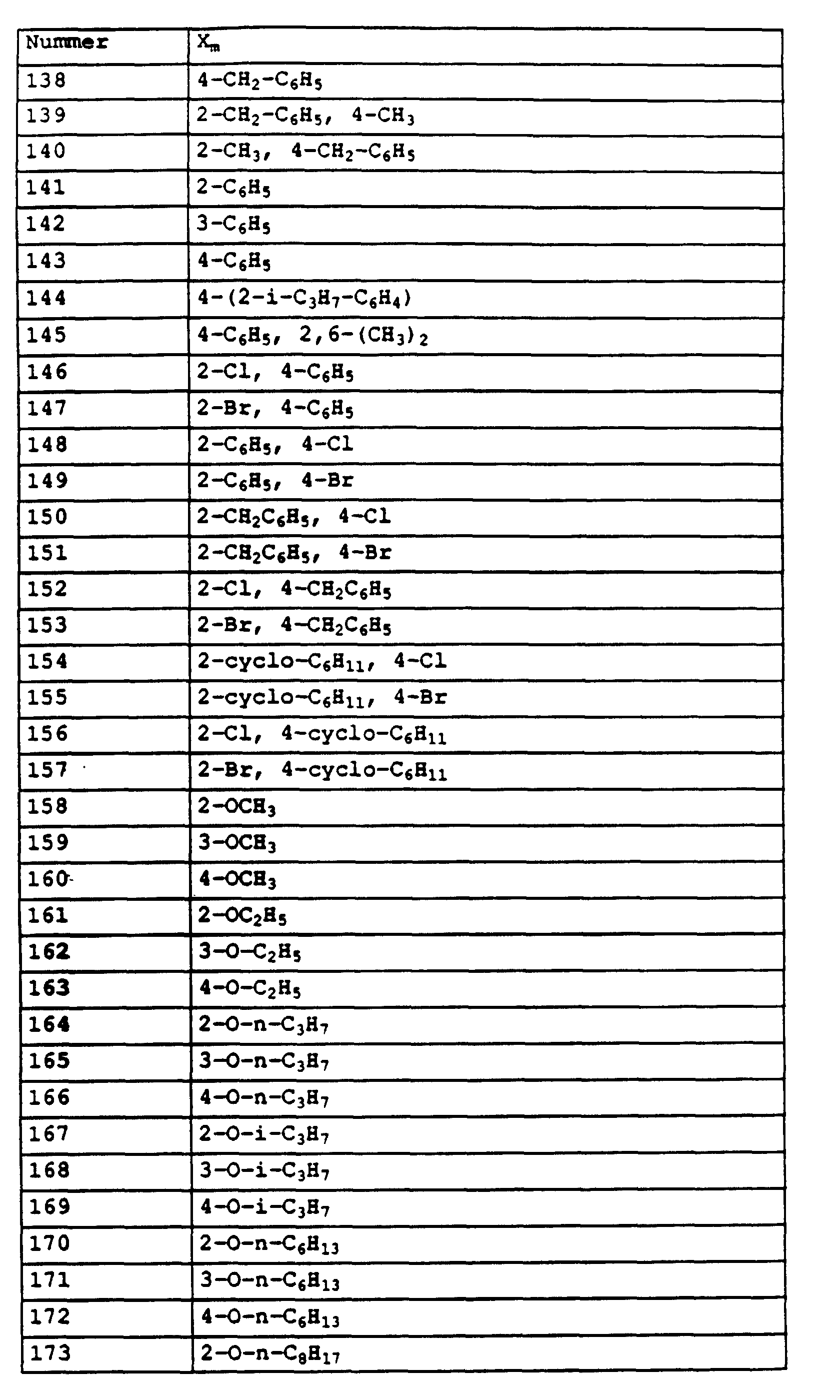

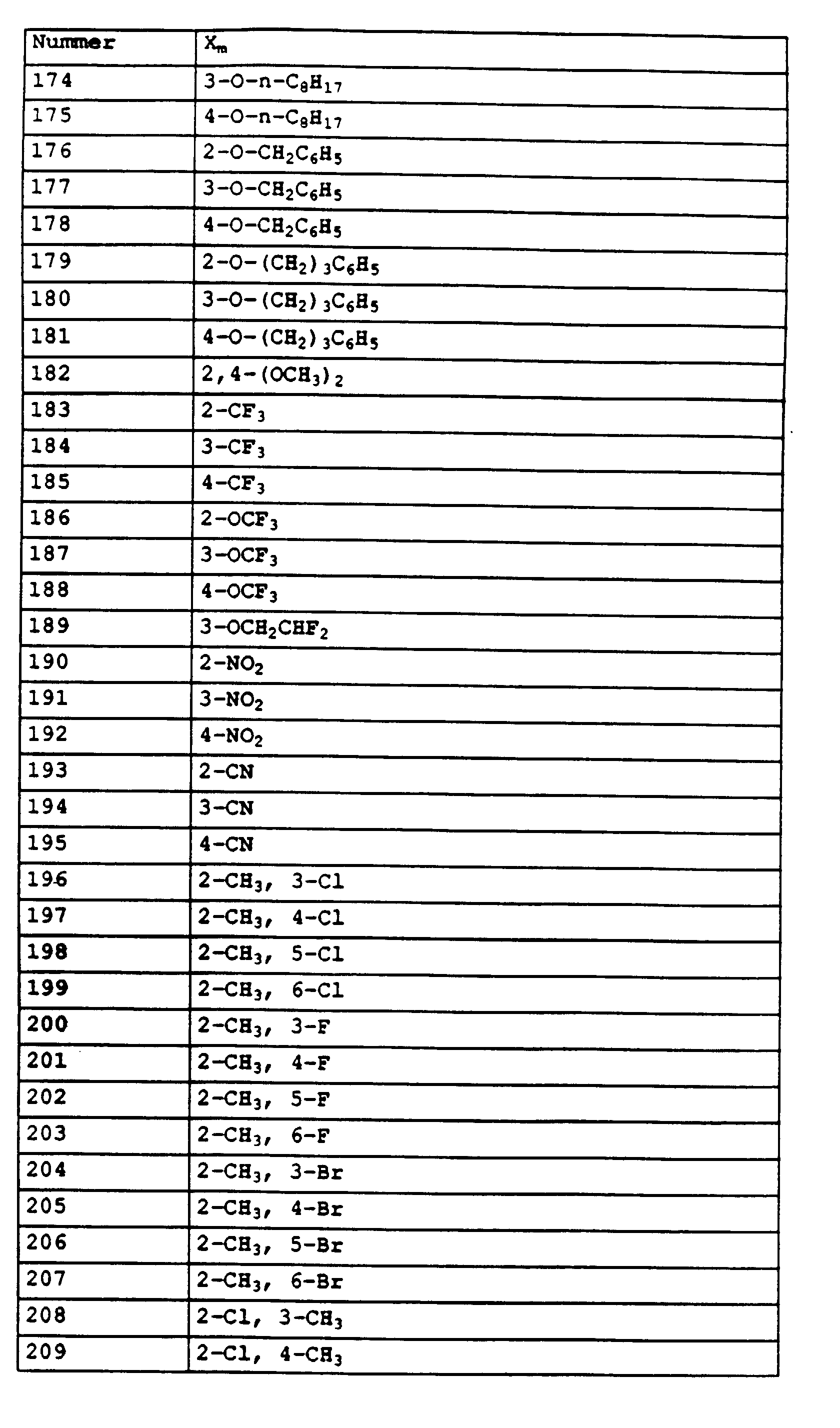

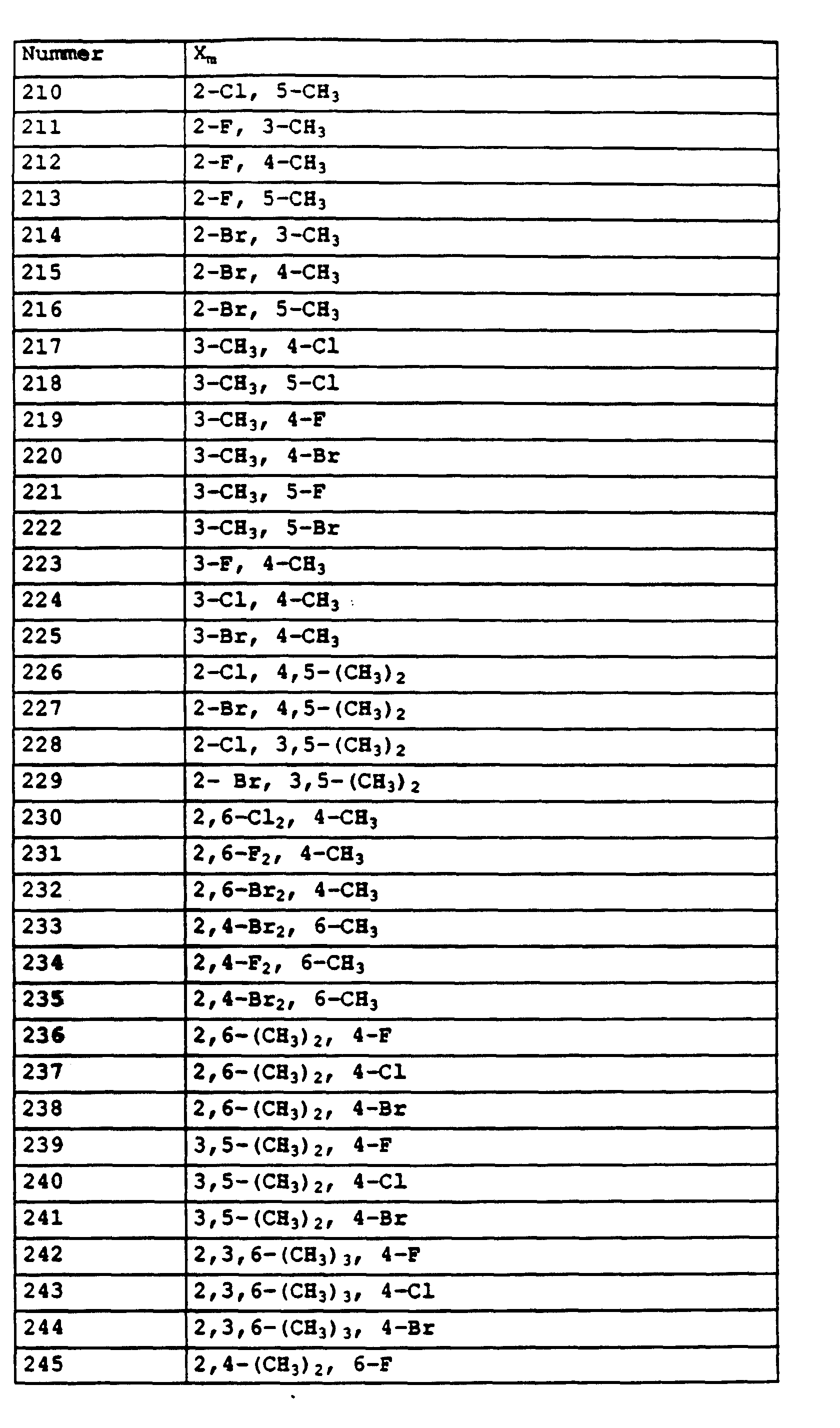

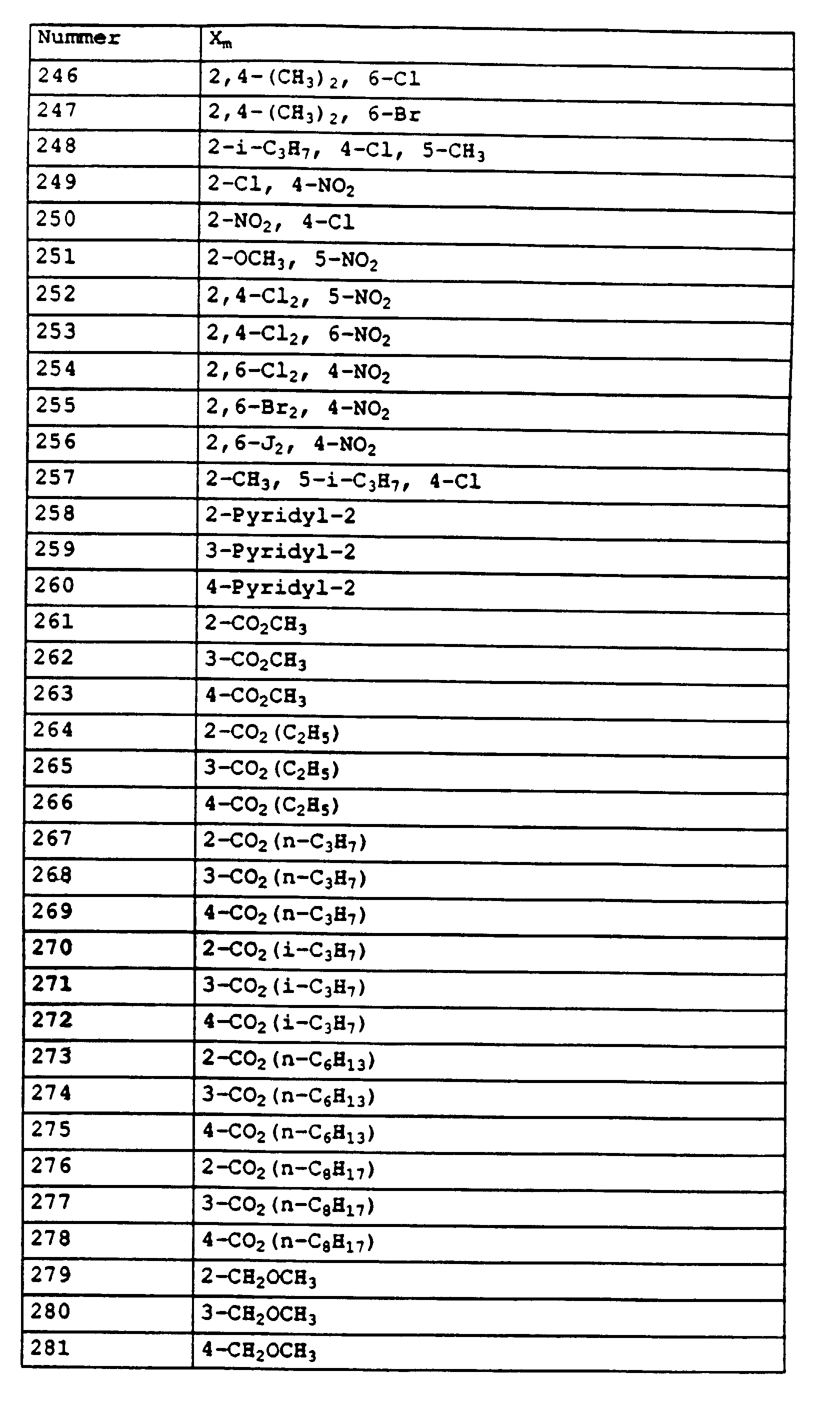

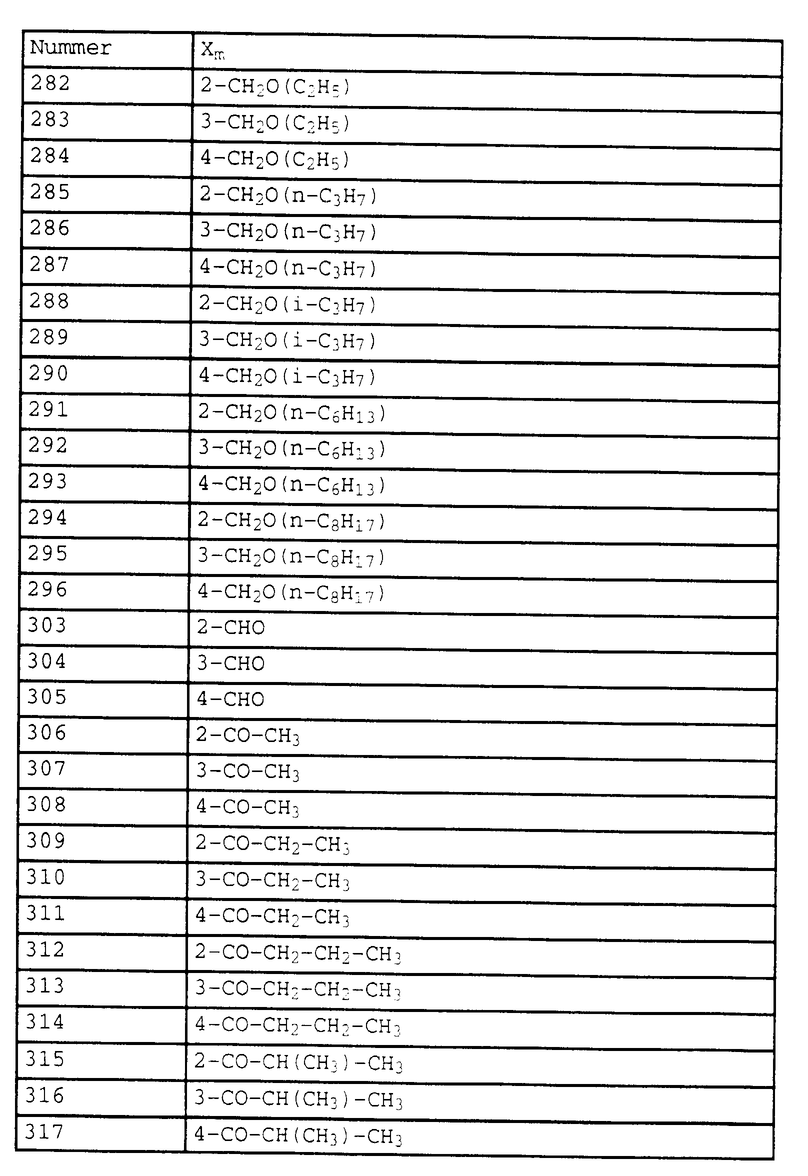

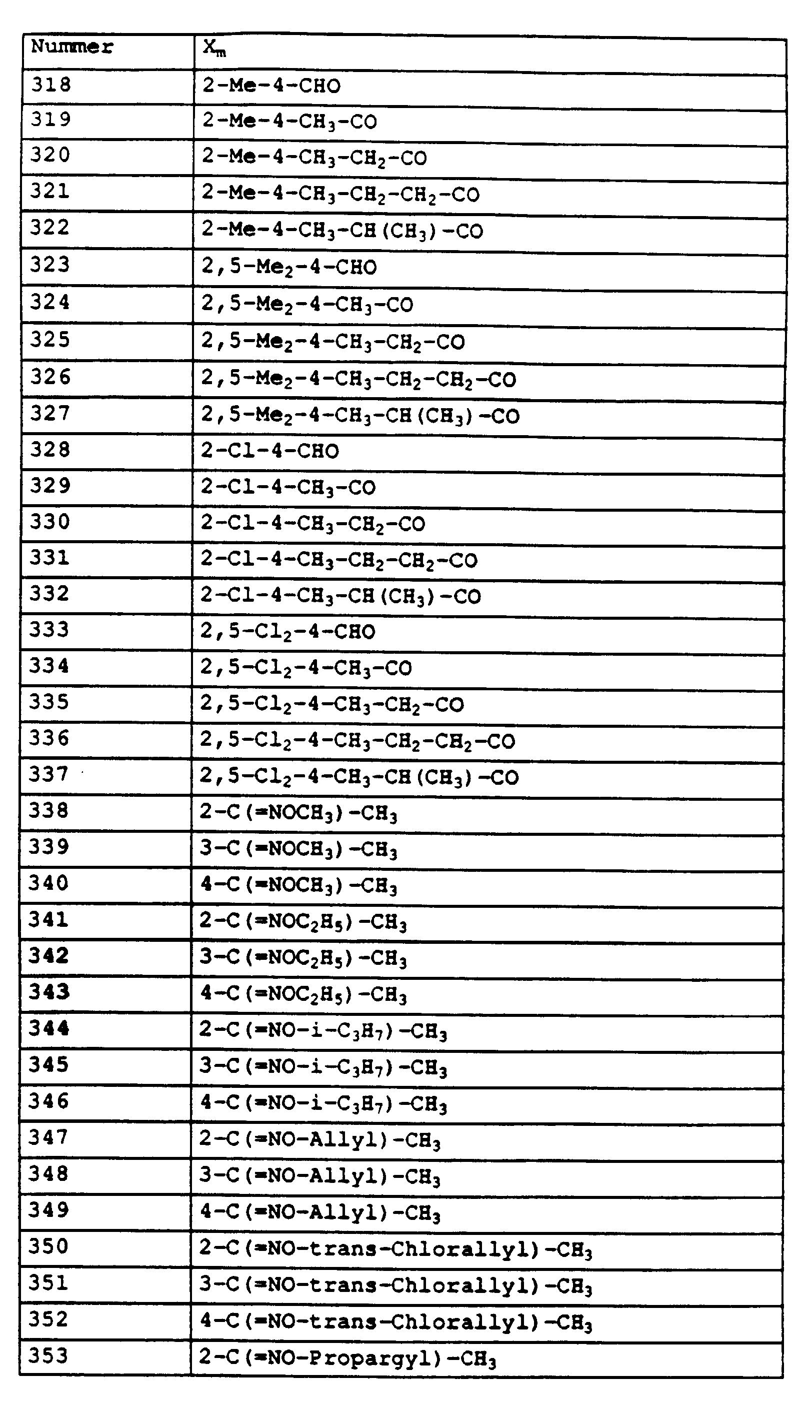

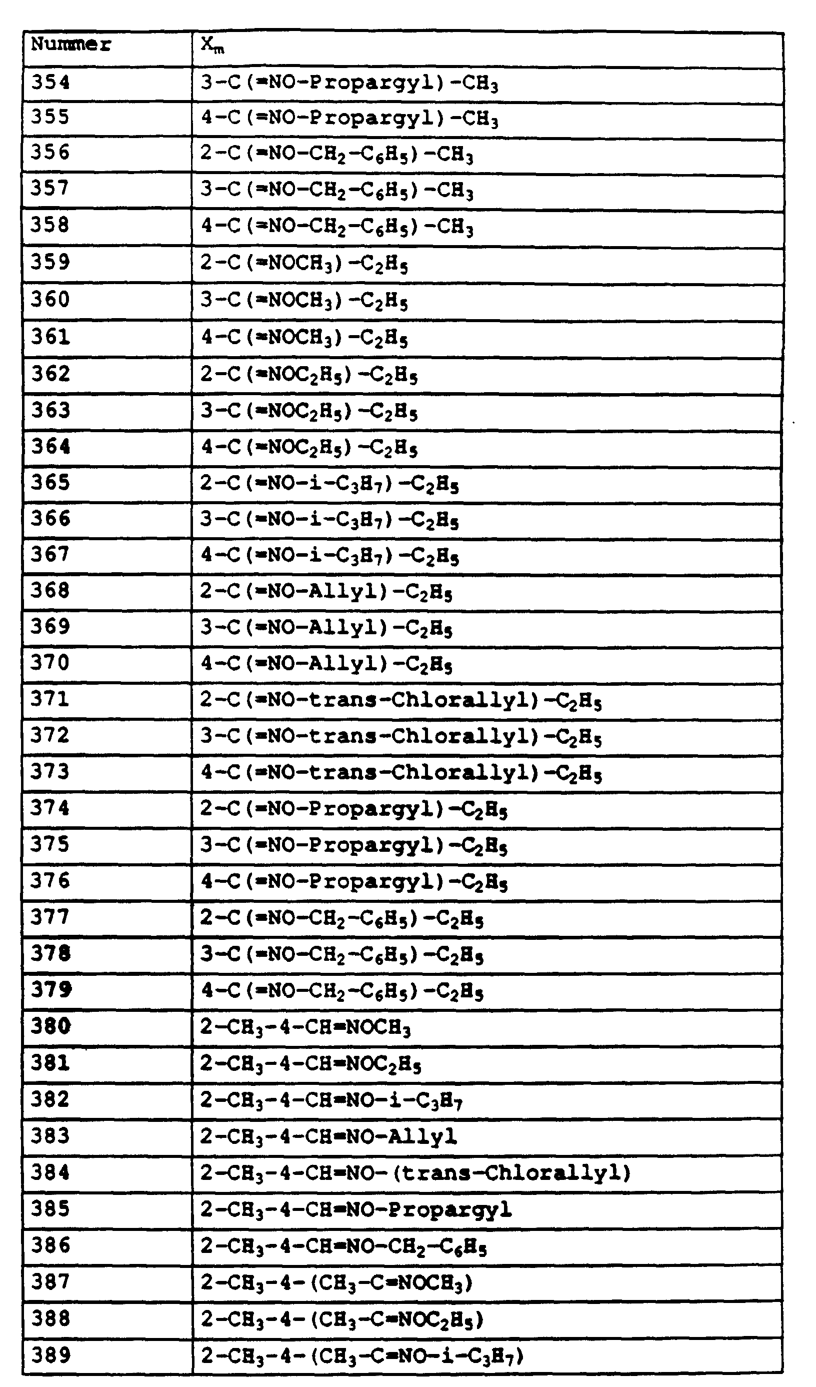

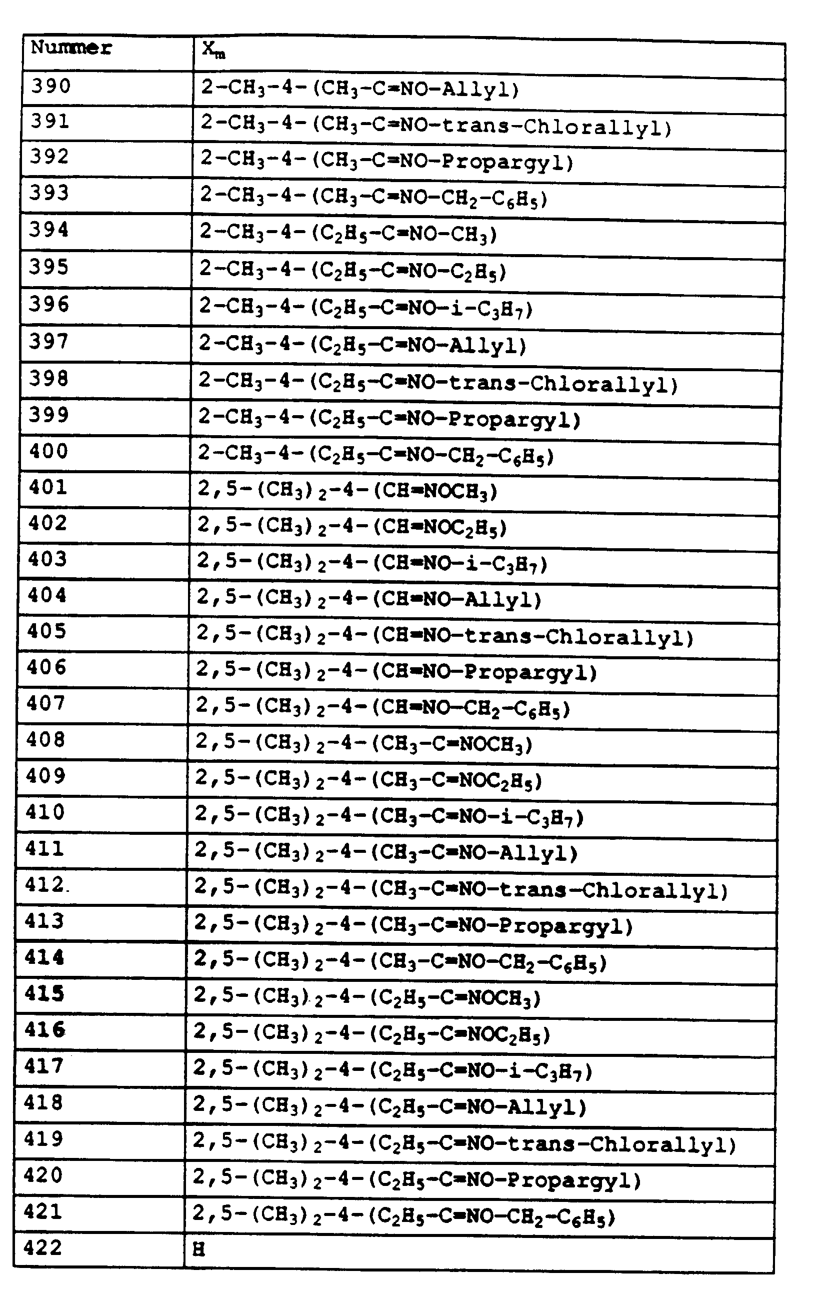

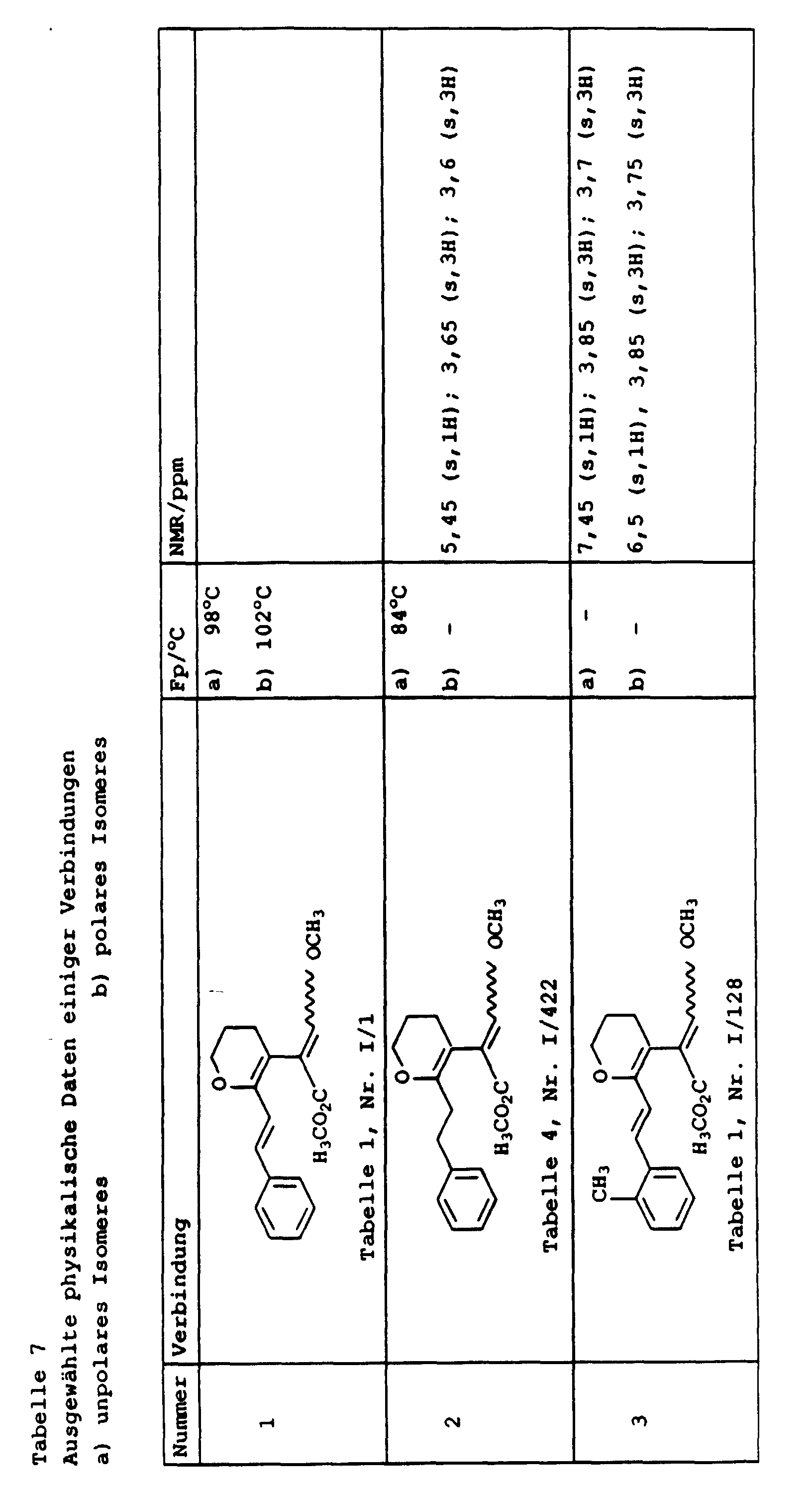

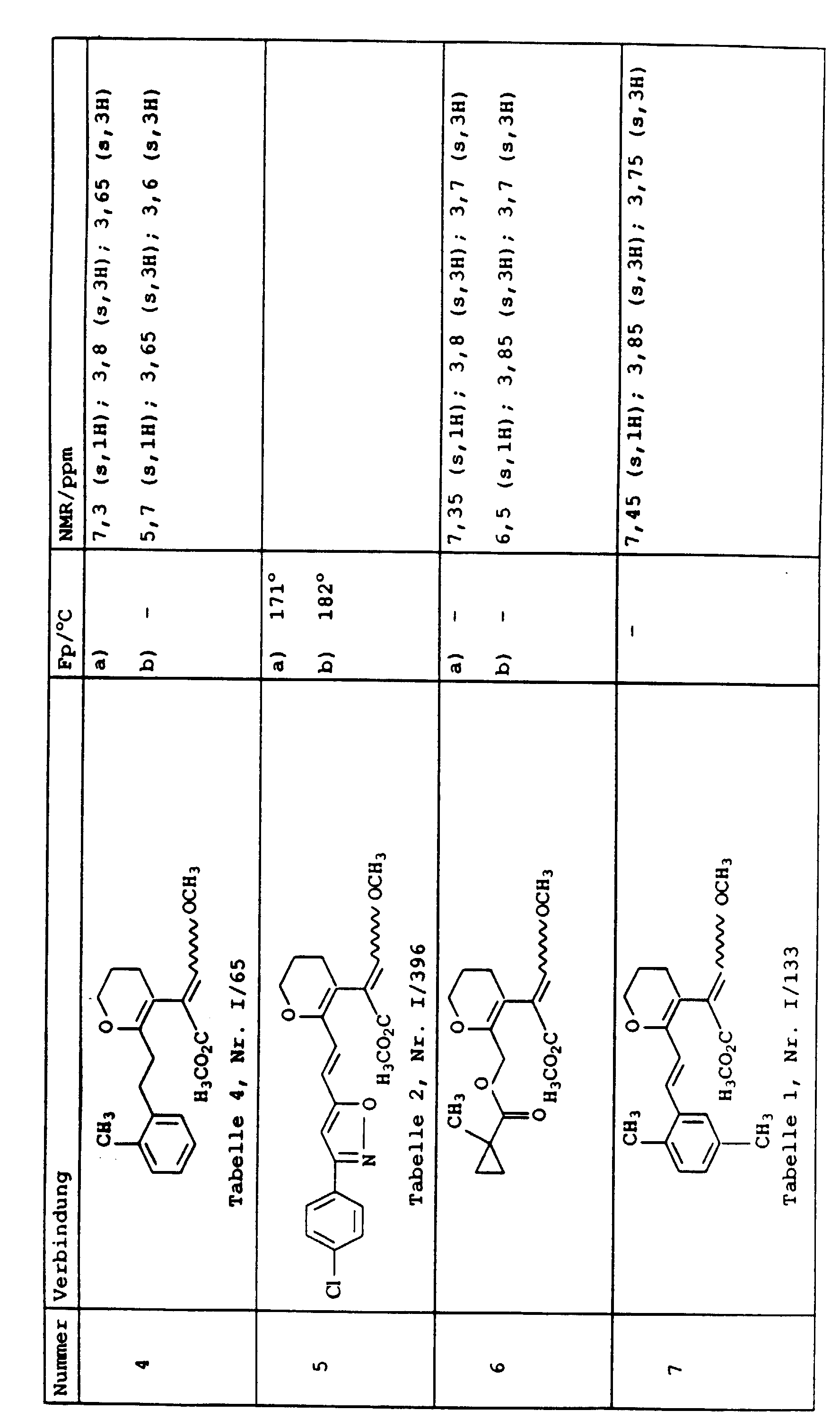

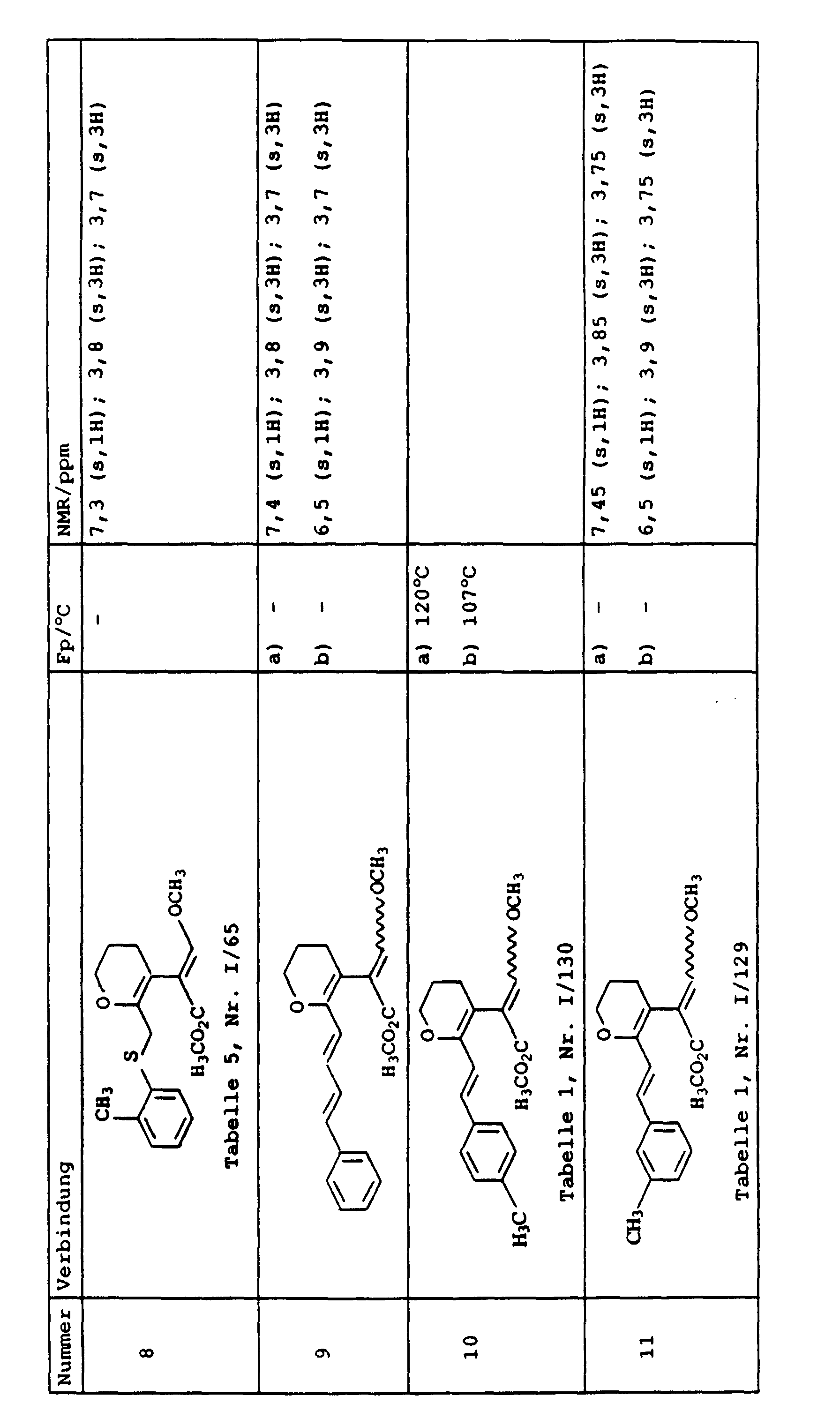

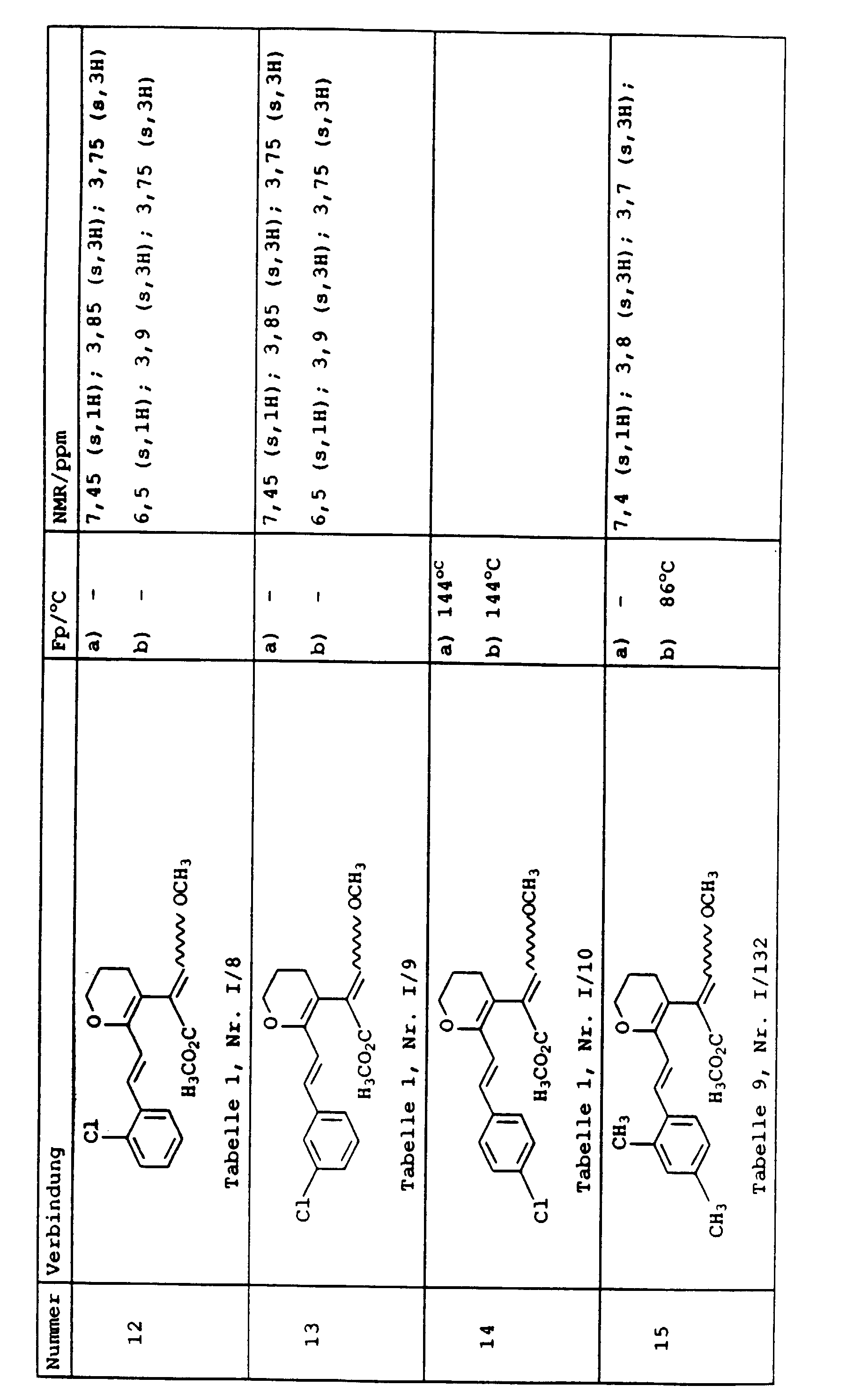

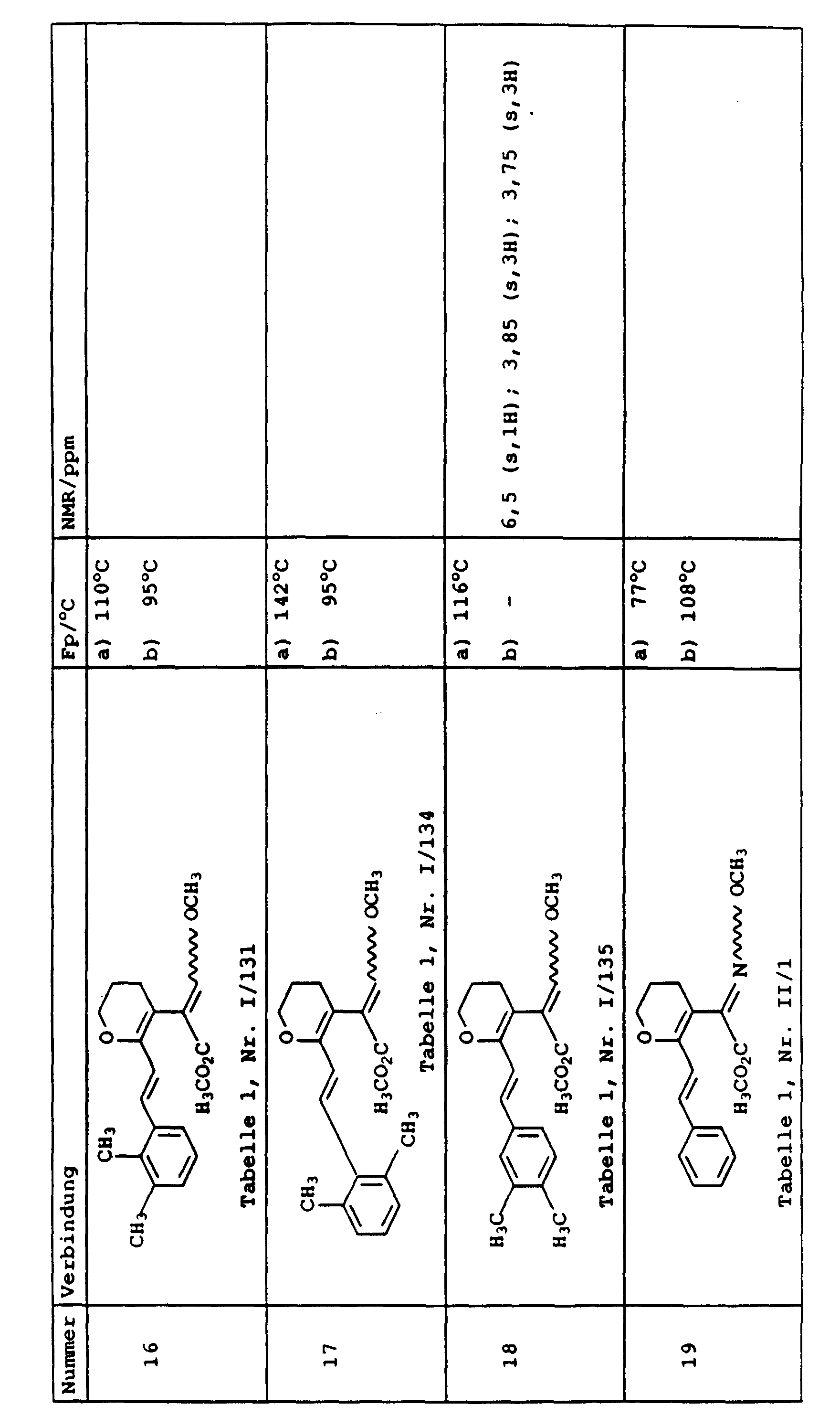

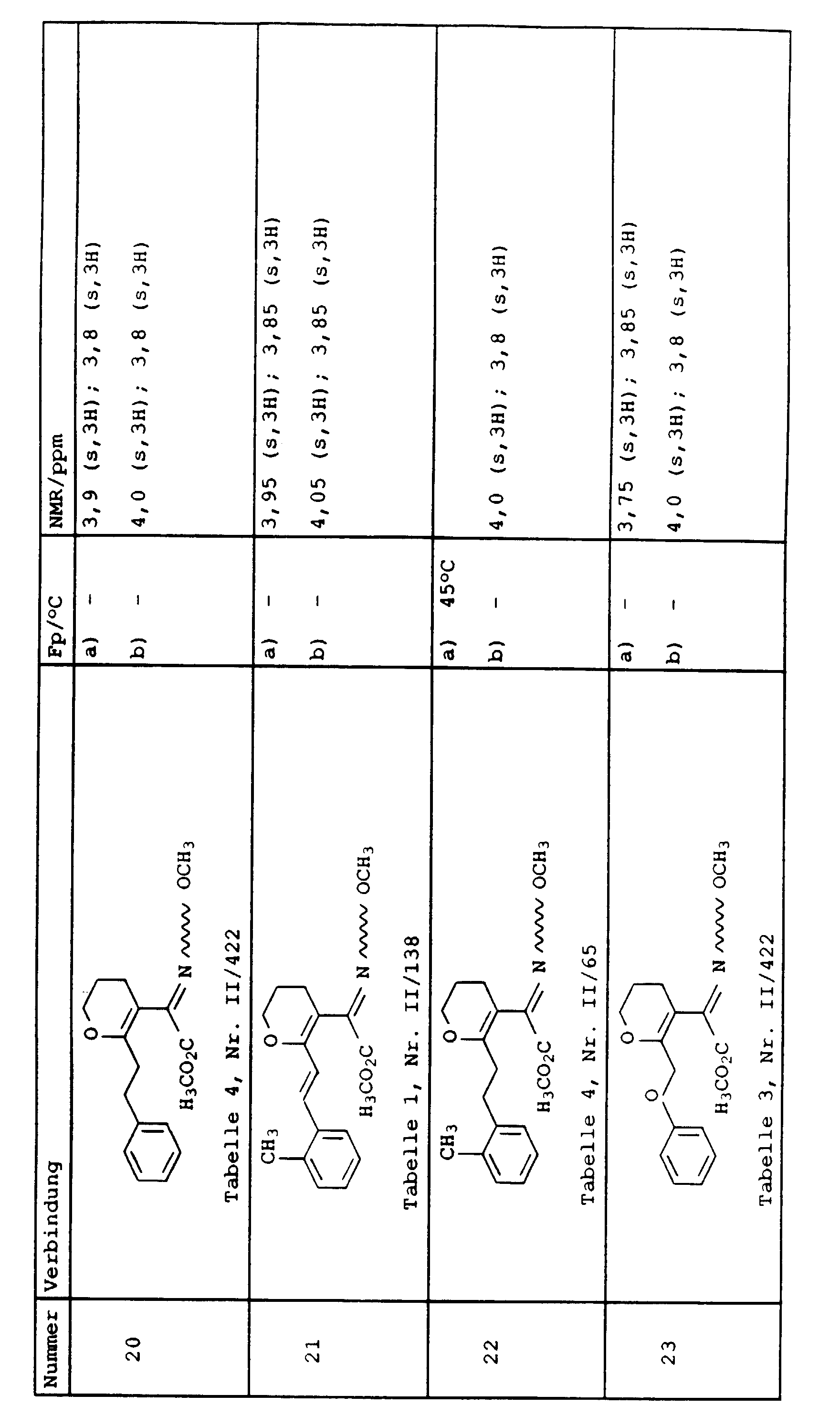

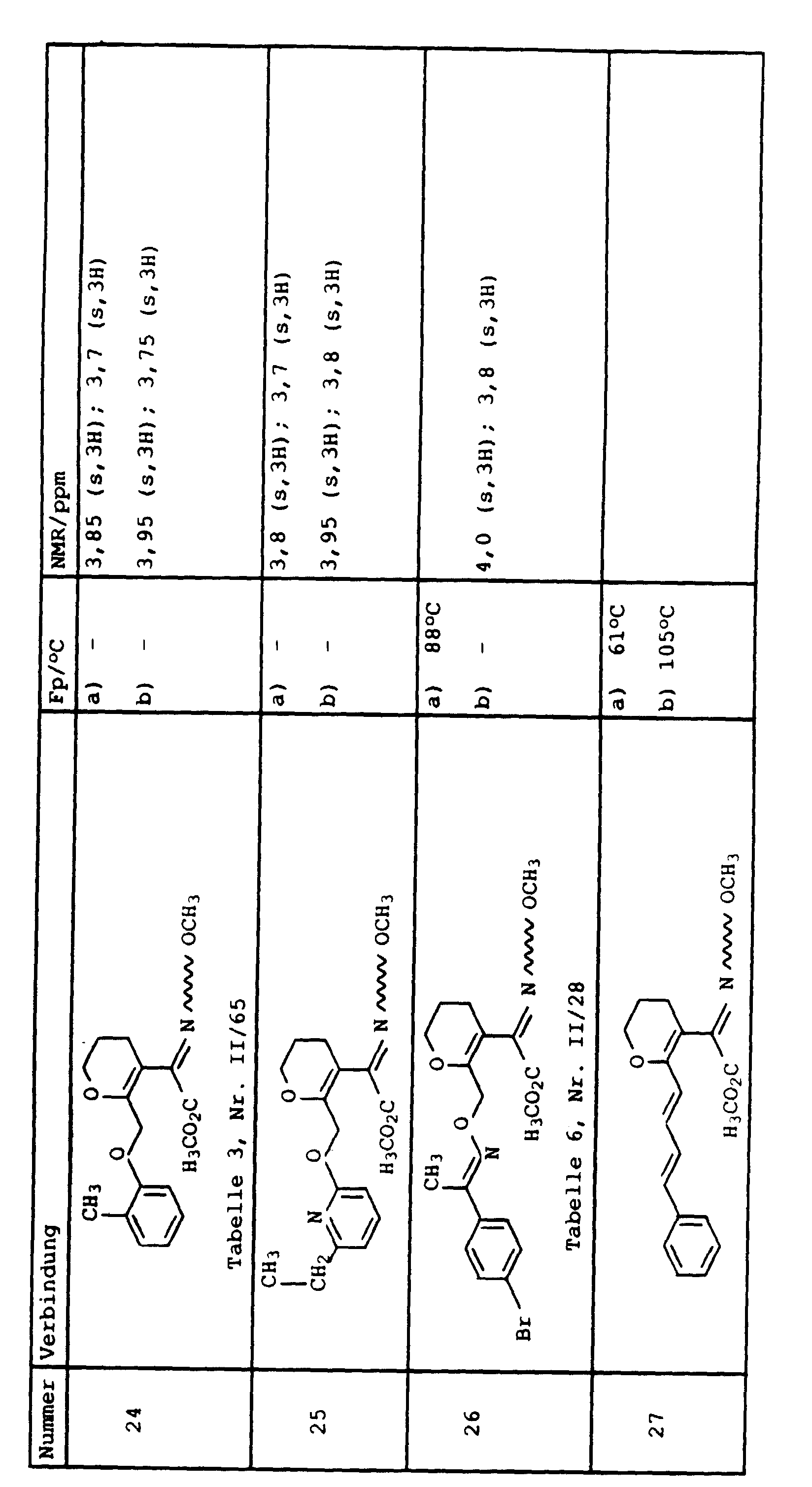

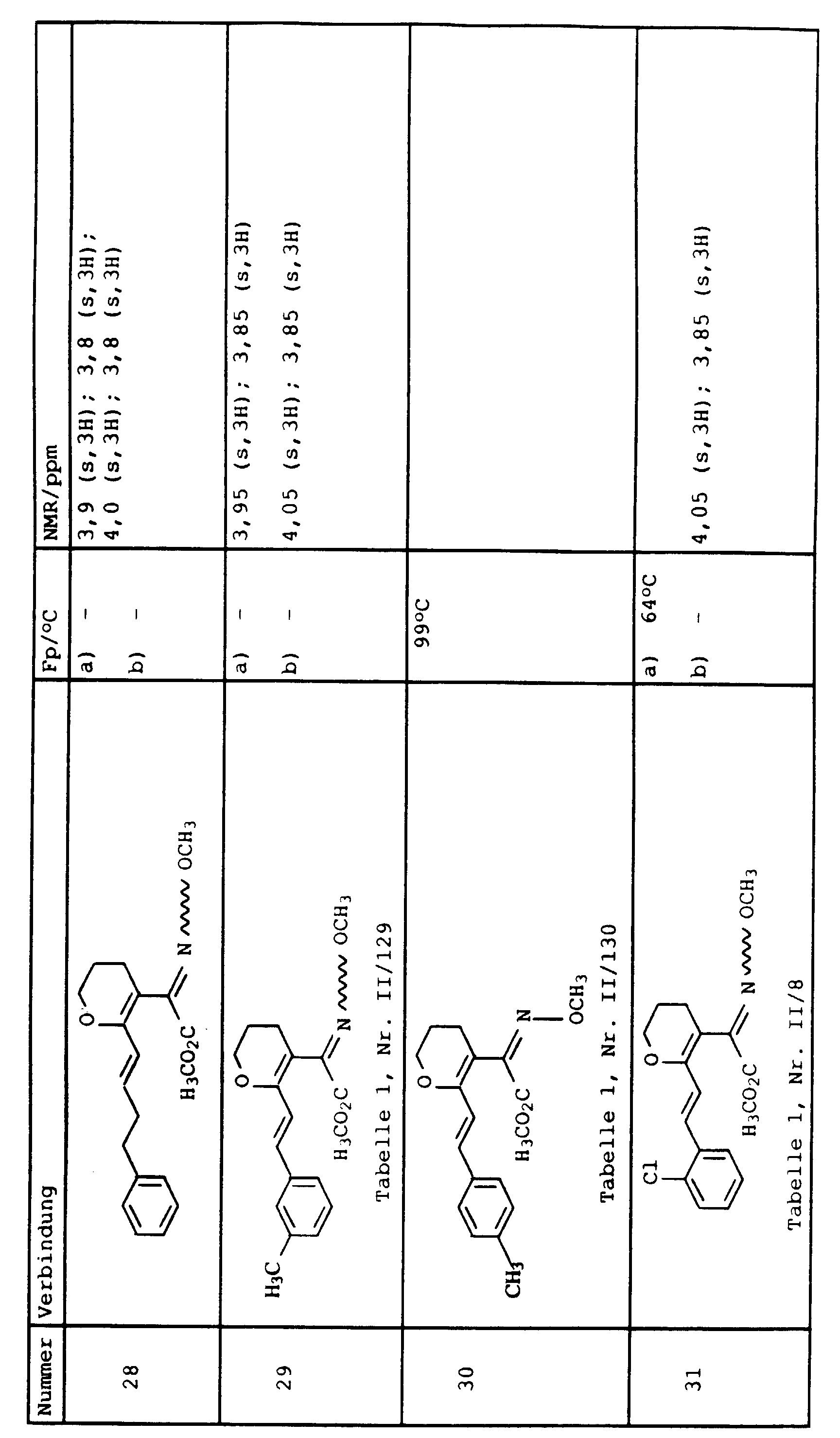

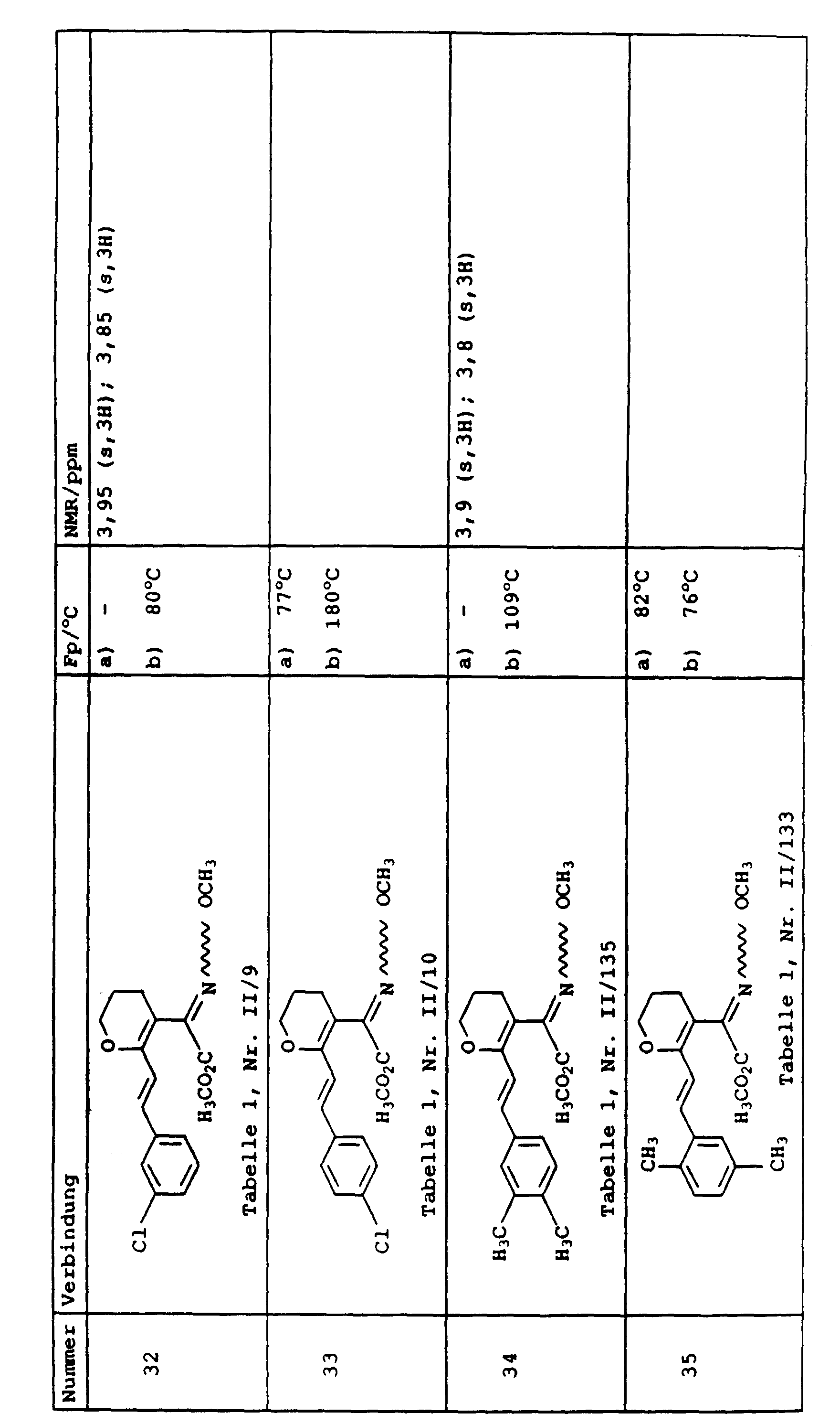

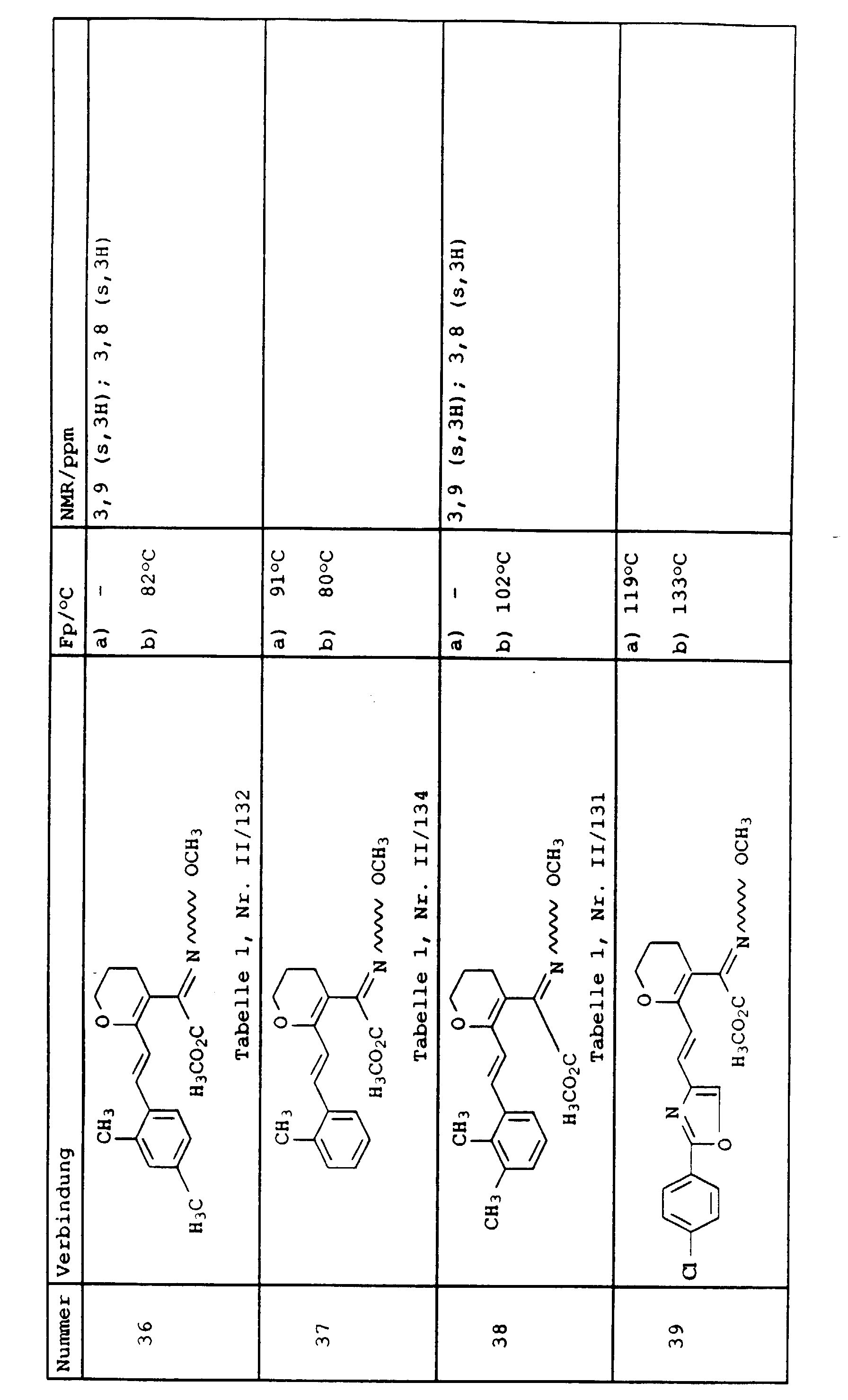

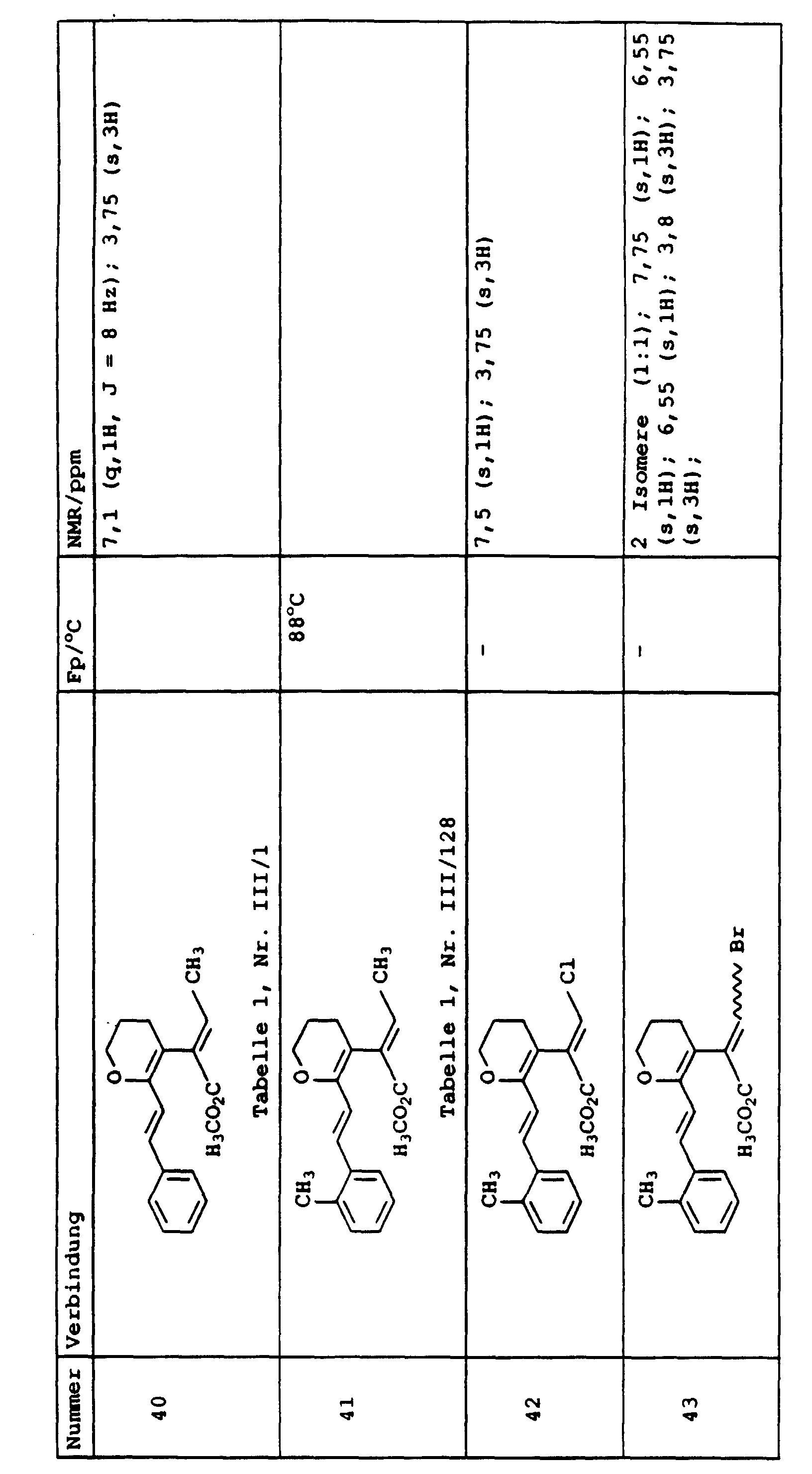

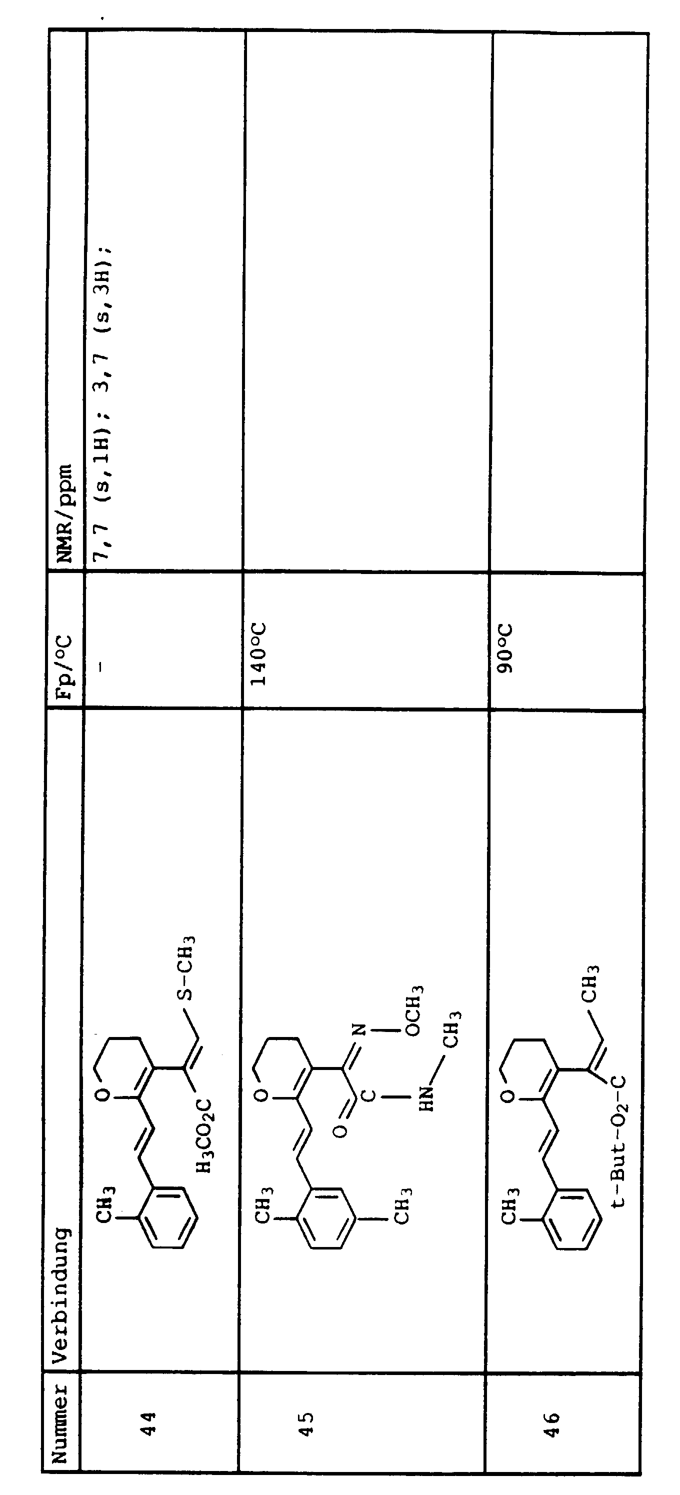

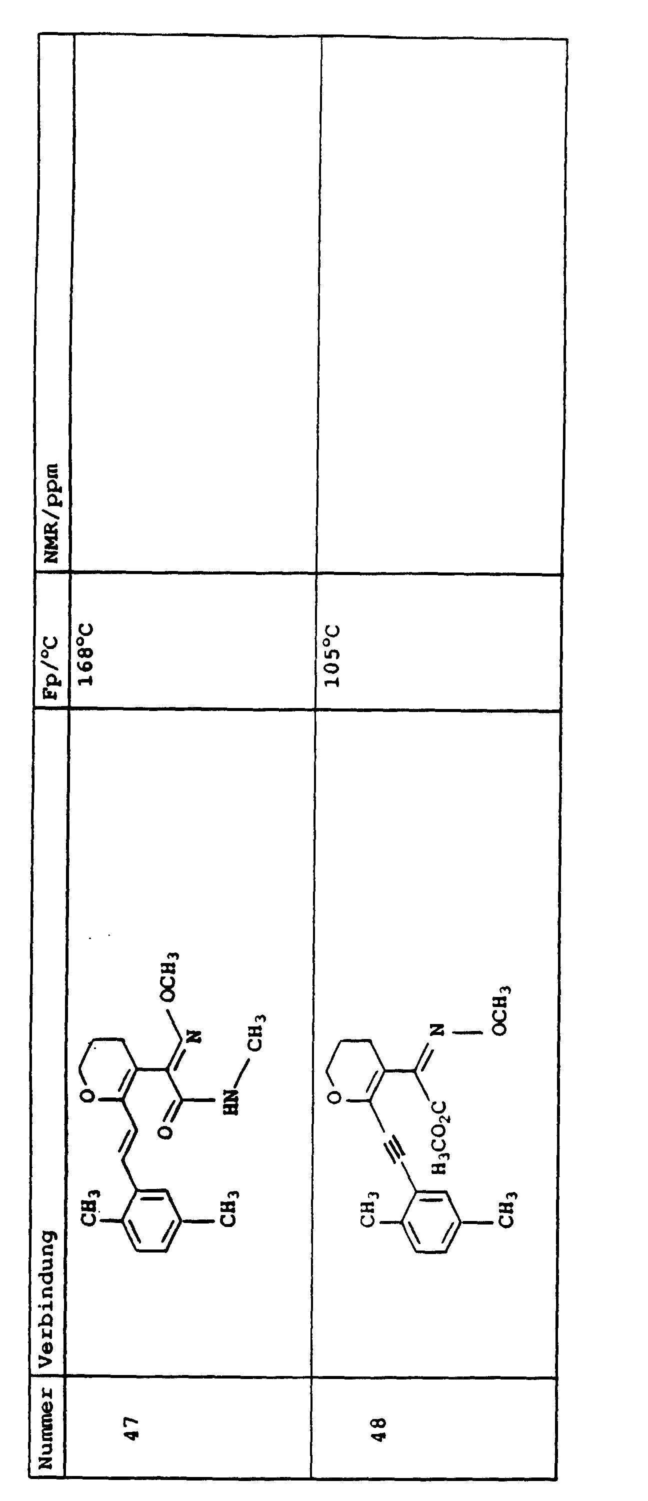

Die neuen Verbindungen eignen sich als Fungizide, Insektizide, Nematizide und zur Regulierung des Pflanzenwachstums.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holzund Nußschalenmehl, Cellulosepulver oder andere feste Trägerstoffe.

Beispiele für solche Zubereitungen sind:
I. eine Lösung aus 90 Gew.-Teilen der Verbindung Tabelle 1, Nr. I/la und 10 Gew.-Teilen N-Methyl-apyrrolidon, die zur Anwendung in Form kleinster Tropfen geeignet ist;
II. eine Mischung aus 20 Gew.-Teilen der Verbindung Tabelle 2, Nr. I/396b, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Tabelle 7, Nr. 6a, , 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Tabelle 1, Nr. I/133, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Tabelle 7, Nr. 9b, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Tabelle Nr. I/130a und 97 Gew.-Teilen feinteiligem Kaolin; dieses Stäubemittel enthält 3 Gew.-% Wirkstoff;
VII. eine innige Mischung aus 30 Gew.-Teilen der Verbindung Tabelle 1, Nr. I/129b, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Tabelle 1, Nr. I/8a, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Tabelle 1, Nr. I/8b, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N' -propylen-bis-dithiocarbamat),
Zink-(N,N'-propylen-bis-dithiocarbamat),
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-βbis-(dimethylamino)-phosphinyl'-3-phenyl-1,2,4-triazol,
2,3-Dicyano-1,4-dithioanthrachinon,
2-Thio-1,3-dithioloβ4,5-b'chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,

N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,

N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-ylethyl]-1H-1,2,4-triazol
1-[2-(2,4-Dichlorphenyl)-4-n-propyl-1,3-dioxolan-2-ylethyl]-1H-1,2,4-triazol
N-(n-Propyl)-N-(2,4,6-trichlorphenoxyethyl)-N'-imidazol-ylharnstoff,
1-(4-Chlorphenoxy)-3,3-dimethyl-1-(1H-1,2,4-triazol-1-yl)-2-butanon,
α-(2-Chlorphenyl) -α-(4-chlorphenyl) -5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-83-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl)]glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorhenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

### Anwendungsbeispiele

Als Vergleichswirkstoffe wurde 3,4-Dihydro-6-methyl-2H-pyran-5-carboxanilid (A) - bekannt aus Pesticide Manual, achte Auflage, Seite 902 - benutzt.

### Anwendungsbeispiel 1

### Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in einer feuchten Kammer aufgestellt. Dann erfolgte die Beurteilung des Ausmaßes des Pilzausbruchs auf den Blattunterseiten.

Das Ergebnis zeigt, daß die Wirkstoffe aus Tabelle 7, Nr. 1a, 5b, 6a, 7, 9b, 10a, 11b, 12a, 12b, 13a, 13b, 14a, 14b, 15a, 17a, 17b, 18a, 27a, 27b, 28a, 29b, 30, 31a, 31b, 32a, 32b, 34a, 34b, 35a, 35b, 36a, 37a, 37b, 39a, 39b und 42 bei der Anwendung als 250 ppm Wirkstoff enthaltende wäßrige Spritzbrühe eine bessere fungizide Wirkung zeigen (95 %) als der bekannte Vergleichswirkstoff A(55 %).

### Anwendungsbeispiel 2

### Wirksamkeit gegen Pyricularia oryzae

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden spräter mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. nach 6 Tagen wurde das Ausmaß des Krankheitsbefalls ermittelt.

Das Ergebnis zeigt, daß die Wirkstoffe aus Tabelle 7, Nr. 3a, 6a, 7, 12a, 12b, 13a, 13b, 14b, 15a, 15b, 16b, 17a, 17b, 18a, 21, 25b, 31a, 31b, 32a, 34a, 34b, 35a, 35b, 36a, 36b, 37a, 37b, 42, 43 und 44 bei der Anwendung als 250 ppm Wirkstoff enthaltende wäßrige Spritzbrühe eine bessere fungizide Wirkung zeigen (95 %) als der bekannte Vergleichswirkstoff A (40 %).

## Patentansprüche

1. Dihydropyran der Formel 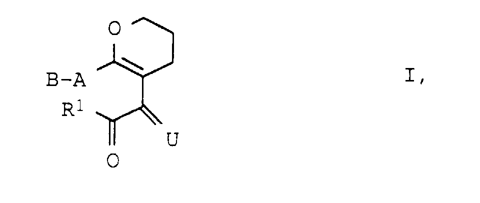 in der
U =CH-OR², =CH-SR², =CH-R², =CH-Halogen oder =N-OR² und
A eine Einfachbindung -CH₂-, -(CH₂-CH₂)ₙ-, -(CR⁹=CR⁸)ₘ-CH=CH, -C≡C-,-OCH₂, -SCH₂-, -NR⁶CH₂-, -C(=O)-OCH₂- oder R⁷C=NO-CH₂- bedeutet,
n 1, 2 oder 3 und
m 0 oder 1 bedeutet,
B Wasserstoff,
Cycloalkyl mit 3 bis 10 C-Atomen, Aryl mit 6, 10 oder 14 Kohlenstoffatomen, Heteroaryl mit 5 bis 14 Ringatomen, davon 1 bis 4 Heteroatome aus der Gruppe N, O, S, Heterocyclyl mit 5 bis 15 Ringatomen, davon 1 bis 4 Heteroatome aus der Gruppe N, O, S, wobei die Ringe gesättigt oder partiell ungesättigt sind oder Cycloalkenyl mit 5 bis 14 C-Atomen bedeutet, wobei diese Gruppen jeweils einen bis vier der unter R¹⁰ genannten Reste tragen können, soweit diese von den vorstehend genannten Resten verschieden sind,
R¹ OR³ oder NR⁴R⁵ bedeutet,
R², R³ und R⁶ Methyl oder Ethyl bedeuten,
R⁴, R⁵, R⁷, R⁸ und R⁹ Wasserstoff, Methyl oder Ethyl bedeuten,
R¹⁰ Halogen, Cyano, Nitro oder CHO
R¹¹, OR¹¹, =NOR¹¹, CO₂R¹¹, SR¹¹, COR¹¹, SOR¹¹ oder SO₂R¹¹ bedeutet,
R¹¹ Alkyl, Alkenyl, Alkinyl, Cycloalkyl mit 3 bis 10 C-Atomen, Aryl mit 6, 10 oder 14 Kohlenstoffatomen, Hetaryl mit 5 bis 14 Ringatomen, davon 1 bis 4 Heteroatome aus der Gruppe N, O, S, Heterocyclyl mit 5 bis 14 Ringatomen, davon 1 bis 4 Heteroatome aus der Gruppe N, O, S, wobei die Ringe gesättigt oder partiell ungesättigt sind oder Cycloalkenyl mit 5 bis 14 C-Atomen bedeutet, wobei diese Gruppen einen bis vier der unter R¹² genannten Reste tragen können, soweit diese von den Resten R¹¹ verschieden sind,
R¹² Halogen, Cyano, Nitro, Alkyl, Haloalkyl, Alkoxy, Haloalkoxy, Alkoxyimino, Alkoxycarbonyl, Alkylcarbonyl, Alkenyloxyimino und Aryl bedeutet.

2. Dihydropyran der Formel I gemäß Anspruch 1, in der A für -(CH=CH)ₙ-CH=CH- steht.

3. Verbindung der Formel III 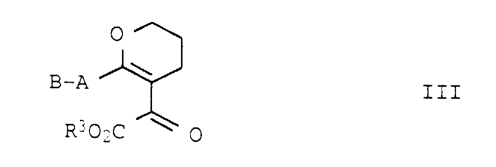 in der A, B und R³ die in Anspruch 1 genannte Bedeutung haben.

4. Verfahren zur Herstellung eines Dihydropyrans gemäß Anspruch 1 der Formel VI 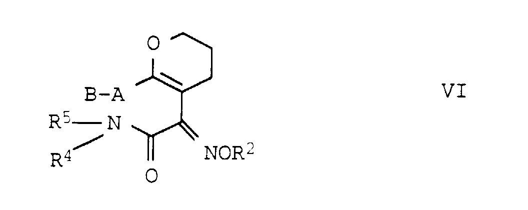 in der A, B, R², R⁴ und R⁵ die in Anspruch 1 genannte Bedeutung haben, dadurch gekennzeichnet, daß man Verbindungen der Formel VII 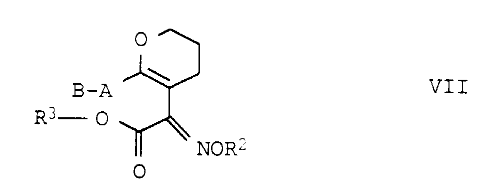 umsetzt mit Aminen der Formel HNR⁴R⁵, wobei R³ die in Anspruch 1 genannte Bedeutung hat.

5. Verfahren zur Herstellung eines Dihydropyrans gemäß Anspruch 1 der Formel VIII 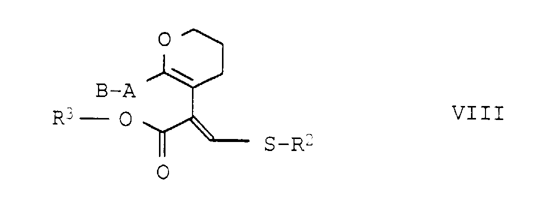 in der A, B, R² und R³ die in Anspruch 1 genannte Bedeutung haben, dadurch gekennzeichnet, daß man eine Verbindung der Formel IX, 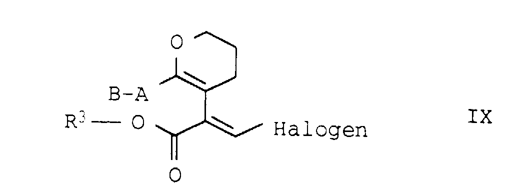 in der A, B und R³ die in Anspruch 1 genannte Bedeutung besitzen und Halogen Chlor oder Brom bedeutet, bevorzugt unter alkalischen Bedingungen, umsetzt mit einer Verbindung der Formel R²-SH und R² die in Anspruch 1 genannte Bedeutung hat.

6. Verfahren zur Herstellung eines Dihydropyrans gemäß Anspruch 1 der Formel I 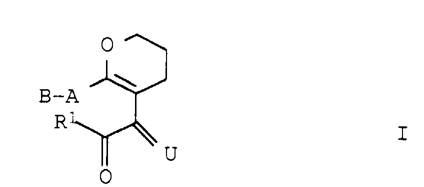 in der A, B und R¹ die in Anspruch 1 genannte Bedeutung haben und in der
U = CH-OR², = CH₂, = CHR² oder = CH-Halogen ist, dadurch gekennzeichnet, daß man Ketoester der Formel III 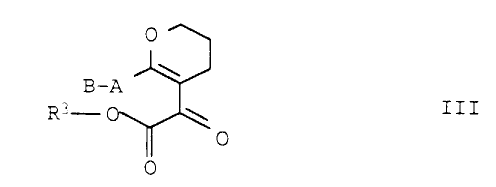 wobei R³ die in Anspruch 1 gegebene Bedeutung hat,
mit dem entsprechenden Phosphonat oder Phosphoniumsalz in einer Wittig-Reaktion umsetzt.

7. Verfahren zur Herstellung eines Dihydropyrans der Formel VII, 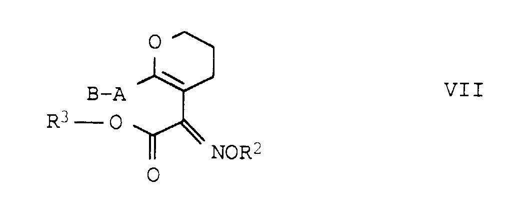 in der A, R², und R³ die in Anspruch 1 genannte Bedeutung besitzen, dadurch gekennzeichnet, daß man ein Alkoxyamin der Formel H₂N-OR² umsetzt mit einem Ketoester der Formel III.

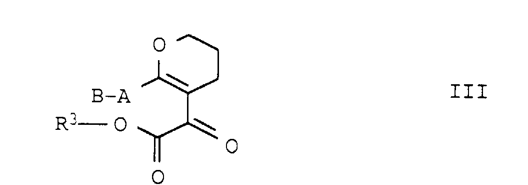

8. Verfahren zur Herstellung eines Dihydropyrans gemäß Anspruch 1 der Formel III, 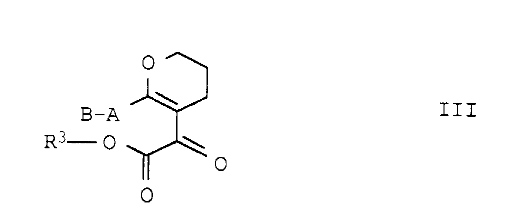 in der A, B und R³ die in Anspruch 1 genannte Bedeutung haben, dadurch gekennzeichnet, daß man Oxalsäurechloride der Formel R³-O-CO-CO-Cl umsetzt mit einem Dihydropyran der Formel IV.

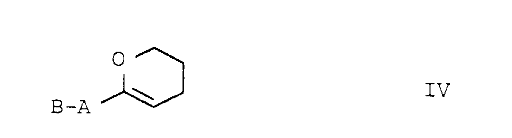

9. Verfahren zur Herstellung eines Dihydropyrans gemäß Anspruch 1 der Formel XIII, 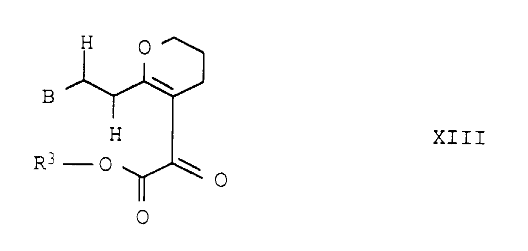 in der B und R³ die in Anspruch 1 genannte Bedeutung besitzen, gekennzeichnet dadurch, daß man ein Dihydropyran der Formel XIV 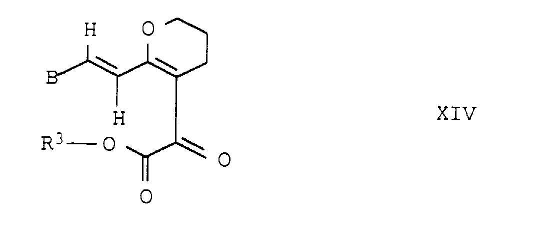 mit Wasserstoff in Gegenwart geeigneter Katalysatoren (z.B. Pd, Pt oder Ni) hydriert.

10. Fungizid, enthaltend einen inerten Trägerstoff und eine fungizid wirksame Menge eines Dihydropyrans der Formel I gemäß Anspruch 1.

11. Verfahren zur Bekämpfung von Pilzen, dadurch gekennzeichnet, daß man die Pilze oder die von Pilzbefall bedrohten Materialien, Pflanzen, Saatgut oder den Erdboden mit einer fungizid wirksamen Menge einer Verbindung der Formel I gemäß Anspruch 1 behandelt.

12. Verbindung der Formel I gemäß Anspruch 1, in der U für CH-OCH₃, A für -CH=CH- und B für Phenyl steht und R¹ Methoxy bedeutet.

13. Verbindung der Formel I gemäß Anspruch 1, in der U für CH-OCH₃, A für -CH₂-CH₂- und B für Phenyl steht und R¹ Methoxy bedeutet.

14. Verbindung der Formel I gemäß Anspruch 1, in der U für CH-OCH₃, A für -CH=CH- und B für 2-Methylphenyl steht und R¹ Methoxy bedeutet.

15. Verbindung der Formel I gemäß Anspruch 1, in der U für CH-OCH₃, A für -CH₂-CH₂- und B für 2-Methylphenyl steht und R¹ Methoxy bedeutet.

## Claims

1. A dihydropyran of the formula I 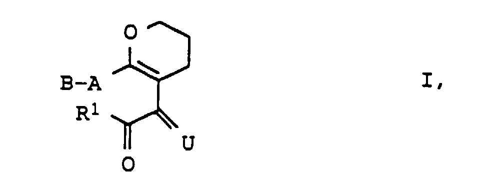 where U is =CH-OR², =CH-SR², =C-R², =CH-halogen or =N-OR² and
A is a single bond or -CH₂-, -(CH₂-CH₂)ₙ-, -(CR⁹=CR⁸)ₘ-CH=CH, -C≡C-, -OCH₂, -SCH₂-, -NH⁶CH₂-, -C (=O) -OCH₂- or R⁷C=NO-CH₂-,
n is 1, 2 or 3 and
m is 0 or 1,
B is hydrogen,
cycloalkyl of 3 to 10 carbon atoms, aryl of 6, 10 or 14 carbon atoms, hetaryl having 5 to 14 ring atoms, including 1 to 4 heteroatoms selected from the group consisting of N, O and S, heterocyclyl having 5 to 15 ring atoms, including 1 to 4 heteroatoms selected from the group consisting of N, 0 and S, the rings being saturated or partially unsaturated, or cycloalkenyl of 5 to 14 carbon atoms, where each of these groups may carry from one to four of the radicals stated under R¹⁰, provided that these differ from the abovementioned radicals,
R¹ is OR³ or NH⁴R⁵,
R², R³ and R⁶ are each methyl or ethyl,
R⁴, R⁵, R⁷, R⁸ and R⁹ are each hydrogen, methyl or ethyl,
R¹⁰ is halogen, cyano, nitro or CHO, R¹¹, OR¹¹, =NOR¹¹, CO₂R¹¹, SR¹¹, COR¹¹, SOR¹¹ or SO₂R¹¹,
R¹¹ is alkyl, alkenyl, alkynyl, cycloalkyl of 3 to 10 carbon atoms, aryl of 6, 10 or 14 carbon atoms, hetaryl having 5 to 14 ring atoms, including 1 to 4 heteroatoms selected from the group consisting of N, O and S, heterocyclyl having 5 to 14 ring atoms, including 1 to 4 heteroatoms selected from the group consisting of N, O and S, the rings being saturated or partially unsaturated, or cycloalkenyl of 5 to 14 carbon atoms, where these groups may carry from one to four of the radicals as stated under R¹², provided that these differ from the radicals R¹¹, and
R¹² is halogen, cyano, nitro, alkyl, haloalkyl, alkoxy, haloalkoxy, alkoximino, alkoxycarbonyl, alkylcarbonyl, alkenyloximino or aryl.

2. A dihydropyran of the formula I as claimed in claim 1, in which A is -(CH=CH)ₙ-CH=CH-.

3. A compound of the formula III 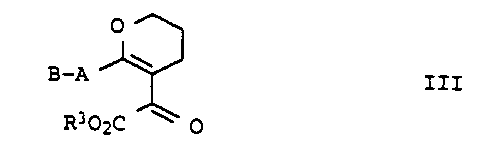 where A, B and R³ have the meanings stated in claim 1.

4. A process for the preparation of a dihydropyran as claimed in claim 1 of the formula VI 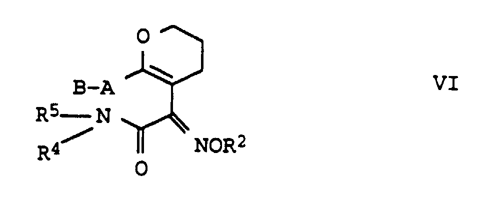 where A, B, R², R⁴ and R⁵ have the meanings stated in claim 1, wherein a compound of the formula VII 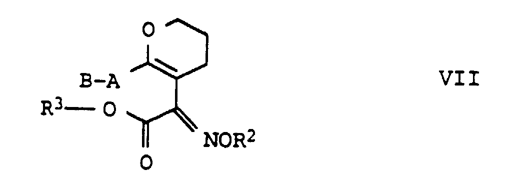 is reacted with an amine of the formula HNR⁴R⁵, and R³ has the meanings stated in claim 1.

5. A process for the preparation of a dihydropyran as claimed in claim 1 of the formula VIII 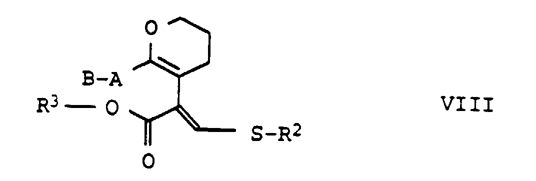 where A, B, R² and R³ have the meanings stated in claim 1, wherein a compound of the formula IX 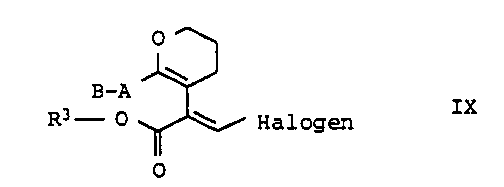 where A, B and R³ have the meanings stated in claim 1 and halogen is chlorine or bromine, is reacted, preferably under alkaline conditions, with a compound of the formula R²-SH, and R² has the meanings stated in claim 1.

6. A process for the preparation of a dihydropyran as claimed in claim 1 of the formula I 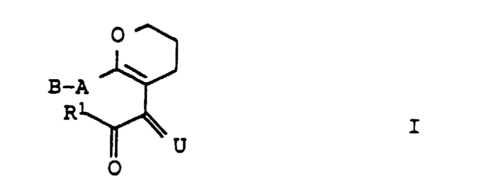 where A, B and R¹ have the meanings stated in claim 1 and U is =CH-OR², =CH₂, =CHR² or =CH-halogen, wherein a ketoester of the formula III 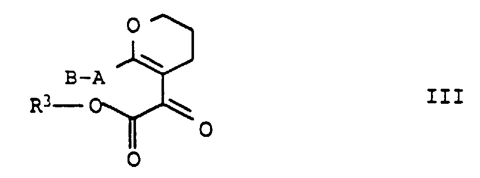 where R³ has the meanings given in claim 1, is reacted with the corresponding phosphonate or phosphonium salt in a Wittig reaction.

7. A process for the preparation of a dihydropyran of the formula VII 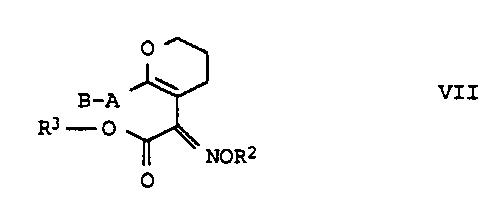 where A, R² and R³ have the meanings stated in claim 1, wherein an alkoxyamine of the formula H₂N-OR² is reacted with a ketoester of the formula III

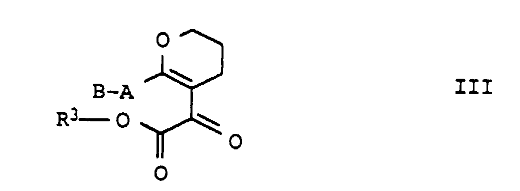

8. A process for the preparation of a dihydropyran as claimed in claim 1 of the formula III 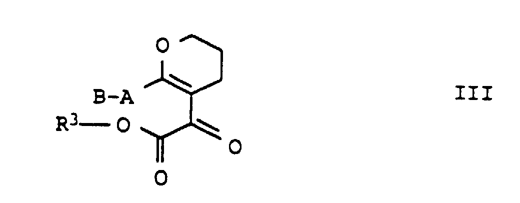 where A, B and R³ have the meanings stated in claim 1, wherein an oxalyl chloride of the formula R³-O-CO-CO-Cl is reacted with a dihydropyran of the formula IV

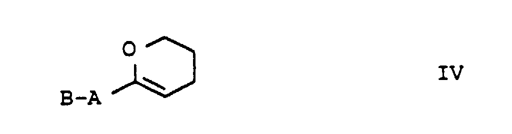

9. A process for the preparation of a dihydropyran as claimed in claim 1 of the formula XIII 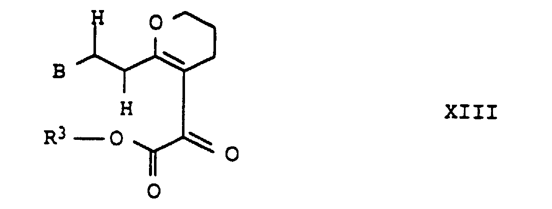 where B and R³ have the meanings stated in claim 1, wherein a dihydropyran of the formula XIV 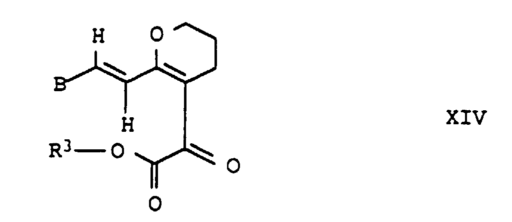 is hydrogenated with hydrogen in the presence of a suitable catalyst (eg. Pd, Pt or Ni).

10. A fungicide containing an inert carrier and a fungicidal amount of a dihydroypran of the formula I as claimed in claim 1.

11. A method for controlling fungi, wherein the fungi or the materials, plants or seed threatened by fungal attack or the soil is or are treated with a fungicidal amount of a compound of the formula I as claimed in claim 1.

12. A compound of the formula I as claimed in claim 1, in which U is CH-OCH₃, A is -CH=CH-, B is phenyl and R¹ is methoxy.

13. A compound of the formula I as claimed in claim 1, in which U is CH-OCH₃, A is -CH₂-CH₂-, B is phenyl and R¹ is methoxy.

14. A compound of the formula I as claimed in claim 1, in which U is CH-OCH₃, A is -CH=CH-, B is 2-methylphenyl and R¹ is methoxy.

15. A compound of the formula I as claimed in claim 1, in which U is CH-OCH₃, A is -CH₂-CH₂-, B is 2-methylphenyl and R₁ is methoxy.

## Revendications

1. Dihydropyrannes de formule I 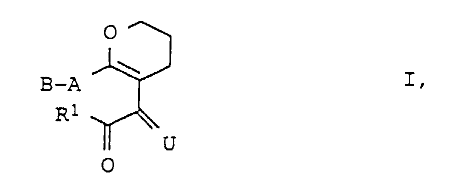 dans laquelle
U représente =CH-OR², =CH-SR², =CH-R², -CH-halogène ou =N-OR² et
A représente une liaison simple, -CH₂-, -(CH₂-CH₂)ₙ-, -(CR⁹=CR⁸)m- CH=CH, -C≡C-, -OCH₂, -SCH₂-, -NR⁶CH₂-, -C(=0) -OCH₂- ou R⁷C=NO-CH₂
n est égal à 1, 2 ou 3 et
m est égal à O ou 1,
B représente l'hydrogène, un groupe cycloalkyle en C3-C10, aryle à 6, 10 ou 14 atomes de carbone, hétéroaryle à 5 à 14 atomes cycliques dont 1 à 4 hétéroatomes choisis parmi N, O et S, hétérocyclyle à 5 à 15 atomes cycliques dont 1 à 4 hétéroatomes choisis parmi N, O et S, les cycles étant saturés ou partiellement insaturés, ou cycloalcényle en C5-C14, ces groupes pouvant porter chacun 1 à 4 des substituants mentionnés en référence à R¹⁰, pour autant que ceux-ci soient différents de ceux mentionnés ci-dessus,
R¹ représente OR³ ou NR⁴R⁵,
R², R³ et R⁶ représentent des groupes méthyle ou éthyle,
R⁴, R⁵, R⁷, R⁸ et R⁹ représentent l'hydrogène, des groupes méthyle ou éthyle,
R¹⁰ représente un halogène, un groupe cyano, nitro ou CHO
R¹¹, OR¹¹, =NOR¹¹, CO₂R¹¹, SR¹¹, COR¹¹, SOR¹¹ ou SO₂R¹¹
R11 représente un groupe alkyle, alcényle, alcynyle, cycloalkyle en C3-C10, aryle à 6, 10 ou 14 atomes de carbone, hétéroaryle contenant 5 à 14
atomes cycliques dont 1 à 4 hétéroatomes choisis parmi N, O et S, les cycles pouvant être saturés ou partiellement insaturés, ou cycloalcényle en C5-C14, ces groupes pouvant porter 1 à 4 des substituants mentionnés en référence à R¹² pour autant que ceux-ci soient différents des substituants R¹¹,
R¹² représente un halogène, un groupe cyano, nitro, alkyle, halogénoalkyle, alcoxy, halogénoalcoxy, alcoxyimino, alcoxycarbonyle, alkylcarbonyle, alcényloxyimino ou aryle.

2. Dihydropyrannes de formule I selon la revendication 1 dans laquelle A représente -(CH=CH)ₙ-CH=CH-.

3. Composé de formule III 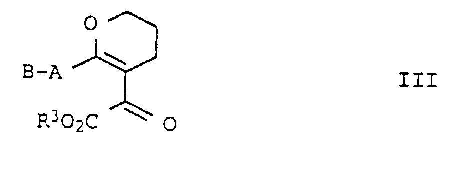 dans laquelle A, B et R³ ont les significations indiquées dans la revendication 1.

4. Procédé de préparation d'un dihydropyranne selon la revendication 1, répondant à la formule VI 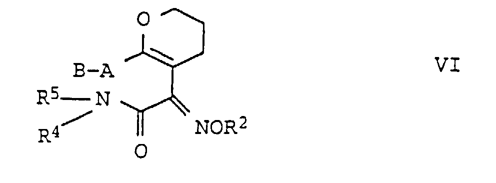 dans laquelle A, B, R², R⁴ et R⁵ ont les significations indiquées dans la revendication 1, caractérisé par le fait que l'on fait réagir des composés de formule VII. 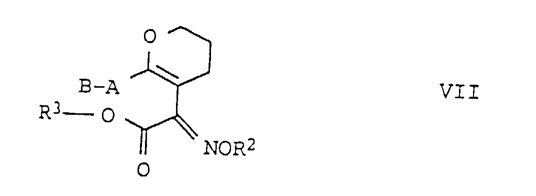 avec des amines de formule HNR⁴R⁵, R³ ayant les significations indiquées dans la revendication 1.

5. Procédé de préparation d'un dihydropyranne selon la revendication 1, répondant à la formule VIII 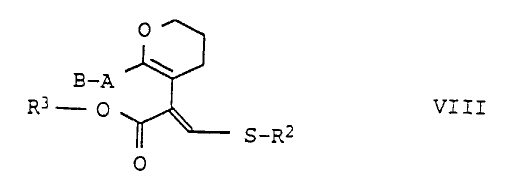 dans laquelle A, B, R² et R³ ont les significations indiquées dans la revendication 1, caractérisé par le fait que l'on fait réagir un composé de formule IX 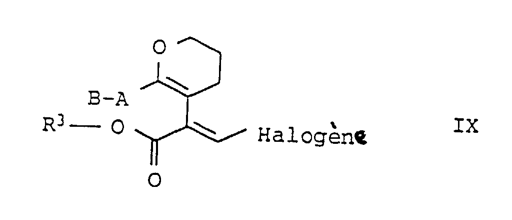 dans laquelle A, B et R³ ont les significations indiquées dans la revendication 1 et l'halogène signalé est le chlore ou le brome, de préférence en milieu alcalin, avec un composé de formule R²-SH, R² ayant les significations indiquées dans la revendication 1.

6. Procédé de préparation d'un dihydropyranne selon la revendication 1, répondant à la formule I 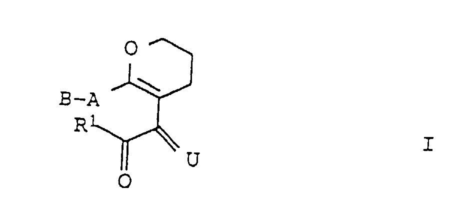 dans laquelle A, B et R¹ ont les significations indiquées dans la revendication 1 et U représente =CH-OR², =CH₂, =CHR² ou -CH-halogène, caractérisé par le fait que l'on fait réagir dans une réaction de Wittig des cétoesters de formule III 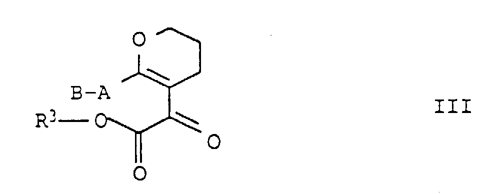 dans laquelle R³ a les significations indiquées dans la revendication 1, avec le phosphonate ou le sel de phosphonium correspondant.

7. Procédé de préparation d'un dihydropyranne de formule VII 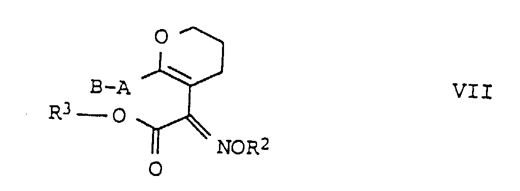 dans laquelle A, R² et R³ ont les significations indiquées dans la revendication 1, caractérisé par le fait que l'on fait réagir une alcoxyamine de formule H₂N-OR² avec un céto-ester de formule III.

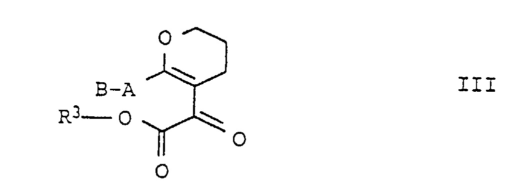

8. Procédé de préparation d'un dihydropyranne selon la revendication 1, répondant à la formule III 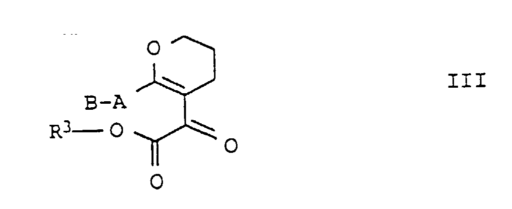 dans laquelle A, B et R³ ont les significations indiquées dans la revendication 1, caractérisé par le fait que l'on fait réagir des chlorures d'acides oxaliques de formule R³-O-CO-CO-Cl avec un dihydropyranne de formule IV

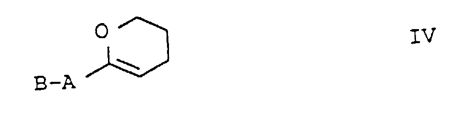

9. Procédé de préparation d'un dihydropyranne selon la revendication 1, répondant à la formule XIII 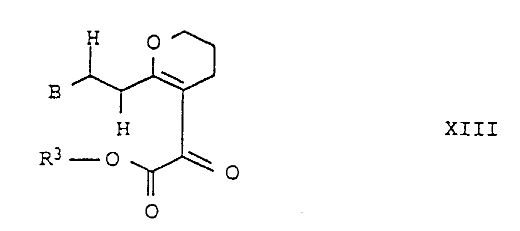 dans laquelle B et R³ ont les significations indiquées dans la revendication 1, caractérisé par le fait que l'on réduit un dihydropyranne de formule XIV 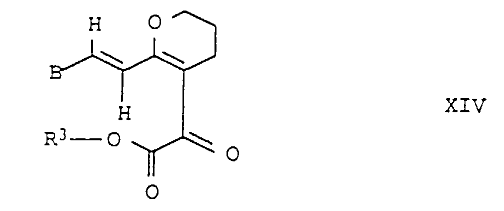 par l'hydrogène en présence de catalyseurs appropriés (par exemple Pd, Pt ou Ni).

10. Produit fongicide contenant un véhicule inerte et une quantité fongicide efficace d'un dihydropyranne de formule I selon la revendication 1.

11. Procédé pour combattre les mycètes, caractérisé par le fait que l'on traite les mycètes ou les matériaux, végétaux, semences ou sols menacés d'une attaque par les mycètes par une quantité fongicide efficace d'un composé I selon la revendication 1.

12. Composé de formule I selon la revendication 1, dans laquelle U représente CH-OCH₃, A représente -CH=CH-, B représente un groupe phényle et R¹ un groupe méthoxy.

13. Composé de formule I selon la revendication 1, dans laquelle U représente CH-OCH₃, A représente -CH₂-CH₂-, B représente un groupe phényle et R¹ un groupe méthoxy.

14. Composé de formule I selon la revendication 1, dans laquelle U représente CH-OCH₃, A représente -CH=CH-, B représente un groupe 2-méthylphényle et R¹ un groupe méthoxy.

15. Composé de forume I selon la revendication 1, dans laquelle U représente CH-OCH₃, A représente -CH₂-CH₂-, B représente un groupe 2-méthylphényle et R¹ un groupe méthoxy.
